(19) Europäisches Patentamt
European Patent Office
Office européen des brevets

(11) EP 2 500 040 A1

(12) **EUROPEAN PATENT APPLICATION**

(43) Date of publication:
**19.09.2012 Bulletin 2012/38**

(51) Int Cl.:
***A61K 49/00*** *(2006.01)* ***C07K 7/08*** *(2006.01)*

(21) Application number: **12159175.4**

(22) Date of filing: **24.12.2003**

(84) Designated Contracting States:
**AT BE BG CH CY CZ DE DK EE ES FI FR GB GR HU IE IT LI LU MC NL PT RO SE SI SK TR**

(30) Priority: **13.01.2003 US 341577**

(62) Document number(s) of the earlier application(s) in accordance with Art. 76 EPC:
**10178206.8 / 2 281 580**
**03800223.4 / 1 585 549**

(71) Applicant: **Bracco Imaging S.p.A**
**20134 Milano (IT)**

(72) Inventors:
- **SWENSON, Rolf**
  **Princeton, NJ New Jersey 08540 (US)**
- **LATTUADA, Luciano**
  **1-10010 Ivrea (IT)**
- **LINDER, Karen**
  **Kingston, NJ New Jersey 08528 (US)**
- **MARINELLI, Edmund**
  **Tucson, AZ Arizona 85704 (US)**
- **CAPPELLETTI, Enrico**
  **20816 Ceriano Laghetto (IT)**
- **TWEEDLE, Michael**
  **Bexley, OH Ohio 43209 (US)**
- **RAJU, Natarajan**
  **Kendall Park, NJ New Jersey 08824 (US)**
- **NANJAPPAN, Palaniappa**
  **Princeton, NJ New Jersey 08540 (US)**

(74) Representative: **Merli, Silvia**
**Intellectual Property Department**
**Bracco Imaging SpA**
**Via XXV Aprile 4**
**20097 San Donato Milanese (MI) (IT)**

Remarks:
This application was filed on 13-03-2012 as a divisional application to the application mentioned under INID code 62.

# (54) Improved Gastrin Releasing Peptide Receptor-Binding Compounds

(57) New and improved compounds for use in diagnostic imaging or therapy having the formula M-N-O-P-G, wherein M is an optical label or a metal chelator (in the form complexed with a metal radionuclide or not), N-O-P is the linker, and G is the GRP receptor targeting peptide. Methods for imaging a patient and/or providing radiotherapy or phototherapy to a patient using the compounds of the invention are also provided. Methods and kits for preparing a diagnostic imaging agent from the compound is further provided. Methods and kits for preparing a radiotherapeutic agent is further provided.

EP 2 500 040 A1

SEQUENCE LISTING

<110> Cappelletti, et al.

<120> Improved Gastrin Releasing Peptide Compounds

<130> 57637-1022

<140> 10/542,202
<141> 2005-07-13

<150> PCT/US03/41328
<151> 2003-12-24

<160> 18

<170> PatentIn version 3.3

<210> 1
<211> 8
<212> PRT
<213> Artificial

<220>
<223> Description of Artificial Sequence: This peptide is known as bombesin (i.e., BBN (7-14)) and binds to gastrin releasing peptide (GRP) receptors.

<400> 1

Gln Trp Ala Val Gly His Leu Met
1               5

<210> 2
<211> 8
<212> PRT
<213> Artificial

<220>
<223> Description of Artifical Sequnce: GRP Receptor Targeting Peptide

<220>
<221> MOD_RES
<222> (8)..(8)
<223> M-NH2

<400> 2

Glu Trp Ala Val Gly His Leu Met
1               5

<210> 3
<211> 13
<212> PRT
<213> Artificial

<220>
<223> Description of Artifical Sequnce: GRP Receptor Targeting Peptide

<220>
<221> MOD_RES

<222> (13)..(13)
<223> M-NH2

<400> 3

Gln Arg Leu Gly Asn Gln Trp Ala Val Gly His Leu Met
1 5 10

<210> 4
<211> 13
<212> PRT
<213> Artificial

<220>
<223> Description of Artifical Sequnce: GRP Receptor Targeting Peptide

<220>
<221> MOD_RES
<222> (13)..(13)
<223> M-NH2

<400> 4

Gln Arg Tyr Gly Asn Gln Trp Ala Val Gly His Leu Met
1 5 10

<210> 5
<211> 13
<212> PRT
<213> Artificial

<220>
<223> Description of Artifical Sequnce: GRP Receptor Targeting Peptide

<220>
<221> MOD_RES
<222> (13)..(13)
<223> M-NH2

<400> 5

Gln Lys Tyr Gly Asn Gln Trp Ala Val Gly His Leu Met
1 5 10

<210> 6
<211> 7
<212> PRT
<213> Artificial

<220>
<223> Description of Artifical Sequnce: GRP Receptor Targeting Peptide

<220>
<221> MOD_RES
<222> (7)..(7)
<223> L-NH-Pentyl

<400> 6

Gln Trp Ala Val Gly His Leu
1 5

<210> 7
<211> 7
<212> PRT
<213> Artificial

<220>
<223> Description of Artifical Sequnce: GRP Receptor Targeting Peptide

<220>
<221> MOD_RES
<222> (7)..(7)
<223> M-NH2

<400> 7

Gln Trp Ser Val His Leu Met
1 5

<210> 8
<211> 8
<212> PRT
<213> Artificial

<220>
<223> Description of Artifical Sequnce: GRP Receptor Targeting Peptide

<220>
<221> MOD_RES
<222> (8)..(8)
<223> L-NH2

<400> 8

Gln Trp Ala Val Gly His Leu Leu
1 5

<210> 9
<211> 8
<212> PRT
<213> Artificial

<220>
<223> Description of Artifical Sequnce: GRP Receptor Targeting Peptide

<220>
<221> MOD_RES
<222> (5)..(5)
<223> bAla

<220>
<221> MOD_RES
<222> (8)..(8)
<223> Nle-NH2

<400> 9

Gln Trp Ala Val Ala His Phe Leu
1               5

<210>  10
<211>  7
<212>  PRT
<213>  Artificial

<220>
<223>  Description of Artifical Sequnce:  GRP Receptor Targeting Peptide

<220>
<221>  MOD_RES
<222>  (7)..(7)
<223>  L-NH2

<400>  10

Gln Trp Ala Gly His Phe Leu
1               5

<210>  11
<211>  8
<212>  PRT
<213>  Artificial

<220>
<223>  Description of Artifical Sequnce:  GRP Receptor Targeting Peptide

<220>
<221>  MOD_RES
<222>  (8)..(8)
<223>  M-NH2

<400>  11

Leu Trp Ala Val Gly Ser Phe Met
1               5

<210>  12
<211>  8
<212>  PRT
<213>  Artificial

<220>
<223>  Description of Artifical Sequnce:  GRP Receptor Targeting Peptide

<220>
<221>  MOD_RES
<222>  (8)..(8)
<223>  M-NH2

<400>  12

His Trp Ala Val Gly His Leu Met
1               5

<210>  13

<211> 8
<212> PRT
<213> Artificial

<220>
<223> Description of Artifical Sequnce: GRP Receptor Targeting Peptide

<220>
<221> MOD_RES
<222> (8)..(8)
<223> M-NH2

<400> 13

Gln Trp Ala Val Gly His Phe Met
1 5

<210> 14
<211> 7
<212> PRT
<213> Artificial

<220>
<223> Description of Artifical Sequnce: GRP Receptor Targeting Peptide

<220>
<221> MOD_RES
<222> (7)..(7)
<223> M-NH2

<400> 14

Gln Trp Ala Val His Leu Met
1 5

<210> 15
<211> 9
<212> PRT
<213> Artificial

<220>
<223> Description of Artifical Sequnce: GRP Receptor Targeting Peptide

<220>
<221> MOD_RES
<222> (7)..(7)
<223> MeH

<400> 15

Gln Trp Ala Val Gly Asn His Leu Met
1 5

<210> 16
<211> 8
<212> PRT
<213> Artificial

<220>

```
<223>  Description of Artifical Sequnce:  GRP Receptor Targeting Peptide


<220>
<221>  MOD_RES
<222>  (8)..(8)
<223>  M-NH2

<400>  16

Leu Trp Ala Thr Gly His Phe Met
1               5


<210>  17
<211>  8
<212>  PRT
<213>  Artificial

<220>
<223>  Description of Artifical Sequnce:  GRP Receptor Targeting Peptide


<220>
<221>  MOD_RES
<222>  (8)..(8)
<223>  M-NH2

<400>  17

Gln Trp Ala Val Gly His Phe Met
1               5


<210>  18
<211>  11
<212>  PRT
<213>  Artificial

<220>
<223>  Description of Artifical Sequnce:  GRP Receptor Targeting Peptide


<220>
<221>  MOD_RES
<222>  (11)..(11)
<223>  M-NH2

<400>  18

Leu Gly Asn Gln Trp Ala Val Gly His Leu Met
1               5                   10
```

**Description**

## FIELD OF THE INVENTION

This invention relates to novel gastrin releasing peptide (GRP) compounds which are useful as diagnostic imaging agents or radiotherapeutic agents. These GRP compounds are labeled with radionuclides or labels detectable by in vivo light imaging and include the use of novel linkers between the label and the targeting peptide, which provides for improved pharmacokinetics.

## BACKGROUND OF THE INVENTION

The use of radiopharmaceuticals (*e.g.,* diagnostic imaging agents, radiotherapeutic agents) to detect and treat cancer is well known. In more recent years, the discovery of site-directed radiopharmaceuticals for cancer detection and/or treatment has gained popularity and continues to grow as the medical profession better appreciates the specificity, efficacy and utility of such compounds.

These newer radiopharmaceutical agents typically consist of a targeting agent connected to a metal chelator, which can be chelated to (*e.g.,* complexed with) a diagnostic metal radionuclide such as, for example, technetium or indium, or a therapeutic metal radionuclide such as, for example, lutetium, yttrium, or rhenium. The role of the metal chelator is to hold (*i.e.,* chelate) the metal radionuclide as the radiopharmaceutical agent is delivered to the desired site. A metal chelator which does not bind strongly to the metal radionuclide would render the radiopharmaceutical agent ineffective for its desired use since the metal radionuclide would therefore not reach its desired site. Thus, further research and development led to the discovery of metal chelators, such as that reported in U.S. Pat. No. 5,662,885 to Pollak et. al., hereby incorporated by reference, which exhibited strong binding affinity for metal radionuclides and the ability to conjugate with the targeting agent. Subsequently, the concept of using a "spacer" to create a physical separation between the metal chelator and the targeting agent was further introduced, for example in U.S. Pat. 5,976,495 to Pollak et. al., hereby incorporated by reference.

The role of the targeting agent, by virtue of its affinity for certain binding sites, is to direct the diagnostic agent, such as a radiopharmaceutical agent containing the metal radionuclide, to the desired site for detection or treatment. Typically, the targeting agent may include a protein, a peptide, or other macromolecule which exhibits a specific affinity for a given receptor. Other known targeting agents include monoclonal antibodies (MAbs), antibody fragments ($F_{ab}$ 's and $(F_{ab})_2$ 's), and receptor-avid peptides. Donald J. Buchsbaum, "Cancer Therapy with Radiolabeled Antibodies; Pharmacokinetics of Antibodies and Their Radiolabels; Experimental Radioimmunotherapy and Methods to Increase Therapeutic Efficacy," CRC Press, Boca Raton, Chapter 10, pp. 115-140, (1995); Fischman, et al. "A Ticket to Ride: Peptide Radiopharmaceuticals," The Journal of Nuclear Medicine, vol. 34, No.12, (Dec. 1993).

In recent years, it has been learned that some cancer cells contain gastrin releasing peptide (GRP) receptors (GRP-R) of which there are a number of subtypes. In particular, it has been shown that several types of cancer cells have over-expressed or uniquely expressed GRP receptors. For this reason, much research and study have been done on GRP and GRP analogues which bind to the GRP receptor family. One such analogue is bombesin (BBN), a 14 amino acid peptide (i.e., tetradecapeptide) isolated from frog skin which is an analogue of human GRP and which binds to GRP receptors with high specificity and with an affinity similar to GRP.

Bombesin and GRP analogues may take the form of agonists or antagonists. Binding of GRP or BBN agonists to the GRP receptor increases the rate of cell division of these cancer cells and such agonists are internalized by the cell, while binding of GRP or BBN antagonists generally does not result in either internalization by the cell or increased rates of cell division. Such antagonists are designed to competitively inhibit endogenous GRP binding to GRP receptors and reduce the rate of cancer cell proliferation. *See, e.g.,* Hoffken, K.; Peptides in Oncology II, Somatostatin Analogues and Bombesin Antagonists (1993), pp. 87-112. For this reason, a great deal of work has been, and is being pursued to develop BBN or GRP analogues that are antagonists. *E.g.*, Davis et al., Metabolic Stability and Tumor Inhibition of Bombesin/GRP Receptor Antagonists, Peptides, vol. 13, pp. 401-407, 1992.

In designing an effective compound for use as a diagnostic or therapeutic agent for cancer, it is important that the drug have appropriate in vivo targeting and pharmacokinetic properties. For example, it is preferable that for a radiopharmaceutical, the radiolabeled peptide have high specific uptake by the cancer cells (*e.g.,* via GRP receptors). In addition, it is also preferred that once the radionuclide localizes at a cancer site, it remains there for a desired amount of time to deliver a highly localized radiation dose to the site.

Moreover, developing radiolabeled peptides that are cleared efficiently from normal tissues is also an important factor for radiopharmaceutical agents. When biomolecules (*e.g.,* MAb, $F_{ab}$ or peptides) labeled with metallic radionuclides (via a chelate conjugation), are administered to an animal such as a human, a large percentage of the metallic radionuclide (in some chemical form) can become "trapped" in either the kidney or liver parenchyma (i.e., is not excreted into the urine or bile). Duncan et al.; Indium-111-Diethylenetriaminepentaacetic Acid-Octreotide Is Delivered in Vivo to Pancreatic,

Tumor Cell, Renal, and Hepatocyte Lysosomes, Cancer Research 57, pp. 659-671, (Feb. 15, 1997). For the smaller radiolabeled biomolecules (*i.e.,* peptides or $F_{ab}$), the major route of clearance of activity is through the kidneys which can also retain high levels of the radioactive metal (i.e., normally >10-15% of the injected dose). Retention of metal radionuclides in the kidney or liver is clearly undesirable. Conversely, clearance of the radiopharmaceutical from the blood stream too quickly by the kidney is also undesirable if longer diagnostic imaging or high tumor uptake for radiotherapy is needed.

Subsequent work, such as that in U.S. Pat. 6,200,546 and US 2002/0054855 to Hoffman, et. al, hereby incorporated by reference, has attempted to overcome this problem by forming a compound having the general formula X-Y-B wherein X is a group capable of complexing a metal, Y is a covalent bond on a spacer group and B is a bombesin agonist binding moiety. Such compounds were reported to have high binding affinities to GRP receptors, and the radioactivity was retained inside of the cells for extended time periods. In addition, in vivo studies in normal mice have shown that retention of the radioactive metal in the kidneys was lower than that known in the art, with the majority of the radioactivity excreted into the urine.

New and improved radiopharmaceutical and other diagnostic compounds which have improved pharmacokinetics and improved kidney excretion (*i.e.,* lower retention of the radioactive metal in the kidney) have now been found for diagnostic imaging and therapeutic uses. For diagnostic imaging, rapid renal excretion and low retained levels of radioactivity are critical for improved images. For radiotherapeutic use, slower blood clearance to allow for higher tumor uptake and better tumor targeting with low kidney retention are critical.

## SUMMARY OF THE INVENTION

In an embodiment of the present invention, there is provided new and improved compounds for use in diagnostic imaging or radiotherapy. The compounds include a chemical moiety capable of complexing a medically useful metal ion or radionuclide (metal chelator) attached to a GRP receptor targeting peptide by a linker or spacer group. In another embodiment, these compounds include an optical label (e.g. a photolabel or other label detectable by light imaging, optoacoustical imaging or photoluminescence) attached to a GRP receptor targeting peptide by a linker or spacer group.

In general, compounds of the present invention may have the formula:

$$M\text{-}N\text{-}O\text{-}P\text{-}G$$

wherein M is the metal chelator (in the form complexed with a metal radionuclide or not), or the optical label, N-O-P is the linker, and G is the GRP receptor targeting peptide.

The metal chelator M may be any of the metal chelators known in the art for complexing with a medically useful metal ion or radionuclide. Preferred chelators include DTPA, DOTA, DO3A, HP-DO3A, EDTA, TETA, EHPG, HBED, NOTA, DOTMA, TETMA, PDTA, TTHA, LICAM, MECAM, or peptide chelators, such as, for example, those discussed herein. The metal chelator may or may not be complexed with a metal radionuclide, and may include an optional spacer such as a single amino acid. Preferred metal radionuclides for scintigraphy or radiotherapy include $^{99m}Tc$, $^{51}Cr$, $^{67}Ga$, $^{68}Ga$, $^{47}Sc$, $^{51}Cr$, $^{167}Tm$, $^{141}Ce$, $^{111}In$, $^{168}Yb$ , $^{175}Yb$ $^{140}La$, $^{90}Y$, $^{88}Y$, $^{153}Sm$, $^{166}Ho$, $^{165}Dy$, $^{166}Dy$, $^{62}Cu$, $^{64}Cu$, $^{67}Cu$, $^{97}Ru$, $^{103}Ru$, $^{186}Re$, $^{188}Re$, $^{203}Pb$, $^{211}B$, $^{212}Bi$, $^{213}Bi$, $^{214}Bi$, $^{105}Rh$, $^{109}Pd$ , $^{117m}Sn$, $^{149}Pm$, $^{161}Tb$, $^{177}Lu$, $^{198}Au$ and $^{199}Au$. The choice of metal will be determined based on the desired therapeutic or diagnostic application. For example, for diagnostic purposes the preferred radionuclides include $^{64}Cu$, $^{67}Ga$, $^{68}Ga$, $^{99m}Tc$, and $^{111}In$, with $^{99m}Tc$, and $^{111}In$ being particularly preferred. For therapeutic purposes, the preferred radionuclides include $^{64}Cu$, $^{90}Y$,$^{105}Rh$, $^{111}In$, $^{117m}S_n$, $^{149}Pm$, $^{153}Sm$, $^{161}Tb$, $^{166}Dy$, $^{166}Ho$, $^{175}Yb$, $^{177}Lu$, $^{186/188}Re$, and $^{199}Au$, with $^{177}Lu$ and $^{90}Y$ being particularly preferred. A most preferred chelator used in compounds of the invention is 1-substituted 4,7,10-tricarboxymethyl 1,4,7,10 tetraazacyclododecane triacetic acid (D03A).

The optical label M may be any of various optical labels known in the art. Preferred labels include, without limitation, optical dyes, including organic chromophores or fluorophores, such as cyanine dyes light absorbing compounds, light reflecting and scattering compounds, and bioluminescent molecules.

In one embodiment, the linker N-O-P contains at least one non-alpha amino acid.

In another embodiment, the linker N-O-P contains at least one substituted bile acid.

In yet another embodiment, the linker N-O-P contains at least one non-alpha amino acid with a cyclic group.

The GRP receptor targeting peptide may be GRP, bombesin or any derivatives or analogues thereof. In a preferred embodiment, the GRP receptor targeting peptide is a GRP or bombesin analogue which acts as an agonist. In a particularly preferred embodiment, the GRP receptor targeting peptide is a bombesin agonist binding moiety disclosed in U.S. Pat. 6,200,546 and US 2002/0054855, incorporated herein by reference.

There is also provided a novel method of imaging using the compounds of the present invention.

A single or multi-vial kit that contains all of the components needed to prepare the diagnostic or therapeutic

agents of the invention is provided in an exemplary embodiment of the present invention.

There is further provided a novel method for preparing a diagnostic imaging agent comprising the step of adding to an injectable imaging medium a substance containing the compounds of the present invention.

A novel method of radiotherapy using the compounds of the invention is also provided, as is a novel method for preparing a radiotherapeutic agent comprising the step of adding to an injectable therapeutic medium a substance comprising a compound of the invention.

## BRIEF DESCRIPTION OF THE DRAWINGS

**FIG. 1A** is a graphical representation of a series of chemical reactions for the synthesis of intermediate **C** ((3β, 5β)-3-(9*H*-Fluoren-9-ylmethoxy)aminocholan-24-oic acid), from A (Methyl-(3β, 5β)-3-aminocholan-24-ate) and B ((3β, 5β)-3-aminocholan-24-oic acid), as described in Example I.

**FIG. 1B** is a graphical representation of the sequential reaction for the synthesis of *N*-[(3β,5β)-3-[[[[[4,7,10-Tris (carboxymethyl)-1,4,7,10-tetraazacyclododec-1-yl]acetyl]amino] acetyl]amino] cholan-24-yl]-L-glutaminyl-L-tryptophyl-L-alanyl-L-valyl-glycyl-L-histidyl-L-leucyl-L-methioninamide (**L62**), as described in Example I.

**FIG. 2A** is a graphical representation of the sequential reaction for the synthesis of *N*-[4-[[[[[4,7,10-Tris(carboxymethyl)-1,4,7,10-tetraazacyclododec-1-yl]acetyl]amino]acetyl]amino]benzoyl]-L-glutaminyl-L-tryptophyl-L-alanyl-L-valyl-glycyl-L-histidyl-L-leucyl-L-methioninamide (**L70**), as described in Example II.

**FIG. 2B** is a general graphical representation of the sequential reaction for the synthesis of *N*-[4-[2-[[[4,7,10-Tris(carboxymethyl)-1,4,7,10-tetraazacyclododec-1-yl]acetyl]amino]ethoxy]benzoyl]-L-glutaminyl-L-tryptophyl-L-alanyl-L-valyl-glycyl-L-histidyl-L-leucyl-L-methioninamide (**L73**), *N*-[3-[[[[4,7,10-Tris(carboxymethyl)-1,4,7,10-tetraazacyclododec-1-yl]acetyl]amino]methyl]benzoyl]-L-glutaminyl-L-tryptophyl-L-alanyl-L-valyl-glycyl-L-histidyl-L-leucyl-L-methioninamide (**L115**), and *N*-[4-[[[[4,7,10-Tris(carboxymethyl)-1,4,7,10-tetraazacyclododec-1-yl]acetyl]amino]methyl] phenylacetyl]-L-glutaminyl-L-tryptophyl-L-alanyl-L-valyl-glycyl-L-histidyl-L-leucyl-L-methioninamide (**L116**), as described in Example II.

**FIG. 2C** is a chemical structure of the linker used in the synthesis reaction of FIG. 2B for synthesis of *N*-[4-[2-[[[4,7,10-Tris(carboxymethyl)-1,4,7,10-tetraazacyclododec-1-yl]acetyl]amino]ethoxy]benzoyl]-L-glutaminyl-L-tryptophyl-L-alanyl-L-valyl-glycyl-L-histidyl-L-leucyl-L-methioninamide (**L73**), as described in Example II.

**FIG. 2D** is a chemical structure of the linker used in the synthesis reaction of FIG. 2B for synthesis of *N*-[3-[[[[4,7,10-Tris(carboxymethyl)-1,4,7,10-tetraazacydododec-1-yl]acetyl]amino]methyl]benzoyl]-L-glutaminyl-L-tryptophyl-L-alanyl-L-valyl-glycyl-L-histidyl-L-leucyl-L-methioninamide (**L115**), as described in Example II.

**FIG. 2E** is a chemical structure of the linker used in the synthesis reaction of FIG. 2B for synthesis of *N*-[4-[[[[4,7,10-Tris(carboxymethyl)-1,4,7,10-tetraazacyclododec-1-yl]acetyl]amino]methyl]phenylacetyl]-L-glutaminyl-L-tryptophyl-L-alanyl-L-valyl-glycyl-L-histidyl-L-leucyl-L-methioninamide (**L116**), as described in Example II.

**FIG. 2F** is a graphical representation of the sequential reaction for the synthesis of *N*-[[4,7,10-Tris(carboxymethyl)-1,4,7,10-tetraazacyclododec-1-yl]acetyl]glycyl-4-piperidinecarbonyl-L-glutaminyl-L-tryptophyl-L-alanyl-L-valyl-glycyl-L-histidyl-L-leucyl-L-methioninamide (**L74**), as described in Example II.

**FIG. 3A** is a graphical representation of a series of chemical reactions for the synthesis of intermediate (3β, 5β)-3-[[(9*H*-Fluoren-9-ylmethoxy)amino]acetyl]amino-12-oxocholan-24-oic acid (**C**), as described in Example III.

**FIG. 3B** is a graphical representation of the sequential reaction for the synthesis of *N*-[(3β,5β)-3-[[[[[4,7,10-Tris (carboxymethyl)-1,4,7,10-tetraazacyclododec-1-yl]acetyl]amino]acetyl]amino]-12,24-dioxocholan-24-yl]-L-glutaminyl-L-tryptophyl-L-alanyl-L-valyl-glycyl-L-histidyl-L-leucyl-L-methioninamide (**L67**), as described in Example III.

**FIG. 3C** is a chemical structure of (3β,5β)-3-Amino-12-oxocholan-24-oic acid (**B**), as described in Example III.

**FIG. 3D** is a chemical structure of (3β,5β)-3-[[(9*H*-Fluoren-9-ylmethoxy)amino]acetyl]amino-12-oxocholan-24-oic acid (**C**), as described in Example III.

**FIG 3E** is a chemical structure of N-[(3β,5β)-3-[[[[[4,7,10-Tris(carboxymethyl)-1,4,7,10-tetraazacyclododec-1-yl]acetyl]amino]acetyl]amino]-12,24-dioxocholan-24-yl]-L-glutaminyl-L-tryptophyl-L-alanyl-L-valyl-glycyl-L-histidyl-L-leucyl-L-methioninamide (**L67**), as described in Example III.

**FIG. 4A** is a graphical representation of a sequence of reactions to obtain intermediates (3 β, 5β, 12α)-3-[[(9H-Fluoren-9-ylmethoxy)amino]acetyl]amino-12-hydroxycholan-24-oic acid (**3a**) and (3β,5β,7α,12α)-3-[[(9H-Fluoren-9-ylmethoxy)amino]acetyl]amino-7,12-dihydroxycholan-24-oic acid (**3b**), as described in Example IV.

**FIG. 4B** is a graphical representation of the sequential reaction for the synthesis of *N*-[(3β,5β,12α)-3-[[[[[4,7,10-Tris(carboxymethyl)-1,4,7,10-tetraazacyclododec-1-yl]acetyl]amino]acetyl]amino]-12-hydroxy-24-oxocholan-24-yl]-L-glutaminyl-L-tryptophyl-L-alanyl-L-valyl-glycyl-L-histidyl-L-leucyl-L-methioninamide (**L63**), as described in Example IV.

**FIG. 4C** is a graphical representation of the sequential reaction for the synthesis of *N*-[(3β,5β,7α,12α)-3-[[[[[4,7,10-Tris(carboxymethyl)-1,4,7,10-tetraazacyclo dodec-1-yl]acetyl]amino]acetyl]amino]-7,12-dihydroxy-24-oxocholan-24-yl]-L-glutaminyl-L-tryptophyl-L-alanyl-L-valyl-glycyl-L-histidyl-L-leucyl-L-methioninamide (**L64**), a s described in Example IV.

**FIG. 4D** is a chemical structure of (3β,5β, 7α, 12α)-3-amino-7,12-dihydroxycholan-24-oic acid (2b), as described in Example IV.

**FIG. 4E** is a chemical structure of (3β,5β,12α)-3-[[(9H-Fluoren-9-ylmethoxy)amino]acetyl]amino-12-hydroxycholan-24-oic acid (**3a**), as described in Example IV;

**FIG. 4F** is a chemical structure of (3β,5β,7α,12α)-3-[[(9H-Fluoren-9-ylmethoxy)amino]acetyl]amino-7,12-dihydroxycholan-24-oic acid (**3b**), as described in Example IV.

**FIG. 4G** is a chemical structure of *N*-[(3β,5β,12α)-3-[[[[4,7,10-Tris(carboxymethyl)-1,4,7,10-tetraazacyclododec-1-yl]acetyl]amino]acetyl]amino]-12-hydroxy-24-oxocholan-24-yl]-L-glutaminyl-L-tryptophyl-L-alanyl-L-valyl-glycyl-L-histidyl-L-leucyl-L-methioninamide (**L63**), as described in Example IV.

**FIG. 4H** is a chemical structure of *N*-[(3β,5β,7α,12α)-3-[[[[4,7,10-Tris(carboxymethyl)-1,4,7,10-tetraazacyclo dodec-1-yl]acetyl]amino]acetyl]amino]-7,12-dihydroxy-24-oxocholan-24-yl]-L-glutaminyl-L-tryptophyl-L-alanyl-L-valyl-glycyl-L-histidyl-L-leucyl-L-methioninamide (**L64**), as described in Example IV.

**FIG. 5A** is a general graphical representation of the sequential reaction for the synthesis of 4-[[[[4,7,10-Tris (carboxymethyl)-1,4,7,10-tetraazacyclododec-1-yl]acetyl]amino]methyl]benzoyl-L-glutaminyl-L-tryptophyl-L-alanyl-L-valyl-glycyl-L-histidyl-L-leucyl-L-methioninamide (**L71**); and *Trans*-4-[[[[4,7,10-tris(carboxymethyl)-1,4,7,10-tetraazacyclododec-1-yl]acetyl]amino]methyl]cyclohexylcarbonyl-L-glutaminyl-L-tryptophyl-L-alanyl-L-valyl-glycyl-L-histidyl-L-leucyl-L-methioninamide (**L72**) as described in Example V, wherein the linker is from FIG 5B and 5C, respectively.

**FIG. 5B** is a chemical structure of the linker used in compound **L71** as shown in Fig. 5A and as described in Example V.

**FIG. 5C** is a chemical structure of the linker used in compound **L72** as shown in Fig. 5A and as described in Example V.

**FIG. 5D** is a chemical structure of Rink amide resin functionalised with bombesin[7-14] (**B**), as described in Example V.

**FIG. 5E** is a chemical structure of *Trans*-4-[[[(9H-fluoren-9-ylmethoxy)carbonyl]amino]methyl]cyclohexanecarboxylic acid (**D**), as described in Example V;

**FIG. 6A** is a graphical representation of a sequence of reactions for the synthesis of intermediate linker 2-[[[9*H*-Fluoren-9-ylmethoxy)carbonyl]amino]methyl]benzoic acid (**E**), as described in Example VI.

**FIG. 6B** is a graphical representation of a sequence of reactions for the synthesis of intermediate linker 4-[[[9*H*-Fluoren-9-ylmethoxy)carbonyl]amino]methyl]-3-nitrobenzoic acid (**H**), as described in Example VI.

**FIG. 6C** is a graphical representation of the synthesis of *N*-[2-[[[[4,7,10-Tris(carboxymethyl)-1,4,7,10-tetraazacyclododec-1-yl]acetyl]amino]methyl]benzoyl]-L-glutaminyl-L-tryptophyl-L-alanyl-L-valyl-glycyl-L-histidyl-L-leucyl-L-methioninamide (L75), as described in Example VI.

**FIG. 6D** is a graphical representation of the synthesis of *N*-[4-[[[[4,7,10-Tris(carboxymethyl)-1,4,7,10-tetraazacyclododec-1-yl]acetyl]amino]methyl]-3-nitrobenzoyl]-L-glutaminyl-L-tryptophyl-L-alanyl-L-valyl-glycyl-L-histidyl-L-leucyl-L-methioninamide (**L76**), as described in Example VI.

**FIG. 7A** is a graphical representation of a sequence of reactions for the synthesis of intermediate linker [4-[[[9*H*-Fluoren-9-ylmethoxy)carbonyl]amino]methyl]phenoxy]acetic acid (**E**), as described in Example VII.

**FIG. 7B** is a graphical representation of the synthesis of *N*-[[4-[[[[4,7,10-Tris(carboxymethyl)-1,4,7,10-tetraazacyclododec-1-yl]acetyl]amino]methyl]phenoxy]acetyl]-L-glutaminyl-L-tryptophyl-L-alanyl-L-valyl-glycyl-L-histidyl-L-leucyl-L-methioninamide (**L124**), as described in Example VII.

**FIG. 7C** is a chemical structure of *N*-[[4-[[[[4,7,10-Tris(carboxymethyl)-1,4,7,10-tetraazacyclododec-1-yl]acetyl]amino]methyl]phenoxy]acetyl]-L-glutaminyl-L-tryptophyl-L-alanyl-L-valyl-glycyl-L-histidyl-L-leucyl-L-methioninamide (**L124**), as described in Example VII.

**Fig. 8A** is a graphical representation of a sequence of reactions for the synthesis of intermediate 4-[[[9*H*-Fluoren-9-ylmethoxy)carbonyl]amino]methyl]-3-methoxybenzoic acid (**E**), as described in Example VIII.

**FIG. 8B** is a graphical representation of the synthesis of *N*-[4-[[[[4,7,10-Tris(carboxymethyl)-1,4,7,10-tetraazacyclododec-1-yl]acetyl]amino]methyl]-3-methoxybenzoyl]-L-glutaminyl-L-tryptophyl-L-alanyl-L-valyl-glycyl-L-histidyl-L-leucyl-L-methioninamide, (**L125**), as described in Example VIII.

**FIG. 8C** is a chemical structure of *N*-[4-[[[[4,7,10-Tris(carboxymethyl)-1,4,7,10-tetraazacyclododec-1-yl]acetyl]amino]methyl]-3-methoxybenzoyl]-L-glutaminyl-L-tryptophyl-L-alanyl-L-valyl-glycyl-L-histidyl-L-leucyl-L-methioninamide, (**L125**), as described in Example VIII.

**FIG. 9A** is a graphical representation of a reaction for the synthesis of 3-[[[(9*H*-Fluoren-9-ylmethoxy)carbonyl]amino]acetyl]aminobenzoic acid, **(B),** as described in Example IX.

**FIG. 9B** is a graphical representation of a reaction for the synthesis of 6-[[[(9*H*-Fluoren-9-ylmethoxy)carbonyl]amino]acetyl]aminonaphthoic acid (**C**), as described in Example IX.

**FIG. 9C** is a graphical representation of a reaction for the synthesis of 4-[[[[(9*H*-Fluoren-9-ylmethoxy)carbonyl]amino]acetyl]methylamino]benzoic acid, (**D**), as described in Example IX.

**FIG. 9D** is a graphical representation of a reaction for the synthesis of *N*-[4-[[[[[4,7,10-Tris(carboxymethyl)-1,4,7,10-tetraazacyclododec-1-yl]acetyl]amino]acetyl]amino]phenylacetyl]-L-glutaminyl-L-tryptophyl-L-alanyl-L-valyl-glycyl-L-histidyl-L-leucyl-L-methioninamide, (**L146**); *N*-[3-[[[[[4,7,10-Tris(carboxymethyl)-1,4,7,10-tetraazacyclododec-1-yl]acetyl]amino]acetyl]amino]benzoyl]-L-glutaminyl-L-tryptophyl-L-alanyl-L-valyl-glycyl-L-histidyl-L-leucyl-L-methioninamide (**L233**); *N*-[6-[[[[[4,7,10-Tris(carboxymethyl)-1,4,7,10-tetraazacyclododec-1-yl]acetyl]amino]acetyl] amino] naphthoyl]-L-glutaminyl-L-tryptophyl-L-alanyl-L-valyl-glycyl-L-histidyl-L-leucyl-L-methioninamide, (**L234**), and *N*-[4-[[[[[4,7,10-Tris(carboxymethyl)-1,4,7,10-tetraazacyclododec-1-yl]acetyl]amino]acetyl] methylamino]benzoyl]-L-glutaminyl-L-tryptophyl-L-alanyl-L-valyl-glycyl-L-histidyl-L-leucyl-L-methioninamide, (**L235**), as described in Example IX.

**FIG. 10A** is a graphical representation of a reaction for the synthesis of 7-[[Bis(1,1-dimethylethoxy)phosphinyl] methyl]-1,4,7,10-tetraazacyclododecane-1,4,10-triacetic acid 4,10-bis(1,1-dimethylethyl) ester **H**, as described in Example X.

**FIG. 10B** is a graphical representation of a reaction for the synthesis of *N*-[4-[[[[[4,10-Bis(carboxymethyl)-7-(dihydroxyphosphinyl)methyl-1,4,7,10-tetraazacyclododec-1-yl]acetyl]amino]acetyl]amino]benzoyl]-L-glutaminyl-L-tryptophyl-L-alanyl-L-valyl-glycyl-L-histidyl-L-leucil-L-methioninamide, (**L237**), as described in Example X.

**FIG. 11A** is a graphical representation of a reaction for the synthesis of *N,N*-Dimethylglycyl-L-serinyl-[S-[(acetylamino)methyl]]-L-cysteinyl-glycyl-4-aminobenzoyl-L-glutaminyl-L-tryptophyl-L-alanyl-L-valyl-glycyl-L-histidyl-L-leucyl-L-methioninamide (**L238**), as described in Example XI.

**FIG. 11B** is a graphical representation of a reaction for the synthesis of *N,N*-Dimethylglycyl-L-serinyl-[S-[(acetylamino)methyl]]-L-cysteinyl-glycyl-(3β,5β,7α,12α)-3-amino-7,12-dihydroxy-24-oxocholan-24-yl-L-glutaminyl-L-tryptophyl-L-alanyl-L-valyl-glycyl-L-histidyl-L-leucyl-L-methioninamide, (**L239**), as described in Example XI.

**FIG. 12A** is a graphical representation of a reaction for the synthesis of 4-[[[(9*H*-Fluoren-9-ylmethoxy)carbonyl] amino]acetyl]amino-3-methoxybenzoic acid (**A**), as described in Example XII.

**FIG. 12B** is a graphical representation of a reaction for the synthesis of 4-[[[(9*H*-Fluoren-9-ylmethoxy)carbonyl] amino]acetyl]amino-3-chlorobenzoic acid, (**D**), as described in Example XII.

**FIG. 12C** is a graphical representation of a reaction for the synthesis of 4-[[[(9*H*-Fluoren-9-ylmethoxy)carbonyl] amino]acetyl]amino-3-methylbenzoic acid (**E**), as described in Example XII.

**FIG. 12D** is a chemical structure of *N*-[4-[[[[4,7,10-Tris(carboxymethyl)-1,4,7,10-tetraazacyclododec-1-yl] acetyl]glycyl] amino]-3-methoxybenzoyl]-L-glutaminyl-L-tryptophyl-1-alanyl-L-valyl-glycyl-L-histidyl-L-leucyl-L-methioninamide (**L240**) as described in Example XII.

**FIG. 12E** is a chemical structure of compound *N*-[4-[[[[4,7,10-Tris(carboxymethyl)-1,4,7,10tetraazacyclododec-1-yl] acetyl]glycyl]amino]3-chlorobenzoyl]L-glutaminyl-L-tryptophyl-1-alanyl-L-valyl-glycyl-L-histidyl-L-leucyl-L-methioninamide, (**L241**) as described in Example XII.

**FIG. 12F** is a chemical structure of N-[4-[[[[4,7,10-Tris(carboxymethyl)-1,4,7,10tetraazacyclododec-1-yl] acetyl] glycyl] amino] 3-methylbenzoyl] L-glutaminyl-L-tryptophyl-1-alanyl-L-valyl-glycyl-L-histidyl-leucyl-L-methioninamide (**L242**), as described in Example XII.

**FIG. 13A** is a graphical representation of a reaction for the synthesis of 4-[*N,N'*-Bis[2-[(9-*H*-fluoren-9-ylmethoxy) carbonyl]aminoethyl]amino]-4-oxobutanoic acid, (**D**), as described in Example XIII.

**FIG. 13B** is a graphical representation of a reaction for the synthesis of *N*-[4-[[4-[Bis[2-[[[4,7,10-tris(carboxymethyl)-1,4,7,10-tetraazacyclododec-1-yl]acetyl]amino]ethyl]amino-1,4-dioxobutyl]amino]benzoyl]-L-glutaminyl-L-tryptophyl-L-alanyl-L-valyl-glycyl-L-histidyl-L-leucyl-L-methioninamide, (**L244**), as described in Example XIII.

**FIG. 13C** is a chemical structure of compound **L244,** as described in Example XIII.

**FIG. 14A** and **FIG. 14B** are graphical representations of the binding and competition curves described in Example XLIII.

**FIG. 15A** is a graphical representation of the results of radiotherapy experiments described in Example LV.

**FIG. 15B** is a graphical representation of the results of other radiotherapy experiments described in Example LV.

**FIG. 16** is a chemical structure of N-[4-[[[[4,7,10-Tris(carboxymethyl)-1,4,7,10tetraazacyclododec-1-yl] acetyl] glycyl]amino]-L-Lysinyl-(3,6,9)-trioxaundecane-1,11-dicarboxylic acid-3,7-dideoxy-3-aminocholic acid)-L-arginyl-L-glutaminyl-L-triptophyl-L-alanyl-L-valyl-glycyl-L-histidyl-L-leucyl-L-methioninamide (**L65**).

**FIG. 17** is a chemical structure of *N*-[2-S-[[[[[12α-Hydroxy-17α-(1-methyl-3-carboxypropyl)etiocholan-3β-carbamoylmethoxyethoxyethoxyacetyl]-amino -6-[4,7,10-tris(carboxymethyl)-1,4,7,10-tetraazacyclododec-1-yl]acetyl] amino]acetyl]amino] hexanoyl-L-glutaminyl-L-tryptophyl-L-alanyl-L-valyl-glycyl-L-histidyl-L-leucyl-L-methioninamide (**L66**).

**FIG. 18A** is a chemical structure of *N*-[4-[[[[[4,7,10-Tris(carboxymethyl)-1,4,7,10-tetraazacyclododec-1-yl] acetyl]amino]acetyl]amino]benzoyl]-L-glutaminyl-L-tryptophyl-L-alanyl-L-valyl-glycyl-L-histidyl-L-leucyl-L-methioninamide (**L70**).

**FIG. 18B** is a chemical structure *N*-[4-[[[[[4,7,10-Tris(carboxymethyl)-1,4,7,10-tetraazacyclododec-1-yl]-3-carboxypropionyl]amino]acetyl]amino]benzoyl]-L-glutaminyl-L-tryptophyl-L-alanyl-L-valyl-glycyl-L-histidyl-L-leucyl-L-methioninamide (**L114**).

**FIG. 18C** is a chemical structure *N*-[4-[[4,7,10-Tris(carboxymethyl)-1,4,7,10-tetraazacyclododec-1-yl]-2-hydroxy- 3- propoxy] benzoyl]-L- glutaminyl- L- tryptophyl- L- alanyl- L- valyl- glycyl- L- histidyl- L- leucyl- L- methioninamide (**L144**).

**FIG. 18D** is a chemical structure *N*-[(3β,5β,7α,12α)-3-[[[[[4,7,10-Tris(carboxymethyl)-1,4,7,10-tetraazacyclododec-1-yl]acetyl]amino]ethoxyethoxy]acetyl]amino]-7,12-dihydroxycholan-24-yl]-L-glutaminyl-L-tryptophyl-L-alanyl-L-valyl-glycyl-L-histidyl-L-leucyl-L-methioninamide (**L69**).

**FIG. 18E** is a chemical structure of *N*-[4-[[[[[4,7,10-Tris(carboxymethyl)-1,4,7,10-tetraazacyclododec-1-yl] acetyl] amino] acetyl] amino] phenylacetyl]-L- glutaminyl- L- tryptophyl- L- alanyl- L- valyl- glycyl- L- histidyl- L- leucyl- L- methioninamide (**L146**).

**FIG. 19** dicloses chemical structures of intermediates which may be used to prepare compounds **L64** and **L70** as described in Example LVI.

**FIG. 20** is a graphical representation of the preparation of **L64** using segment coupling as described in Example LVI.

**FIG. 21** is a graphical representation of the preparation of (IR)-1-(Bis{2-[bis(carboxymethyl)amino]ethyl}amino) propane-3-carboxylicacid-1-carboxyl-glycyl-4-aminobenzoyl-L-glutaminyl-L-tryptophyl-L-alanyl-L-valyl-glycyl-L-histidyl-L-leucyl-L-methioninamide (L201).

**FIG. 22A** is a graphical representation of chemical structure of chemical intermediates used to prepare L202.

**FIG. 22B** is a graphical representation of the preparation of N-[(3β,5β,12α)-3-[[[[4,7,10-Tris(carboxymethyl)-1,4,7,10-tetraazacyclododec-1-yl]acetyl]amino]acetyl]amino]-4-hydrazinobenzoyl-L-glutaminyl-L-tryptophyl-L-alanyl-L-valyl-glycyl-L-histidyl-L-leucyl-L-methioninamide (L202).

**FIG. 23A** is a graphical representation of chemical structure of chemical intermediates used to prepare L203.

**FIG. 23B** is a graphical representation of the preparation of N-[(3β,5β,12α)-3-[[[4,7,10-Tris(carboxymethyl)-1,4,7,10-tetraazacyclododec-1-yl]acetyl]amino]-4-aminobenzoyl-L-glutaminyl-L-tryptophyl-L-alanyl-L-valyl-glycyl-L-histidyl-L-leucyl-L-methioninamide (L203).

**FIG. 24** is a graphical representation of the preparation of N-[(3β,5β,12α)-3-[[[4,7,10-Tris(carboxymethyl)-1,4,7,10-tetraazacyclododec-1-yl]acetyl]amino]-4-aminobenzoyl-glycyl-L-glutaminyl-L-tryptophyl-L-alanyl-L-valyl-glycyl-L-histidyl-L-leucyl-L-methioninamide (L204).

**FIG. 25** is a graphical representation of the preparation of N-[(3β,5β,12α)-3-[[[4,7,10-Tris(carboxymethyl)-1,4,7,10-tetraazacyclododec-1-yl]acetyl]amino]-4-aminobenzoyl-glycyl-L-glutaminyl-L-tryptophyl-L-alanyl-L-valyl-glycyl-L-histidyl-L-leucyl-L-methioninamide (L205).

**FIG. 26A** is a graphical representation of chemical structures of chemical intermediates used to prepare L206.

**FIG. 26B** is a graphical representation of the preparation of N-[(3β,5β,12α)-3-[[[[4,7,10-Tris(carboxymethyl)-1,4,7,10-tetraazacyclododec-1-yl]acetyl]amino]acetyl]amino]- [4'-Amino-2'-methyl biphenyl-4-carboxyl]-L-glutaminyl-L-tryptophyl-L-alanyl-L-valyl-glycyl-L-histidyl-L-leucyl-L-methioninamide (L206).

**FIG. 27A** is a graphical representation of chemical structures of chemical intermediates used to prepare L207.

**FIG. 27B** is a graphical representation of the preparation of N-[(3β,5β,12α)-3-[[[[4,7,10-Tris(carboxymethyl)-1,4,7,10-tetraazacyclododec-1-yl]acetyl]amino]acetyl]amino]- [3'-amino-biphenyl-3-carboxyl]-L-glutaminyl-L-tryptophyl-L-alanyl-L-valyl-glycyl-L-histidyl-L-leucyl-L-methioninamide (L207).

**FIG. 28** is a graphical representation of the preparation of N-[(3β,5β,12α)-3-[[[[4,7,10-Tris(carboxymethyl)-1,4,7,10-tetraazacyclododec-1-yl]acetyl]amino]acetyl]amino]- [1,2-diaminoethyl-terephthalyl]-L-glutaminyl-L-tryptophyl-L-alanyl-L-valyl-glycyl-L-histidyl-L-leucyl-L-methioninamide (L208).

**FIG. 29A** is a graphical representation of chemical structures of chemical intermediates used to prepare L209.

**FIG. 29B** is a graphical representation of the preparation of L209.

**FIG. 30A** is a graphical representation of chemical structures of chemical intermediates used to prepare L210.

**FIG. 30B** is a chemical structure of L210.

**FIG. 31** is a chemical structure of N-[(3β,5β,12α)-3-[[[4,7,10-Tris(carboxymethyl)-1,4,7,10-tetraazacyclododec-1-yl]acetyl]amino]-glycyl-glycyl-4-aminobenzoyl-L-glutaminyl-L-tryptophyl-L-alanyl-L-valyl-glycyl-L-histidyl-L-leucyl-L-methioninamide L211.

**FIG. 32** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutamyl-L-tryptophyl-L-alanyl-L-valyl-glycyl-L-histidyl-L-leucyl-L-methioninamide L212.

**FIG. 33** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-tryptophyl-L-alanyl-L-valyl-glycyl-L-histidyl-L-leucyl-L-methionine carboxylate L213.

**FIG. 34** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-D-phenylalanyl-L-glutaminyl-L-tryptophyl-L-alanyl-L-valyl-glycyl-L-histidyl-L-leucyl-L-methioninamide L214.

**FIG. 35** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-arginyl-L-leucyl-glycyl-L-asparginyl-L-glutaminyl-L-tryptophyl-L-alanyl-L-valyl-glycyl-L-histidyl-L-leucyl-L-methioninamide L215.

**FIG. 36** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-arginyl-L-tyrosinyl-glycyl-L-asparginyl-L-glutaminyl-L-tryptophyl-L-alanyl-L-valyl-glycyl-L-histidyl-L-leucyl-L-methioninamide L216.

**FIG. 37** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-lysyl-L-tyrosinyl-glycyl-L-glutaminyl-L-tryptophyl-L-alanyl-L-valyl-glycyl-L-histidyl-L-leucyl-L-methioninamide L217.

**FIG. 38** is a chemical structure of L218.

**FIG. 39** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-D-phenylalanyl-L-glutaminyl-L-tryptophyl-L-alanyl-L-valyl-glycyl-L-histidyl-L-leucyl-aminopentyl, L219.

**FIG. 40** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-tryptophyl-L-serinyl-L-valyl-D-alanyl-L-histidyl-L-leucyl-L-methioninamide, L220.

**FIG. 41** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-D-phenylalanyl-L-glutaminyl-L-tryptophyl-L-alanyl-L-valyl-glycyl-L-histidyl-L-leucyl-L-leucinamide, L221.

**FIG. 42** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-D-tyrosinyl-L-glutaminyl-L-tryptophyl-L-alanyl-L-valyl-betaalanyl-L-histidyl-L-phenylalanyl-L-norleucinamide, L222.

**FIG. 43** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-phenylalanyl-L-glutaminyl-L-tryptophyl-L-alanyl-L-valyl-betaalanyl-L-histidyl-L-phenylalanyl-L-norleucinamide, L223.

**FIG. 44** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-tryptophyl-L-alanyl-glycyl-L-histidyl-L-phenylalanyl-L-leucinamide, L224.

**FIG. 45** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-leucyl-L-tryptophyl-L-alanyl-L-valinyl-glycyl-L-serinyl-L-phenylalanyl-L-methioninamide, L225.

**FIG. 46** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-histidyl-L-tryptophyl-L-alanyl-L-valyl-glycyl-L-histidyl-L-leucyl-L-methioninamide, L226.

**FIG. 47** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-leucyl-L-tryptophyl-L-alanyl-L-valyl-glycyl-L-serinyl-L-phenylalanyl-L-methioninamide L227.

**FIG. 48** is a chemical structure of N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-tryptophyl-L-alanyl-L-valyl-glycyl-L-histidyl-L-phenylalanyl-L-methioninamide, L228.

**FIG. 49** is a graphical representation of a reaction for the synthesis of (3β,5β ,7α,12α)-3-(9H-Fluoren-9-ylmethoxy)amino-7,12-dihydroxycholan-24-oic acid (**B**) as described in Example LVII.

**FIG. 50** is a graphical representation of a reaction for the synthesis of N-[3β,5β ,7α,12α)-3-[[[2-[2-[[[4,7,10-Tris (carboxymethyl)-1,4,7,10-tetraazacyclododec-1-yl]acetyl]amino]ethoxy]ethoxy]acetyl]amino]-7,12-dihydroxy-24-oxocholan-24-yl]-L-glutaminyl-L-tryptophyl-L-alanyl-L-valyl-glycyl-L-histidyl-L-leucyl-L-methioninamide, (**L69**), as described in Example LVII.

**FIG. 51** is a graphical representation of a reaction for the synthesis of N-[4-[2-Hydroxy-3-[4,7,10-tris(carboxymethyl)-1,4,7,10-tetraazacyclododec-1-yl]propoxy]benzoyl]-L-glutaminyl-L-tryptophyl-L-alanyl-L-valyl-glycyl-L-histidyl-L-leucyl-L-methioninamide (L144), as described in Example LVIII.

## DETAILED DESCRIPTION OF THE INVENTION

In the following description, various aspects of the present invention will be further elaborated. For purposes of explanation, specific configurations and details are set forth in order to provide a thorough understanding of the present invention. However, it will also be apparent to one skilled in the art that the present invention may be practiced without the specific details. Furthermore, well known features may be omitted or simplified in order not to obscure the present invention.

In an embodiment of the present invention, there are provided new and improved compounds for use in diagnostic imaging or radiotherapy. The compounds include an optical label or a chemical moiety capable of complexing a medically useful metal ion or radionuclide (metal chelator) attached to a GRP receptor targeting peptide by a linker or

spacer group.

In general, compounds of the present invention may have the formula:

$$M\text{-}N\text{-}O\text{-}P\text{-}G$$

wherein M is the metal chelator (in the form complexed with a metal radionuclide or not), or an optical label, N-O-P is the linker, and G is the GRP receptor targeting peptide. Each of the metal chelator, optical label, linker, and GRP receptor targeting peptide is described in the discussion that follow.

In another embodiment of the present invention, there is provided a new and improved linker or spacer group which is capable of linking an optical label or a metal chelator to a GRP receptor targeting peptide. In general, linkers of the present invention may have the formula:

$$N\text{-}O\text{-}P$$

wherein each of N, O and P are defined throughout the specification.

Compounds meeting the criteria defined herein were discovered to have improved pharmacokinetic properties compared to other GRP receptor targeting peptide conjugates known in the art. For example, compounds containing the linkers of the present invention were retained in the bloodstream longer, and thus had a longer half life than prior known compounds. The longer half life was medically beneficial because it permitted better tumor targeting which is useful for diagnostic imaging, and especially for therapeutic uses, where the cancerous cells and tumors receive greater amounts of the radiolabeled peptides.

1A. Metal Chelator

The term "metal chelator" refers to a molecule that forms a complex with a metal atom, wherein said complex is stable under physiological conditions. That is, the metal will remain complexed to the chelator backbone *in vivo.* More particularly, a metal chelator is a molecule that complexes to a radionuclide metal to form a metal complex that is stable under physiological conditions and which also has at least one reactive functional group for conjugation with the linker N-O-P. The metal chelator M may be any of the metal chelators known in the art for complexing a medically useful metal ion or radionuclide. The metal chelator may or may not be complexed with a metal radionuclide. Furthermore, the metal chelator can include an optional spacer such as, for example, a single amino acid (*e.g.,* Gly) which does not complex with the metal, but which creates a physical separation between the metal chelator and the linker.

The metal chelators of the invention may include, for example, linear, macrocyclic, terpyridine, and $N_3S$, $N_2S_2$, or $N_4$ chelators (*see also,* U.S. 5,367,080, U.S. 5,364,613, U.S. 5,021,556, U.S. 5,075,099, U.S. 5,886,142, the disclosures of which are incorporated by reference herein in their entirety), and other chelators known in the art including, but not limited to, HYNIC, DTPA, EDTA, DOTA, TETA, and bisamino bisthiol (BAT) chelators (*see also* U.S. 5,720,934). For example, $N_4$ chelators are described in U.S. Patent Nos. 6,143,274; 6,093,382; 5,608,110; 5,665,329; 5,656,254; and 5,688,487, the disclosures of which are incorporated by reference herein in their entirety. Certain $N_3S$ chelators are described in PCT/CA94/00395, PCT/CA94/00479, PCT/CA95/00249 and in U.S. Patent Nos. 5,662,885; 5,976,495; and 5,780,006, the disclosures of which are incorporated by reference herein in their entirety. The chelator may also include derivatives of the chelating ligand mercapto-acetyl-glycyl-glycyl-glycine (MAG3), which contains an $N_3S$, and $N_2S_2$ systems such as MAMA (monoamidemonoaminedithiols), DADS ($N_2S$ diaminedithiols), CODADS and the like. These ligand systems and a variety of others are described in Liu and Edwards, Chem Rev. 1999, 99, 2235-2268 and references therein, the disclosures of which are incorporated by reference herein in their entirety.

The metal chelator may also include complexes containing ligand atoms that are not donated to the metal in a tetradentate array. These include the boronic acid adducts of technetium and rhenium dioximes, such as those described in U.S. Patent Nos. 5,183,653; 5,387,409; and 5,118,797, the disclosures of which are incorporated by reference herein, in their entirety.

Examples of preferred chelators include, but are not limited to, diethylenetriamine pentaacetic acid (DTPA), 1,4,7,10-tetraazacyclotetradecane-1,4,7,10-tetraacetic acid (DOTA), 1-substituted 1,4,7,-tricarboxymethyl 1,4,7,10-tetraazacyclododecane triacetic acid (DO3A), ethylenediaminetetraacetic acid (EDTA), 4-carbonylmethyl-10-phosponomethyl-1,4,7,10-Tetraazacyclododecane-1,7-diacetic acid (Cm4pm10d2a); and 1,4,8,11-tetraazacyclotetradecane-1,4,8,11-tetraacetic acid (TETA). Additional chelating ligands are ethylenebis-(2-hydroxy-phenylglycine) (EHPG), and derivatives thereof, including 5-C1-EHPG, 5-Br-EHPG, 5-Me-EHPG, 5-t-Bu-EHPG, and 5-sec-Bu-EHPG; benzodiethylenetriamine pentaacetic acid (benzo-DTPA) and derivatives thereof, including dibenzo-DTPA, phenyl-DTPA, diphenyl-DTPA, benzyl-DTPA, and dibenzyl-DTPA; bis-2 (hydroxybenzyl)-ethylene-diaminediacetic acid (HBED) and derivatives thereof; the class of macrocyclic compounds which contain at least 3 carbon atoms, more preferably at least 6, and at least two heteroatoms (O and/or N), which macrocyclic compounds can consist of one ring, or two or three rings joined together at the hetero ring elements, e.g., benzo-DOTA, dibenzo-DOTA, and benzo-NOTA, where NOTA is 1,4,7-triazacyclononane N,N',N"-triacetic acid, benzo-TETA, benzo-DOTMA, where DOTMA is 1,4,7,10-tetraazacyclotetradecane-1,4,7, 10-tetra(methyl tetraacetic acid), and benzo-TETMA, where TETMA is 1,4,8,11- tetraazacyclotetradecane-1,4,8,11-(methyl tetraacetic acid); derivatives of 1,3-propylenediaminetetraacetic acid (PDTA) and triethylenetetraaminehexaacetic acid (TTHA); derivatives of 1,5,10-N,N',N"-tris(2,3-dihydroxybenzoyl)-tricatecholate (LICAM) and 1,3,5-N,N',N"-tris(2,3-dihydroxybenzoyl) aminomethylbenzene (MECAM). Examples of representative chelators and chelating groups contemplated by the present invention are described in WO 98/18496, WO 86/06605, WO 91/03200, WO 95/28179, WO 96/23526, WO 97/36619, PCT/US98/01473, PCT/US98/20182, and U.S. 4,899,755, U.S. 5,474,756, U.S. 5,846,519 and U.S. 6,143,274, each of which is hereby incorporated by reference in its entirety.

Particularly preferred metal chelators include those of Formula 1, 2 and 3 (for $^{111}$In and radioactive lanthanides, such as, for example $^{177}$Lu, $^{90}$Y, $^{153}$Sm, and $^{166}$Ho) and those of Formula 4, 5 and 6 (for radioactive $^{99m}$Tc, $^{186}$Re, and $^{188}$Re) set forth below. These and other metal chelating groups are described in U.S. Patent Nos. 6,093,382 and 5,608,110, which are incorporated by reference herein in their entirety. Additionally, the chelating group of formula 3 is described in, for example, U.S. Patent No. 6,143,274; the chelating group of formula 5 is described in, for example, U.S. Patent Nos. 5,627,286 and 6,093,382, and the chelating group of formula 6 is described in, for example, U.S. Patent Nos. 5,662,885; 5,780,006; and 5,976,495, all of which are incorporated by reference. Specific metal chelators of formula 6 include N,N-dimethylGly-Ser-Cys; N,N-dimethylGly-Thr-Cys ; N,N-diethylGly-Ser-Cys; N,N-dibenzylGly-Ser-Cys; and other variations thereof. For example, spacers which do not actually complex with the metal radionuclide such as an extra single amino acid Gly, may be attached to these metal chelators (*e.g.,* N,N-dimethylGly-Ser-Cys-Gly; N,N-dimethylGly-Thr-Cys-Gly; N,N-diethylGly-Ser-Cys-Gly; N,N-dibenzylGly-Ser-Cys-Gly). Other useful metal chelators such as all of those disclosed in U.S. Pat. No. 6,334,996, also incorporated by reference (*e.g.,* Dimethylgly-L-t-Butylgly-L-Cys-Gly; Dimethylgly-D-t-Butylgly-L-Cys-Gly; Dimethylgly-L-t-Butylgly-L-Cys, etc.)

Furthermore, sulfur protecting groups such as Acm (acetamidomethyl), trityl or other known alkyl, aryl, acyl, alkanoyl, aryloyl, mercaptoacyl and organothiol groups may be attached to the cysteine amino acid of these metal chelators.

Additionally, other useful metal chelators include:

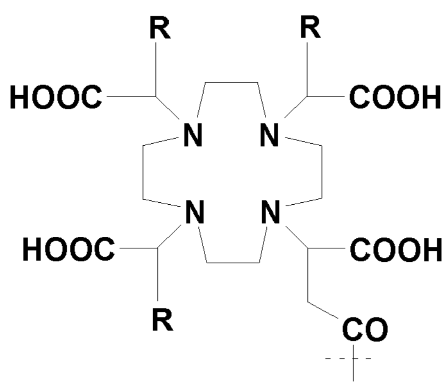

(1)

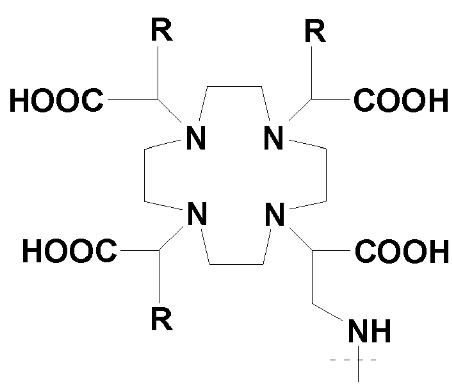

(2)

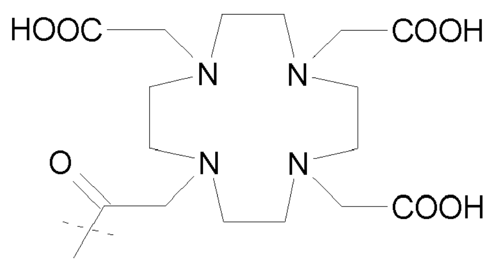

(3)

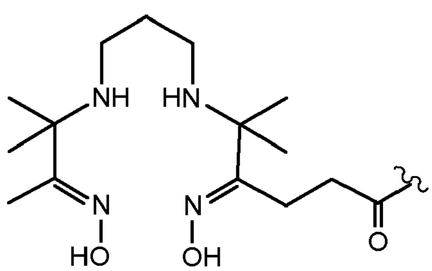

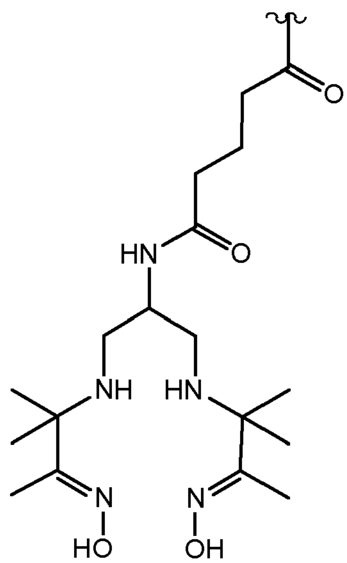

(4a) (4b)

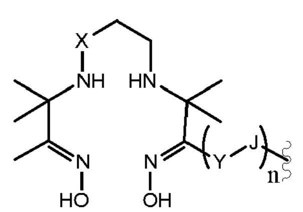

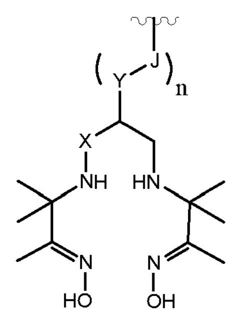

(5a) (5b)

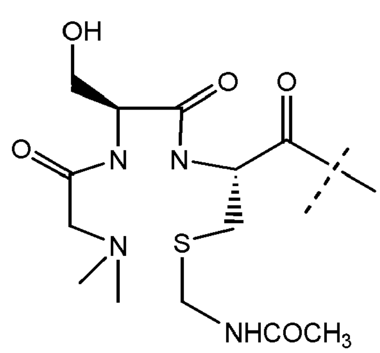

(6)

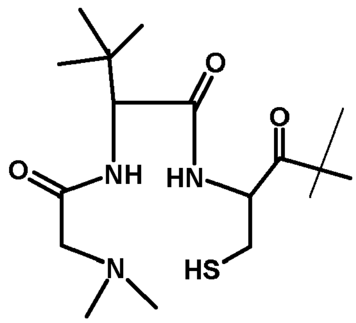

(7)

In the above Formulas 1 and 2, R is alkyl, preferably methyl. In the above Formulas 5a and 5b, X is either $CH_2$ or O; Y is $C_1$-$C_{10}$ branched or unbranched alkyl; aryl, aryloxy, arylamino, arylaminoacyl; arylalkyl - where the alkyl group or groups attached to the aryl group are $C_1$-$C_{10}$ branched or unbranched alkyl groups, $C_1$-$C_{10}$ branched or unbranched hydroxy or polyhydroxyalkyl groups or polyalkoxyalkyl or polyhydroxy-polyalkoxyalkyl groups; J is optional, but if present is C(=O)-, OC(=O)-, $SO_2$-, NC(=O)-, NC(=S)-, N(Y), NC(=$NCH_3$)-, NC(=NH)-, N=N-, homopolyamides or heteropolyamines derived from synthetic or naturally occurring amino acids; all where n is 1-100. Other variants of these structures are described, for example, in U.S. Patent No. 6,093,382. In Formula 6, the group S-$NHCOCH_3$ may be replaced with SH or S-Z wherein Z is any of the known sulfur protecting groups such as those described above. Formula 7 illustrates one embodiment of t-butyl compounds useful as a metal chelator. The disclosures of each of the foregoing patents, applications and references are incorporated by reference herein, in their entirety.

In a preferred embodiment, the metal chelator includes cyclic or acyclic polyaminocarboxylic acids such as DOTA (1,4,7,10-tetraazacyclododecane -1,4,7,10-tetraacetic acid), DTPA (diethylenetriaminepentaacetic acid), DTPA-bismethylamide, DTPA-bismorpholineamide, Cm4pm10d2a (4-carbonylmethyl-10-phosponomethyl-1,4,7,10-Tetraazacyclododecane-1,7-diacetic acid), DO3A *N*-[[4,7,10-Tris(carboxymethyl)-1,4,7,10-tetraazacyclododec-1-yl]acetyl, HP-DO3A, DO3A-monoamide and derivatives thereof.

Preferred metal radionuclides for scintigraphy or radiotherapy include $^{99m}$Tc, $^{51}$Cr, $^{67}$Ga, $^{68}$Ga, $^{47}$Sc, $^{51}$Cr, $^{167}$Tm, $^{141}$Ce, $^{111}$In, $^{168}$Yb, $^{175}$Yb, $^{140}$La, $^{90}$Y, $^{88}$Y, $^{153}$Sm, $^{166}$Ho, $^{165}$Dy, $^{166}$Dy, $^{62}$Cu, $^{64}$Cu $^{67}$Cu, $^{97}$Ru, $^{103}$Ru, $^{186}$Re, $^{188}$Re, $^{203}$Pb, $^{211}$Bi, $^{212}$Bi , $^{213}$Bi, $^{214}$Bi, $^{105}$Rh, $^{109}$Pd, $^{117m}$Sn, $^{149}$Pm, $^{161}$Tb, $^{177}$Lu, $^{198}$Au and $^{199}$Au and oxides or nitrides thereof. The choice of metal will be determined based on the desired therapeutic or diagnostic application. For example, for diagnostic purposes (*e.g.,* to diagnose and monitor therapeutic progress in primary tumors and metastases), the preferred radionuclides include $^{64}$Cu, $^{67}$Ga, $^{68}$Ga, $^{99m}$Tc, and $^{111}$In, with $^{99m}$Tc and $^{111}$In being especially preferred. For therapeutic purposes (*e.g.,* to provide radiotherapy for primary tumors and metastasis related to cancers of the prostate, breast, lung, etc.), the preferred radionuclides include $^{64}$Cu, $^{90}$Y, $^{105}$Rh, $^{111}$In, $^{117m}$Sn, $^{149}$Pm, $^{153}$Sm, $^{161}$Tb, $^{166}$Dy, $^{166}$Ho, $^{175}$Yb, $^{177}$Lu, $^{186/188}$Re, and $^{199}$Au, with $^{177}$Lu and $^{90}$Y being particularly preferred. $^{99m}$Tc is particularly useful and is a preferred for diagnostic radionuclide because of its low cost, availability, imaging properties, and high specific activity. The nuclear and radioactive properties of $^{99m}$Tc make this isotope an ideal scintigraphic imaging agent. This isotope has a single photon energy of 140 keV and a radioactive half-life of about 6 hours, and is readily available from a $^{99}$Mo-$^{99m}$Tc generator. For example, the $^{99m}$Tc labeled peptide can be used to diagnose and monitor therapeutic progress in primary tumors and metastases. Peptides labeled with $^{177}$Lu, $^{90}$Y or other therapeutic radionuclides can be used to provide radiotherapy for primary tumors and metastasis related to cancers of the prostate, breast, lung, etc.

1B. <u>Optical Labels</u>

In an exemplary embodiment, the compounds of the invention may be conjugated with photolabels, such as optical dyes, including organic chromophores or fluorophores, having extensive delocalized ring systems and having absorption or emission maxima in the range of 400-1500 nm. The compounds of the invention may alternatively be derivatized with a bioluminescent molecule. The preferred range of absorption maxima for photolabels is between 600 and 1000 nm to minimize interference with the signal from hemoglobin. Preferably, photoabsorption labels have large molar absorptivities, *e.g.* > $10^5$ $cm^{-1}M^{-1}$, while fluorescent optical dyes will have high quantum yields. Examples of optical dyes include, but are not limited to those described in WO 98/18497, WO 98/18496, WO 98/18495, WO 98/18498, WO 98/53857, WO 96/17628, WO 97/18841, WO 96/23524, WO 98/47538, and references cited therein. For example, the photolabels may be covalently linked directly to compounds of the invention, such as, for example, compounds comprised of GRP receptor targeting peptides and linkers of the invention. Several dyes that absorb and emit light in the visible

and near-infrared region of electromagnetic spectrum are currently being used for various biomedical applications due to their biocompatibility, high molar absorptivity, and/or high fluorescence quantum yields. The high sensitivity of the optical modality in conjunction with dyes as contrast agents parallels that of nuclear medicine, and permits visualization of organs and tissues without the undesirable effect of ionizing radiation. Cyanine dyes with intense absorption and emission in the near-infrared (NIR) region are particularly useful because biological tissues are optically transparent in this region. For example, indocyanine green, which absorbs and emits in the NIR region has been used for monitoring cardiac output, hepatic functions, and liver blood flow and its functionalized derivatives have been used to conjugate biomolecules for diagnostic purposes (R. B. Mujumdar, L. A. Ernst, S. R. Mujumdar, et al., Cyanine dye labeling reagents: Sulfoindocyanine succinimidyl esters. Bioconjugate Chemistry, 1993, 4(2), 105-111; Linda G. Lee and Sam L. Woo. "N-Heteroaromatic ion and iminium ion substituted cyanine dyes for use as fluorescent labels", U.S. Pat. No. 5,453,505; Eric Hohenschuh, et al. "Light imaging contrast agents", WO 98/48846; Jonathan Turner, et al. "Optical diagnostic agents for the diagnosis of neurodegenerative diseases by means of near infra-red radiation", WO 98/22146; Kai Licha, et al. "In-vivo diagnostic process by near infrared radiation", WO 96/17628; Robert A. Snow, et al., Compounds, WO 98/48838. Various imaging techniques and reagents are described in U.S. Patents 6,663,847, 6,656,451, 6,641,798, 6,485,704, 6,423,547, 6,395,257, 6,280,703, 6,277,841, 6,264,920, 6,264,919, 6,228,344, 6,217,848, 6,190,641, 6,183,726, 6,180,087, 6,180,086, 6,180,085, 6,013,243, and published U.S. Patent Applications 2003185756, 20031656432, 2003158127, 2003152577, 2003143159, 2003105300, 2003105299, 2003072763, 2003036538, 2003031627, 2003017164, 2002169107, 2002164287, and 2002156117.

2A. Linkers Containing At Least One Non-alpha Amino Acid

In one embodiment of the invention, the linker N-O-P contains at least one non-alpha amino acid. Thus, in this embodiment of the linker N-O-P, N is 0 (where 0 means it is absent), an alpha or non-alpha amino acid or other linking group; O is an alpha or non-alpha amino acid; and
P is 0, an alpha or non-alpha amino acid or other linking group,
wherein at least one of N, O or P is a non-alpha amino acid.
Thus, in one example, N = Gly, O = a non-alpha amino acid, and P= 0.

Alpha amino acids are well known in the art, and include naturally occurring and synthetic amino acids.

Non-alpha amino acids are also known in the art and include those which are naturally occurring or synthetic.
Preferred non-alpha amino acids include: 8-amino-3,6-dioxaoctanoic acid;
N-4-aminoethyl-N-1-acetic acid; and
polyethylene glycol derivatives having the formula $NH_2$-$(CH_2CH_2O)$n-$CH_2$ or $NH_2$-$(CH_2CH_2O)$n-$CH_2CH_2CO_2H$ where n = 2 to 100.

Examples of compounds having the formula M-N-O-P-G which contain linkers with at least one non-alpha amino acid are listed in Table 1. These compounds may be prepared using the methods disclosed herin, particularly in the Examples, as well as by similar methods known to one skilled in the art.

**TABLE 1**

| Compounds Containing Linkers With At Least One Non-alpha Amino Acid | | | | | | | | | |
|---|---|---|---|---|---|---|---|---|---|
| **Compound** | **HPLC method[1]** | **HPLC RT[2]** | **MS[3]** | **IC50[5]** | **M** | **N** | **0** | **P** | **G*** |
| L1 | 10-40%B | 5.43 | 1616.6 | 5 | N,N-dimethylglycine -Ser-Cys (Acm)-Gly | Lys | 8-amino-3,6-dioxaoctanoic acid | none | BBN(7-14) |
| L2 | 10-40%B | 5.47 | 1644.7 | 3 | N,N-dimethylglycine -Ser-Cys (Acm)-Gly | Arg | 8-amino-3,6-dioxaoctanoic acid | none | BBN(7-14) |
| L3 | 10-40%B | 5.97 | 1604.6 | >50 | N,N-dimethylglycine -Ser-Cys (Acm)-Gly | Asp | 8-amino-3,6-dioxaoctanoic acid | none | BBN(7-14) |
| L4 | 10-40%B | 5.92 | 1575.5 | 4 | N,N-dimethylglycine -Ser-Cys (Acm)-Gly | Ser | 8-amino-3,6-dioxaoctanoic acid | none | BBN(7-14) |
| L5 | 10-40%B | 5.94 | 1545.5 | 9 | N,N-dimethylglycine -Ser-Cys (Acm)-Gly | Gly | 8-amino-3,6-dioxaoctanoic acid | none | BBN(7-14) |
| L6 | 10-30%B | 7.82 | 1639(M+Na) | >50 | N,N-dimethylglycine -Ser-Cys (Acm)-Gly | Glu | 8-amino-3,6-dioxaoctanoic acid | none | BBN(7-14) |

(continued)

| Compound | HPLC method [1] | HPLC RT[2] | MS[3] | IC50[5] | M | N | O | P | G* |
|---|---|---|---|---|---|---|---|---|---|
| L7 | 10-30%B | 8.47 | 1581 (M+Na) | 7 | N,N-dimethylglycine -Ser-Cys (Acm)-Gly | Dala | 8-amino-3,6-dioxaoctanoic acid | none | BBN(7-14) |
| L8 | 10-30%B | 6.72 | 1639 (M+Na) | 4 | N,N-dimethylglycine -Ser-Cys (Acm)-Gly | 8-amino-3,6-dioxaoctanoic acid | Lys | none | BBN(7-14) |
| L9 | 10-30%B | 7.28 | 823.3 (M+2/2) | 6 | N,N-dimethylglycine -Ser-Cys (Acm)-Gly | 8-amino-3,6-dioxaoctanoic acid | Arg | none | BBN(7-14) |
| L10 | 10-30%B | 7.94 | 1625.6 (M+Na) | >50 | N,N-dimethylglycine -Ser-Cys (Acm)-Gly | 8-amino-3,6-dioxaoctanoic acid | Asp | none | BBN(7-14) |
| L11 | 10-30%B | 7.59 | 1575.6 | 36 | N,N-dimethylglycine -Ser-Cys (Acm)-Gly | 8-amino-3,6-dioxaoctanoic acid | Ser | none | BBN(7-14) |
| L12 | 10-30%B | 7.65 | 1567.5 (M+Na) | >50 | N,N-dimethylglycine -Ser-Cys (Acm)-Gly | 8-amino-3,6-dioxaoctanoic acid | Gly | none | BBN(7-14) |
| L13 | 10-30%B | 7.86 | 1617.7 | >50 | N,N-dimethylglycine -Ser-Cys (Acm)-Gly | 8-amino-3,6-dioxaoctanoic acid | Glu | none | BBN(7-14) |
| L14 | 10-30%B | 7.9 | 1581.7 (M+Na) | 11 | N,N-dimethylglycine -Ser-Cys (Acm)-Gly | 8-amino-3,6-dioxaoctanoic acid | Dala | none | BBN(7-14) |

(continued)

| Compound | HPLC method [1] | HPLC RT[2] | MS[3] | IC50[5] | M | N | O | P | G* |
|---|---|---|---|---|---|---|---|---|---|
| L15 | 10-30%B | 7.84 | 1656.8 (M+Na) | 11.5 | N,N-dimethylglycine -Ser-Cys (Acm)-Gly | 8-amino-3,6-dioxaoctanoic acid | 8-amino-3,6-dioxaoctanoic acid | none | BBN(7-14) |
| L16 | 10-30%B | 6.65 | 1597.4 (M+Na) | 17 | N,N-dimethylglycine -Ser-Cys (Acm)-Gly | 8-amino-3,6-dioxaoctanoic acid | 2,3-diaminopropionic acid | none | BBN(7-14) |
| L17 | 10-30%B | 7.6 | 1488.6 | 8 | N,N-dimethylglycine -Ser-Cys (Acm)-Gly | none | 8-amino-3,6-dioxaoctanoic acid | none | BBN(7-14) |
| L18 | 10-30%B | 7.03 | 1574.6 | 7.8 | N,N-dimethylglycine -Ser-Cys (Acm)-Gly | 2,3-diaminopropionic acid | 8-amino-3,6-dioxaoctanoic acid | none | BBN(7-14) |
| L19 | 10-35%B | 5.13 | 1603.6 | >50 | N,N-dimethylglycine -Ser-Cys (Acm)-Gly | Asp | 8-amino-3,6-dioxaoctanoic acid | Gly | BBN(7-14) |
| L20 | 10-35%B | 5.19 | 1603.6 | 37 | N,N-dimethylglycine -Ser-Cys (Acm)-Gly | 8-amino-3,6-dioxaoctanoic acid | Asp | Gly | BBN(7-14) |
| L21 | 10-35%B | 5.04 | 1575.7 | 46 | N, Ndimethylglycine -Ser-Cys (Acm)-Gly | 8-amino-3,6-dioxaoctanoic acid | Ser | Gly | BBN(7-14) |
| L22 | 10-35%B | 4.37 | 1644.7 | 36 | N,N-dimethylglycine -Ser-Cys (Acm)-Gly | 8-amino-3,6-dioxaoctanoic acid | Arg | Gly | BBN(7-14) |

(continued)

| Compound | HPLC method [1] | HPLC RT[2] | MS[3] | IC50[5] | M | N | O | P | G* |
|---|---|---|---|---|---|---|---|---|---|
| L23 | 10-35%B | 5.32 | 1633.7 | >50 | N,N-dimethylglycine -Ser-Cys (Acm)-Gly | 8-amino-3,6-dioxaoctanoic acid | 8-amino-3,6-dioxaoctanoic acid | Gly | BBN(7-14) |
| L24 | 10-35%B | 4.18 | 1574.6 | 38 | N,N-dimethylglycine -Ser-Cys (Acm)-Gly | 8-amino-3,6-dioxaoctanoic acid | 2,3-diammopropionic acid | Gly | BBN(7-14) |
| L25 | 10-35%B | 4.24 | 1616.6 | 26 | N,N-dimethylglycine -Ser-Cys (Acm)-Gly | 8-amino-3,6-dioxaoctanoic acid | Lys | Gly | BBN(7-14) |
| L26 | 10-35%B | 4.45 | 1574.6 | 30 | N,N-dimethylglycine -Ser-Cys (Acm)-Gly | 2,3-diaminopropionic acid | 8-amino-3,6-dioxaoctanoic acid | Gly | BBN(7-14) |
| L27 | 10-35%B | 4.38 | 1627.3 | >50 | N,N-dimethylglycine -Ser-Cys (Acm)-Gly | N-4-aminoethyl-N-1-piperazineacetic acid | Asp | none | BBN(7-14) |
| L28 | 10-35%B | 4.1 | 1600.3 | 25 | N,N-dimethylglycine -Ser-Cys (Acm)-Gly | N-4-ammoethyl-N-1-piperazineacetic acid | Ser | none | BBN(7-14) |
| L29 | 10-35%B | 3.71 | 1669.4 | 36 | N,N-dimethylglycine -Ser-Cys (Acm)-Gly | N-4-aminoethyl-N-1-piperazineacetic acid | Arg | none | BBN(7-14) |
| L30 | 10-35%B | 4.57 | 1657.2 | 36 | N,N-dimethylglycine -Ser-Cys (Acm)-Gly | N-4-aminoethyl-N-1-piperazineacetic acid | 8-amino-3,6-dioxaoctanoic acid | none | BBN(7-14) |

(continued)

| Compound | HPLC method [1] | HPLC RT[2] | MS[3] | IC50[5] | M | N | O | P | G* |
|---|---|---|---|---|---|---|---|---|---|
| L31 | 10-35%B | 3.69 | 1598.3 | >50 | N,N-dimethylglycine -Ser-Cys (Acm)-Gly | N-4-aminoethyl-N-1-piperazineacetic acid | 2,3-diaminopropionic acid | none | BBN(7-14) |
| L32 | 10-35%B | 3.51 | 1640.3 | 34 | N,N-dimethylglycine -Ser-Cys (Acm)-Gly | N-4-aminoethyl-N-1-piperazineacetic acid | Lys | none | BBN(7-14) |
| L33 | 10-35%B | 4.29 | 1584.5 | >50 | N,N-dimethylglycine -Ser-Cys (Acm)-Gly | N-1-piperazineacetic acid | Asp | none | BBN(7-14) |
| L34 | 10-35%B | 4.07 | 1578.7 (M+Na) | 38 | N,N-dimethylglycine -Ser-Cys (Acm)-Gly | N-1-piperazineacetic acid | Ser | none | BBN(7-14) |
| L35 | 10-35%B | 3.65 | 1625.6 | 26 | N,N-dimethylglycine -Ser-Cys (Acm)-Gly | N-1-piperazineacetic acid | Arg | none | BBN(7-14) |
| L36 | 10-35%B | 4.43 | 1636.6 | 7 | N,N-dimethylglycine -Ser-Cys (Acm)-Gly | N-1-piperazineacetic acid | 8-amino-3,6-dioxaoctanoic acid | none | BBN(7-14) |
| L37 | 10-35%B | 3.66 | 1555.7 | 23 | N,N-dimethylglycine -Ser-Cys (Acm)-Gly | N-1-piperazineacetic acid | 2,3-diaminopropionic acid | none | BBN(7-14) |
| L38 | 10-35%B | 3.44 | 1619.6 | 7 | N,N-dimethylglycine -Ser-Cys (Acm)-Gly | N-1-piperazineacetic acid | Lys | none | BBN(7-14) |

(continued)

| Compound | HPLC method [1] | HPLC RT[2] | MS[3] | IC50[5] | M | N | O | P | G* |
|---|---|---|---|---|---|---|---|---|---|
| L42 | 305-0%B | 5.65 | 1601.6 | 25 | N,N-dimethylglycine -Ser-Cys (Acm)-Gly | 4-Hydroxyproline | 8-amino-3,6-dioxaoctanoic acid | none | BBN(7-14) |
| L48 | 30-50%B | 4.47 | 1600.5 | 40 | N,N-dimethylglycine -Ser-Cys (Acm)-Gly | 4-aminoproline | 8-amino-3,6-dioxaoctanoic acid | none | BBN(7-14) |
| L51 | 15-35%B | 5.14 | 1673.7 | 49 | N,N-dimethylglycine -Ser-Cys (Acm)-Gly | Lys | 8-amino-3,6-dioxaoctanoic acid | Gly | BBN(7-14) |
| L52 | 15-35%B | 6.08 | 1701.6 | 14 | N,N-dimethylglycine -Ser-Cys (Acm)-Gly | Arg | 8-amino-3,6-dioxaoctanoic acid | Gly | BBN(7-14) |
| L53 | 15-35%B | 4.16 | 1632.6 | 10 | N,N-dimethylglycine -Ser-Cys (Acm)-Gly | Ser | 8-amino-3,6-dioxaoctanoic acid | Gly | BBN(7-14) |
| L54 | 15-35%B | 4.88 | 1661.6 | >50 | N,N-dimethylglycine -Ser-Cys (Acm)-Gly | Asp | 8-amino-3,6-dioxaoctanoic acid | Gly | BBN(7-14) |
| L55 | 15-35%B | 4.83 | 1683.4 (M+Na) | 43 | N,N-dimethylglycine -Ser-Cys (Acm)-Gly | 8-amino-3,6-dioxaoctanoic acid | Asp | Gly | BBN(7-14) |
| L56 | 15-35%B | 4.65 | 1655.7 (M+Na) | 4 | N,N-dimethylglycine -Ser-Cys (Acm)-Gly | 8-amino-3,6-dioxaoctanoic acid | Ser | Gly | BBN(7-14) |

(continued)

| Compound | HPLC method [1] | HPLC RT[2] | MS[3] | IC50[5] | M | N | O | P | G* |
|---|---|---|---|---|---|---|---|---|---|
| L57 | 15-35%B | 4.9 | 1701.8 | 50 | N,N-dimethylglycine -Ser-Cys (Acm)-Gly | 8-amino-3,6-dioxaoctanoic acid | Arg | Gly | BBN(7-14) |
| L58 | 15-35%B | 4.22 | 846.4 (M+H/2) | >50 | N,N-dimethylglycine -Ser-Cys (Acm)-Gly | 8-amino-3,6-dioxaoctanoic acid | 8-amino-3,6-dioxaoctanoic acid | Gly | BBN(7-14) |
| L59 | 15-35%B | 4.03 | 1635.5 | 42 | N,N-dimethylglycine -Ser-Cys (Acm)-Gly | 8-amino-3,6-dioxaoctanoic acid | 2,3-diaminopropionic acid | Gly | BBN(7-14) |
| L60 | 15-35%B | 4.11 | 1696.6 (M+Na) | 20 | N,N-dimethylglycine -Ser-Cys (Acm)-Gly | 8-amino-3,6-dioxaoctanoic acid | Lys | Gly | BBN(7-14) |
| L61 | 15-35%B | 4.32 | 1631.4 | 43 | N,N-dimethylglycine -Ser-Cys (Acm)-Gly | 2,3-diaminopropionic acid | 8-amino-3,6-dioxaoctanoic acid | Gly | BBN(7-14) |
| L78 | 20-40%B | 6.13 | 1691.4 (M+Na) | 35 | D03A-monoamide | 8-amino-3,6-dioxaoctanoic acid | Diaminopropionic acid | none | BBN(7-14) |
| L79 | 20-40%B | 7.72 | 1716.8 (M+Na) | 42 | D03A-monoamide | 8-amino-3,6-dioxaoctanoic acid | Biphenylalamine | none | BBN(7-14) |
| L80 | 20-40%B | 7.78 | 1695.9 | >50 | D03A-monoamide | 8-amino-3,6-dioxaoctanoic acid | Diphenylalanine | none | BBN(7-14) |

(continued)

| Compound | HPLC method [1] | HPLC RT[2] | MS[3] | IC50[5] | M | N | O | P | G* |
|---|---|---|---|---|---|---|---|---|---|
| L81 | 20-40%B | 7.57 | 1513.6 | 37.5 | D03A-monoamide | 8-amino-3,6-dioxaoctanoic acid | 4-Benzoylphenylalanine | none | BBN(7-14) |
| L92 | 15-30%B | 5.63 | 1571.6 | 5 | D03A-monoamide | 5-aminopentanoic acid | 8-amino-3,6-dioxaoctanoic acid | none | BBN(7-14) |
| L94 | 20-36%B | 4.19 | 1640.8 (M+Na) | 6.2 | D03A-monoamide | 8-amino-3,6-dioxaoctanoic acid | D-Phenylalanine | none | BBN(7-14) |
| L110 | 15-45%B | 5.06 | 1612.7 | 36 | D03A-monoamide | 8-aminooctanoic acid | 8-amino-3,6-dioxaoctanoic acid | none | BBN(7-14) |
| L209 | 20-40%B over 6 minutes | 4.62 | 3072.54 | 37 | D03A-monoamide | E(G8-amino-3,6-dioxaoctanoic acid-8-amino-3,6-dioxaoctanoic acid QWAVGHLM-$NH_2$) | 8-aminooctanoic acid | 8-aminoocta noic acid | BBN(7-14) |
| L210 | 20-50%B over 10 minutes | 6.18 | 3056.76 | 11 | D03A-monoamide | E(G-Aoa-Aoa-QWAVGHLM-$NH_2$) | 8-aminooctanoic acid | 8-aminoocta noic acid | BBN(7-14) |

*BBN(7-14) is [SEQ ID NO:1]
[1] HPLC method refers to the 10 minute time for the HPLC gradient.
[2] HPLC RT refers to the retention time of the compound in the HPLC.
[3] MS refers to mass spectra where molecular weight is calculated from mass/unit charge (m/e).
[4] $IC_{50}$ refers to the concentration of compound to inhibit 50% binding of iodinated bombesin to a GRP receptor on cells.

2B. Linkers Containing At Least One Substituted Bile Acid

In another embodiment of the present invention, the linker N-O-P contains at least one substituted bile acid. Thus, in this embodiment of the linker N-O-P,
N is 0 (where 0 means it is absent), an alpha amino acid, a substituted bile acid or other linking group;
O is an alpha amino acid or a substituted bile acid; and
P is 0, an alpha amino acid, a substituted bile acid or other linking group, wherein at least one ofN, O or P is a substituted acid.

Bile acids are found in bile (a secretion of the liver) and are steroids having a hydroxyl group and a five carbon atom side chain terminating in a carboxyl group. In substituted bile acids, at least one atom such as a hydrogen atom of the bile acid is substituted with another atom, molecule or chemical group. For example, substituted bile acids include those having a 3-amino, 24-carboxyl function optionally substituted at positions 7 and 12 with hydrogen, hydroxyl or keto functionality.

Other useful substituted bile acids in the present invention include substituted cholic acids and derivatives thereof. Specific substituted cholic acid derivatives include:

(3β,5β)-3-aminocholan-24-oic acid;

(3β,5β,12α)-3-amino-12-hydroxycholan-24-oic acid;

(3β,5β,7α,12α)-3-amino-7,12-dihydroxycholan-24-oic acid;

Lys-(3,6,9)-trioxaundecane-1,11-dicarbonyl-3,7-dideoxy-3-aminocholic acid);

(3β,5β,7α)-3-amino-7-hydroxy-12-oxocholan-24-oic acid; and

(3β,5β,7α)-3-amino-7-hydroxycholan-24-oic acid.

Examples of compounds having the formula M-N-O-P-G which contain linkers with at least one substituted bile acid are listed in Table 2. These compounds may be prepared using the methods disclosed herein, particularly in the Examples, as well as by similar methods known to one skilled in the art.

**TABLE 2**

**Compounds Containing Linkers With At Least One Substituted Bile Acid**

| Compound | HPLC method[1] | HPLC RT[2] | MS[3] | IC50[5] | M | N | O | P | G* |
|---|---|---|---|---|---|---|---|---|---|
| L62 | 20-80%B | 3.79 | 1741.2 | >50 | D03A-monoamide | Gly | (3β,5β-3-aminocholan-24-oic acid | none | BBN(7-14) |
| L63 | 20-80%B | 3.47 | 1757.0 | 23 | D03A-monoamide | Gly | (3β,5β,12α)-3-amino-12-hydroxycholan-24-oic acid | none | BBN(7-14) |
| L64 | 20-50%B | 5.31 | 1773.7 | 8.5 | D03A-monoamide | Gly | (3β,5β,7α,12α)-3-amino-7,12-dihydroxycholan-24-oic acid | none | BBN(7-14) |
| L65 | 20-80%B | 3.57 | 2246.2 | >50 | DO3 A-monoamide | Gly | Lys-(3,6,9-tnoxaundecane-1,11-dicarbonyl-3,7-dideoxy-3-aminocholic acid) | Arg | BBN(7-14) |
| L66 | 20-80% | 3.79 | 2245.8 | >50 | (3β,5β,7α,12α)-3-amino-7,12-dihydroxycholan-24-oic acid-3,6,9-trioxaundecane-1,11-dicarbonyl | | Lys(DO3 A-monoamide-Gly) | Arg | BBN(7-14) |
| L67 | 20-80% | 3.25 | 1756.9 | 4.5 | DO3 A-monoamide | Gly | (3β,5β,7α,12α)-3-amino-12-oxacholan-24-oic acid | none | BBN(7-14) |
| L69 | 20-80% | 3.25 | 1861.27 | 8 | DO3 A-monoamide | 1-amino-3,6-dioxaoctanoic acid | (3β,5β,7α,12α)-3-amino-7,12-dihydroxycholan-24-oic acid | none | BBN(7-14) |

*BBN(7-14) is [SEQ ID NO:1]
[1] HPLC method refers to the 10 minute time for the HPLC gradient.
[2] HPLC RT refers to the retention time of the compound in the HPLC.
[3] MS refers to mass spectra where molecular weight is calculated from mass/unit charge (m/e).
[4] $IC_{50}$ refers to the concentration of compound to inhibit 50% binding of iodinated bombesin to a GRP receptor on cells.

2C. Linkers Containing At Least One Non-Alpha Amino Acid With A Cyclic Group

In yet another embodiment of the present invention, the linker N-O-P contains at least one non-alpha amino acid with a cyclic group. Thus, in this embodiment of the linker N-O-P,
N is 0 (where 0 means it is absent), an alpha amino acid, a non-alpha amino acid with a cyclic group or other linking group;
O is an alpha amino acid or a non-alpha amino acid with a cyclic group; and
P is 0, an alpha amino acid, a non-alpha amino acid with a cyclic group, or other linking group,
wherein at least one of N, O or P is a non-alpha amino acid with a cyclic group.

Non-alpha amino acids with a cyclic group include substituted phenyl, biphenyl, cyclohexyl or other amine and carboxyl containing cyclic aliphatic or heterocyclic moieties.
Examples of such include:

4-aminobenzoic acid
3-aminobenzoic acid
4-aminomethyl benzoic acid
8-aminooctanoic acid trans-4-aminomethylcyclohexane carboxylic acid 4-(2-aminoethoxy)benzoic acid
isonipecotic acid
2-aminomethylbenzoic acid
4-amino-3-nitrobenzoic acid
4-(3-carboxymethyl-2-keto-1-benzimidazolyl-piperidine
6-(piperazin-1-yl)-4-(3H)-quinazolinone-3-acetic acid (2S,5S)-5-amino-1,2,4,5,6,7-hexahydro-azepino[3,21-hi]indole-4-one-2-carboxylic acid
(4S,7R)-4-amino-6-aza-5-oxo-9-thiabicyclo[4.3.0]nonane-7-carboxylic acid
3-carboxymethyl-1-phenyl-1,3,8-triazaspiro[4.5]decan-4-one N1-piperazineacetic acid
N-4-aminoethyl-N-1-piperazineacetic acid
(3 S)-3-amino-1-carboxymethylcaprolactam
(2S,6S,9)-6-amino-2-carboxymethyl-3,8-diazabicyclo-[4,3,0]-nonane-1,4-dione
3-amino-3-deoxycholic acid
4-hydroxybenzoic acid
4-aminophenylacetic acid
3-hydroxy-4-aminobenzoic acid
3-methyl-4-aminobenzoic acid
3-chloro-4-aminobenzoic acid
3-methoxy-4-aminobenzoic acid
6-aminonaphthoic acid
N,N'-Bis(2-aminoethyl)-succinamic acid

Examples of compounds having the formula M-N-O-P-G which contain linkers with at least one alpha amino acid with a cyclic group are listed in Table 3. These compounds may be prepared using the methods disclosed herein, particularly in the Examples, as well as by similar methods known to one skilled in the art.

**TABLE 3**

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50 5** | **M** | **N** | **O** | **P** | **G*** |
| L70 | 10-40%B | 6.15 | 1502.6 | 5 | D03A-monoamide | Gly | 4-aminobenzoic acid | | BBN(7-14) |
| L71 | | | 1482.2 (M+Na) | 7 | D03A-monoamide | none | 4-aminomethyl benzoic acid | none | BBN(7-14) |
| L72 | | | 1504.0 (M+K) | 8 | D03A-monoamide | none | trans-4-aminomethylcyclohexyl carboxylic acid | none | BBN(7-14) |
| L73 | 5-35% | 7.01 | 1489.8 | 5 | D03A-monoamide | none | 4-(2-aminoethoxy)benzoic acid | none | BBN(7-14) |
| L74 | 5-35% | 6.49 | 1494.8 | 7 | D03A-monoamide | Gly | isonipecotic acid | none | BBN(7-14) |
| L75 | 5-35% | 6.96 | 1458.0 | 23 | D03A-monoamide | none | 2-aminomethylbenzoic acid | none | BBN(7-14) |
| L76 | 5-35% | 7.20] | 1502.7 | 4 | D03A-monoamide | none | 4-aminomethyl-3 -nitrobenzoic acid | none | BBN(7-14) |
| L77 | 20-40%B | 6.17 | 1691.8 (M+Na) | 17.5 | D03A-monoamide | 8-amino-3,6-dioxaoctanoic acid | 1-Naphthylalanine | none | BBN(7-14) |
| L82 | 20-40%B | 6.18 | 1584.6 | 8 | D03A-monoamide | none | 4-(3-carboxymethyl-2-keto-1-benzimidazolyl-piperidine | none | BBN(7-14) |
| L83 | 20-40%B | 5.66 | 1597.5 | >50 | D03A-monoamide | none | 6-(piperazin-1-yl)-4-(3H)-quinazolinone-3-acetic acid | none | BBN(7-14) |
| L84 | 20-40%B | 6.31 | 1555.5 | >50 | D03A-monoamide | none | (2S,5S)-5-amino-1,2,4,5,6,7-hexahydro-azepino[3,21-hi] indole-4-one-2-carboxyhc acid | none | BBN(7-14) |
| L85 | 20-40%B | 5.92 | 1525.5 | >50 | D03A-monoamide | none | (4S,7R)-4-amino-6-aza-5-oxo-9-thiabicyclo[4.3.0]nonane-7-carboxylic acid | none | BBN(7-14) |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| Compound | HPLC method[1] | HPLC RT[2] | MS[3] | IC50 5 | M | N | O | P | G* |
| L86 | 20-40%B | 6.46 | 1598.6 | >50 | D03A-monoamide | none | N,N-dimethylglycine | none | BBN(7-14) |
| L87 | 20- | 5.47 | 1593.8 | >50 | D03A- | none | 3-carboxymethyl-1- | none | BBN(7-14) |
| | 40%B | | (M+Na) | | monoamide | | phenyl-1,3,8-triazaspiro[4.5] decan -4-one | | |
| L88 | 20-40%B | 3.84 | 1452.7 | >50 | D03A-monoamide | none | N1-piperazineacetic acid | none | BBN(7-14) |
| L89 | 20-40%B | 5.68 | 1518.5 (M+Na) | 23 | D03A-monoamide | none | N-4-aminoethyl-N-1-piperazine-acetic acid | none | BBN(7-14) |
| L90 | 20-40%B | 7.95 | 1495.4 | 50 | D03A-monoamide | none | (3S)-3-amino-1-carboxymethylcaprol actam | none | BBN(7-14) |
| L91 | 20-40%B | 3.97 | 1535.7 | >50 | D03A-monoamide | none | (2S,6S,9)-6-amino-2-carboxymethyl-3,8-diazabicyclo-[4,3,0]-nonane-1,4-dione | none | BBN(7-14) |
| L93 | 15-30%B | 7.57 | 1564.7 | 5.8 | D03A-monoamide | 5-aminopentanoic acid | trans-4-aminomethylcyclohe xane-1-carboxylic acid | none | BBN(7-14) |
| L95 | 15-35%B | 5.41 | 1604.6 | 14 | D03A-monoamide | trans-4-aminomethylcycloh exane-1-carboxylic acid | D-Phenylalanine | none | BBN(7-14) |
| L96 | 20-36%B | 4.75 | 1612.7 | 35 | D03A-monoamide | 4-aminomethylbenzoi c acid | 8-amino-3,6-dioxaoctanoic acid | none | BBN(7-14) |
| L97 | 15-35%B | 5.86 | 1598.8 | 4.5 | D03A-monoamide | 4-benzoyl-(L)-phenylalanine | trans-4-aminomethylcyclohe xane-1-carboxylic acid | none | BBN(7-14) |
| L98 | 15-35%B | 4.26 | 1622.7 | 16 | D03A-monoamide | trans-4-aminomethylcycloh exane-1-carboxyhc acid | Arg | none | BBN(7-14) |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50 5** | **M** | **N** | **O** | **P** | **G*** |
| L99 | 15-35%B | 4.1 | 1594.7 | 22 | D03A-monoamide | trans-4-aminomethylcycloh exane-1-carboxylic acid | Lys | none | BBN(7-14) |
| L100 | 15-35%B | 4.18 | 1613.6 | 10 | D03A-monoamide | trans-4-aminomethylcycloh exane-1-carboxylic acid | Diphenylalanine | none | BBN(7-14) |
| L101 | 15-35%B | 5.25 | 1536.7 | 25 | D03A-monoamide | trans-4-aminomethylcycloh exane-1-carboxylic acid | 1-Naphthylalanine | none | BBN(7-14) |
| L102 | 15-35%B | 5.28 | 1610.8 | 9.5 | D03A-monoamide | trans-4-aminomethylcycloh exane-1-carboxyhc acid | 8-amino-3,6-dioxaoctanoic acid | none | BBN(7-14) |
| L103 | 15-35%B | 4.75 | 1552.7 | 24 | D03A-monoamide | trans-4-aminomethylcycloh exane-1-carboxylic acid | Ser | none | BBN(7-14) |
| L104 | 15-35%B | 3.91 | 1551.7 | 32 | D03A-monoamide | trans-4-aminomethylcycloh exane-1-carboxyhc acid | 2,3-diaminopropiobic acid | none | BBN(7-14) |
| L105 | 20-45%B | 7.68 | 1689.7 | 3.5 | D03A-monoamide | trans-4-aminomethylcycloh exane-1-carboxylic acid | Biphenylalanine | none | BBN(7-14) |
| L106 | 20-45%B | 6.97 | 1662.7 | 3.8 | D03A-monoamide | trans-4-aminomethylcycloh exane-1-carboxylic acid | (2S,5S)-5-amino-1,2,4,5,6,7-hexahydro-azepino[3,21-hi] indole-4-one-2-carboxylic acid | none | BBN(7-14) |
| L107 | 15-35%B | 5.79 | 1604.7 | 5 | D03A-monoamide | trans-4-aminomethylcycloh exane-1-carboxyhc acid | trans-4-aminomethylcyclohe xane-1-carboxylic acid | none | BBN(7-14) |
| L108 | 15-45%B | 6.38 | 1618.7 | 10 | D03A-monoamide | 8-amino-3,6-dioxaoctanoic acid | Phenylalanine | none | BBN(7-14) |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| Compound | HPLC method[1] | HPLC RT[2] | MS[3] | IC50 5 | M | N | O | P | G* |
| L109 | 15-45%B | 6.85 | 1612.7 | 6 | D03A-monoamide | trans-4-aminomethylcyclohexane-1-carboxyhc acid | Phenylalanine | none | BBN(7-14) |
| L111 | 20-45%B | 3.75 | 1628.6 | 8 | D03A-monoamide | 8-aminooctanoic acid | trans-4-aminomethylcyclohexane-1-carboxylic acid | none | BBN(7-14) |
| L112 | 20-47%B in 9 min | 3.6 | 1536.5 | 4.5 | D03A-monoamide | none | 4'-aminomethyl-biphenyl-1-carboxylic acid | none | BBN(7-14) |
| L113 | 20-47%B in 9 min | 3.88 | 1558.6 (M+N a) | 5 | D03A-monoamide | none | 3'-aminomethyl-biphenyl-3-carboxylic acid | none | BBN(7-14) |
| L114 | 10-40%B | 5.47 | 1582.8 | 4.5 | CMDOTA | Gly | 4-aminobenzoic acid | none | BBN(7-14) |
| L124 | 5-35%B | 7.04 | 1489.9 | 8.0 | D03A-monoamide | none | 4-aminomethylphenoxyacetic acid | none | BBN(7-14) |
| L143 | 5-35%B | 6.85 | 1516.8 | 11 | D03A-monoamide | Gly | 4-aminophenylacetic acid | none | BBN(7-14) |
| L144 | 5-35%B | 6.85 | 1462.7 | 9 | HPD03A | none | 4-phenoxy | none | BBN(7-14) |
| L145 | 20-80%B | 1.58 | 1459.8 | 5 | D03A-monoamide | none | 3-aminomethylbenzoic acid | none | BBN(7-14) |
| L146 | 20-80%B | 1.53 | 1473.7 | 9 | D03A-monoamide | none | 4-aminomethylphenyla celtic acid | none | BBN(7-14) |
| L147 | 20-80%B | 1.68 | 1489.7 | 3.5 | D03A-monoamide | none | 4-aminomethyl-3 -methoxybenzoic acid | none | BBN(7-14) |
| L201 | 10-46%B over 12 minute s | 5.77 | 1563.7 | 36 | Boa*** | none | Gly | 4-aminobenzoic acid | BBN(7-14) |
| L202 | 10-46%B over 12 minute s | 5.68 | 1517.74 | 13 | D03A-monoamide | none | Gly | 4-hydrazinobenzoyl | BBN(7-14) |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50 5** | **M** | **N** | **O** | **P** | **G*** |
| L203 | 1046%B over 12 minute s | 5.98 | 1444.69 | 9 | D03A-monoamide | none | none | 4-aminobenzoic acid | BBN(7-14) |
| L204 | 10-46%B over 12 minute s | 5.82 | 1502.73 | 50 | D03A-monoamide | none | 4-aminobenzoic acid | Gly | BBN(7-14) |
| L205 | 10-46%B over 12 minute s | 5.36 | 1503.72 | 45 | D03A-monoamide | Gly | 6-Aminonicotinic acid | none | BBN(7-14) |
| L206 | 10-46%B over 12 minute s | 7.08 | 1592.85 | 4.5 | D03A-monoamide | Gly | 4'-Amino-2'-methyl biphenyl-4-carboxylic acid | none | BBN(7-14) |
| L207 | 10-46%B over 12 minute s | 7.59 | 1578.83 | 2.5 | D03A-monoamide | Gly | 3'-Arrunobiphenyl-3-carboxylic acid | none | BBN(7-14) |
| L208 | 10-46%B over 12 minute s | 5.9 | 1516.75 | 7.5 | D03A-monoamide | Gly | 1,2-diaminoethyl | Terephthalic acid | BBN(7-14) |
| L211 | 10-46%B over 12 minute s | 5.76 | 1560.77 | 4 | D03A-monoamide | Gly | Gly | 4-aminobenzoic acid | BBN(7-14) |
| L212 | 10-46%B over 12 minute s | 6.05 | 1503.71 | NT* * | D03A-monoamide | none | Gly | 4-aminobenzoic acid | EWAVGHLM-$NH_2$ |
| L213 | 10-46%B over 12 minute s | 5.93 | 1503.71 | NT* * | D03A-monoamide | Gly | 4-aminobenzoic acid | none | QWAVGHLM-OH |
| L214 | 10-46%B over 12 minute s | 7.36 | 1649.91 | NT* * | D03A-monoamide | Gly | 4-aminobenzoic acid | (D)-Phe | BBN(7-14) |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50 5** | **M** | **N** | **O** | **P** | **G*** |
| L215 | 10-46%B over 12 minute s | 5.08 | 2071.37 | NT* * | D03A-monoamide | Gly | 4-aminobenzoic acid | none | QRLGNQWAVG HLM-$NH_2$ |
| L216 | 10-46%B over 12 minute s | 4.94 | 2121.38 | NT* * | D03A-monoamide | Gly | 4-aminobenzoic acid | none | QRYGNQWAVG HLM-$NH_2$ |
| L217 | 10-46%B over 12 minute s | 4.38 | 2093.37 | NT* * | D03A-monoamide | Gly | 4-aminobenzoic acid | none | QKYGNQWAVG HLM-NH2 |
| L218 | 10-46%B over 12 minute s | 6.13 | 2154.45 | NT* * | D03A-monoamide | Gly | 4-aminobenzoic acid | none | See scheme for structure |
| L219 | 10-46%B over 12 minute s | 8.61 | 1588.84 | NT* * | D03A-monoamide | Gly | 4-aminobenzoic acid | (D)-Phe | QWAVGHL-NH-Pentyl |
| L220 | 10-46%B over 12 minute s | 5.96 | 1516.75 | NT* * | D03A-monoamide | Gly | 4-aminobenzoic acid | none | QWSVaHLM-$NH_2$ |
| L221 | 10-46%B over 12 minute s | 7.96 | 1631.87 | NT* * | D03A-monoamide | Gly | 4-aminobenzoic acid | (D)-Phe | QWAVGHLL-$NH_2$ |
| L222 | 10-46%B over 12 minute s | 6.61 | 1695.91 | NT* * | D03A-monoamide | Gly | 4-aminobenzoic acid | (D)-Tyr | QWAV-Bala-HF-Nle-$NH_2$ |
| L223 | 10-46%B over 12 minute s | 7.48 | 1679.91 | NT* * | D03A-monoamide | Gly | 4-aminobenzoic acid | Phe | QWAV-Bala-HF-Nle-$NH_2$ |
| L224 | 10-46%B over 12 minute s | 5.40 | 1419.57 | NT* * | D03A-monoamide | Gly | 4-aminobenzoic acid | none | QWAGHFL-$NH_2$ |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50 5** | **M** | **N** | **O** | **P** | **G*** |
| L225 | 10-46%B over 12 minute s | 8.27 | 1471.71 | NT* * | D03A-monoamide | Gly | 4-aminobenzoic acid | none | LWAVGSFM-$NH_2$ |
| L226 | 10-46%B over 12 minute s | 5.12 | 1523.75 | NT* * | D03A-monoamide | Gly | 4-aminobenzoic acid | none | HWAVGHLM-$NH_2$ |
| L227 | 10-46%B over 12 minute s | 6.61 | 1523.75 | NT* * | D03A-monoamide | Gly | 4-aminobenzoic acid | none | LWAVGSFM-$NH_2$ |
| L228 | 10-46%B over 12 minute s | 5.77 | 1511 | NT* * | D03A-monoamide | Gly | 4-aminobenzoic acid | none | QWAVGHFM-$NH_2$ |
| L233 | 5-35%B over 10 min | 7.04 | 1502.71 | 4.8 | D03A-monoamide | Gly | 3-aminobenzoic acid | none | BBN(7-14) |
| L234 | 20-80% over 10 minute s | 1.95 | 1552.76 | 3 | D03A-monoamide | Gly | 6-aminonaphthoic acid | none | BBN(7-14) |
| L235 | 20-80% over 10 minute s | 1.95 | 1515.72 | 7 | D03A-monoamide | Gly | 4-methylaminobenzoic acid | none | BBN(7-14) |
| L237 | 20-80% over 10 minute s | 1.52 | 1538.68 | 5 | Cm4pm10d2 a | Gly | 4-aminobenzoic acid | none | BBN(7-14) |
| L238 | 5-35%B over 10 min | 7.17 | 1462.70 | 1.5 | N,N-dimethylglycine-Ser-Cys (Acm)-Gly | Gly | 4-aminobenzoic acid | none | BBN(7-14) |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| Compound | HPLC method[1] | HPLC RT[2] | MS[3] | IC50 5 | M | N | O | P | G* |
| L239 | 20-80% over 10 minute s | 3.36 | 1733.16 | 4.5 | N,N-dimethylglycme-Ser-Cys (Acm)-Gly | Gly | 3-amino-3-deoxycholic acid | none | BBN(7-14) |
| L240 | 20-80% over 10 minute s | 1.55 | 1532.73 | 4 | D03A-monoamide | Gly | 3-methoxy-4-aminobenzoic acid | none | BBN(7-14) |
| L241 | 20-80% over 10 minute s | 1.63 | 1535.68 | 4 | D03A-monoamide | Gly | 3-chloro-4-aminobenzoic acid | none | BBN(7-14) |
| L242 | 20-80% over 10 minute s | 1.55 | 1516.75 | 5 | DO3A-monoamide | Gly | 3-methyl-4-aminobenzoic acid | none | BBN(7-14) |
| L243 | 20-80% over 10 minute s | 1.57 | 1518.70 | 14 | DO3A-monoamide | Gly | 3-hydroxy-4-aminobenzoic acid | none | BBN(7-14) |
| L244 | 5-50% over 10 minute s | 4.61 | 1898.16 | >50 | (DO3 A-monoamide)$_2$ | N,N'-Bis(2-aminoethyl)-succinamic acid | none | none | BBN(7-14) |
| L280 | | | | | DO3A-monoamide | Gly | Adca3 | | Q-W-A-V-a-H-L-M-NH2 |
| L281 | | | | | DO3A-monoamide | Gly | Adca3 | f | Q-W-A-V-G-H-L-M-NH2 |
| L282 | | | | | DO3A-monoamide | Gly | Adca3 | f | Q-W-A-V-G-H-L-L-NH2 |
| L283 | | | | | DO3A-monoamide | Gly | Adca3 | f | Q-W-A-V-G-H-L-NH-pentyl |
| L284 | | | | | DO3A-monoamide | Gly | Adca3 | y | Q-W-A-V-Bala-H-F-Nle-NH2 |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50 5** | **M** | **N** | **O** | **P** | **G*** |
| L285 | | | | | DO3A-monoamide | Gly | Adca3 | f | Q-W-A-V-Bala-H-F-Nle-NH2 |
| L286 | | | | | DO3A-monoamide | Gly | Adca3 | | Q-W-A-V-G-H-F-L-NH2 |
| L287 | | | | | DO3A-monoamide | Gly | Adca3 | | Q-W-A-V-G-NMeHis-L-M-NH2 |
| L288 | | | | | DO3A-monoamide | Gly | Adca3 | | L-W-A-V-G-S-F-M-NH2 |
| L289 | | | | | DO3A-monoamide | Gly | Adca3 | | H-W-A-V-G-H-L-M-NH2 |
| L290 | | | | | DO3A-monoamide | Gly | Adca3 | | L-W-A-T-G-H-F-M-NH2 |
| L291 | | | | | DO3A-monoamide | Gly | Adca3 | | Q-W-A-V-G-H-F-M-NH2 |
| L292 | | | | | DO3A-monoamide | Gly | Adca3 | Q-R-L-G-N | Q-W-A-V-G-H-L-M-NH2 |
| L293 | | | | | DO3A-monoamide | Gly | Adca3 | Q-R-Y-G-N | Q-W-A-V-G-H-L-M-NH2 |
| L294 | | | | | DO3A-monoamide | Gly | Adca3 | Q-K-Y-G-N | Q-W-A-V-G-H-L-M-NH2 |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50 5** | **M** | **N** | **O** | **P** | **G*** |
| L295 | | | | | Pglu-Q-Lys (DO3A-monoamide) | Gly | Adca3 | L-G-N | Q-W-A-V-G-H-L-M-NH2 |

*BBN(7-14) is [SEQ ID NO:1]
**NT is defined as "not tested."
***BOA is defined as (1R)-1-(Bis{2-[bis(carboxymethyl)amino]ethyl}amino]propane-1,3-dicarboxylic acid.
[1] HPLC method refers to the 10 minute time for the HPLC gradient.
[2] HPLC RT refers to the retention time of the compound in the HPLC.
[3] MS refers to mass spectra where molecular weight is calculated from mass/unit charge (m/e).
[4] $IC_{50}$ refers to the concentration of compound to inhibit 50% binding of iodinated bombesin to a GRP receptor on cells.

A subset of compounds containing preferred linkers related to amino-(phenyl, biphenyl, cycloalkyl or heterocyclic) carboxylates and various GRP receptor targeting peptides are set forth in Table 4. These compounds may be prepared using the methods disclosed herein, particularly in the Examples, as well as by similar methods known to one skilled in the art.

**TABLE 4**

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50[5]** | **M** | **N** | **O** | **P** | **G*** |
| L214 | 10-46%B over 12 minutes | 7.36 | 1649.91 | NT** | DO3A-monoamide | Gly | 4-aminobenzoic acid | (D)Phe | BBN(7-14) |
| L215 | 10-46%B over 12 minutes | 5.08 | 2071.37 | NT** | DO3A-monoamide | Gly | 4-aminobenzoic acid | none | QRLGNQWAVGHLM-$NH_2$ |
| L216 | 10-46%B over 12 minutes | 4.94 | 2121.38 | NT** | DO3A-monoamide | Gly | 4-aminobenzoic acid | none | QRYGNQWAVGHLM-$NH_2$ |
| L217 | 10-46%B over 12 minutes | 4.38 | 2093.37 | NT** | DO3A-monoamide | Gly | 4-aminobenzoic acid | none | QKYGNQWAVGHLM-NH2 |
| L218 | 10-46%B over 12 minutes | 6.13 | 2154.45 | NT** | DO3A-monoamide | Gly | 4-aminobenzoic acid | none | See FIG 38 |
| L219 | 10-46%B over 12 minutes | 8.61 | 1588.84 | NT** | DO3A-monoamide | Gly | 4-aminobenzoic acid | (D)-Phe | QWAVGHL-NH-Pentyl |
| L220 | 10-46%B over 12 minutes | 5.96 | 1516.75 | NT** | DO3A-monoamide | Gly | 4-aminobenzoic acid | none | QWAVaHLM-$NH_2$ |
| L221 | 10-46%B over 12 minutes | 7.96 | 1631.87 | NT** | DO3A-monoamide | Gly | 4-aminobenzoic acid | (D)-Phe | QWAVGHLL-$NH_2$ |
| L222 | 10-46%B over 12 minutes | 6.61 | 1695.91 | NT** | DO3A-monoamide | Gly | 4-aminobenzoic acid | (D)-Tyr | QWAV-Bala-HF-Nle-$NH_2$ |
| L223 | 10-46%B over 12 minutes | 7.48 | 1679.91 | NT** | DO3A-monoamide | Gly | 4-aminobenzoic acid | Phe | QWAV-Bala-HF-Nle-$NH_2$ |
| L224 | 10-46%B over 12 minutes | 5.40 | 1419.57 | NT** | DO3A-monoamide | Gly | 4-aminobenzoic acid | none | QWAGHFL-$NH_2$ |
| L225 | 10-46%B | 8.27 | 1471.71 | NT** | DO3A- | Gly | 4- | none | LWAVGSFM-$NH_2$ |
| | over 12 minutes | | | | monoamide | | aminobenzoic acid | | |
| L226 | 10-46%B over 12 minutes | 5.12 | 1523.75 | NT** | DO3A-monoamide | Gly | 4-aminobenzoic acid | none | HWAVGHLM-$NH_2$ |
| L227 | 10-46%B over 12 minutes | 6.61 | 1523.75 | NT** | DO3A-monoamide | Gly | 4-aminobenzoic acid | none | LWATGHFM-$NH_2$ |

(continued)

| Compounds Containing Linkers Related To Amino-(Phenyl, Biphenyl, Cycloalkyl Or Heterocyclic) Carboxylates | | | | | | | | | |
|---|---|---|---|---|---|---|---|---|---|
| **Compound** | **HPLC method[1]** | **HPLC RT[2]** | **MS[3]** | **IC50[5]** | **M** | **N** | **O** | **P** | **G*** |
| L228 | 10-46%B over 12 minutes | 5.77 | 1511 | NT** | DO3A-monoamide | Gly | 4-aminobenzoic acid | none | QWAVGHFM-$NH_2$ |

2D. Other Linking Groups

Other linking groups which may be used within the linker N-O-P include a chemical group that serves to couple the GRP receptor targeting peptide to the metal chelator or optical label while not adversely affecting either the targeting function of the GRP receptor targeting peptide or the metal complexing function of the metal chelator or the detectability of the optical label. Suitable other linking groups include peptides (i.e., amino acids linked together) alone, a non-peptide group (e.g., hydrocarbon chain) or a combination of an amino acid sequence and a non-peptide spacer.

In one embodiment, other linking groups for use within the linker N-O-P include L-glutamine and hydrocarbon chains, or a combination thereof.

In another embodiment, other linking groups for use within the linker N-O-P include a pure peptide linking group consisting of a series of amino acids (*e.g.*, diglycine, triglycine, gly-gly-glu, gly-ser-gly, etc.), in which the total number of atoms between the N-terminal residue of the GRP receptor targeting peptide and the metal chelator or the optical label in the polymeric chain is ≤ 12 atoms.

In yet a further embodiment, other linking groups for use within the linker N-O-P can also include a hydrocarbon chain [*i.e.*, $R_1$-$(CH_2)_n$-$R_2$] wherein n is 0-10, preferably n = 3 to 9, $R_1$ is a group (*e.g.*, $H_2N$-, HS-, -COOH) that can be used as a site for covalently linking the ligand backbone or the preformed metal chelator or metal complexing backbone or optical label; and $R_2$ is a group that is used for covalent coupling to the N-terminal $NH_2$-group of the GRP receptor targeting peptide (*e.g.*, $R_2$ is an activated COOH group). Several chemical methods for conjugating ligands (*i.e.*, chelators) or preferred metal chelates to biomolecules have been well described in the literature [Wilbur, 1992; Parker, 1990; Hermanson, 1996; Frizberg et al., 1995]. One or more of these methods could be used to link either the uncomplexed ligand (chelator) or the radiometal chelate or optical label to the linker or to link the linker to the GRP receptor targeting peptides. These methods include the formation of acid anhydrides, aldehydes, arylisothiocyanates, activated esters, or N-hydroxysuccinimides [Wilbur, 1992; Parker, 1990; Hermanson, 1996; Frizberg et al., 1995].

In a preferred embodiment, other linking groups for use within the linker N-O-P may be formed from linker precursors having electrophiles or nucleophiles as set forth below:

LP1: a linker precursor having on at least two locations of the linker the same electrophile E1 or the same nucleophile Nu1;
LP2: a linker precursor having an electrophile E1 and on another location of the linker a different electrophile E2;
LP3: a linker precursor having a nucleophile Nu1 and on another location of the linker a different nucleophile Nu2; or
LP4: a linker precursor having one end functionalized with an electrophile E1 and the other with a nucleophile Nu1.

The preferred nucleophiles Nu1/Nu2 include-OH, -NH, -NR, -SH, -HN-$NH_2$, -RN-$NH_2$, and -RN-NHR', in which R' and R are independently selected from the definitions for R given above, but for R' is not H.

The preferred electrophiles E1/E2 include -COOH, -CH=O (aldehyde), -CR=OR' (ketone), -RN-C=S, -RN-C=O,-S-S-2-pyridyl, -$SO_2$-Y -$CH_2C$(=O)Y, and

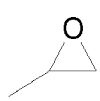 , 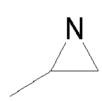 , 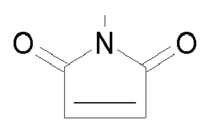

wherein Y can be selected from the following groups:

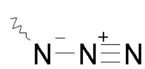

Cl, Br, F

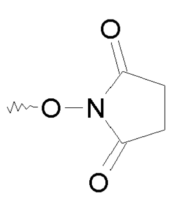 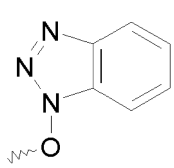 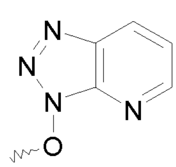 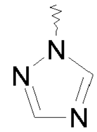 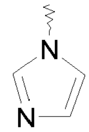 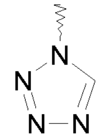

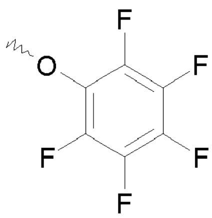
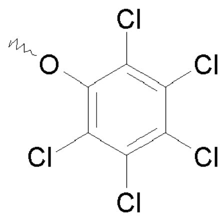
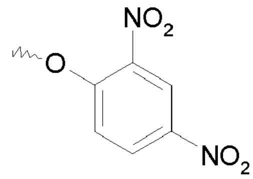
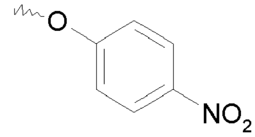

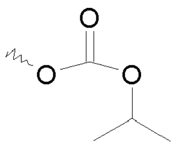
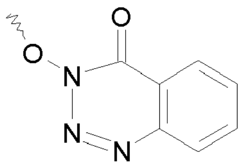
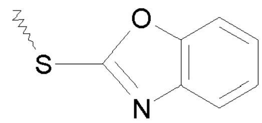
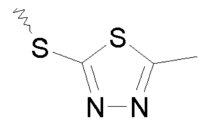

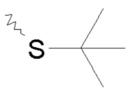
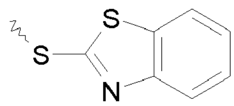
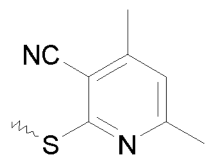
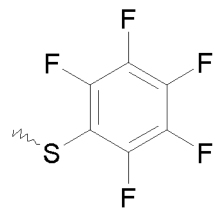

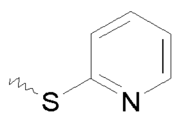

3. <u>GRP receptor targeting peptide</u>

The GRP receptor targeting peptide (*i.e.,* G in the formula M-N-O-P-G) is any peptide, equivalent, derivative or analogue thereof which has a binding affinity for the GRP receptor family.

The GRP receptor targeting peptide may take the form of an agonist or an antagonist. A GRP receptor targeting peptide agonist is known to "activate" the cell following binding with high affinity and may be internalized by the cell. Conversely, GRP receptor targeting peptide antagonists are known to bind only to the GRP receptor on the cell without being internalized by the cell and without "activating" the cell. In a preferred embodiment, the GRP receptor targeting peptide is an agonist.

In a more preferred embodiment of the present invention, the GRP agonist is a bombesin (BBN) analogue and/or a derivative thereof. The BBN derivative or analog thereof preferably contains either the same primary structure of the BBN binding region (*i.e.,* BBN(7-14) [SEQ ID NO:1]) or similar primary structures, with specific amino acid substitutions that will specifically bind to GRP receptors with better or similar binding affinities as BBN alone (*i.e.,* Kd<25nM). Suitable compounds include peptides, peptidomimetics and analogues and derivatives thereof. The presence of L-methionine (Met) at position BBN-14 will generally confer agonistic properties while the absence of this residue at BBN-14 generally confers antagonistic properties [Hoffken, 1994]. Some useful bombesin analogues are disclosed in U.S. Patent Pub. 2003/0224998, incorporated herein in its entirety.

It is well documented in the art that there are a few and selective number of specific amino acid substitutions in the BBN (8-14) binding region (e.g., D-Ala$^{11}$ for L-Gly$^{11}$ or D-Trp$^{8}$ for L-Trp$^{8}$), which can be made without decreasing binding affinity [Leban et al., 1994; Qin et al., 1994; Jensen et al., 1993]. In addition, attachment of some amino acid chains or other groups to the N-terminal amine group at position BBN-8 (i.e., the Trp$^{8}$ residue) can dramatically decrease the binding affinity of BBN analogues to GRP receptors [Davis et al., 1992; Hoffken, 1994; Moody et al., 1996; Coy, et al., 1988; Cai et al., 1994]. In a few cases, it is possible to append additional amino acids or chemical moieties without decreasing binding affinity.

Analogues of BBN receptor targeting peptides include molecules that target the GRP receptors with avidity that is greater than or equal to BBN, as well as muteins, retropeptides and retro-inverso-peptides of GRP or BBN. One of ordinary skill will appreciate that these analogues may also contain modifications which include substitutions, and/or deletions and/or additions of one or several amino acids, insofar that these modifications do not negatively alter the biological activity of the peptides described therein. These substitutions may be carried out by replacing one or more amino acids by their synonymous amino acids. Synonymous amino acids within a group are defined as amino acids

that have sufficient physicochemical properties to allow substitution between members of a group in order to preserve the biological function of the molecule.

Deletions or insertions of amino acids may also be introduced into the defined sequences provided they do not alter the biological functions of said sequences. Preferentially such insertions or deletions should be limited to 1, 2, 3, 4 or 5 amino acids and should not remove or physically disturb or displace amino acids which are critical to the functional conformation. Muteins of the GRP receptor targeting peptides described herein may have a sequence homologous to the sequence disclosed in the present specification in which amino acid substitutions, deletions, or insertions are present at one or more amino acid positions. Muteins may have a biological activity that is at least 40%, preferably at least 50%, more preferably 60-70%, most preferably 80-90% of the peptides described herein. However, they may also have a biological activity greater than the peptides specifically exemplified, and thus do not necessarily have to be identical to the biological function of the exemplified peptides. Analogues of GRP receptor targeting peptides also include peptidomimetics or pseudopeptides incorporating changes to the amide bonds of the peptide backbone, including thioamides, methylene amines, and E-olefins. Also peptides based on the structure of GRP, BBN or their peptide analogues with amino acids replaced by N-substituted hydrazine carbonyl compounds (also known as aza amino acids) are included in the term analogues as used herein.

The GRP receptor targeting peptide can be prepared by various methods depending upon the selected chelator. The peptide can generally be most conveniently prepared by techniques generally established and known in the art of peptide synthesis, such as the solid-phase peptide synthesis (SPPS) approach. Solid-phase peptide synthesis (SPPS) involves the stepwise addition of amino acid residues to a growing peptide chain that is linked to an insoluble support or matrix, such as polystyrene. The C-terminal residue of the peptide is first anchored to a commercially available support with its amino group protected with an N-protecting agent such as a t-butyloxycarbonyl group (Boc) or a fluorenylmethoxycarbonyl (Fmoc) group. The amino protecting group is removed with suitable deprotecting agents such as TFA in the case of Boc or piperidine for Fmoc and the next amino acid residue (in N-protected form) is added with a coupling agent such as N,N'-dicyclohexylcarbodiimide (DCC), or N,N'-diisopropylcarbodiimide (DIC) or 2-(1H-benzotriazol-1-yl)-1,1,3,3-tetramethyluronium hexafluorophosphate (HBTU). Upon formation of a peptide bond, the reagents are washed from the support. After addition of the final residue, the peptide is cleaved from the support with a suitable reagent such as trifluoroacetic acid (TFA) or hydrogen fluoride (HF).

The linker may then be coupled to form a conjugate by reacting the free amino group of the $Trp^8$ residue of the GRP receptor targeting peptide with an appropriate functional group of the linker. The entire construct of chelator, linker and targeting moiety discussed above may also be assembled on resin and then cleaved by agency of suitable reagents such as trifluoroacetic acid or HF, as well.

4. <u>Labeling And Administration Of Radiopharmaceutical Compounds</u>

Incorporation of the metal within the radiopharmaceutical conjugates can be achieved by various methods commonly known in the art of coordination chemistry. When the metal is $^{99m}Tc$, a preferred radionuclide for diagnostic imaging, the following general procedure can be used to form a technetium complex. A peptide-chelator conjugate solution is formed by initially dissolving the conjugate in water, dilute acid, or in an aqueous solution of an alcohol such as ethanol. The solution is then optionally degassed to remove dissolved oxygen. When an -SH group is present in the peptide, a thiol protecting group such as Acm (acetamidomethyl), trityl or other thiol protecting group may optionally be used to protect the thiol from oxidation. The thiol protecting group(s) are removed with a suitable reagent, for example with sodium hydroxide, and are then neutralized with an organic acid such as acetic acid (pH 6.0-6.5). Alternatively, the thiol protecting group can be removed *in situ* during technetium chelation. In the labeling step, sodium pertechnetate obtained from a molybdenum generator is added to a solution of the conjugate with a sufficient amount of a reducing agent, such as stannous chloride, to reduce technetium and is either allowed to stand at room temperature or is heated. The labeled conjugate can be separated from the contaminants $^{99m}TcO_4^-$ and colloidal $^{99m}TcO_2$ chromatographically, for example with a C-18 Sep Pak cartridge [Millipore Corporation, Waters Chromatography Division, 34 Maple Street, Milford, Massachusetts 01757] or by HPLC using methods known to those skilled in the art.

In an alternative method, the labeling can be accomplished by a transchelation reaction. In this method, the technetium source is a solution of technetium that is reduced and complexed with labile ligands prior to reaction with the selected chelator, thus facilitating ligand exchange with the selected chelator. Examples of suitable ligands for transchelation includes tartrate, citrate, gluconate, and heptagluconate. It will be appreciated that the conjugate can be labeled using the techniques described above, or alternatively, the chelator itself may be labeled and subsequently coupled to the peptide to form the conjugate; a process referred to as the "prelabeled chelate" method. Re and Tc are both in row VIIB of the Periodic Table and they are chemical congeners. Thus, for the most part, the complexation chemistry of these two metals with ligand frameworks that exhibit high *in vitro* and *in vivo* stabilities are the same [Eckelman, 1995] and similar chelators and procedures can be used to label with Re. Many $^{99m}Tc$ or $^{186/188}Re$ complexes, which are employed to form stable radiometal complexes with peptides and proteins, chelate these metals in their +5

oxidation state [Lister-James et al., 1997]. This oxidation state makes it possible to selectively place $^{99m}$Tc- or $^{186/188}$Re into ligand frameworks already conjugated to the biomolecule, constructed from a variety of $^{99m}$Tc(V) and/or $^{186/188}$Re (V) weak chelates (e.g., $^{99m}$Tc- glucoheptonate, citrate, gluconate, etc.) [Eckelman, 1995; Lister-James et al., 1997; Pollak et al., 1996].

5. Diagnostic and Therapeutic Uses

When labeled with diagnostically and/or therapeutically useful metals or optical labels, compounds of the present invention can be used to treat and/or detect any pathology involving overexpression of GRP receptors (or NMB receptors) by procedures established in the art of radiodiagnostics, radiotherapeutics and optical imaging. [See e.g., Bushbaum, 1995; Fischman et al., 1993; Schubiger et al., 1996; Lowbertz et al., 1994; Krenning et al., 1994; examples of optical dyes include, but are not limited to those described in WO 98/18497, WO 98/18496, WO 98/18495, WO 98/18498, WO 98/53857, WO 96/17628, WO 97/18841, WO 96/23524, WO 98/47538, and references cited therein.]

GRP-R expression is highly upregulated in a variety of human tumors. See e.g., WO 99/62563. Thus, compounds of the invention may be widely useful in treating and diagnosing cancers, including prostate cancer (primary and metastatic), breast cancer (primary and metastatic), colon cancer, gastric cancer, pancreatic cancer, non small cell lung cancer, small cell lung cancer, gastrinomas, melanomas, glioblastomas, neuroblastomas, uterus leiomyosarcoma tumors, prostatic intraepithelial neoplasias [PIN], and ovarian cancer. Additionally, compounds of the invention may be useful to distinguish between conditions in which GRP receptors are upregulated and those in which they are not (e.g. chronic pancreatitis and ductal pancreatic carcinoma, respectively

The compounds of the invention, which, as explained in more detail in the Examples, show higher uptake in tumors in vivo than compounds without the novel linkers disclosed herein, exhibit an improved ability to target GRP receptor-expressing tumors and thus to image or deliver radiotherapy to these tissues. Indeed, as shown in the Examples, radiotherapy is more effective (and survival time increased) using compounds of the invention.

The diagnostic application of these compounds can be as a first line diagnostic screen for the presence of neoplastic cells using scintigraphic, optical, sonoluminescence or photoacoustic imaging, as an agent for targeting neoplastic tissue using hand-held radiation detection instrumentation in the field of radioimmuno guided surgery (RIGS), as a means to obtain dosimetry data prior to administration of the matched pair radiotherapeutic compound, and as a means to assess GRP receptor population as a function of treatment over time.

The therapeutic application of these compounds can be defined as an agent that will be used as a first line therapy in the treatment of cancer, as combination therapy where these agents could be utilized in conjunction with adjuvant chemotherapy, and/or as a matched pair therapeutic agent. The matched pair concept refers to a single unmetallated compound which can serve as both a diagnostic and a therapeutic agent depending on the radiometal that has been selected for binding to the appropriate chelate. If the chelator cannot accommodate the desired metals, appropriate substitutions can be made to accommodate the different metal while maintaining the pharmacology such that the behavior of the diagnostic compound in vivo can be used to predict the behavior of the radiotherapeutic compound. When utilized in conjunction with adjuvant chemotherapy any suitable chemotherapeutic may be used, including for example, antineoplastic agents, such as platinum compounds (e.g., spiroplatin, cisplatin, and carboplatin), methotrexate, adriamycin, mitomycin, ansamitocin, bleomycin, cytosine, arabinoside, arabinosyl adenine, mercaptopolylysine, vincristine, busulfan, chlorambucil, melphalan (e.g., PAM, a, L -PAM or phennylalanine mustard), mercaptopurine, mitotane. procarbazine hydrochloride, dactinomycin (actinomycin D), daunorubcin hydrochloride, doxorubicin hydrochloride, taxol, mitomycin, plicamycin (mithramycin), aminoglutethimide, estramustine phosphate sodium, flutamide, leuprolide acetate, megestrol acetate, tamoxifen citrate, testolactone, trilostane, amsacrine (m-AMSA), asparaginase (L-asparaginase) *Erwina aparaginase,* etoposide -(VP-16), interferon α-2a, interferon α-2b, teniposide (VM-26), vinblastine sulfate (VLB), and arabinosyl. In certain embodiments, the therapeutic may be monoclonal antibody, such as a monoclonal antibody capable of binding to melanoma antigen.

A conjugate labeled with a radionuclide metal, such as $^{99m}$Tc, can be administered to a mammal, including human patients or subj ects, by intravenous, subcutaneous or intraperitoneal injection in a pharmaceutically acceptable carrier and/or solution such as salt solutions like isotonic saline. Radiolabeled scintigraphic imaging agents provided by the present invention are provided having a suitable amount of radioactivity. In forming $^{99m}$Tc radioactive complexes, it is generally preferred to form radioactive complexes in solutions containing radioactivity at concentrations of from about 0.01 millicurie (mCi) to 100 mCi per mL. Generally, the unit dose to be administered has a radioactivity of about 0.01 mCi to about 100 mCi, preferably 1 mCi to 30 mCi. The solution to be injected at unit dosage is from about 0.01 mL to about 10 mL. The amount of labeled conjugate appropriate for administration is dependent upon the distribution profile of the chosen conjugate in the sense that a rapidly cleared conjugate may need to be administered in higher doses than one that clears less rapidly. *In vivo* distribution and localization can be tracked by standard scintigraphic techniques at an appropriate time subsequent to administration; typically between thirty minutes and 180 minutes depending upon the rate of accumulation at the target site with respect to the rate of clearance at non-target tissue. For example, after

injection of the diagnostic radionuclide-labeled compounds of the invention into the patient, a gamma camera calibrated for the gamma ray energy of the nuclide incorporated in the imaging agent can be used to image areas of uptake of the agent and quantify the amount of radioactivity present in the site. Imaging of the site *in vivo* can take place in a few minutes. However, imaging can take place, if desired, hours or even longer, after the radiolabeled peptide is injected into a patient. In most instances, a sufficient amount of the administered dose will accumulate in the area to be imaged within about 0.1 hour to permit the taking of scintiphotos.

The compounds of the present invention can be administered to a patient alone or as part of a composition that contains other components such as excipients, diluents, radical scavengers, stabilizers, and carriers, all of which are well-known in the art. The compounds can be administered to patients either intravenously or intraperitoneally.

There are numerous advantages associated with the present invention. The compounds made in accordance with the present invention form stable, well-defined $^{99m}Tc$ or $^{186/188}Re$ labeled compounds. Similar compounds of the invention can also be made by using appropriate chelator frameworks for the respective radiometals, to form stable, well-defined products labeled with $^{153}Sm$, $^{90}Y$, $^{166}Ho$, $^{105}Rh$, $^{199}Au$, $^{149}Pm$, $^{177}Lu$, $^{111}In$ or other radiometal. The radiolabeled GRP receptor targeting peptides selectively bind to neoplastic cells expressing GRP receptors, and if an agonist is used, become internalized, and are retained in the tumor cells for extended time periods. The radioactive material that does not reach (i.e., does not bind) the cancer cells is preferentially excreted efficiently into the urine with minimal retention of the radiometal in the kidneys.

6. Optical Imaging, Sonoluminescence, Photoacoustic Imaging and Phototherapy

In accordance with the present invention, a number of optical parameters may be employed to determine the location of a target with *in vivo* light imaging after injection of the subject with an optically-labeled compound of the invention. Optical parameters to be detected in the preparation of an image may include transmitted radiation, absorption, fluorescent or phosphorescent emission, light reflection, changes in absorbance amplitude or maxima, and elastically scattered radiation. For example, biological tissue is relatively translucent to light in the near infrared (NIR) wavelength range of 650-1000 nm. NIR radiation can penetrate tissue up to several centimeters, permitting the use of compounds of the present invention to image target-containing tissue in vivo. The use of visible and near-infrared (NIR) light in clinical practice is growing rapidly. Compounds absorbing or emitting in the visible, NIR, or long-wavelength (UV-A, >350 nm) region of the electromagnetic spectrum are potentially useful for optical tomographic imaging, endoscopic visualization, and phototherapy.

A major advantage of biomedical optics lies in its therapeutic potential. Phototherapy has been demonstrated to be a safe and effective procedure for the treatment of various surface lesions, both external and internal. Dyes are important to enhance signal detection and/or photosensitizing of tissues in optical imaging and phototherapy. Previous studies have shown that certain dyes can localize in tumors and serve as a powerful probe for the detection and treatment of small cancers (D. A. Bellnier et al., Murine pharmacokinetics and antitumor efficacy of the photodynamic sensitizer 2-[1-hexyloxyethyl]-2-devinyl pyropheophorbide-a, J. Photochem. Photobiol., 1993, 20, pp. 55-61; G. A. Wagnieres et al., In vivo fluorescence spectroscopy and imaging for oncological applications, Photochem. Photobiol., 1998, 68, pp. 603-632; J. S. Reynolds et al., Imaging of spontaneous canine mammary tumors using fluorescent contrast agents, Photochem. Photobiol., 1999, 70, pp. 87-94). However, these dyes do not localize preferentially in malignant tissues.

In an exemplary embodiment, the compounds of the invention may be conjugated with photolabels, such as optical dyes, including organic chromophores or fluorophores, having extensive delocalized ring systems and having absorption or emission maxima in the range of 400-1500 nm. The compounds of the invention may alternatively be derivatized with a bioluminescent molecule. The preferred range of absorption maxima for photolabels is between 600 and 1000 nm to minimize interference with the signal from hemoglobin. Preferably, photoabsorption labels have large molar absorptivities, *e.g.* > $10^5$ $cm^{-1}M^{-1}$, while fluorescent optical dyes will have high quantum yields. Examples of optical dyes include, but are not limited to those described in WO 98/18497, WO 98/18496, WO 98/18495, WO 98/18498, WO 98/53857, WO 96/17628, WO 97/18841, WO 96/23524, WO 98/47538, and references cited therein. For example, the photolabels may be covalently linked directly to compounds of the invention, such as, for example, compounds comprised of GRP receptor targeting peptides and linkers of the invention. Several dyes that absorb and emit light in the visible and near-infrared region of electromagnetic spectrum are currently being used for various biomedical applications due to their biocompatibility, high molar absorptivity, and/or high fluorescence quantum yields. The high sensitivity of the optical modality in conjunction with dyes as contrast agents parallels that of nuclear medicine, and permits visualization of organs and tissues without the undesirable effect of ionizing radiation. Cyanine dyes with intense absorption and emission in the near-infrared (NIR) region are particularly useful because biological tissues are optically transparent in this region (B. C. Wilson, Optical properties of tissues. Encyclopedia of Human Biology, 1991, 5, 587-597). For example, indocyanine green, which absorbs and emits in the NIR region has been used for monitoring cardiac output, hepatic functions, and liver blood flow (Y-L. He, H. Tanigami, H. Ueyama, T. Mashimo, and I. Yoshiya, Measurement of blood volume using indocyanine green measured with pulse-spectrometry: Its reproducibility and reliability. Critical Care Medicine, 1998, 26(8), 1446-1451; J. Caesar, S. Shaldon, L. Chiandussi, et al., The use of Indocyanine green in the measurement of hepatic blood flow and as a test of hepatic function. Clin. Sci. 1961, 21, 43-57) and its functionalized derivatives have been used to conjugate biomolecules for diagnostic purposes (R. B. Mujumdar, L. A. Ernst, S. R. Mujumdar, et al., Cyanine dye labeling reagents: Sulfoindocyanine succinimidyl esters. Bioconjugate Chemistry, 1993, 4(2), 105-111; Linda G. Lee and Sam L. Woo. "N-Heteroaromatic ion and iminium ion substituted cyanine dyes for use as fluorescent labels", U.S. Pat. No. 5,453,505; Eric Hohenschuh, et al. "Light imaging contrast agents", WO 98/48846; Jonathan Turner, et al. "Optical diagnostic agents for the diagnosis of neurodegenerative diseases by means of near infra-red radiation", WO 98/22146; Kai Licha, et al. "In-vivo diagnostic process by near infrared radiation", WO 96/17628; Robert A. Snow, et al., Compounds, WO 98/48838.

After injection of the optically-labeled compound, the patient is scanned with one or more light sources (*e.g.*, a laser) in the wavelength range appropriate for the photolabel employed in the agent. The light used may be monochromatic or polychromatic and continuous or pulsed. Transmitted, scattered, or reflected light is detected via a photodetector tuned to one or multiple wavelengths to determine the location of target-containing tissue (*e.g.*, tissue containing GRP) in the subject. Changes in the optical parameter may be monitored over time to detect accumulation of the optically-labeled reagent at the target site (e.g. the tumor or other site with GRP receptors). Standard image processing and detecting devices may be used in conjunction with the optical imaging reagents of the present invention.

The optical imaging reagents described above may also be used for acousto-optical or sonoluminescent imaging performed with optically-labeled imaging agents (see, U.S. 5,171,298, WO 98/57666, and references therein). In acousto-optical imaging, ultrasound radiation is applied to the subject and affects the optical parameters of the transmitted, emitted, or reflected light. In sonoluminescent imaging, the applied ultrasound actually generates the light detected. Suitable imaging methods using such techniques are described in WO 98/57666.

Various imaging techniques and reagents are described in U.S. Patents 6,663,847, 6,656,451, 6,641,798, 6,485,704, 6,423,547, 6,395,257, 6,280,703, 6,277,841, 6,264,920, 6,264,919, 6,228,344, 6,217,848, 6,190,641, 6,183,726, 6,180,087, 6,180,086, 6,180,085, 6,013,243, and published U.S. Patent Applications 2003185756, 20031656432, 2003158127, 2003152577, 2003143159, 2003105300, 2003105299, 2003072763, 2003036538, 2003031627, 2003017164, 2002169107, 2002164287, and 2002156117.

7. <u>Radiotherapy</u>

Radioisotope therapy involves the administration of a radiolabeled compound in sufficient quantity to damage or destroy the targeted tissue. After administration of the compound (by e.g., intravenous, subcutaneous, or intraperitonal injection), the radiolabeled pharmaceutical localizes preferentially at the disease site (in this instance, tumor tissue or other tissue that expresses the GRP receptor). Once localized, the radiolabeled compound then damages or destroys the diseased tissue with the energy that is released during the radioactive decay of the isotope that is administered. As discussed herein, the invention also encompasses use of radiotherapy in combination with adjuvant chemotherapy (or in combination with any other appropriate therapeutic agent).

The design of a successful radiotherapeutic involves several critical factors:

1. selection of an appropriate targeting group to deliver the radioactivity to the disease site;
2. selection of an appropriate radionuclide that releases sufficient energy to damage that disease site, without substantially damaging adjacent normal tissues; and
3. selection of an appropriate combination of the targeting group and the radionuclide without adversely affecting the ability of this conjugate to localize at the disease site. For radiometals, this often involves a chelating group that coordinates tightly to the radionuclide, combined with a linker that couples said chelate to the targeting group, and that affects the overall biodistribution of the compound to maximize uptake in target tissues and minimize uptake in normal, non-target organs.

The present invention provides radiotherapeutic agents that satisfy all three of the above criteria, through proper selection of targeting group, radionuclide, metal chelate and linker.

Radiotherapeutic agents may contain a chelated 3+ metal ion from the class of elements known as the lanthanides (elements of atomic number 57-71) and their analogs (i.e. $M^{3+}$ metals such as yttrium and indium). Typical radioactive metals in this class include the isotopes 90-Yttrium, 111-Indium, 149-Promethium, 153-Samarium, 166-Dysprosium, 166-Holmium, 175-Ytterbium, and 177-Lutetium. All of these metals (and others in the lanthanide series) have very similar chemistries, in that they remain in the +3 oxidation state, and prefer to chelate to ligands that bear hard (oxygen/nitrogen) donor atoms, as typified by derivatives of the well known chelate DTPA (diethylenetriaminepentaacetic acid) and polyaza-polycarboxylate macrocycles such as DOTA (1,4,7,10-tetrazacyclododecane-N, N',N",N"'-tetraacetic acid and its close analogs. The structures of these chelating ligands, in their fully deprotonated form are shown below.

| DTPA | DOTA |
|---|---|
| 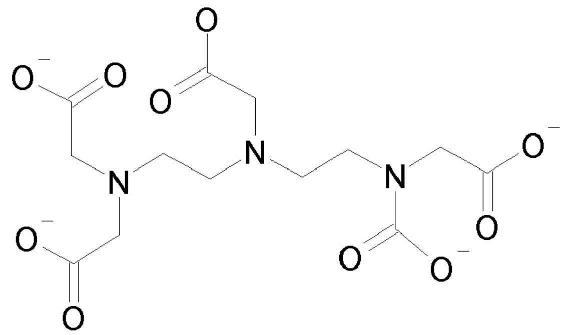 | 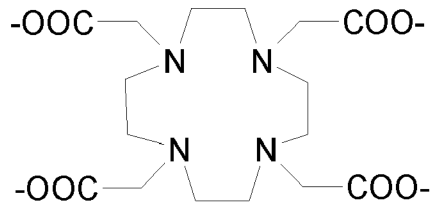 |

These chelating ligands encapsulate the radiometal by binding to it *via* multiple nitrogen and oxygen atoms, thus preventing the release of free (unbound) radiometal into the body. This is important, as *in vivo* dissociation of $3^+$ radiometals from their chelate can result in uptake of the radiometal in the liver, bone and spleen [Brechbiel MW, Gansow OA, "Backbone-substituted DTPA ligands for 90Y radioimmunotherapy", Bioconj. Chem. 1991; 2: 187-194; Li, WP, Ma DS, Higginbotham C, Hoffman T, Ketring AR, Cutler CS, Jurisson, SS, " Development of an in vitro model for assessing the in vivo stability of lanthanide chelates." Nucl. Med. Biol. 2001; 28(2): 145-154; Kasokat T, Urich K. Arzneim.-Forsch, "Quantification of dechelation of gadopentetate dimeglumine in rats". 1992; 42(6): 869-76]. Unless one is specifically targeting these organs, such non-specific uptake is highly undesirable, as it leads to non-specific irradiation of non-target tissues, which can lead to such problems as hematopoietic suppression due to irradiation of bone marrow.

For radiotherapy applications any of the chelators for therapeutic radionuclides disclosed herein may be used. However, forms of the DOTA chelate [Tweedle MF, Gaughan GT, Hagan JT, "1-Substituted-1,4,7-triscarboxymethyl-1,4,7,10-tetraazacyclododecane and analogs." US Patent 4,885,363, Dec. 5, 1989] are particularly preferred, as the DOTA chelate is expected to de-chelate less in the body than DTPA or other linear chelates.

General methods for coupling DOTA-type macrocycles to targeting groups through a linker (e.g. by activation of one of the carboxylates of the DOTA to form an active ester, which is then reacted with an amino group on the linker to form a stable amide bond), are known to those skilled in the art. (See e.g. Tweedle et al. US Patent 4,885,363). Coupling can also be performed on DOTA-type macrocycles that are modified on the backbone of the polyaza ring.

The selection of a proper nuclide for use in a particular radiotherapeutic application depends on many factors, including:

a. Physical half-life - This should be long enough to allow synthesis and purification of the radiotherapeutic construct from radiometal and conjugate, and delivery of said construct to the site of injection, without significant radioactive decay prior to injection. Preferably, the radionuclide should have a physical half-life between about 0.5 and 8 days.

b. Energy of the emission(s) from the radionuclide - Radionuclides that are particle emitters (such as alpha emitters, beta emitters and Auger electron emitters) are particularly useful, as they emit highly energetic particles that deposit their energy over short distances, thereby producing highly localized damage. Beta emitting radionuclides are particularly preferred, as the energy from beta particle emissions from these isotopes is deposited within 5 to about 150 cell diameters. Radiotherapeutic agents prepared from these nuclides are capable of killing diseased cells that are relatively close to their site of localization, but cannot travel long distances to damage adjacent normal tissue such as bone marrow.

c. Specific activity (i.e. radioactivity per mass of the radionuclide) - Radionuclides that have high specific activity (e.g. generator produced 90-Y, 111-In, 177-Lu) are particularly preferred. The specific activity of a radionuclide is determined by its method of production, the particular target that is used to produce it, and the properties of the isotope in question.

Many of the lanthanides and lanthanoids include radioisotopes that have nuclear properties that make them suitable for use as radiotherapeutic agents, as they emit beta particles. Some of these are listed in the table below.

| Isotope | Half -Life (days) | Max b- energy (MeV) | Gamma energy (keV) | Approximate range of b-particle (cell diameters) |
|---|---|---|---|---|
| $^{149}$-Pm | 2.21 | 1.1 | 286 | 60 |

(continued)

| Isotope | Half -Life (days) | Max b- energy (MeV) | Gamma energy (keV) | Approximate range of b-particle (cell diameters) |
|---|---|---|---|---|
| $^{153}$-Sm | 1.93 | 0.69 | 103 | 30 |
| $^{166}$-Dy | 3.40 | 0.40 | 82.5 | 15 |
| $^{166}$-Ho | 1.12 | 1.8 | 80.6 | 117 |
| $^{175}$-Yb | 4.19 | 0.47 | 396 | 17 |
| $^{177}$-Lu | 6.71 | 0.50 | 208 | 20 |
| $^{90}$-Y | 2.67 | 2.28 | - | 150 |
| $^{111}$-In | 2.810 | Auger electron emitter | 173,247 | < 5 μm |
| Pm Promethium, Sm Samarium, Dy Dysprosium, Ho Holmium, Yb Ytterbium, Lu Lutetium, Y Yttnum, In Indium | | | | |

Methods for the preparation of radiometals such as beta-emitting lanthanide radioisotopes are known to those skilled in the art, and have been described elsewhere [e.g. Cutler C S, Smith CJ, Ehrhardt GJ.; Tyler TT, Jurisson SS, Deutsch E. "Current and potential therapeutic uses of lanthanide radioisotopes." Cancer Biother. Radiopharm. 2000; 15(6): 531-545]. Many of these isotopes can be produced in high yield for relatively low cost, and many (e.g. $^{90}$-Y, $^{149}$-Pm, $^{177}$-Lu can be produced at close to carrier-free specific activities (i.e. the vast majority of atoms are radioactive). Since non-radioactive atoms can compete with their radioactive analogs for binding to receptors on the target tissue, the use of high specific activity radioisotope is important, to allow delivery of as high a dose of radioactivity to the target tissue as possible.

Radiotherapeutic derivatives of the invention containing beta-emitting isotopes of rhenium ($^{186}$-Re and $^{188}$-Re) are also particularly preferred.

8. Dosages And Additives

Proper dose schedules for the compounds of the present invention are known to those skilled in the art. The compounds can be administered using many methods which include, but are not limited to, a single or multiple IV or IP injections. For radiopharmaceuticals, one administers a quantity of radioactivity that is sufficient to permit imaging or, in the case of radiotherapy, to cause damage or ablation of the targeted GRP-R bearing tissue, but not so much that substantive damage is caused to non-target (normal tissue). The quantity and dose required for scintigraphic imaging is discussed supra. The quantity and dose required for radiotherapy is also different for different constructs, depending on the energy and half-life of the isotope used, the degree of uptake and clearance of the agent from the body and the mass of the tumor. In general, doses can range from a single dose of about 30-50 mCi to a cumulative dose of up to about 3 Curies.

[Insert dosage info for optical compounds]

The compositions of the invention can include physiologically acceptable buffers, and can require radiation stabilizers to prevent radiolytic damage to the compound prior to injection. Radiation stabilizers are known to those skilled in the art, and may include, for example, para-aminobenzoic acid, ascorbic acid, gentistic acid and the like.

A single, or multi-vial kit that contains all of the components needed to prepare the diagnostic or therapeutic agents of this invention is an integral part of this invention. In the case of radiopharmaceuticals, such kits will often include all necessary ingredients except the radionuclide.

For example, a single-vial kit for preparing a radiopharmaceutical of the invention preferably contains a chelator/linker/targeting peptide conjugate of the formula M-N-O-P-G, a source of stannous salt (if reduction is required, *e.g.*, when using technetium), or other pharmaceutically acceptable reducing agent, and is appropriately buffered with pharmaceutically acceptable acid or base to adjust the pH to a value of about 3 to about 9. The quantity and type of reducing agent used will depend highly on the nature of the exchange complex to be formed. The proper conditions are well known to those that are skilled in the art. It is preferred that the kit contents be in lyophilized form. Such a single vial kit may optionally contain labile or exchange ligands such as glucoheptonate, gluconate, mannitol, malate, citric or tartaric acid and can also contain reaction modifiers such as diethylenetriamine-pentaacetic acid (DPTA), ethylenediamine tetraacetic acid (EDTA), or a, β, or γ-cyclodextrin that serve to improve the radiochemical purity and stability of the final product. The kit may also contain stabilizers, bulking agents such as mannitol, that are designed to aid in the freeze-drying process, and other additives known to those skilled in the art.

A multi-vial kit preferably contains the same general components but employs more than one vial in reconstituting the radiopharmaceutical. For example, one vial may contain all of the ingredients that are required to form a labile Tc (V) complex on addition of pertechnetate (*e.g.* the stannous source or other reducing agent). Pertechnetate is added to this vial, and after waiting an appropriate period of time, the contents of this vial are added to a second vial that contains the chelator and targeting peptide, as well as buffers appropriate to adjust the pH to its optimal value. After a reaction time of about 5 to 60 minutes, the complexes of the present invention are formed. It is advantageous that the contents of both vials of this multi-vial kit be lyophilized. As above, reaction modifiers, exchange ligands, stabilizers, bulking agents, etc. may be present in either or both vials.

## General Preparation Of Compounds

The compounds of the present invention can be prepared by various methods depending upon the selected chelator. The peptide portion of the compound can be most conveniently prepared by techniques generally established and known in the art of peptide synthesis, such as the solid-phase peptide synthesis (SPPS) approach. Because it is amenable to solid phase synthesis, employing alternating FMOC protection and deprotection is the preferred method of making short peptides. Recombinant DNA technology is preferred for producing proteins and long fragments thereof.

Solid-phase peptide synthesis (SPPS) involves the stepwise addition of amino acid residues to a growing peptide chain that is linked to an insoluble support or matrix, such as polystyrene. The C-terminal residue of the peptide is first anchored to a commercially available support with its amino group protected with an N-protecting agent such as a t-butyloxycarbonyl group (Boc) or a fluorenylmethoxycarbonyl (Fmoc) group. The amino protecting group is removed with suitable deprotecting agents such as TFA in the case of Boc or piperidine for Fmoc and the next amino acid residue (in N-protected form) is added with a coupling agent such as diisopropylcarbodiimide (DIC). Upon formation of a peptide bond, the reagents are washed from the support. After addition of the final residue, the peptide is cleaved from the support with a suitable reagent such as trifluoroacetic acid (TFA) or hydrogen fluoride (HF).

## Alternative Preparation of the Compounds via Segment Coupling

The compounds of the invention may also be prepared by the process known in the art as segment coupling or fragment condensation (Barlos, K. and Gatos, D. ; 2002 "Convergent Peptide Synthesis" in Fmoc Solid Phase Synthesis - A Practical Approach; Eds. Chan, W.C. and White, P.D.; Oxford University Press, New York; Chap. 9, pp 215-228). In this method segments of the peptide usually in side-chain protected form, are prepared separately by either solution phase synthesis or solid phase synthesis or a combination of the two methods. The choice of segments is crucial and is made using a division strategy that can provide a manageable number of segments whose C-terminal residues and N-terminal residues are projected to provide the cleanest coupling in peptide synthesis. The C-terminal residues of the best segments are either devoid of chiral alpha carbons (glycine or other moieties achiral at the carbon ∝ to the carboxyl group to be activated in the coupling step) or are compromised of amino acids whose propensity to racemization during activation and coupling is lowest of the possible choices. The choice of N-terminal amino acid for each segment is based on the ease of coupling of an activated acyl intermediate to the amino group. Once the division strategy is selected the method of coupling of each of the segments is chosen based on the synthetic accessibility of the required intermediates and the relative ease of manipulation and purification of the resulting products (if needed). The segments are then coupled together, both in solution, or one on solid phase and the other in solution to prepare the final structure in fully or partially protected form.

The protected target compound is then subjected to removal of protecting groups, purified and isolated to give the final desired compound. Advantages of the segment coupling approach are that each segment can be purified separately, allowing the removal of side products such as deletion sequences resulting from incomplete couplings or those derived from reactions such as side-chain amide dehydration during coupling steps, or internal cyclization of side-chains (such as that of Gln) to the alpha amino group during deprotection of Fmoc groups. Such side products would all be present in the final product of a conventional resin-based 'straight through' peptide chain assembly whereas removal of these materials can be performed, if needed, at many stages in a segment coupling strategy. Another important advantage of the segment coupling strategy is that different solvents, reagents and conditions can be applied to optimize the synthesis of each of the segments to high purity and yield resulting in improved purity and yield of the final product. Other advantages realized are decreased consumption of reagents and lower costs.

Further embodiments of the invention are enlisted as embodiments 20-42, 44-45, 51, 71, 73, 75, 77, 81, 82, 85 as follows: 20. A compound of the general formula:

$$M\text{-}N\text{-}O\text{-}P\text{-}G$$

Wherein :

M is an optical label or a metal chelator, optionally complexed with a radionuclide;
N is 0, an alpha amino acid, a substituted bile acid or other linking group;
O is an alpha amino acid or a substituted bile acid; and
P is 0, an alpha amino acid, a substituted bile acid or other linking group; and
G is a GRP receptor targeting peptide, and
wherein at least one ofN, O or P is a substituted bile acid.

21. The compound of embodiment 20, wherein G is an agonist or a peptide which confers agonist activity.
22. The compound of embodiment 20, wherein the substituted bile acid is selected from the group consisting of:
3β-amino-3-deoxycholic acid;
(3β,5β)-3-aminocholan-24-oic acid;
(3β,5β,12α)-3-amino-12-hydroxycholan-24-oic acid; (3β,5β,7α,12α)-3-amino-7,12-dihydroxycholan-24-oic acid; Lys-(3,6,9)-trioxaundecane-1,11-dicarbonyl-3,7-dideoxy-3-aminocholic acid);
(3β,5β,7α,12α)-3-amino-7-hydroxy-12-oxocholan-24-oic acid; and (3β,5β,7α)-3-amino-7-hydroxycholan-24-oic acid.
23. The compound of embodiment 20, wherein M is selected from the group consisting of: DTPA, DOTA, DO3A, HPDO3A, EDTA, TETA and CMDOTA.
24. The compound of embodiment 20, wherein M is selected from the group consisting of EHPG and derivatives thereof.
25. The compound of embodiment 20, wherein M is selected from the group consisting of 5-Cl-EHPG, 5-Br-EHPG, 5-Me-EHPG, 5-t-Bu-EHPG, and 5-sec-Bu-EHPG.
26. The compound of embodiment 20, wherein M is selected from the group consisting of benzodiethylenetriamine pentaacetic acid (benzo-DTPA) and derivatives thereof.
27. The compound of embodiment 20, wherein M is selected from the group consisting of dibenzo-DTPA, phenyl-DTPA, diphenyl-DTPA, benzyl-DTPA, and dibenzyl DTPA.
28. The compound of embodiment 20, wherein M is selected from the group consisting of HBED and derivatives thereof.
29. The compound of embodiment 20, wherein M is a macrocyclic compound which contains at least 3 carbon atoms and at least two heteroatoms (O and/or N), which macrocyclic compounds can consist of one ring, or two or three rings joined together at the hetero ring elements.
30. The compound of embodiment 20, wherein M is selected from the group consisting of benzo-DOTA, dibenzo-DOTA, and benzo-NOTA, benzo-TETA, benzo-DOTMA, and benzo-TETMA.
31. The compound of embodiment 20, wherein M is selected from the group consisting of derivatives of 1,3-propylenediaminetetraacetic acid (PDTA) and triethylenetetraaminehexaacetic acid (TTHA); derivatives of 1,5,10-N,N', N"-tris(2,3-dihydroxybenzoyl)-tricatecholate (LICAM) and 1,3,5-N,N',N"-tris(2,3-dihydroxybenzoyl) aminomethylbenzene (MECAM).
32. A compound of embodiment 20 selected from the group consisting of:

DO3A-monoamide-Gly-(3β,5β)-3-aminocholan-24-oic acid-BBN(7-14) wherein the BBN(7-14) sequence is SEQ. ID NO: 1;
DO3A-monoamide-Gly-(3β,5β,12α)-3-amino-12-hydroxycholan-24-oic acid-BBN(7-14) wherein the BBN(7-14) sequence is SEQ. ID NO: 1;
DO3A-monoamide-Gly-(3β,5β,7α,12α)-3-amino-7,12-dihydroxycholan-24-oic acid-BBN(7-14) wherein the BBN(7-14) sequence is SEQ. ID NO: 1;
DO3A-monoamide-Gly-Lys-(3,6,9)-trioxaundecane-1,11-dicarbonyl-3,7-dideoxy-3-aminocholic acid)-Arg-BBN (7-14) wherein the BBN(7-14) sequence is SEQ. ID NO: 1;
(3β,5β,7α,12α)-3-amino-7,12-dihydroxycholan-24-oic acid-3,6,9-trioxaundecane-1,11-dicarbonyl Lys(DO3A-monoamide-Gly)-Arg-BBN(7-14) wherein the BBN(7-14) sequence is SEQ. ID NO: 1;
DO3A-monoamide-Gly-(3β,5β,7α,12α)-3-amino-12-oxocholan-24-oic acid-BBN(7-14) wherein the BBN(7-14) sequence is SEQ. ID NO: 1; and
DO3A-monoamide-1-amino-3,6-dioxaoctanoic acid-(3β,5β,7α,12α)-3-amino-7,12-dihydroxycholan-24-oic acid-BBN(7-14) wherein the BBN(7-14) sequence is SEQ. ID NO: 1.

33. The compound of any one of embodiments 20, 21 or 22, wherein the optical label is selected from the group consisting of organic chromophores, organic fluorophores, light-absorbing compounds, light-reflecting compounds, light-scattering compounds, and bioluminescent molecules.

34. A method of imaging comprising the steps of:

administering to a patient a diagnostic imaging agent comprising the compound of embodiment 20 wherein M is a metal chelator complexed with a diagnostic radionuclide, and imaging said patient.

35. A method of imaging comprising the steps of:

administering to a patient a diagnostic imaging agent comprising the compound of embodiment 32, and imaging said patient.

36. A method of imaging comprising the steps of:

administering to a patient a diagnostic imaging agent comprising the compound of embodiment 20 wherein M is an optical label, and imaging said patient.

37. A method of imaging comprising the steps of:

administering to a patient a diagnostic imaging agent comprising the compound of embodiment 33, and imaging said patient.

38. A method for preparing a diagnostic imaging agent comprising the step of adding to an injectable medium a substance comprising the compound of embodiment 20.

39. A method of treating a patient comprising the step of administering to a patient a radiotherapeutic agent comprising the compound of embodiment 20 complexed with a therapeutic radionuclide.

40. A method of preparing a radiotherapeutic agent comprising the step of adding to an injectable medium a substance comprising the compound of embodiment 20.

41. A compound DO3A-monoamide-Gly-(3β,5β,7α,12α)-3-amino-7,12-dihydroxycholan-24-oic acid-BBN(7-14) wherein the BBN(7-14) sequence is SEQ. ID NO: 1.

42. A method of imaging comprising the steps of:

administering to a patient a diagnostic imaging agent comprising DO3A-monoamide-Gly-(3β,5β,7α,12α)-3-amino-7,12-dihydroxycholan-24-oic acid-BBN(7-14) complexed with a diagnostic radionuclide, wherein the BBN (7-14) sequence is SEQ. ID NO: 1, and imaging said patient.

44. A method of treating a patient comprising the step of administering to a patient a radiotherapeutic agent comprising the compound of embodiment 41 complexed with a therapeutic radionuclide.

45. A method of preparing a radiotherapeutic agent comprising the step of adding to an injectable medium a substance comprising the compound of embodiment 41.

51. A compound of the general formula:

$$M\text{-}N\text{-}O\text{-}P\text{-}G$$

wherein

M is an optical label or a metal chelator optionally complexed with a radionuclide;

N is 0, an alpha amino acid, a non-alpha amino acid with a cyclic group or other linking group;

O is an alpha amino acid or a non-alpha amino acid with a cyclic group;

P is 0, an alpha amino acid, a non-alpha amino acid with a cyclic group, or other linking group; and

G is a GRP receptor targeting peptide,

wherein at least one ofN, O or P is a non-alpha amino acid with a cyclic group.

71. A method of synthesizing DO3A-monoamide-Gly-(3β,5β,7α,12α)-3-amino-7,12-dihydroxycholan-24-oic acid-BBN(7-14) wherein the BBN(7-14) sequence is SEQ. ID NO: 1 comprising the steps of:

(a) shaking a solution in a solid phase peptide synthesis vessel, said solution comprising a resin and at least one peptide building ingredient,

(b) flushing said solution, and

(c) washing said resin with DMA,

wherein said at least one peptide building ingredient includes DMA morpholine, (3β,5β,7α,12α)-3-[[(9H-fluoren-9-ylmethoxy)amino]acetyl]amino-7,12-dihydroxycholan-24-oic acid, HOBT, DIC, HATU or mixtures thereof, and wherein each of steps (a), (b) and (c) are repeated until the compound DO3A-monoamide-Gly-(3β,5β,7α,12α)-3-amino-7,12-dihydroxycholan-24-oic acid-BBN(7-14) wherein the BBN(7-14) sequence is SEQ. ID NO: 1 is obtained.

73. A method for labeling DO3A-monoamide-Gly-(3β,5β,7α,12α)-3-amino-7,12-dihydroxycholan-24-oic acid-BBN (7-14) wherein the BBN(7-14) sequence is SEQ. ID NO: 1 comprising the steps of incubating a first solution comprising DO3A-monoamide-Gly-(3β,5β,7α,12α)-3-amino-7,12-dihydroxycholan-24-oic acid-BBN(7-14) wherein the BBN (7-14) sequence is SEQ. ID NO: 1, ammonium acetate, a radioactive metal precursor selected from the group consisting of $^{177}LuCl_3$ or $^{111}InCl_3$, HCI, and adding to said first solution a second solution comprising $Na_2EDTA \cdot 2H_2O$ and water to obtain a radiochemical purity greater than 95%.

75. A method of synthesizing DO3A-monoamide-Gly-(3β,5β,7α,12α)-3-amino-7,12-dihydroxycholan-24-oic acid-BBN(7-14) wherein the BBN(7-14) sequence is SEQ. ID NO: 1 by coupling of individual amino acids, protected amino acids or modified amino acids, with any required additional treatments with reagents or processing steps before or after the coupling steps in solution.

77. A method of synthesizing DO3 A-monoamide-Gly-(3β,5β,7α,12α)-3-amino-7,12-dihydroxycholan-24-oic acid-BBN(7-14) wherein the BBN(7-14) sequence is SEQ. ID NO: 1 by coupling of individual amino acids protected amino acids or modified amino acids, with any required additional treatments with reagents or processing steps before or after the coupling steps in solution.

81. A compound of the general formula:

$$M\text{-}N\text{-}O\text{-}P\text{-}G$$

wherein

M is D03A, optionally complexed with a radionuclide;

N is 0, an alpha or non-alpha amino acid or other linking group;

O is an alpha or non-alpha amino acid; and

P is 0, an alpha or non-alpha amino acid or other linking group,

and G is a GRP receptor targeting peptide,

wherein at least one ofN, O or P is (3β,5β,12α)-3-amino-12-hydroxycholan-24-oic acid.

82. The compound of embodiment 81, wherein the GRP receptor targeting peptide is selected from the group consisting of QWAVGHLM-OH, QRLGNQWAVGHLM-$NH_2$, QRYGNQWAVGHLM-$NH_2$, QKYGNQWAVGHLM-NH2, QWAVGHL-NH-Pentyl, QWSVaHLM-$NH_2$, QWAVGHLL-$NH_2$, QWAV-Bala-HF-Nle-NH2 QWAV-Bala-HF-Nle-$NH_2$, QWAGHFL-$NH_2$, LWAVGSFM-$NH_2$, HWAVGHLM-$NH_2$, LWAVGSFM-$NH_2$, and QWAVGHFM-$NH_2$.

85. A method of phototherapy comprising administering to a patient a compound of embodiment 20 wherein M is an optical label useful in phototherapy.

86. Compound of embodiment 51 selected from the group consisting of:

DOTA-Gly-3-amino-3-deoxycholic acid-Q-W-A-V-a-H-L-M-NH2
DOTA-Gly-3-amino-3-deoxycholic acid-f-Q-W-A-V-Gly-H-L-M-NH2
DOTA-Gly-3-amino-3-deoxycholic acid-f-Q-W-A-V-Gly-H-L-L-NH2
DOTA-Gly-3-amino-3-deoxycholic acid-f-Q-W-A-V-Gly-H-L-NH-pentyl
DOTA-Gly-3-amino-3-deoxycholic acid-y-Q-W-A-V-Bala-H-F-Nle-NH2
DOTA-Gly-3-amino-3-deoxycholic acid-f-Q-W-A-V-Bala-H-F-Nle-NH2
DOTA-Gly-3-amino-3-deoxycholic acid-Q-W-A-V-Gly-H-F-L-NH2
DOTA-Gly-3-amino-3-deoxycholic acid-Q-W-A-V-Gly-NMeHis-L-M-NH2
DOTA-Gly-3-amino-3-deoxycholic acid-L-W-A-V-Gly-S-F-M-NH2
DOTA-Gly-3-amino-3-deoxycholic acid-H-W-A-V-Gly-H-L-M-NH2
DOTA-Gly-3-amino-3-deoxycholic acid-L-W-A-T-Gly-H-F-M-NH2
DOTA-Gly-3-amino-3-deoxycholic acid-Q-W-A-V-Gly-H-F-M-NH2
DOTA-Gly-3-amino-3-deoxycholic acid-Q-R-L-Gly-N-Q-W-A-V-Gly-H-L-M-NH2
DOTA-Gly-3-amino-3-deoxycholic acid-Q-R-Y-Gly-N-Q-W-A-V-Gly-H-L-M-NH2
DOTA-Gly-3-amino-3-deoxycholic acid-Q-K-Y-Gly-N-Q-W-A-V-Gly-H-L-M-NH2
Pglu-Q-Lys(DOTA-Gly-3-amino-3-deoxycholic acid)-L-Gly-N-Q-W-A-V-Gly-H-L-M-NH2 87. The method of any one of embodiments 39, 44, 49 further comprising administering a chemotherapeutic or other therapeutic agent.

## EXAMPLES

The following examples are provided as examples of different methods which can be used to prepare various compounds of the present invention. Within each example, there are compounds identified in single bold capital letter (*e.g.*, **A**, **B**, **C**), which correlate to the same labeled corresponding compounds in the drawings identified.

### General Experimental

A. Definitions of abbreviations used

The following common abbreviations are used throughout this specification: 1,1-dimethylethoxycarbonyl (Boc or Boc);
9-fluorenylmethyloxycarbonyl (Fmoc);
1-hydroxybenozotriazole (HOBT);
N,N'-diisopropylcarbodiimide (DIC);
N-methylpyrrolidinone (NMP);
acetic anhydride ($Ac_2O$);
(4,4-dimethyl-2,6-dioxocyclohex-1-ylidene)-3-methylbutyl (iv-Dde);
trifluoroacetic acid (TFA);
Reagent B (TFA:$H_2O$:phenol:triisopropylsilane, 88:5:5:2);
diisopropylethylamine (DIEA);
O-(1H-benzotriazole-1-yl)-N,N,N',N'-tetramethyluronium
hexafluorophosphate (HBTU);
O-(7-azabenzotriazol-1-yl)-1,1,3,3-tetramethyluronium
hexafluorphosphate (HATU);
N-hydroxysuccinimide (NHS);
solid phase peptide synthesis (SPPS);
dimethylsulfoxide (DMSO);
dichloromethane (DCM);
dimethylformamide (DMF);
dimethylacetamide (DMA);
1,4,7,10-tetraazacyclotetradecane-1,4,7,10-tetraacetic acid (DOTA);
Triisopropylsilane (TIPS);
1,4,7,10-tetraazacyclotetradecane-1,4,7,10-tetraacetic acid (DOTA)
(1R)-1-[1,4,7,10-tetraaza-4,7,10-tris(carboxymethyl)cyclododecyl]
ethane-1,2-dicarboxylic acid (CMDOTA);
fetal bovine serum (FBS);
human serum albumin (HSA);
human prostate cancer cell line (PC3);
isobutylchloroformate (IBCF);
tributyl amine (TBA);
radiochemical purity (RCP); and
high performance liquid chromatography (HPLC).

B. Materials

The Fmoc-protected amino acids used were purchased from Nova-Biochem (San Diego, CA, USA), Advanced Chem Tech (Louisville, KY., USA), Chem-Impex International (Wood Dale Ill., USA), and Multiple Peptide Systems (San Diego, CA., USA). Other chemicals, reagents and adsorbents required for the syntheses were procured from Aldrich Chemical Co. (Milwaukee, WI, USA) and VWR Scientific Products (Bridgeport, NJ., USA). Solvents for peptide synthesis were obtained from Pharmco Co. (Brookfield CT., USA). Columns for HPLC analysis and purification were obtained from Waters Co. (Milford, MA., USA). Experimental details are given below for those that were not commercially available.

C. Instrumentation for Peptide Synthesis

Peptides were prepared using an Advanced ChemTech 496 Ω MOS synthesizer, an Advanced ChemTech 357 FBS synthesizer and/or by manual peptide synthesis. However the protocols for iterative deprotection and chain extension employed were the same for all.

D. Automated synthesis with the Symphony instrument (made by Rainin)

The synthesis was run with Symphony Version 3 supplied by Protein Technologies Inc. Novagel TGR resin, with a substitution of 0.25 mmol/g, was used, and each well contained 0.2 g of the resin (50 μmol). The amino acids were dissolved in NMP and the concentration was 0.25M. A 0.25M solution of HBTU and N-Methylmorpholine in DMF was prepared and used for the coupling. All the couplings were carried out for 2.0 h. The cleavage was done outside the machine by transferring the resin to another reaction vessel and using reagent B as in the manual synthesis

E. Instrumentation Employed for Analysis and Purification

Analytical HPLC was performed using a Shimadzu-LC-10A dual pump gradient analytical LC system employing Shimadzu-ClassVP software version 4.1 for system control, data acquisition, and post run processing. Mass spectra were acquired on a Hewlett-Packard Series 1100 MSD mass spectrometer interfaced with a Hewlett-Packard Series 1100 dual pump gradient HPLC system fitted with an Agilent Technologies 1100 series autosampler fitted for either direct flow injection or injection onto a Waters Associates XTerra MS C18 column (4.6 mm x 50 mm, 5μ particle, 120Å pore). The instrument was driven by a HP Kayak workstation using 'MSD Anyone' software for sample submission and HP Chemstation software for instrument control and data acquisition. In most cases the samples were introduced via direct injection using a 5 μL injection of sample solution at a concentration of 1 mg/mL and analyzed using positive ion electrospray to obtain m/e and m/z (multiply charged) ions for confirmation of structure. $^{1}H$-NMR spectra were obtained on a Varian Innova spectrometer at 500 MHz. $^{13}C$-NMR spectra were obtained on the same instrument at 125.73 MHz. Generally the residual $^{1}H$ absorption, or in the case of $^{13}C$-NMR, the $^{13}C$ absorption of the solvent employed, was used as an internal reference; in other cases tetramethylsilane (μ = 0.00 ppm) was employed. Resonance values are given in μ units. Micro-analysis data was obtained from Quantitative Technologies Inc. Whitehouse NJ. Preparative HPLC was performed on a Shimadzu-LC-8A dual pump gradient preparative HPLC system employing Shimadzu-ClassVP software version 4.3 for system control, data acquisition, fraction collection and post run processing

F. General Procedure for Peptide Synthesis

Rink Amide-Novagel HL resin (0.6 mmol/g) was used as the solid support.

G. Coupling Procedure

In a typical experiment, the first amino acid was loaded onto 0.1 g of the resin (0.06 mmol). The appropriate Fmoc-amino acid in NMP (0.25M solution; 0.960 mL was added to the resin followed by N-hydroxybenzotriazole (0.5M in NMP; 0.48 mL)) and the reaction block (in the case of automated peptide synthesis) or individual reaction vessel (in the case of manual peptide synthesis) was shaken for about 2 min. To the above mixture, diisopropylcarbodiimide (0.5M in NMP; 0.48 mL) was added and the reaction mixture was shaken for 4h at ambient temperature. Then the reaction block or the individual reaction vessel was purged of reactants by application of a positive pressure of dry nitrogen.

H. Washing Procedure

Each well of the reaction block was filled with 1.2 mL of NMP and the block was shaken for 5 min. The solution was drained under positive pressure of nitrogen. This procedure was repeated three times. The same procedure was used, with an appropriate volume of NMP, in the case of manual synthesis using individual vessels.

I. Removal of Fmoc Group

The resin containing the Fmoc-protected amino acid was treated with 1.5 mL of 20% piperidine in DMF (v/v) and the reaction block or individual manual synthesis vessel was shaken for 15 min. The solution was drained from the resin. This procedure was repeated once and the resin was washed employing the washing procedure described above.

J. Final coupling of ligands (DOTA and CMDOTA)

The N-terminal amino group of the resin bound peptide linker construct was deblocked and the resin was washed. A 0.25M solution of the desired ligand and HBTU in NMP was made, and was treated with a two-fold equivalency of DIEA. The resulting solution of activated ligand was added to the the resin (1.972 mL; 0.48 mmol) and the reaction mixture was shaken at ambient temperature for 24-30 h. The solution was drained and the resin was washed. The final wash of the resin was conducted with 1.5 mL dichloromethane (3X).

K. Deprotection and purification of the final peptide

A solution of reagent B (2 mL; 88:5:5:2 - TFA:Phenol:Water:TIPS) was added to the resin and the reaction block or individual vessel was shaken for 4.5h at ambient temperature. The resulting solution containing the deprotected peptide was drained into a vial. This procedure was repeated two more times with 1 mL of reagent B. The combined filtrate was concentrated under reduced pressure using a Genevac HT-12 series II centrifugal concentrator. The residue in each vial was then triturated with 2 mL of $Et_2O$ and the supernatant was decanted. This procedure was repeated twice to provide the peptides as colorless solids. The crude peptides were dissolved in water/acetonitrile and purified using either a Waters XTerra MS C18 preparative HPLC column (50 mm x 19 mm, 5 micron particle size, 120Å pore size) or a Waters-YMC C18 ODS column (250 mm x 30 mm i.d., 10 micron particle size. 120 Å pore size). The fractions with the products were collected and analyzed by HPLC. The fractions with >95% purity were pooled and the peptides isolated by lyophilization..
Conditions for Preparative HPLC (Waters XTerra Column):

Elution rate: 50 mL/min
Detection: UV, λ = 220 nm
Eluent A: 0.1% aq. TFA ; Solvent B: Acetonitrile (0.1% TFA).
Conditions for HPLC Analysis:
Column: Waters XTerra (Waters Co..; 4.6 x 50 mm; MS C18; 5 micron particle, 120 Å pore).
Elution rate: 3 mL/min; Detection: UV, λ = 220 nm.
Eluent A:0.1% aq. TFA ; Solvent B: Acetonitrile (0.1% TFA).

**Example I - Figures 1A-B**

**Synthesis of L62**

Summary: As shown in Figures 1A-B, L62 was prepared using the following steps: Hydrolysis of (3β,5β)-3-aminocholan-24-oic acid methyl ester **A** with NaOH gave the corresponding acid **B**, which was then reacted with Fmoc-Cl to give intermediate **C**. Rink amide resin functionalised with the octapeptide Gln-Trp-Ala-Val-Gly-His-Leu-Met-$NH_2$ (BBN[7-14] [SEQ ID NO:1]) was sequentially reacted with **C**, Fmoc-glycine and DOTA tri-*t*-butyl ester. After cleavage and deprotection with reagent B the crude was purified by preparative HPLC to give L62. Overall yield: 2.5%. More details are provided below:

A. Rink amide resin functionalised with bombesin[7-14] (*A*)
In a solid phase peptide synthesis vessel (see enclosure No. 1) Fmoc-aminoacid (24 mmol), *N*-hydroxybenzotriazole (HOBt) (3.67 g; 24 mmol), and *N,N'*-diisopropylcarbodiimide (DIC) (3.75 mL; 24 mmol) were added sequentially to a suspension of Rink amide NovaGel™ resin (10 g; 6.0 mmol) **A** in DMF (45 mL). The mixture was shaken for 3 h at room temperature using a bench top shaker, then the solution was emptied and the resin was washed with DMF (5 x 45 mL). The resin was shaken with 25% piperidine in DMF (45 mL) for 4 min, the solution was emptied and fresh 25% piperidine in DMF (45 mL) was added. The suspension was shaken for 10 min, then the solution was emptied and the resin was washed with DMF (5 x 45 mL).
This procedure was applied sequentially for the following amino acids: *N*-α-Fmoc-L-methionine, *N*-α-Fmoc-L-leucine, *N*-α-Fmoc-N-im-trityl-L-histidine, *N*-α-Fmoc-glycine, *N*-α-Fmoc-L-valine, *N*-α-Fmoc-L-alanine, *N*-α-Fmoc-*N*-in-Boc-L-tryptophan.
In the last coupling reaction *N*-α-Fmoc-*N*-γ-trityl-L-glutamine (14.6 g; 24 mmol), HOBt (3.67 g; 24 mmol), and DIC (3.75 mL; 24 mmol) were added to the resin in DMF (45 mL). The mixture was shaken for 3 h at room temperature, the solution was emptied and the resin was washed with DMF (5 x 45 mL), $CH_2Cl_2$ (5 x 45 mL) and vacuum dried.
B. Preparation of intermediates **B** and **C** (FIG 1A):

1. Synthesis of (3β,5β)-3-Aminocholan-24-oic acid (**B**)
A 1 M solution of NaOH (16.6 mL; 16.6 mmol) was added dropwise to a solution of (3β,5β)-3-aminocholan-24-oic acid methyl ester (5.0 g; 12.8 mmol) in MeOH (65 mL) at 45 ˚C. After 3 h stirring at 45 ˚C, the mixture was concentrated to 25 mL and $H_2O$ (40 mL) and 1 M HCl (22 mL) were added. The precipitated solid was filtered, washed with $H_2O$ (2 x 50 mL) and vacuum dried to give **B** as a white solid (5.0 g; 13.3 mmol). Yield 80%.
2. Synthesis of (3β,5β)-3-(9*H*-Fluoren-9-ylmethoxy)aminocholan-24-oic acid (**C**)
A solution of 9-fluorenylmethoxycarbonyl chloride (0.76 g; 2.93 mmol) in 1,4-dioxane (9 mL) was added dropwise to a suspension of (3β,5β)-3-aminocholan-24-oic acid **B** (1.0 g; 2.66 mmol) in 10% aq. $Na_2CO_3$ (16 mL) and 1,4-dioxane (9 mL) stirred at 0 ˚C. After 6 h stirring at room temperature $H_2O$ (90 mL) was added, the aqueous

phase washed with $Et_2O$ (2 x 90 mL) and then 2 M HCl (15 mL) was added (final pH: 1.5). The aqueous phase was extracted with EtOAc (2 x 100 mL), the organic phase dried over $Na_2SO_4$ and evaporated. The crude was purified by flash chromatography to give C as a white solid (1.2 g; 2.0 mmol). Yield 69%.

C. Synthesis of **L62** (*N*-[(3β,5β)-3-[[[[[4,7,10-Tris(carboxymethyl)-1,4,7,10-tetraazacyclododec-1-yl]acetyl]amino] acetyl]amino ]-cholan-24-yl]-L-glutaminyl-L-tryptophyl-L-alanyl-L-valyl-glycyl-L-histidyl-L-leucyl-L-methioninamide) (FIG 1B):

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was shaken for 20 min then the solution was emptied and the resin washed with DMA (5 x 7 mL). (3β,5β)-3-(9*H*-Fluoren-9-ylmethoxy)aminocholan-24-oic acid **C** (0.72 g; 1.2 mmol), *N*-hydroxybenzotriazole (HOBT) (0.18 g; 1.2 mmol), *N,N'*-diisopropylcarbodiimide (DIC) (0.19 mL; 1.2 mmol) and DMA (7 mL) were added to the resin, the mixture shaken for 24 h at room temperature, and the solution was emptied and the resin washed with DMA (5 x 7 mL). The resin was then shaken with 50% morpholine in DMA (7 mL) for 10 min, the solution was emptied, fresh 50% morpholine in DMA (7 mL) was added and the mixture shaken for another 20 min. The solution was emptied and the resin washed with DMA (5 x 7 mL). *N*-α-Fmoc-glycine (0.79 g; 1.2 mmol), HOBT (0.18 g; 1.2 mmol), DIC (0.19 mL: 1.2 mmol) and DMA (7 mL) were added to the resin. The mixture was shaken for 3 h at room temperature, the solution was emptied and the resin washed with DMA (5 x 7 mL). The resin was then shaken with 50% morpholine in DMA (7 mL) for 10 min, the solution was emptied, fresh 50% morpholine in DMA (7 mL) was added and the mixture shaken for another 20 min. The solution was emptied and the resin washed with DMA (5 x 7 mL) followed by addition of 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), DIEA (0.40 mL; 2.4 mmol) and DMA (7 mL) to the resin. The mixture was shaken for 24 h at room temperature, the solution was emptied and the resin washed with DMA (5 x 7 mL), $CH_2Cl_2$ (5 x 7 mL) and vacuum dried. The resin was shaken in a flask with reagent B (25 mL) for 4.5 h. The resin was filtered and the solution was evaporated under reduced pressure to afford an oily crude which was triturated with $Et_2O$ (20 mL) gave a precipitate. The precipitate was collected by centrifugation and washed with $Et_2O$ (3 x 20 mL), then analysed by HPLC and purified by preparative HPLC. The fractions containing the product were lyophilised to give L62 (6.6 mg; $3.8 \times 10^{-3}$ mmol) as a white solid. Yield 4.5%.

**Example II - Figures 2A-F**

**Synthesis of L70, L73, L74, L115 and L116**

Summary: The products were obtained by coupling of the octapeptide Gln-Trp-Ala-Val-Gly-His-Leu-Met-$NH_2$ (BBN[7-14] [SEQ ID NO:1]) (with appropriate side chain protection) on the Rink amide resin with different linkers, followed by functionalization with DOTA tri-*t*-butyl ester. After cleavage and deprotection with reagent B the final products were purified by preparative HPLC. Overall yields 3-9%.

A. Synthesis of **L70 (FIG. 2A)**:

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin washed with DMA (5 x 7 mL). Fmoc-4-aminobenzoic acid (0.43 g; 1.2 mmol), HOBT (0.18 g; 1.2 mmol), DIC (0.19 mL; 1.2 mmol) and DMA (7 mL) were added to the resin, the mixture shaken for 3 h at room temperature, the solution was emptied and the resin washed with DMA (5 x 7 mL). The resin was then shaken with 50% morpholine in DMA (7 mL) for 10 min, the solution was emptied, fresh 50% morpholine in DMA (7 mL) was added and the mixture was shaken for 20 min. The solution was emptied and the resin washed with DMA (5 x 7 mL). Fmoc-glycine (0.36 g; 1.2 mmol) HATU (0.46 g; 1.2 mmol) and DIEA (0.40 mL; 2.4 mmol) were stirred for 15 min in DMA (7 mL) then the solution was added to the resin, the mixture shaken for 2 h at room temperature, the solution was emptied and the resin washed with DMA (5 x 7 mL). The resin was then shaken with 50% morpholine in DMA (7 mL) for 10 min, the solution was emptied, fresh 50% morpholine in DMA (7 mL) was added and the mixture shaken for 20 min. The solution was emptied and the resin washed with DMA (5 x 7 mL). 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, the solution was emptied and the resin washed with DMA (5 x 7 mL), $CH_2Cl_2$ (5 x 7 mL) and vacuum dried. The resin was shaken in a flask with reagent B (25 mL) for 4 h. The resin was filtered and the filtrate solution was evaporated under reduced pressure to afford an oily crude that was triturated with $Et_2O$ (5 mL). The precipitate was collected by centrifugation and washed with $Et_2O$ (5 x 5 mL), then analysed by HPLC and purified by preparative HPLC. The fractions containing

the product were lyophilised to give **L70** as a white fluffy solid (6.8 mg; 0.005 mmol). Yield 3%.

B. Synthesis of **L73**, **L115** and **L116 (FIGS. 2B - 2E)**:

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin washed with DMA (5 x 7 mL). Fmoc-linker-OH (1.2 mmol), HOBT (0.18 g; 1.2 mmol), DIC (0.19 mL; 1.2 mmol) and DMA (7 mL) were added to the resin, the mixture was shaken for 3 h at room temperature, the solution was emptied and the resin was washed with DMA (5 x 7 mL). The resin was shaken with 50% morpholine in DMA (7 mL) for 10 min, the solution was emptied, fresh 50% morpholine in DMA (7 mL) was added and the mixture was shaken for 20 min. The solution was emptied and the resin washed with DMA (5 x 7 mL). 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, the solution was emptied and the resin washed with DMA (5 x 7 mL), $CH_2Cl_2$ (5 x 7 mL) and vacuum dried. The resin was shaken in a flask with reagent B (25 mL) for 4 h. The resin was filtered and the solution was evaporated under reduced pressure to afford an oily crude that was triturated with $Et_2O$ (5 mL). The precipitate was collected by centrifugation and washed with $Et_2O$ (5 x 5 mL), then analysed by HPLC and purified by preparative HPLC. The fractions containing the product were lyophilised.

C. Synthesis of **L74 (FIG. 2F)**:

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin was washed with DMA (5 x 7 mL). Fmoc-isonipecotic acid (0.42 g; 1.2 mmol), HOBT (0.18 g; 1.2 mmol), DIC (0.19 mL; 1.2 mmol) and DMA (7 mL) were added to the resin, the mixture was shaken for 3 h at room temperature, the solution was emptied and the resin was washed with DMA (5 x 7 mL). The resin was shaken with 50% morpholine in DMA (7 mL) for 10 min, the solution was emptied, fresh 50% morpholine in DMA (7 mL) was added and the mixture was shaken for 20 min. The solution was emptied and the resin was washed with DMA (5 x 7 mL). Fmoc-glycine (0.36 g; 1.2 mmol), HOBT (0.18 g; 1.2 mmol), DIC (0.19 mL; 1.2 mmol) and DMA (7 mL) were added to the resin, the mixture was shaken for 3 h at room temperature, the solution was emptied and the resin washed with DMA (5 x 7 mL). The resin was then shaken with 50% morpholine in DMA (7 mL) for 10 min, the solution was emptied, fresh 50% morpholine in DMA (7 mL) was added and the mixture shaken for 20 minutes. The solution was emptied and the resin was washed with DMA (5 x 7 mL). 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin.

The mixture was shaken for 24 h at room temperature, the solution was emptied and the resin was washed with DMA (5 x 7 mL), $CH_2Cl_2$ (5 x 7 mL) and vacuum dried. The resin was shaken in a flask with reagent B (25 mL) for 4 h. The resin was filtered and the solution was evaporated under reduced pressure to afford an oily crude that was triturated with $Et_2O$ (5 mL). The precipitate was collected by centrifugation and washed with $Et_2O$ (5 x 5 mL), then analysed by HPLC and purified by HPPLC. The fractions containing the product were lyophilised to give **L74** as a white fluffy solid (18.0 mg; 0.012 mmol). Yield 8%.

**Example III - Figures 3A-E**

**Synthesis of L67**

Summary: Hydrolysis of (3β,5β)-3-amino-12-oxocholan-24-oic acid methyl ester A with NaOH gave the corresponding acid B, which was then reacted with Fmoc-Glycine to give intermediate C. Rink amide resin functionalised with the octapeptide Gln-Trp-Ala-Val-Gly-His-Leu-Met-$NH_2$ (BBN[7-14] [SEQ ID NO:1]) was sequentially reacted with C, and DOTA tri-t-butyl ester. After cleavage and deprotection with reagent B the crude was purified by preparative HPLC to give L67. Overall yield: 5.2%.

A. Synthesis (3β,5β)-3-Amino-12-oxocholan-24-oic acid, (**B**) **(FIG 3A)**

A 1 M solution of NaOH (6.6 mL; 6.6 mmol) was added dropwise to a solution of (3β,5β)-3-amino-12-oxocholan-24-oic acid methyl ester **A** (2.1 g; 5.1 mmol) in MeOH (15 mL) at 45 ˚C. After 3 h stirring at 45 ˚C, the mixture was concentrated to 25 mL then $H_2O$ (25 mL) and 1 M HC1 (8 mL) were added. The precipitated solid was filtered, washed with $H_2O$ (2 x 30 mL) and vacuum dried to give **B** as a white solid (1.7 g; 4.4 mmol). Yield 88%.

B. Synthesis of (3β,5β)-3-[[(9*H*-Fluoren-9-ylmethoxy)amino]acetyl]amino-12-oxocholan-24-oic acid (**C**) (FIG 3A)

Tributylamine (0.7 mL; 3.1 mmol) was added dropwise to a solution of N-α-Fmoc-glycine (0.9 g; 3.1 mmol) in THF (25 mL) stirred at 0 ˚C. Isobutyl chloroformate (0.4 mL; 3.1 mmol) was subsequently added and, after 10 min, a

suspension of tributylamine (0.6 mL; 2.6 mmol) and (3β,5β)-3-amino-12-oxocholan-24-oic acid **B** (1.0 g; 2.6 mmol) in DMF (30 mL) was added dropwise, over 1 h, into the cooled solution. The mixture was allowed to warm up and after 6 h the solution was concentrated to 40 mL, then $H_2O$ (50 mL) and 1 N HCl (10 mL) were added (final pH: 1.5). The precipitated solid was filtered, washed with $H_2O$ (2 x 50 mL), vacuum dried and purified by flash chromatography to give **C** as a white solid (1.1 g; 1.7 mmol). Yield 66%.

C. Synthesis of **L67** (*N*-[(3β,5β)-3-[[[[[4,7,10-Tris(carboxymethyl)-1,4,7,10-tetraazacyclododec-1-yl]acetyl]amino]acetyl]amino]-12,24-dioxocholan-24-yl]-L-glutaminyl-L-tryptophyl-L-alanyl-L-valyl-glycyl-L-histidyl-L-leucyl-L-methioninamide) (FIG 3B and 3E).

Resin **D** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin was washed with DMA (5 x 7 mL). (3β,5β)-3-[[(9*H*-Fluoren-9-ylmethoxy)amino]acetyl]amino]-12-oxocholan-24-oic acid **C** (0.80 g; 1.2 mmol), HOBT (0.18 g; 1.2 mmol), DIC (0.19 mL; 1.2 mmol) and DMA (7 mL) were added to the resin, the mixture was shaken for 24 h at room temperature, the solution was emptied and the resin was washed with DMA (5 x 7 mL). The resin was shaken with 50% morpholine in DMA (7 mL) for 10 min, the solution was emptied, fresh 50% morpholine in DMA (7 mL) was added and the mixture was shaken for 20 min. The solution was emptied and the resin was washed with DMA (5 x 7 mL). 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, the solution was emptied and the resin was washed with DMA (5 x 7 mL), $CH_2Cl_2$ (5 x 7 mL) and vacuum dried. The resin was shaken in a flask with reagent B (25 mL) for 4.5 h. The resin was filtered and the solution was evaporated under reduced pressure to afford an oily crude that was triturated with $Et_2O$ (20 mL).

**Example IV - Figures 4A-H**

**Synthesis of L63 and L64**

Summary: Hydrolysis of (3β,5β,7α,12α)-3-amino-7,12-dihydroxycholan-24-oic acid methyl ester 1b with NaOH gave the intermediate **2b**, which was then reacted with Fmoc-glycine to give **3b**. Rink amide resin functionalised with the octapeptide Gln-Trp-Ala-Val-Gly-His-Leu-Met-$NH_2$ (BBN[7-14] [SEQ ID NO:1]) was reacted with 3b and then with DOTA tri-t-butyl ester. After cleavage and deprotection with reagent B the crude was purified by preparative HPLC to give **L64**. The same procedure was repeated starting from intermediate **2a**, already available, to give **L63**. Overall yields: 9 and 4%, respectively.

A. Synthesis of (3β,5β,7α,12α)-3-Amino-7,12-dihydroxycholan-24-oic acid, (**2b**) FIG. 4A)

A 1 M solution of NaOH (130 mL; 0.13 mol) was added dropwise to a solution of (3β,5β,7α,12α)-3-amino-7,12-dihydroxycholan-24-oic acid methyl ester **1b** (42.1 g; 0.10 mol) in MeOH (300 mL) heated at 45 ˚C. After 3 h stirring at 45˚C, the mixture was concentrated to 150 mL and $H_2O$ (350 mL) was added. After extraction with $CH_2Cl_2$ (2 x 100 mL) the aqueous solution was concentrated to 200 mL and 1 M HC1 (150 mL) was added. The precipitated solid was filtered, washed with $H_2O$ (2 x 100 mL) and vacuum dried to give **2b** as a white solid (34.8 g; 0.08 mol). Yield 80%.

B. Synthesis of (3β,5β,12α)-3-[[(9H-Fluoren-9-ylmethoxy)amino]acetyl]amino-12-hydroxycholan-24-oic acid, (**3a**) (FIG 4A)

Tributylamine (4.8 mL; 20.2 mmol) was added dropwise to a solution of N-α-Fmoc-glycine (6.0 g; 20.2 mmol) in THF (120 mL) stirred at 0˚C. Isobutyl chloroformate (2.6 mL; 20.2 mmol) was subsequently added and, after 10 min, a suspension of tributylamine (3.9 mL; 16.8 mmol) and (3β,5β,12α)-3-amino-12-hydroxycholan-24-oic acid **2a** (6.6 g; 16.8 mmol) in DMF (120 mL) was added dropwise, over 1 h, into the cooled solution. The mixture was allowed to warm up and after 6 h the solution was concentrated to 150 mL, then $H_2O$ (250 mL) and 1 N HCl (40 mL) were added (final pH: 1.5). The precipitated solid was filtered, washed with $H_2O$ (2 x 100 mL), vacuum dried and purified by flash chromatography to give **3a** as a white solid (3.5 g; 5.2 mmol). Yield 31%.

C. Synthesis of (3β,5β,7α,12α)-3-[[(9H-Fluoren-9-ylmethoxy)amino]acetyl]amino-7,12-dihydroxycholan-24-oic acid, (**3b**) (FIG 4A)

Tributylamine (3.2 mL; 13.5 mmol) was added dropwise to a solution of N-α-Fmoc-glycine (4.0 g; 13.5 mmol) in THF (80 mL) stirred at 0˚C. Isobutyl chloroformate (1.7 mL; 13.5 mmol) was subsequently added and, after 10 min, a suspension of tributylamine (2.6 mL; 11.2 mmol) and (3β,5β,7α,12α)-3-amino-7,12-dihydroxycholan-24-oic acid **3a** (4.5 g; 11.2 mmol) in DMF (80 mL) was added dropwise, over 1 h, into the cooled solution. The mixture was allowed to warm up and after 6 h the solution was concentrated to 120 mL, then $H_2O$ (180 mL) and 1 N HCl (30 mL) were added (final pH: 1.5). The precipitated solid was filtered, washed with $H_2O$ (2 x 100 mL), vacuum dried

and purified by flash chromatography to give **3a** as a white solid (1.9 g; 2.8 mmol). Yield 25%.
In an alternative method, (3β,5β,7α,12α)-3-[[(9H-Fluoren-9-ylmethoxy)amino]acetyl]amino-7,12-dihydroxycholan-24-oic acid, (**3b**) can be prepared as follows:

N-Hydroxysuccinimide (1.70 g, 14.77 mmol) and DIC (1.87 g, 14.77 mmol) were added sequentially to a stirred solution of Fmoc-Gly-OH (4.0 g, 13.45 mmol) in dichloromethane (15 mL); the resulting mixture was stirred at room temperature for 4 h. The N,N'-diisopropylurea formed was removed by filtration and the solid was washed with ether (20 mL). The volatiles were removed and the solid Fmoc-Gly-succinimidyl ester formed was washed with ether (3 x 20 mL). Fmoc-Gly-succinimidyl ester was then redissolved in dry DMF (15 mL) and 3-aminodeoxycholic acid (5.21 g, 12.78 mmol) was added to the clear solution. The reaction mixture was stirred at room temperature for 4 h, water (200 mL) was added and the precipitated solid was filtered, washed with water, dried and purified by silica gel chromatography (TLC (silica): ($R_f$: 0.50, silica gel, $CH_2Cl_2/CH_3OH$, 9:1) (eluant: $CH_2Cl_2/CH_3OH$ (9:1)) to give (3β,5β,7α,12α)-3-[[(9H-Fluoren-9-ylmethoxy)amino]acetyl]amino-7,12-dihydroxycholan-24-oic acid as a colorless solid. Yield: 7.46 g (85 %).

D. <u>Synthesis of **L63** (*N*-[(3β,5β,12α)-3-[[[[4,7,10-Tris(carboxymethyl)-1,4,7,10-tetraazacyclododec-1-yl] acetyl] amino]acetyl]amino]-12-hydroxy-24-oxocholan-24-yl]-L-glutaminyl-L-tryptophyl-L-alanyl-L-valyl-glycyl-L-histidyl-L-leucyl-L-methioninamide) (FIG. 4B)</u>

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin washed with DMA (5 x 7 mL). (3β,5β,12α)-3-[[(9H-Fluoren-9-ylmethoxy)amino]acetyl]amino-12-hydroxycholan-24-oic acid **3a** (0.82 g; 1.2 mmol), HOBT (0.18 g; 1.2 mmol), DIC (0.19 mL; 1.2 mmol) and DMA (7 mL) were added to the resin, the mixture was shaken for 24 h at room temperature, the solution was emptied and the resin was washed with DMA (5 x 7 mL). The resin was then shaken with 50% morpholine in DMA (7 mL) for 10 min, the solution was emptied, fresh 50% morpholine in DMA (7 mL) was added and the mixture was shaken for 20 min. The solution was emptied and the resin washed with DMA (5 x 7 mL). 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, the solution was emptied and the resin washed with DMA (5 x 7 mL), $CH_2Cl_2$ (5 x 7 mL) and vacuum dried. The resin was shaken in a flask with reagent B (25 mL) for 4 h. The resin was filtered and the solution was evaporated under reduced pressure to afford an oily crude that after treatment with $Et_2O$ (5 mL) gave a precipitate. The precipitate was collected by centrifugation and washed with $Et_2O$ (5 x 5 mL), then analysed and purified by HPLC. The fractions containing the product were lyophilised to give **L63** as a white fluffy solid (12.8 mg; 0.0073 mmol). Yield 9%.

E. <u>Synthesis of **L64** (*N*-[(3β,5β,7α,12α)-3-[[[[4,7,10-Tris(carboxymethyl)-1,4,7,10-tetraazacyclododec-1-yl] acetyl] amino]acetyl]amino]-7,12-dihydroxy-24-oxocholan-24-yl]-L-glutaminyl-L-tryptophyl-L-alanyl-L-valyl-glycyl-L-histidyl-L-leucyl-L-methioninamide) (FIG 4C)</u>

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min, the solution was emptied and the resin was washed with DMA (5 x 7 mL). (3β,5β,7α,12α)-3-[[(9H-Fluoren-9-ylmethoxy)amino]acetyl]amino-7,12-dihydroxycholan-24-oic acid **3b** (0.81 g; 1.2 mmol), HOBT (0.18 g; 1.2 mmol), DIC (0.19 mL; 1.2 mmol) and DMA (7 mL) were added to the resin, the mixture was shaken for 24 h at room temperature, the solution was emptied and the resin was washed with DMA (5 x 7 mL). The resin was shaken with 50% morpholine in DMA (7 mL) for 10 min, the solution was emptied, fresh 50% morpholine in DMA (7 mL) was added and the mixture was shaken for 20 min. The solution was emptied and the resin was washed with DMA (5 x 7 mL). 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, the solution was emptied and the resin washed with DMA (5 x 7 mL), $CH_2Cl_2$ (5 x 7 mL) and vacuum dried. The resin was shaken in a flask with reagent B (25 mL) for 4 h. The resin was filtered and the solution was evaporated under reduced pressure to afford an oily crude that was triturated with $Et_2O$ (5 mL). The precipitate was collected by centrifugation and washed with $Et_2O$ (5 x 5 mL). Then it was dissolved in $H_2O$ (20 mL), and $Na_2CO_3$ (0.10 g; 0.70 mmol) was added; the resulting mixture was stirred 4 h at room temperature. This solution was purified by HPLC, the fractions containing the product lyophilised to give **L64** as a white fluffy solid (3.6 mg; 0.0021 mmol). Yield 4%.

**Example V - Figures 5A-E**

**Synthesis of L71 and L72**

Summary: The products were obtained in two steps. The first step was the solid phase synthesis of the octapeptide Gln-Trp-Ala-Val-Gly-His-Leu-Met-$NH_2$ (BBN[7-14] [SEQ ID NO:1]) (with appropriate side chain protecting groups) on the Rink amide resin discussed supra. The second step was the coupling with different linkers followed by functionalization with DOTA tri-*t*-butyl ester. After cleavage and deprotection with reagent B the final products were purified by preparative HPLC. Overall yields 3-9%.

A. Bombesin [7-14] functionalisation and cleavage procedure (FIGS 5A and 5D)
The resin **B** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin was washed with DMA (5 x 7 mL). The Fmoc-linker-OH (1.2 mmol), HOBT (0.18 g; 1.2 mmol), DIC (0.19 mL; 1.2 mmol) and DMA (7 mL) were added to the resin. The mixture was shaken for 3 h at room temperature, the solution was emptied and the resin washed with DMA (5 x 7 mL). The resin was then shaken with 50% morpholine in DMA (7 mL) for 10 min, the solution was emptied, fresh 50% morpholine in DMA (7 mL) was added and the mixture was shaken for 20 min. The solution was emptied and the resin was washed with DMA (5 x 7 mL). 1,4,7,10-Tetraazacyclododecane-1,4,7,10-tetraacetic acid tris(1,1-dimethylethyl) ester adduct with NaCl **C** (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature. The solution was emptied and the resin washed with DMA (5 x 7 mL), $CH_2Cl_2$ (5 x 7 mL) and vacuum dried. The resin was shaken in a flask with reagent B (25 mL) for 4 h. The resin was filtered and the filtrate was evaporated under reduced pressure to afford an oily crude that was triturated with ether (5 mL). The precipitate was collected by centrifugation and washed with ether (5 x 5 mL), then analyzed by analytical HPLC and purified by preparative HPLC. The fractions containing the product were lyophilized.
B. Products

1. **L71** (4-[[[[4,7,10-Tris(carboxymethyl)-1,4,7,10-tetraazacyclododec-1-yl]acetyl]amino]methyl]benzoyl-L-glutaminyl-L-tryptophyl-L-alanyl-L-valyl-glycyl-L-histidyl-L-leucyl-L-methioninamide)
The product was obtained as a white fluffy solid (7.3 mg; 0.005 mmol). Yield 7.5%.
2. **L72** (*Trans*-4-[[[[4,7,10-tris(carboxymethyl)-1,4,7,10-tetraazacyclododec-1-yl]acetyl]amino] methyl]cyclohexylcarbonyl-L-glutaminyl-L-tryptophyl-L-alanyl-L-valyl-glycil-L-histidyl-L-leucyl-L-methioninamide)
The product was obtained as a white fluffy solid (7.0 mg; 0.005 mmol). Yield 4.8%.

C. *Trans*-4-[[[(9H-fluoren-9-ylmethoxy)carbonyl]amino]methyl]cyclohexanecarboxylic acid, **(D)** (FIG. 5E)
A solution of *N*-(9-fluorenylmethoxycarbonyloxy)succinimide (4.4 g; 14.0 mmol) in 1,4-dioxane (40 mL) was added dropwise to a solution of *trans*-4-(aminomethyl)cyclohexanecarboxylic acid (2.0 g; 12.7 mmol) in 10% $Na_2CO_3$ (30 mL) cooled to 0 ˚C. The mixture was then allowed to warm to ambient temperature and after 1 h stirring at room temperature was treated with 1 N HCl (32 mL) until the final pH was 2. The resulting solution was extracted with n-BuOH (100 mL); the volatiles were removed and the crude residue was purified by flash chromatography to give **D** as a white solid (1.6 g; 4.2 mmol). Yield 33%.

**Example VI - Figures 6A-F**

**Synthesis of L75 and L76**

Summary: The two products were obtained by coupling of the octapeptide Gln-Trp-Ala-Val-Gly-His-Leu-Met-NH2 (BBN[7-14] [SEQ ID NO:1]) (A) on the Rink amide resin with the two linkers E and H, followed by functionalization with DOTA tri-t-butyl ester. After cleavage and deprotection with reagent B the final products were purified by preparative HPLC. Overall yields: 8.5% (L75) and 5.6% (L76).

A. 2-[(1,3-Dihydro-1,3-dioxo-2*H*-isoindol-2-yl)methyl]benzoic acid, **(C)** (FIG. 6A) The product was synthesized following the procedure reported in the literature (Bornstein, J; Drummon, P. E.; Bedell, S. F. Org Synth. Coll. Vol. IV 1963, 810-812).
B. 2-(Aminomethyl)benzoic acid, **(D)** (FIG 6A)
A 40% solution of methylamine (6.14 mL; 7.1mmol) was added to 2-[(1,3-dihydro-1,3-dioxo-2*H*-isoindol-2-yl)methyl] benzoic acid **C** (2 g; 7.1 mmol) and then EtOH (30 mL) was added. After 5 minutes stirring at room temperature the

reaction mixture was heated at 50 ˚C. After 2.5 h, the mixture was cooled and the solvent was evaporated under reduced pressure. The crude product was suspended in 50 mL of absolute ethanol and the suspension was stirred at room temperature for 1 h. The solid was filtered and washed with EtOH to afford 2-(aminomethyl)benzoic acid **D** (0.87 g; 5.8 mmol). Yield 81%.

C. 2-[[[9*H*-Fluoren-9-ylmethoxy)carbonyl]amino]methyl]benzoic acid, **(E)** (FIG 6A) The product was synthesized following the procedure reported in the literature (Sun, J-H.; Deneker, W. F. Synth. Commun. 1998, 28, 4525-4530).

D. 4-(Aminomethyl)-3-nitrobenzoic acid, **(G)** (FIG 6B)

4-(Bromomethyl)-3-nitrobenzoic acid (3.2 g; 12.3 mmol) was dissolved in 8% $NH_3$ in EtOH (300 mL) and the resulting solution was stirred at room temperature. After 22 h the solution was evaporated and the residue suspended in $H_2O$ (70 mL). The suspension was stirred for 15 min and filtered. The collected solid was suspended in $H_2O$ (40 mL) and dissolved by the addition of few drops of 25% aq. $NH_4OH$ (pH 12), then the pH of the solution was adjusted to 6 by addition of 6 N HCl. The precipitated solid was filtered, and washed sequentially with MeOH (3 x 5 mL), and $Et_2O$ (10 mL) and was vacuum dried (1.3 kPa; $P_2O_5$) to give 4-(aminomethyl)-3-nitrobenzoic acid as a pale brown solid (1.65 g; 8.4 mmol). Yield 68%.

E. 4-[[[9*H*-Fluoren-9-ylmethoxy)carbonyl]amino]methyl]-3-nitrobenzoic acid, **(H)** (FIG. 6B)

4-(Aminomethyl)-3-nitrobenzoic acid **G** (0.8 g; 4 mmol) was dissolved in 10% aq. $Na_2CO_3$ (25 mL) and 1,4-dioxane (10 mL) and the solution was cooled to 0 ˚C. A solution of 9-fluorenylmethyl chloroformate (Fmoc-Cl) (1.06 g; 4 mmol) in 1,4-dioxane (10 mL) was added dropwise for 20 min. After 2 h at 0-5 ˚C and 1 h at 10 ˚C the reaction mixture was filtered and the solution was acidified to pH 5 by addition of 1 N HCl. The precipitate was filtered, washed with $H_2O$ (2 x 2 mL) dried under vacuum (1.3 kPa; $P_2O_5$) to give 4-[[[9*H*-fluoren-9-ylmethoxy)carbonyl]amino]methyl]-3-nitrobenzoic acid as a white solid (1.6 g; 3.7 mmol). Yield 92%.

F. **L75** (*N*-[2-[[[[4,7,10-Tris(carboxymethyl)-1,4,7,10-tetraazacydododec-1-yl]acetyl]amino]methyl]benzoyl]-L-glutaminyl-L-tryptophyl-L-alanyl-L-valyl-glycyl-L-histidyl-L-leucyl-L-methioninamide) (FIG. 6C)

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin washed with DMA (5 x 7 mL). 2-[[[9*H*-Fluoren-9-ylmethoxy)carbonyl]amino]methyl]benzoic acid, **E** (0.45 g; 1.2 mmol), *N*-hydroxybenzotriazole (HOBT) (0.18 g; 1.2 mmol), *N*,*N*'-diisopropylcarbodiimide (DIC)

(0.19 mL; 1.2 mmol) and DMA (7 mL) were added to the resin, the mixture shaken for 24 h at room temperature, the solution was emptied and the resin was washed with DMA (5 x 7 mL). The resin was then shaken with 50% morpholine in DMA (7 mL) for 10 min, the solution was emptied, fresh 50% morpholine in DMA (7 mL) was added and the mixture shaken for 20 min. The solution was emptied and the resin washed with DMA (5 x 7 mL). 1,4,7,10-Tetraazacyclododecane-1,4,7,10-tetraacetic acid tris(1,1-dimethylethyl) ester adduct with NaCl (DOTA tri-t-butyl ester) (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, the solution was emptied and the resin was washed with DMA (5 x 7 mL), $CH_2Cl_2$ (5 x 7 mL) and vacuum dried. The resin was shaken in a flask with reagent B (25 mL) for 4.5 h. The resin was filtered and the filtrate was evaporated under reduced pressure to afford an oily crude that after treatment with $Et_2O$ (20 mL) gave a precipitate. The resulting precipitate was collected by centrifugation and was washed with $Et_2O$ (3 x 20 mL) to give **L75** (190 mg; 0.13 mmol) as a white solid. Yield 44%.

G. L76 (*N*-[4-[[[[4,7,10-Tris(carboxymethyl)-1,4,7,10-tetraazacyclododec-1-yl]acetyl]amino]methyl]-3-nitrobenzoyl]-L-glutaminyl-L-tryptophyl-L-alanyl-L-valyl-glycyl-L-histidyl-L-leucyl-L-methioninamide) (FIG. 6D)

Resin **A** (0.5 g; 0.3 mmol) was shaken in a solid phase peptide synthesis vessel with 50% morpholine in DMA (7 mL) for 10 min, the solution was emptied and fresh 50% morpholine in DMA (7 mL) was added. The suspension was stirred for 20 min then the solution was emptied and the resin was washed with DMA (5 x 7 mL). 4-[[[9H-Fluoren-9-ylmethoxy)carbonyl]amino]methyl]-3-nitrobenzoic acid, **H** (0.50 g;1.2 mmol), HOBT (0.18 g; 1.2 mmol), DIC (0.19 mL; 1.2 mmol) and DMA (7 mL) were added to the resin, the mixture was shaken for 24 h at room temperature, the solution was emptied and the resin was washed with DMA (5 x 7 mL). The resin was then shaken with 50% morpholine in DMA (7 mL) for 10 min, the solution was emptied, fresh 50% morpholine in DMA (7 mL) was added and the mixture was shaken for 20 min. The solution was emptied and the resin was washed with DMA (5 x 7 mL). DOTA tri-t-butyl ester (0.79 g; 1.2 mmol), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, the solution was emptied and the resin was washed with DMA (5 x 7 mL), $CH_2Cl_2$ (5 x 7 mL) and vacuum dried. The resin was shaken in a flask with reagent B (25 mL) for 4.5 h. The resin was filtered and the solution was evaporated under reduced pressure to afford an oily crude that was triturated with $Et_2O$ (20 mL). The precipitate was collected by centrifugation and was washed with $Et_2O$ (3 x 20 mL) to give a solid (141 mg) which was analysed by HPLC. A 37 mg portion of the crude was purified by preparative HPLC. The fractions containing the product were lyophilised to give **L76** (10.8 mg; 7.2 x $10^{-3}$ mmol) as a white solid. Yield 9%.

## Example VII - Figures 7A-C

### Synthesis of L124

Summary: 4-Cyanophenol A was reacted with ethyl bromoacetate and $K_2CO_3$ in acetone to give the intermediated B, which was hydrolysed with NaOH to the corresponding acid C. Successive hydrogenation of C with $H_2$ and $PtO_2$ at 355 kPa in EtOH/$CHCl_3$ gave the corresponding aminoacid D, which was directly protected with FmocOSu to give E. Rink amide resin functionalised with the octapeptide Gln-Trp-Ala-Val-Gly-His-Leu-Met-$NH_2$ (BBN[7-14] [SEQ ID NO:1]) was reacted with E and then with DOTA tri-*t*-butyl ester. After cleavage and deprotection with reagent B the crude was purified by preparative HPLC to give L124. Overall yield: 1.3%

A. Synthesis of (4-Cyanophenoxy)acetic acid ethyl ester, **(B)** (FIG 7A)
The product was synthesized following the procedure reported in the literature (Archimbault, P.; LeClerc, G.; Strosberg, A. D.; Pietri-Rouxel, F. PCT Int. Appl. WO 980005, 1998).
B. Synthesis of (4-Cyanophenoxy)acetic acid, **(C)** (FIG 7A)
A 1 N solution of NaOH (7.6 mL; 7.6 mmol) was added dropwise to a solution of (4-cyanophenoxy)acetic acid ethyl ester **B** (1.55 g; 7.6 mmol) in MeOH (15 mL). After 1 h the solution was acidified with 1 N HCl (7.6 mL; 7.6 mmol) and evaporated. The residue was taken up with water (20 mL) and extracted with $CHCl_3$ (2 x 30 mL). The organic phases were evaporated and the crude was purified by flash chromatography to give (4-cyanophenoxy)acetic acid **C** (0.97 g; 5.5 mmol) as a white solid. Yield 72%.
C. Synthesis of [4-[[[9*H*-Fluoren-9-ylmethoxy)carbonyl]amino]methyl]phenoxy]acetic acid, **(E)** (FIG 7A)
$PtO_2$ (150 mg) was added to a solution of (4-cyanophenoxy)acetic acid **C** (1.05 g; 5.9 mmol) in EtOH (147 mL) and $CHCl_3$ (3 mL). The suspension was stirred 30 h under a hydrogen atmosphere (355 kPa; 20 ˚C). The mixture was filtered through a Celite® pad and the solution evaporated under vacuum. The residue was purified by flash chromatography to give acid **D** (0.7 g) which was dissolved in $H_2O$ (10 mL), MeCN (2 mL) and $Et_3N$ (0.6 mL) at 0 ˚C, then a solution of *N*-(9-fluorenylmethoxycarbonyloxy)succinimide (1.3 g; 3.9 mmol) in MeCN (22 mL) was added dropwise. After stirring 16 h at room temperature the reaction mixture was filtered and the volatiles were removed under vacuum. The residue was treated with 1 N HCl (10 mL) and the precipitated solid was filtered and purified by flash chromatography to give [4-[[[9*H*-fluoren-9-ylmethoxy)carbonyl]amino]methyl]phenoxy]acetic acid **E** (0.56 g; 1.4 mmol) as a white solid. Overall yield 24%.
D. Synthesis of **L124** (*N*-[[4-[[[[4,7,10-Tris(carboxymethyl)-1,4,7,10-tetraazacyclododec-1-yl]acetyl] amino]methyl] phenoxy]acetyl]-L-glutaminyl-L-tryptophyl-L-alanyl-L-valyl-glycyl-L-histidyl-L-leucyl-L-methioninamide) (FIG. 7B)
Resin **A** (480 mg; 0.29 mmol) was shaken in a solid phase peptide synthesis vessel with 50% morpholine in DMA (7 mL) for 10 min, the solution was emptied and fresh 50 % morpholine in DMA (7 mL) was added. The suspension was stirred for 20 min, the solution was emptied and the resin was washed with DMA (5 x 7 mL). [4-[[[9*H*-Fluoren-9-ylmethoxy)carbonyl]amino]methyl]phenoxy]acetic acid **E** (480 mg; 1.19 mmol), *N*-hydroxybenzotriazole (HOBT) (182 mg; 1.19 mmol), *N,N*'-diisopropylcarbodiimide (DIC)
(185 μL; 1.19 mmol) and DMA (7 mL) were added to the resin, the mixture was shaken for 24 h at room temperature, the solution was emptied and the resin was washed with DMA (5 x
7 mL). The resin was then shaken with 50% morpholine in DMA (6 mL) for 10 min, the
solution was emptied, fresh 50% morpholine in DMA (6 mL) was added and the mixture was shaken for 20 min. The solution was emptied and the resin was washed with DMA (5 x 7 mL). 1,4,7,10-Tetraazacyclododecane-1,4,7,10-tetraacetic acid tris(1,1-dimethylethyl) ester adduct with NaCl (750 mg; 1.19 mmol), HOBT (182 mg; 1.19 mmol), DIEA (404 μL; 2.36 mmol), DIC (185 μL; 1.19 mmol) and DMA (6 mL) were added to the resin. The mixture was shaken for 24 h at room temperature, the solution was emptied, the resin was washed with DMA (2 x 7 mL), $CH_2Cl_2$ (5 x 7 mL) and vacuum dried. The resin was shaken in a flask with Reagent B (25 mL) for 4 h. The resin was filtered and the filtrate was evaporated under reduced pressure to afford an oily crude that was triturated with $Et_2O$ (5 mL). The precipitate was collected by centrifugation and washed with $Et_2O$ (5 x 5 mL) to give a solid (148 mg) which was analysed by HPLC. A 65 mg portion of the crude was purified by preparative HPLC. The fractions containing the product were lyophilised to give **L124** (FIG. 7C) as a white solid (15 mg; 0.01 mmol). Yield 7.9%.

## Example VIII - Figures 8A-C

### Synthesis of L125

Summary: 4-(Bromomethyl)-3-methoxybenzoic acid methyl ester A was reacted with $NaN_3$ in DMF to give the intermediate azide B, which was then reduced with $Ph_3P$ and $H_2O$ to amine C. Hydrolysis of C with NaOH gave acid D,

which was directly protected with FmocOSu to give E. Rink amide resin functionalised with the octapeptide Gln-Trp-Ala-Val-Gly-His-Leu-Met-$NH_2$ (BBN[7-14] [SEQ ID NO:1]) (A) was reacted with E and then with DOTA tri-*t*-butyl ester. After cleavage and deprotection with reagent B the crude was purified by preparative HPLC to give L125. Overall yield: 0.2%.

A. Synthesis of 4-(Azidomethyl)-3-methoxybenzoic acid methyl ester, **(B)** (FIG. 8A)
A solution of 4-(bromomethyl)-3-methoxybenzoic acid methyl ester (8 g; 31 mmol) and $NaN_3$(2 g; 31 mmol) in DMF (90 mL) was stirred overnight at room temperature. The volatiles were removed under vacuum and the crude product was dissolved in EtOAc (50 mL). The solution was washed with water (2 x 50 mL) and dried. The volatiles were evaporated to provide 4-(azidomethyl)-3-methoxybenzoic acid methyl ester (6.68 g; 30 mmol). Yield 97%.
B. 4-(Aminomethyl)-3-methoxybenzoic acid methyl ester, **(C)** (FIG. 8A) Triphenylphosphine (6.06 g; 23 mmol) was added to a solution of (4-azidomethyl)-3-methoxybenzoic acid methyl ester **B** (5 g; 22 mmol) in THF (50 mL): hydrogen evolution and formation of a white solid was observed. The mixture was stirred under nitrogen at room temperature. After 24 h more triphenylphosphine (0.6 g; 2.3 mmol) was added. After 24 h the azide was consumed and $H_2O$ (10 mL) was added. After 4 h the white solid disappeared. The mixture was heated at 45 ˚C for 3 h and was stirred overnight at room temperature. The solution was evaporated to dryness and the crude was purified by flash chromatography to give 4-(aminomethyl)-3-methoxybenzoic acid methyl ester **C** (1.2 g; 6.1 mmol). Yield 28%.
C. 4-[[[9*H*-Fluoren-9-ylmethoxy)carbonyl]amino]methyl]-3-methoxybenzoic acid **(E)** (FIG. 8A)
A 1 N solution of NaOH (6.15 mL; 6.14 mmol) was added dropwise to a solution of 4-(aminomethyl)-3-methoxybenzoic acid methyl ester **C** (1.2 g; 6.14 mmol) in MeOH (25 mL) heated at 40 ˚C. After stirring 8 h at 45 ˚C the solution was stirred over night at room temperature. A 1 N solution of NaOH (0.6 mL; 0.6 mmol) was added and the mixture heated at 40 ˚C for 4 h. The solution was concentrated, acidified with 1 N HCl (8 mL; 8 mmol), extracted with EtOAc (2 x 10 mL) then the aqueous layer was concentrated to 15 mL. This solution (pH 4.5) was cooled at 0 ˚C and $Et_3N$ (936 μL; 6.75 mmol) was added (pH 11). A solution of *N*-(9-fluorenylmethoxycarbonyloxy)succinimide (3.04 g; 9 mmol) in MeCN (30 mL) was added dropwise (final pH 9) and a white solid precipitated. After stirring 1 h at room temperature the solid was filtered, suspended in 1N HCl (15 mL) and the suspension was stirred for 30 min. The solid was filtered to provide 4-[[[9*H*-fluoren-9-ylmethoxy)carbonyl]amino]methyl]-3-methoxybenzoic acid **E** as a white solid (275 mg; 0.7 mmol).
The filtrate was evaporated under vacuum and the resulting white residue was suspended in 1N HCl (20 mL) and stirred for 30 minutes. The solid was filtered and purified by flash chromatography to give more acid **E** (198 mg; 0.5 mmol). Overall yield 20%.
D. **L125** (*N*-[4-[[[[4,7,10-Tris(carboxymethyl)-1,4,7,10-tetraazacyclododec-1-yl]acetyl]amino]methyl]-3-methoxybenzoyl]-L-glutaminyl-L-tryptophyl-L-alanyl-L-valyl-glycyl-L-histidyl-L-leucyl-L-methioninamide) (FIG. 8B)
Resin **A** (410 mg; 0.24 mmol) was shaken in a solid phase peptide synthesis vessel with 50% morpholine in DMA (7 mL) for 10 min, the solution was emptied and fresh 50 % morpholine in DMA (7 mL) was added. The suspension was stirred for 20 min then the solution was emptied and the resin was washed with DMA (5 x 7 mL). 4-[[[9*H*-Fluoren-9-ylmethoxy)carbonyl]amino]methyl]-3-methoxybenzoic acid **E** (398 mg; 0.98 mmol), HOBT (151 mg; 0.98 mmol), DIC (154 μL; 0.98 mmol) and DMA (6 mL) were added to the resin; the mixture was shaken for 24 h at room temperature, the solution was emptied and the resin was washed with DMA (5 x 7 mL). The resin was then shaken with 50% morpholine in DMA (6 mL) for 10 min, the solution was emptied, fresh 50% morpholine in DMA (6 mL) was added and the mixture was shaken for 20 min. The solution was emptied and the resin washed with DMA (5 x 7 mL). 1,4,7,10-Tetraazacyclododecane-1,4,7,10-tetraacetic acid tris(1,1-dimethylethyl) ester adduct with NaCl (618 mg; 0.98 mmol), HOBT (151 mg; 0.98 mmol), DIC (154 μL; 0.98 mmol), DIEA (333 μL; 1.96 mmol) and DMA (6 mL) were added to the resin. The mixture was shaken for 24 h at room temperature, the solution was emptied and the resin was washed with DMA (5 x 7 mL), $CH_2Cl_2$ (5 x 7 mL) and vacuum dried. The resin was shaken in a flask with reagent B (25 mL) for 4 h. The resin was filtered and the solution was evaporated under reduced pressure to afford an oily crude that was triturated with $Et_2O$ (5 mL). The resulting precipitate was collected by centrifugation, was washed with $Et_2O$ (5 x 5 mL), was analysed by HPLC and purified by preparative HPLC. The fractions containing the product were lyophilised to give **L125** (FIG. 8C) as a white solid (15.8 mg; 0.011 mmol). Yield 4.4%.

**Example IX - Figures 9A - 9D**

**Synthesis of L146, L233, L234, and L235**

Summary: The products were obtained in several steps starting from the octapeptide GlnTrpAlaValGlyHisLeuMetNH$_2$(BBN[7-14]) (A) on the Rink amide resin. After final cleavage and deprotection with reagent B the crudes were purified by preparative HPLC to give L146, L233, L234 and L235. Overall yields: 10%, 11%, 4.5%, 5.7% respectively.

A. 3-[[[(9*H*-Fluoren-9-ylmethoxy)carbonyl]amino]acetyl]aminobenzoic acid, B (FIG. 9A)
A solution of 3-aminobenzoic acid (0.5 g; 3.8 mmol) and *N*-ethyldiisopropylamine (DIEA) (0.64 mL; 3.8 mmol) in THF (20 mL) was added dropwise to a solution of Fmoc-glycine chloride (1.2 g; 4.0 mmol) (3) in THF (10 mL) and $CH_2Cl_2$ (10 mL). After 24 h stirring at room temperature 1 M HCl (50 mL) was added (final pH: 1.5). The precipitate was filtered, washed with $H_2O$ (2 x 100 mL), vacuum dried and crystallised from $CHCl_3/CH_3OH$ (1:1) to give **B** as a white solid (0.7 g; 1.6 mmol). Yield 43%.

B. *N*-[3-[[[[[4,7,10-Tris(carboxymethyl)-1,4,7,10-tetraazacyclododec-1-yl]acetyl]amino]acetyl]amino]benzoyl]-L-glutaminyl-L-tryptophyl-L-alanyl-L-valyl-glycyl-L-histidyl-L-leucyl-L-methioninamide, L233 (FIG. 9D)
Resin **A** (0.5 g; 0.3 mmol) was shaken in a solid phase peptide synthesis vessel with 50% morpholine in DMA (7 mL) for 10 min, the solution emptied and fresh 50% morpholine in DMA (7 mL) was added.
The suspension was stirred for another 20 min then the solution was emptied and the resin washed with DMA (5 x 7 mL). 3-[[[(9*H*-Fluoren-9-ylmethoxy)carbonyl]amino]acetyl]aminobenzoic acid, **B** (0.50 g;1.2 mmol), HOBT (0.18 g; 1.2 mmol), DIC (0.19 mL; 1.2 mmol) and DMA (7 mL) were added to the resin, the mixture shaken for 6 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). DOTA tri-t-butyl ester adduct with NaCl[2] (0.79 g; 1.2 mmol) (5), HOBT (0.18 g; 1.2 mmol), DIC (0.19 mL: 1.2 mmol), DIEA (0.40 mL; 2.4 mmol) and DMA (7 mL) were added to the resin. The mixture was shaken for 24 h at room temperature, emptied and the resin washed with DMA (5 x 7 mL), $CH_2Cl_2$ (5 x 7 mL) and vacuum dried. The resin was shaken in a flask with Reagent B (25 mL) for 4.5 h. The resin was filtered and the solution was evaporated under reduced pressure to afford an oily crude that after treatment with $Et_2O$ (20 mL) gave a precipitate. The precipitate was collected by centrifugation and washed with $Et_2O$ (3 x 20 mL) to give a solid (152 mg) which was analysed by HPLC. An amount of crude (50 mg) was purified by preparative HPLC. The fractions containing the product were lyophilised to give L233 (17.0 mg; 11.3 x $10^{-3}$ mmol) as a white solid. Yield 11%.

C. *N*-[4-[[[[[4,7,10-Tris(carboxymethyl)-1,4,7,10-tetraazacyclododec-1-yl]acetyl]amino]acetyl]amino]phenylacetyl]-L- glutaminyl-L-tryptophyl-L-alanyl-L-valyl-glycyl-L-histidyl-L-leucyl-L-methioninamide, L146 (FIG 9D)
Resin A (0.5 g; 0.3 mmol) was shaken in a solid phase peptide synthesis vessel with 50% morpholine in DMA (7 mL) for 10 min, the solution flushed off and fresh 50% morpholine in DMA (7 mL) was added. The suspension was stirred for another 20 min then the solution was flushed off and the resin washed with DMA (5 x 7 mL). Fmoc-4-aminophenylacetic acid (0.45 g; 1.2 mmol), HOBt (0.18 g; 1.2 mmol), DIC (0.19 mL; 1.2 mmol) and DMA (7 mL) were added to the resin, the mixture shaken for 6 h at room temperature, flushed off and the resin washed with DMA (5 x 7 mL). The resin was then shaken with 50% morpholine in DMA (7 mL) for 10 min, the solution flushed off, fresh 50% morpholine in DMA (7 mL) was added and the mixture shaken for another 20 min. The solution was flushed off and the resin washed with DMA (5 x 7 mL). Fmoc-glycine (0.36 g; 1.2 mmol), HATU (0.46 g; 1.2 mmol) and DIEA (0.40 mL; 2.4 mmol) were stirred for 15 min in DMA (7 mL) then the solution was added to the resin, the mixture shaken for 2 h at room temperature, flushed off and the resin washed with DMA (5 x 7 mL). The resin was then shaken with 50% morpholine in DMA (7 mL) for 10 min, the solution flushed off, fresh 50% morpholine in DMA (7 mL) was added and the mixture shaken for another 20 min. The solution was flushed off and the resin washed with DMA (5 x 7 mL). DOTA tri-t-butyl ester adduct with NaCl (0.79 g; 1.2 mmol), HOBt (0.18 g; 1.2 mmol), DIC (0.19 mL; 1.2 mmol), DIEA (0.40 mL; 2.4 mmol) and DMA (7 mL) were added to the resin. The mixture was shaken for 24 h at room temperature, flushed off and the resin washed with DMA (5 x 7 mL), CH2Cl2 (5 x 7 mL) and vacuum dried. The resin was shaken in a flask with Reagent B (25 mL) for 4.5 h. The resin was filtered and the solution was evaporated under reduced pressure to afford an oily crude that after treatment with Et2O (20 mL) gave a precipitate. The precipitate was collected by centrifugation and washed with Et2O (3 x 20 mL) to give a solid (203 mg) which was analysed by HPLC. An amount of crude (50 mg) was purified by preparative HPLC. The fractions containing the product were lyophilised to give **L146** (11.2 mg; 7.4 x 10-3 mmol) as a white solid. Yield 10%.

D. 6-[[[(9*H*-Fluoren-9-ylmethoxy)carbonyl]amino]acetyl]aminonaphthoic acid, C (FIG. 9B)
A solution of 6-aminonaphthoic acid (500 mg; 2.41 mmol); and DIEA (410 μL 2.41 mmol) in THF (20 mL) was added dropwise to a solution of Fmoc-glycine chloride (760 mg; 2.41 mmol) in $CH_2Cl_2$/THF 1:1 (10 mL) and stirred at room temperature. After 24 h the solvent was evaporated under vacuum. The residue was taken up with 0.5 N HCl (50 mL) and stirred for 1 h. The white solid precipitated was filtered and dried. The white solid was suspended in methanol (30 mL) and boiled for 5 min, then was filtered to give product C (690 mg; 1.48 mmol). Yield 62%.

E. *N*-[6-[[[[[4,7,10-Tris(carboxymethyl)-1,4,7,10-tetraazacyclododec-1-yl]acetyl]amino]acetyl] amino] naphthoyl] -L-glutaminyl-L-tryptophyl-L-alanyl-L-valyl-glycyl-L-histidyl-L-leucyl-L-methioninamide, L234
Resin **A** (500 mg; 0.3 mmol) was shaken in a solid phase peptide synthesis vessel with 50% morpholine in DMA (7 mL) for 10 min, the solution emptied and fresh 50 % morpholine in DMA (7 mL) was added. The suspension was stirred for another 20 min then the solution was emptied and the resin washed with DMA (5 x 7 mL). 6-[[[(9*H*-Fluoren-9-ylmethoxy)carbonyl]amino]acetyl]aminonaphthoic acid **C** (560 mg; 1.2 mmol), HOBT (184 mg; 1.2 mmol), DIC (187 μL; 1.2 mmol) and DMA (7 mL) were added to the resin, the mixture shaken for 6 h at room temperature, emptied and the resin washed with DMA (5 x 7 mL). The resin was then shaken with 50% morpholine in DMA (6 L) for 10 min, the solution emptied, fresh 50% morpholine in DMA (7 mL) was added and the mixture shaken for another 20 min. The solution was emptied and the resin washed with DMA (5 x 7 mL). DOTA tri-t-butyl ester adduct with NaCl (757 mg; 1.2 mmol), HOBT (184 mg; 1.2 mmol), DIC (187 μL; 1.2 mmol), and DIEA (537 μL; 2.4 mmol) and DMA (7 mL) were added to the resin. The mixture was shaken in a flask, emptied and the resin washed with DMA (2 x 7 mL), $CH_2Cl_2$ (5 x 7 mL) and vacuum dried. The resin was shaken in a flask with Reagent B (25 mL) for 4.5 h. The resin was filtrated and the solution was evaporated under reduced pressure to afford an oil crude that after treatment with $Et_2O$ (20 mL) gave a precipitate. The precipitate was collected by centrifugation and washed with $Et_2O$ (3 x 20 mL) to give a solid (144 mg) which was analysed by HPLC. An amount of crude (54 mg) was purified by preparative HPLC. The fractions containing the product were lyophilised to give L234 (8 mg; 5.1 x $10^{-3}$ mmol) as a white solid. Yield 4.5%.

F. 4-[[[[(9*H*-Fluoren-9-ylmethoxy)carbonyl]amino]acetyl]methylamino]benzoic acid, D (FIG. 9C) A solution of 4-*N*-methylaminonaphthoic acid (500 mg; 3.3 mmol) and DIEA (562 μL 3.3 mmol) in THF (20 mL) was added to a solution of Fmoc-glycine chloride (1.04 g; 3.3 mmol) in $CH_2Cl_2$/THF 1:1 (10 mL) and stirred at room temperature. After 24 h the solvent was evaporated under vacuum. The residue was taken up with 0.5 N HCl (30 mL) and was stirred for 3 h at 0 °C. The white solid precipitated was filtered and dried. The crude was purified by flash chromatography to give compound **D** (350 mg; 0.81 mmol). Yield 25%.

G. *N*-[4-[[[[[4,7,10-Tris(carboxymethyl)-1,4,7,10-tetraazacyclododec-1-yl]acetyl]amino]acetyl] methylamino]benzoyl]-L-glutaminyl-L-tryptophyl-L-alanyl-L-valyl-glycyl-L-histidyl-L-leucyl-L-methioninamide, L235 (FIG. 9D)
Resin **A** (500 mg; 0.3 mmol) was shaken in a solid phase peptide synthesis vessel with 50% morpholine in DMA (7 mL) for 10 min, the solution emptied and fresh 50 % morpholine in DMA (7 mL) was added. The suspension was stirred for another 20 min then the solution was emptied and the resin washed with DMA (5 x 7 mL). 4-[[[[9H-Fluoren-9-ylmethoxy)carbonyl]amino]acetyl]-*N*-methyl]amino-benzoic acid **D** (510 mg; 1.2 mmol), HOBT (184 mg; 1.2 mmol), DIC (187 μL; 1.2 mmol) and DMA (7 mL) were added to the resin, the mixture shaken for 6 h at room temperature, emptied and the resin washed with DMA (5 x 7 mL). The resin was then shaken with 50% morpholine in DMA (7mL) for 10 min, the solution emptied, fresh 50% morpholine in DMA (7 mL) was added and the mixture shaken for another 20 min. The solution was emptied and the resin washed with DMA (5 x 7 mL). DOTA tri-t-butyl ester adduct with NaCl (757 mg; 1.2 mmol), HOBT (184 mg; 1.2 mmol), DIC (187 μL; 1.2 mmol), and DIEA (537 μL; 2.4 mmol) and DMA (7 mL) were added to the resin. The mixture was shaken in a flask, emptied and the resin washed with DMA (2 x 7 mL), $CH_2Cl_2$ (5 x 7 mL) and vacuum dried. The resin was shaken in a flask with Reagent B (25 mL) for 4.5 h. The resin was filtrated and the solution was evaporated under reduced pressure to afford an oil crude that after treatment with $Et_2O$ (20 mL) gave a precipitate.
The precipitate was collected by centrifugation and washed with $Et_2O$ (3 x 20 mL) to give a solid (126 mg) which was analysed by HPLC. An amount of crude (53 mg) was purified by preparative HPLC. The fractions containing the product were lyophilised to give L235 (11 mg; 7.2 x $10^{-3}$ mmol) as a white solid. Yield 5.7%.

**EXAMPLE X - Figures 10A-B**

**Synthesis of L237**

Summary: 1-Formyl-1,4,7,10-tetraazacyclododecane (A) was selectively protected with benzyl chloroformate at pH 3 to give B, which was alkylated with t-butyl bromoacetate and deformylated with hydroxylamine hydrochloride to give D. Reaction with $P(OtBu)_3$ and paraformaldehyde gave E, which was deprotected by hydrogenation and alkylated with benzyl bromoacetate to give G, which was finally hydrogenated to H. Rink amide resin functionalized with the octapeptide $GlnTrpAlaValGlyHisLeuMetNH_2$ (BBN[7-14]) (A) was sequentially reacted with Fmoc-4-aminobenzoic acid, Fmoc-glycine and **H.** After cleavage and deprotection with reagent B the crude was purified by preparative HPLC to give L237. Overall yield 0.21%.

A. 7-Formyl-1,4,7,10-tetraazacyclododecane-1-carboxylic acid phenylmethyl ester dihydrochloride, B (FIG. 10A)
1-Formyl-1,4,7,10-tetraazacyclododecane **A** (14 g; 69.9 mmol) was dissolved in $H_2O$ (100 mL) and 12 N HCl (11 mL) was added until pH 3 then 1,4-dioxane (220 mL) was added. A solution of benzyl chloroformate (13.8 g; 77 mmol) in 1,4-dioxane (15 mL) was slowly added dropwise in 3.5 h, constantly maintaining the reaction mixture at pH 3 by continuous addition of 2 N NaOH (68.4 mL) with a pHstat apparatus. At the end of the addition the reaction was stirred for 1 h then washed with *n*-hexane (4 x 100 mL) and $^iPr_2O$ (4 x 100 mL). The aqueous phase was brought to pH 13 by addition of 10 N NaOH (6.1 mL) and extracted with $CHCl_3$ (4 x 100 mL). The organic phase was washed with brine (100 mL), dried ($Na_2SO_4$), filtered and evaporated. The oily residue was dissolved in acetone (200 mL)

and 6 N HCl (26 mL) was added. The solid precipitated was filtered, washed with acetone (2 x 50 mL) and dried under vacuum to give compound **B** (23.6 g; 58 mmol) as a white crystalline solid. Yield 83%.

B. 4-(Phenylmethoxy)carbonyl-1,4,7,10-tetraazacyclododecane-1,7-diacetic acid bis(1,1-dimethylethyl) ester, D (FIG. 10A)

A solution of **B** (14.4 g; 35.3 mmol) in $H_2O$ (450 mL) and 1 N NaOH (74 mL; 74 mmol) was stirred for 20 min then extracted with $CHCl_3$ (4 x 200 mL). The organic layer was evaporated to obtain an oily residue (12.3 g) which was dissolved in $CH_3CN$ (180 mL) and N-ethyldiisopropylamine (DIEA) (15 mL; 88.25 mmol). A solution of t-butyl bromoacetate (16.8 g; 86.1 mmol) in $CH_3CN$ (15 mL) was added dropwise to the previous solution in 2.5 h. After 20 h at room temperature the solvent was evaporated and the oily residue was dissolved in $CHCl_3$ (150 mL) and washed with $H_2O$ (5 x 100 mL). The organic layer was dried ($Na_2SO_4$), filtered and evaporated to dryness to give **C** as a yellow oil. Crude **C** (22 g) was dissolved in EtOH (250 mL), $NH_2OH{\cdot}HCl$ (2.93g; 42.2 mmol) was added and the solution heated to reflux. After 48 h the solvent was evaporated and the residue dissolved in $CH_2Cl_2$ (250 mL), washed with $H_2O$ (3 x 250 mL) then with brine (3 x 250 mL). The organic layer was dried ($Na_2SO_4$), filtered and evaporated. The oily residue (18.85 g) was purified by flash chromatography. The fractions containing the product were collected and evaporated to obtain a glassy white solid (17.62 g) which was dissolved in $H_2O$ (600 mL) and 1 N NaOH (90 mL; 90 mmol) and extracted with $CHCl_3$ (3 x 250 ml). The organic layer was dried ($Na_2SO_4$) and evaporated to dryness to give **D** (16.6 g; 31 mmol) as an oil. Yield 88%.

C. 4-(Phenylmethoxy)carbonyl-10-[[bis(1,1-dimethylethoxy)phosphinyl]methyl]-1,4,7,10-tetraazacyclododecane-1,7-diacetic acid bis(1,1-dimethylethyl) ester, E (FIG. 10A)

A mixture of compound **D** (13.87 g; 26 mmol), $P(OtBu)_3$ (7.6 g; 28.6 mmol) (10) and paraformaldeyde (0.9 g; 30 mmol) was heated at 60 °C. After 16 h more $P(OtBu)_3$ (1 g; 3.76 mmol) and paraformaldeyde (0.1 g; 3.33 mmol) were added. The reaction was heated at 60 °C for another 20 h then at 80 °C for 8 h under vacuum to eliminate the volatile impurities. The crude was purified by flash chromatography to give **E** (9.33 g; 8 mmol) as an oil. Yield 31%.

D. 7-[[Bis(1,1-dimethylethoxy)phosphinyl]methyl]-1,4,7,10-tetraazacyclododecane-1,4,10-triacetic acid 1-phenylmethyl 4,10-bis(1,1-dimethylethyl) ester, G (FIG. 10A)

To the solution of **E** (6.5 g; 5.53 mmol) in $CH_3OH$ (160 mL) 5% Pd/C (1 g; 0.52 mmol) was added and the mixture was stirred under hydrogen atmosphere at room temperature. After 4 h (consumed $H_2$ 165 mL;6.7 mmol) the mixture was filtered through a Millipore® filter (FT 0.45 μm) and the solution evaporated under reduced pressure. The crude (5.9 g) was purified by flash chromatography to give **F** (4.2 g) as an oil. Benzyl bromoacetate (1.9 g; 8.3 mmol) dissolved in $CH_3CN$ (8 mL) was added dropwise in 1 h to a solution of **F** (4.2 g) in $CH_3CN$ (40 mL) and DIEA (1.5 mL; 8.72 mmol). After 36 h at room temperature the solvent was evaporated and the residue (5.76 g) dissolved in $CHCl_3$ (100 mL), washed with $H_2O$ (2 x 100 mL) then with brine (2 x 70 mL). The organic layer was dried ($Na_2SO_4$), filtered and evaporated. The crude (5.5 g) was purified twice by flash chromatography, the fractions were collected and evaporated to dryness to afford **G** (1.12 g; 1.48 mmol) as an oil. Yield 27%.

E. 7-[[Bis(1,1-dimethylethoxy)phosphinyl]methyl]-1,4,7,10-tetraazacyclododecane-1,4,10-triacetic acid 4,10-bis (1,1-dimethylethyl) ester, **H** FIG. 10A)

5% Pd/C (0.2 g; 0.087 mmol) was added to a solution of **G** (1.12 g; 1.48 mmol) in $CH_3OH$ (27 mL) and the mixture was stirred under hydrogen atmosphere at room temperature. After 2 h (consumed $H_2$ 35 mL; 1.43 mmol) the mixture was filtered through a Millipore® filter (FT 0.45 μm) and the solution evaporated to dryness to give **H** (0.94 g; 1.41 mmol) as a pale yellow oil. Yield 97%.

F. *N*-[4-[[[[[4,10-Bis(carboxymethyl)-7-(dihydroxyphosphinyl)methyl-1,4,7,10-tetraazacyclododec-1-yl]acetyl]amino]acetyl]amino]benzoyl]-L-glutaminyl-L-tryptophyl-L-alanyl-L-valyl-glycyl-L-histidyl-L-leucil-L-methioninamide, L 237 (FIG. 10B)

Resin **A** (330 mg; 0.20 mmol) (17) was shaken in a solid phase peptide synthesis vessel with 50% morpholine in DMA (5 mL) for 10 min, the solution emptied and fresh 50 % morpholine in DMA (5 mL) was added. The suspension was stirred for another 20 min then the solution was emptied and the resin washed with DMA (5 x 5 mL). Fmoc-4-aminobenzoic acid (290 mg; 0.80 mmol), HOBT (120 mg; 0.80 mmol), DIC (130 μL; 0.80 mmol) and DMA (5 mL) were added to the resin, the mixture shaken for 3 h at room temperature, emptied and the resin washed with DMA (5 x 5 mL). The resin was then shaken with 50% morpholine in DMA (5 mL) for 10 min, the solution emptied, fresh 50% morpholine in DMA (5 mL) was added and the mixture shaken for another 20 min. The solution was emptied and the resin washed with DMA (5 x 5 mL). Fmoc-glycine (240 mg; 0.8 mmol), HATU (310 mg; 0.8 mmol) and DIEA (260 μL; 1.6 mmol) were stirred for 15 min in DMA (5 mL) then the solution was added to the resin, the mixture shaken for 2 h at room temperature, emptied and the resin washed with DMA (5 x 5 mL). The resin was then shaken with 50% morpholine in DMA (5 mL) for 10 min, the solution emptied, fresh 50% morpholine in DMA (5 mL) was added and the mixture shaken for another 20 min. The solution was emptied and the resin washed with DMA (5 x 5 mL). H (532 mg; 0.80 mmol), HOBT (120 mg; 0.80 mmol), DIC (130 μL; 0.80 mmol), and DIEA (260 μL; 1.6 mmol) and DMA (5 mL) were added to the resin. The mixture was shaken in a flask for 40 h at room temperature, emptied and the resin washed with DMA (5 x 5 mL), $CH_2Cl_2$ (5 x 5 mL) and vacuum dried. The resin was shaken in a flask

with Reagent B (25 mL) for 4 h. The resin was filtered and the solution was evaporated under reduced pressure to afford an oily crude that after treatment with $Et_2O$ (20 mL) gave a precipitate. The precipitate was collected by centrifugation and washed with $Et_2O$ (3 x 20 mL) to give a solid (90 mg) which was analysed by HPLC. An amount of crude (50 mg) was purified by preparative HPLC. The fractions containing the product were lyophilised to give L237 (6 mg; $3.9 \times 10^{-3}$ mmol) as a white solid. Yield 3.5%.

## EXAMPLE XI - Figures 11A-B

### Synthesis of L238 and L239

Summary: The products were obtained in several steps starting from the octapeptide GlnTrpAlaValGlyHisLeuMet$NH_2$ (BBN[7-14]) (A) on the Rink amide resin. After cleavage and deprotection with reagent B the crude was purified by preparative HPLC to give L238 and L239. Overall yields: 14 and 9%, respectively.

A. *N*,*N*-Dimethylglycyl-L-seryl-[S-[(acetylamino)methyl]]-L-cysteinyl-glycyl-4-aminobenzoyl-L-glutaminyl-L-tryptophyl-L-alanyl-L-valyl-glycyl-L-histidyl-L-leucyl-L-methioninamide, L238 (FIG. 11A)

Resin **A** (0.5 g; 0.3 mmol) was shaken in a solid phase peptide synthesis vessel with 50% morpholine in DMA (7 mL) for 10 min, the solution emptied and fresh 50% morpholine in DMA (7 mL) was added. The suspension was stirred for another 20 min then the solution was emptied and the resin washed with DMA (5 x 7 mL). Fmoc-4-aminobenzoic acid (0.43 g; 1.2 mmol), HOBT (0.18 g; 1.2 mmol), DIC (0.19 mL; 1.2 mmol) and DMA (7 mL) were added to the resin, the mixture shaken for 3 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). Fmoc-glycine (0.36 g; 1.2 mmol), HATU (0.46 g; 1.2 mmol) and N-ethyldiisopropylamine (0.40 mL; 2.4 mmol) were stirred for 15 min in DMA (7 mL) then the solution was added to the resin, the mixture shaken for 2 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N*-α-Fmoc-S-acetamidomethyl-L-cysteine (0.50 g; 1.2 mmol), HOBT (0.18 g; 1.2 mmol), DIC (0.19 mL; 1.2 mmol) and DMA (7 mL) were added to the resin, the mixture shaken for 3 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N*-α-Fmoc-O-t-butyl-L-serine (0.46 g; 1.2 mmol), HOBT (0.18 g; 1.2 mmol), DIC (0.19 mL: 1.2 mmol), and DMA (7 mL) were added to the resin, the mixture was shaken for 3 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min.

The solution was emptied and the resin washed with DMA (5 x 7 mL). N,N-Dimethylglycine (0.12 g; 1.2 mmol), HATU (0.46 g; 1.2 mmol) and N-ethyldiisopropylamine (0.40 mL; 2.4 mmol) were stirred for 15 min in DMA (7 mL) then the solution was added to the resin. The mixture was shaken for 2 h at room temperature, emptied and the resin washed with DMA (5 x 7 mL), $CH_2Cl_2$ (5 x 7 mL) and vacuum dried. The resin was shaken in a flask with reagent B (25 mL) for 4.5 h. The resin was filtered and the solution was evaporated under reduced pressure to afford an oily crude that after treatment with $Et_2O$ (20 mL) gave a precipitate. The precipitate was collected by centrifugation and washed with $Et_2O$ (3 x 20 mL) to give a solid (169 mg) which was analysed by HPLC. An amount of crude (60 mg) was purified by preparative HPLC. The fractions containing the product were lyophilised to give L238 (22.0 mg; 0.015 mmol) as a white solid. Yield 14%.

B. *N*,*N*-Dimethylglycyl-L-seryl-[S-[(acetylamino)methyl]]-L-cysteinyl-glycyl-(3β,5β,7α,12α)-3-amino-7,12-dihydroxy-24-oxocholan-24-yl-L-glutaminyl-L-tryptophyl-L-alanyl-L-valyl-glycyl-L-histidyl-L-leucyl-L-methioninamide, L239 (FIG. 11B)

Resin **A** (0.5 g; 0.3 mmol) was shaken in a solid phase peptide synthesis vessel with 50% morpholine in DMA (7 mL) for 10 min, the solution emptied and fresh 50% morpholine in DMA (7 mL) was added. The suspension was stirred for another 20 min then the solution was emptied and the resin washed with DMA (5 x 7 mL). (3β,5β,7α,12α)-3-[[(9H-Fluoren-9-ylmethoxy)amino]acetyl]amino-7,12-dihydroxycholan-24-oic acid **B** (0.82 g; 1.2 mmol) (7), HOBT (0.18 g; 1.2 mmol), DIC (0.19 mL; 1.2 mmol) and DMA (7 mL) were added to the resin, the mixture shaken for 24 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N*-α-Fmoc-S-acetamidomethyl-L-cysteine (0.50 g; 1.2 mmol), HOBT (0.18 g; 1.2 mmol), DIC (0.19 mL; 1.2 mmol)

and DMA (7 mL) were added to the resin, the mixture was shaken for 3 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N*-α-Fmoc-O-t-butyl-L-serine (0.46 g; 1.2 mmol), HOBT (0.18 g; 1.2 mmol), DIC (0.19 mL: 1.2 mmol), and DMA (7 mL) were added to the resin, the mixture was shaken for 3 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N*,*N*-Dimethylglycine (0.12 g; 1.2 mmol), HATU (0.46 g; 1.2 mmol) and *N*-ethyldiisopropylamine (0.40 mL; 2.4 mmol) were stirred for 15 min in DMA (7 mL) then the solution was added to the resin. Resin **A** (0.5 g; 0.3 mmol) was shaken in a solid phase peptide synthesis vessel with 50% morpholine in DMA (7 mL) for 10 min, the solution emptied and fresh 50% morpholine in DMA (7 mL) was added. The suspension was stirred for another 20 min then the solution was emptied and the resin washed with DMA (5 x 7 mL). (3β,5β,7α,12α)-3-[[(9H-Fluoren-9-ylmethoxy) amino]acetyl]amino-7,12-dihydroxycholan-24-oic acid **B** (0.82 g; 1.2 mmol) HOBT (0.18 g; 1.2 mmol), DIC (0.19 mL; 1.2 mmol) and DMA (7 mL) were added to the resin, the mixture shaken for 24 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N*-α-Fmoc-S-acetamidomethyl-L-cysteine (0.50 g; 1.2 mmol), HOBT (0.18 g; 1.2 mmol), DIC (0.19 mL; 1.2 mmol) and DMA (7 mL) were added to the resin, the mixture was shaken for 3 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N*-α-Fmoc-O-t-butyl-L-serine (0.46 g; 1.2 mmol), HOBT (0.18 g; 1.2 mmol), DIC (0.19 mL: 1.2 mmol), and DMA (7 mL) were added to the resin, the mixture was shaken for 3 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). *N,N*-Dimethylglycine (0.12 g; 1.2 mmol), HATU (0.46 g; 1.2 mmol) and *N*-ethyldiisopropylamine (0.40 mL; 2.4 mmol) were stirred for 15 min in DMA (7 mL) then the solution was added to the resin.

**EXAMPLE XII - Figures 12A-F**

**Synthesis of L240, L241, L242**

Summary: The products were obtained in several steps starting from the octapeptide GlnTrpAlaValGlyHisLeu $MetNH_2$ (BBN[7-14]) (A) on the Rink amide resin. After cleavage and deprotection with reagent B the crudes were purified by preparative HPLC to give L240, L241, and L242. Overall yields: 7.4, 3.2, 1.3% respectively.

A. 4-[[[(9***H***-Fluoren-9-ylmethoxy)carbonyl]amino]acetyl]amino-3-methoxybenzoic acid A (FIG. 12A)

A solution of 4-amino-3-methoxybenzoic acid (1.0 g; 5.9 mmol); and N-ethyldiisopropylamine (1.02 mL 5.9 mmol) in THF (20 mL) was added dropwise to a solution of Fmoc-glycylchloride (1.88 g; 5.9 mmol) in $CH_2Cl_2$ /THF 1:1 (20 mL) and stirred at room temperature under $N_2$. After 3 h the solvent was evaporated under vacuum. The residue was taken up with 0.5 N HCl (50 mL), was stirred for 1 h at 0 °C then filtered and dried. The white solid was suspended in MeOH (30 mL) and stirred for 1 h, then was filtered and suspended in a solution of $CHCl_3$/hexane 1:4 (75 mL). The suspension was filtered to give compound A as a with solid (1.02 g; 2.28 mmol). Yield 39%.

B. *N*-[4-[[[[4,7,10-Tris(carboxymethyl)-1,4,7,10tetraazacyclododec-1-yl] acetyl]glycyl]amino]-3-methoxybenzoyl]-L-glutaminyl-L-tryptophyl-1-alanyl-L-valyl-glycyl-L-histidyl-L-leucyl-L-methioninamide L240

Resin **A** (0.5 g; 0.3 mmol) was shaken in a solid phase peptide synthesis vessel with 50% morpholine in DMA (7 mL) for 10 min, the solution emptied and fresh 50% morpholine in DMA (7 mL) was added. The suspension was stirred for another 20 min then the solution was emptied and the resin washed with DMA (5 x 7 mL). 4-[[[(9H-Fluoren-9-ylmethoxy)carbonyl] amino] acetyl] amino-3-methoxybenzoic acid, **A** (0.50 g; 1.2 mmol), HOBT (0.18 g; 1.2 mmol), DIC (0.19 mL; 1.2 mmol) and DMA (7 mL) were added to the resin, the mixture shaken for 5 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL).1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), N-ethyldiisopropylamine (0.40 mL; 2.4 mmol) and DMA (7 mL) were added to the resin. The mixture was shaken for 24 h at room temperature, emptied and the resin washed with DMA (5 x 7

mL), $CH_2Cl_2$ (5 x 7 mL) and vacuum dried. The resin was shaken in a flask with Reagent B (25 mL) for 4.5 h. The resin was filtered and the solution was evaporated under reduced pressure to afford an oil crude that after treatment with $Et_2O$ (20 mL) gave a precipitate. The precipitate was collected by centrifugation and washed with $Et_2O$ (5 x 20 mL) to give a solid (152 mg) which was analysed by HPLC. An amount of crude (52 mg) was purified by preparative HPLC. The fractions containing the product were lyophilised to give L240 (12.0 mg; 7.8 x $10^{-3}$ mmol) as a white solid. Yield 7.4%.

C. 4-amino-3-chlorobenzoic acid C (FIG. 12B)

1 N NaOH (11 mL; 11 mmol) was added to a solution of methyl 4-amino-3-chlorobenzoate (2 g; 10.8 mmoli) in MeOH (20 mL) at 45 ˚C. The reaction mixture was stirred for 5 h at 45 ˚C and overnight at room temperature. More 1N NaOH was added (5 mL; 5 mmol) and the reaction was stirred at 45 ˚C for 2 h. After concentration of solvent was added 1N HCl (16 ml). The solid precipitate was filtered and dried to give 4-amino-3-chlorobenzoic acid, **C,** as a with solid (1,75 g; 10.2 mmol). Yield 94.6%.

D. 4-[[[(9*H*-Fluoren-9-ylmethoxy)carbonyl]amino]acetyl]amino-3-chlorobenzoic acid, D (FIG. 12B)

A solution of 4-amino-3-chlorobenzoic acid (1.5 g; 8.75 mmol) and N-ethyldiisopropylamine (1.46 mL 8.75 mmol) in THF (50 mL) was added dropwise to a solution of Fmoc-glycylchloride (2.76 g; 8.75 mmol) in $CH_2Cl_2$ /THF 1:1 (30 mL) and stirred at room temperature under $N_2$. After 3 h the solvent was evaporated under vacuum. The residue was taken up with 0.5N HCl (50 mL), filtered and dried.

The white solid was suspended in MeOH (30 mL) and stirred for 1 h, then was filtered and dried to give 4-[[[(9H-fluoren-9-ylmethoxy)carbonyl]amino]acetyl]amino-3-chlorobenzoic acid (2.95 g; 6.5 mmol). Yield 75%.

E. *N*-[4-[[[[4,7,10-Tris(carboxymethyl)-1,4,7,10tetraazacyclododec-1-yl] acetyl]glycyl]amino]3-chlorobenzoyl]L-glutaminyl-L-tryptophyl-1-alanyl-L-valyl-glycyl-L-histidyl-L-leucyl-L-methioninamide, L241 (FIG. 12E)

Resin A (0.5 g; 0.3 mmol) was shaken in a solid phase peptide synthesis vessel with 50% morpholine in DMA (7 mL) for 10 min, the solution emptied and fresh 50% morpholine in DMA (7 mL) was added. The suspension was stirred for another 20 min then the solution was emptied and the resin washed with DMA (5 x 7 mL). 4-[[[(9*H*-Fluoren-9-ylmethoxy)carbonyl]amino]acetyl]amino-3-chlorobenzoic acid, **D** (0.54 g; 1.2 mmol), HOBT (0.18 g; 1.2 mmol), DIC (0.19 mL; 1.2 mmol) and DMA (7 mL) were added to the resin, the mixture shaken for 5 h at room temperature, emptied and the resin washed with DMA (5 x 7 mL).

The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). 1,4,7,10-Tetraazacyclododecane-1,4,7,10-tetraacetic acid tris(1,1-dimethylethyl) ester adduct with NaCl (0.79 g; 1.2 mmol), HOBT (0.18 g; 1.2 mmol), DIC (0.19 mL: 1.2 mmol), *N*-ethyldiisopropylamine (0.40 mL; 2.4 mmol) and DMA (7 mL) were added to the resin. The mixture was shaken for 40 h at room temperature, emptied and the resin washed with DMA (5 x 7 mL), $CH_2Cl_2$ (5 x 7 mL) and vacuum dried. The resin was shaken in a flask with Reagent B (25 mL) for 4.5 h. The resin was filtered and the solution was evaporated under reduced pressure to afford an oil crude that after treatment with $Et_2O$ (20 mL) gave a precipitate. The precipitate was collected by centrifugation and washed with $Et_2O$ (5 x 20 mL) to give a solid (151 mg) which was analysed by HPLC. An amount of crude (56 mg) was purified by preparative HPLC. The fractions containing the product were lyophilised to give L241 (5.6 mg; 3.6 x $10^{-3}$ mmol) as a white solid. Yield 3.2%.

F. 4-[[[(9*H*-Fluoren-9-ylmethoxy)carbonyl]amino]acetyl]amino-3-methylbenzoic acid, E (FIG. 12C)

A solution of 4-amino-3-methylbenzoic acid (0.81 g; 5.35 mmol) and N-ethyldiisopropylamine (0.9 mL 5.35 mmol) in THF (30 mL) was added dropwise to a solution of Fmoc-glycylchloride (1.69 g; 5.35 mmol) in $CH_2Cl_2$ /THF 1:1 (20 mL) and stirred at room temperature under $N_2$. After 3 h the solvent was evaporated under vacuum. The residue was taken up with HCl 0.5 N (50 mL)and was stirred for 3 h at 0˚C. then was filtered and dried. The white solid was suspended in MeOH (50 mL) and stirred for 1 h, then filtered and dried to give compound **E** (1.69 g; 3.9 mmol). Yield 73%.

G. N-[4-[[[[4,7,10-Tris(carboxymethyl)-1,4,7,10-tetraazacyclododec-1-yl] acetyl]glycyl]amino]3-methylbenzoyl]L-glutaminyl-L-tryptophyl-L-alanyl-L-valyl-glycyl-L-histidyl-L-leucyl-L-methioninamide L242 (FIG. 12F) Resin A (0.5 g; 0.3 mmol) was shaken in a solid phase peptide synthesis vessel with 50% morpholine in DMA (7 mL) for 10 min, the solution emptied and fresh 50% morpholine in DMA (7 mL) was added. The suspension was stirred for another 20 min then the solution was emptied and the resin washed with DMA (5 x 7 mL). 4-[[(9H-Fluoren-9-ylmethoxy) amino]acetyl]amino-3-methylbenzoic acid, E (0.52 g; 1.2 mmol), HOBT (0.18 g; 1.2 mmol), DIC (0.19 mL; 1.2 mmol) and DMA (7 mL) were added to the resin, the mixture shaken for 5 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL).1,4,7,10-Tetraazacyclododecane-1,4,7,10-tetraacetic acid tris(1,1-dimethylethyl) ester adduct with NaCl (0.76 g; 1.2 mmol), HOBT (0.18 g; 1.2 mmol), DIC (0.19 mL; 1.2 mmol), *N*-ethyldiisopropylamine (0.40 mL; 2.4 mmol) and DMA (7 mL) were added to the resin. The mixture was shaken for 40 h at room temperature, emptied and the resin washed with DMA (5 x 7 mL), $CH_2Cl_2$ (5 x 7 mL)

and vacuum dried. The resin was shaken in a flask with Reagent B (25 mL) for 4.5 h. The resin was filtered and the solution was evaporated under reduced pressure to afford an oil crude that after treatment with $Et_2O$ (20 mL) gave a precipitate. The precipitate was collected by centrifugation and washed with $Et_2O$ (5 x 20 mL) to give a solid (134 mg) which was analysed by HPLC. An amount of crude (103 mg) was purified by preparative HPLC. The fractions containing the product were lyophilised to give L242 (4.5 mg; 2.9 x $10^{-3}$ mmol) as a white solid. Yield 1.3%.

**EXAMPLE XIII - Figures 13A-C**

**Synthesis of L244**

Summary: The product was obtained in several steps starting from the octapeptide GlnTrpAlaValGlyHisLeuMet$NH_2$ (BBN[7-14]) on the Rink amide resin (A). The final coupling step with DOTA tri-t-butyl ester was done in solution phase after cleavage and deprotection with reagent B of Linker-Bombesin [7-14]. The crude was purified by preparative HPLC to give L244. Overall yield: 0.4%.

A. *N,N'*-(Iminodi-2,1-ethanediyl)bis[2,2,2-trifluoroacetamide], A (FIG. 13A) Trifluoroacetic acid ethyl ester (50 g; 0.35 mol) was dropped into a solution of diethylenetriamine (18 g; 0.175 mol) in THF (180 mL) at 0˚C in 1 h. After 20 h at room temperature, the mixture was evaporated to an oily residue (54 g). The oil was crystallized from $Et_2O$ (50 mL), filtered, washed with cooled $Et_2O$ (2 x 30 mL) and dried to obtain **A** as a white solid (46 g; 0.156 mol). Yield 89%.

B. 4-[*N,N'*-Bis[2-(trifluoroacetyl)aminoethyl]amino]-4-oxobutanoic acid, B (FIG. 13A) Succinic anhydride (0.34 g; 3.4 mmol) was added in a solution of **A** (1 g; 3.4 mmol) in THF (5 mL) at room temperature. After 28 h the crude was concentrated to residue (1.59 g), washed with EtOAc (2 x 10 mL) and 1 N HCl (2 x 15 mL). The organic layer was dried on $Na_2SO_4$, filtered and evaporated to give an oily residue (1.3 g) that was purified by flash chromatography (5) to afford **B** as an oil (0.85 g; 2.15 mmol). Yield 63%.

C. 4-[*N,N'*- Bis[2-[(9-H-fluoren-9-ylmethoxy)carbonyl] aminoethyl] amino]-4-oxobutanoic acid, D (FIG. 13A) Succinic anhydride (2 g; 20 mmol) was added in a solution of **A** (5 g; 16.94 mmol) in THF (25 mL) at room temperature. After 28 h the crude was concentrated to residue (7 g), washed in ethyl acetate (100 mL) and in 1 N HCl (2 x 50 mL). The organic layer was dried on $Na_2SO_4$, filtered and evaporated to give crude **B** as an oily residue (6.53 g). 2 N NaOH (25 mL) was added to suspension of crude **B** (5 g) in EtOH (35 mL) obtaining a complete solution after 1 h at room temperature. After 20 h the solvent was evaporated to obtain **C** as an oil (8.48 g). A solution of 9-fluorenylmethyl chloroformate (6.54 g, 25.3 mmol) in 1,4-dioxane (30 mL), was dropped in the solution of **C** in 10% aq. $Na_2CO_3$ (30 mL) in 1 h at 0˚C. After 20 h at r.t. a gelatinous suspension was obtained and filtered to give a white solid (3.5 g) and a yellow solution. The solution was evaporated and the remaining aqueous solution was diluted in $H_2O$ (150 mL) and extracted with EtOAc (70 mL). Fresh EtOAc (200 mL) was added to aqueous phase, obtaining a suspension which was cooled to 0˚C and acidified to pH 2 with conc. HCl. The organic layer was washed with $H_2O$ (5 x 200 mL) until neutral pH, then dried to give a glassy solid (6.16 g). The compound was suspended in boiling *n*-Hexane (60 mL) for 1 h, filtered to give **D** as a white solid (5.53 g, 8.54 mmol). Overall yield 50%.

D. *N*-[4-[[4-[Bis[2-[[[4,7,10-tris(carboxymethyl)-1,4,7,10-tetraazacyclododec-1-yl]acetyl]amino]ethyl]amino-1,4-dioxobutyl]amino]benzoyl]-L-glutaminyl-L-tryptophyl-L-alanyl-L-valyl-glycyl-L-histidyl-L-leucyl-L-methioninamide, L244 (FIG. 13B) Resin A (0.5 g; 0.3 mmol) was shaken in a solid phase peptide synthesis vessel with 50% morpholine in DMA (7 mL) for 10 min, the solution emptied and fresh 50% morpholine in DMA (7 mL) was added. The suspension was stirred for another 20 min then the solution was emptied and the resin washed with DMA (5 x 7 mL). 4-[NN'-Bis[2-[(9-H-fluoren-9-yl methoxy) carbonyl] aminoethyl] amino]-4-oxo butanoic acid (777.3 mg; 1.2 mmol), HOBT (184 mg; 1.2 mmol), DIC (187 μL; 1.2 mmol) and DMA (7 mL) were added to the resin, the mixture shaken for 40 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for 20 min. The solution was emptied and the resin washed with DMA (2 x 7 mL) and with $CH_2Cl_2$ (5 x 7 mL) then it was shaken in a flask with Reagent B (25 mL) for 4.5 h. The resin was filtered and the solution was evaporated under reduced pressure to afford an oily crude that after treatment with $Et_2O$ (20 mL) gave a precipitate. The precipitate was collected by centrifugation and washed with $Et_2O$ (5 x 20 mL) to give **F** as a white solid (140 mg). DOTA tri-t-butyl ester (112 mg; 0.178 mmol) HATU (70 mg; 0.178 mmol) and DIEA (60 μL; 0.356 mmol) were added to a solution of **F** (50 mg; 0.0445 mmol) in DMA (3 mL) and $CH_2Cl_2$ (2 mL) and stirred for 24 h at room temperature. The crude was evaporated to reduced volume (1 mL) and shaken with Reagent B (25 mL) for 4.5 h. After evaporation of the solvent, the residue was treated with $Et_2O$ (20 mL) to give a precipitate. The precipitate was collected by centrifugation and washed with $Et_2O$ (5 x 20 mL) to afford a beige solid (132 mg) that was analyzed by HPLC. An amount of crude (100 mg) was purified by preparative HPLC. The fractions containing the product were lyophilized to give L244 (FIG. 13C) (3.5 mg; 1.84 x $10^{-3}$mmol) as a white solid. Yield 0.8 %.

**General Experimentals for Examples XIV-Example XLII**

**L201-L228**

Manual Couplings

6.0 equivalents of the appropriately protected amino acid was treated with 6.0 equivalents each of HOBT and DIC and activated outside the reaction vessel. This activated carboxylic acid in NMP was then transferred to the resin containing the amine and the reaction was carried out for 4-6 h and then the resin was drained and washed.

Special coupling of Fmoc-Gly-OH to 4-aminobenzoic acid and Aminobiphenylcarboxylic acid amides:

Fmoc-Gly-OH (10.0 equiv.) was treated with HATU (10.0 equiv.) and DIEA (20.0 equiv.) in NMP (10 mL of NMP was used for one gram of the amino acid by weight) and the solution was stirred for 10-15 min at RT before transferring to the vessel containing the amine loaded resin. The volume of the solution was made to 15.0 ml for every gram of the resin. The coupling was continued for 20h at RT and the resin was drained of all the reactants. This procedure was repeated one more time and then washed with NMP before moving on to the next step.

Preparation of D03A monoamide:

8.0 equivalents of DOTA mono acid was dissolved in NMP and treated with 8.0 equivalents of HBTU and 16.0 equivalents of DIEA. This solution was stirred for 15 min at RT and then transferred to the amine on the resin and the coupling was continued for 24h at RT. The resin was then drained, washed and then the peptide was cleaved and purified.

Cleavage of the crude peptides from the resin and purification:

The resin was suspended in Reagent B (15.0 ml/g) and shaken for 4h at RT. The resin was then drained and washed with 2 x 5 mL of Reagent B again and combined with the previous filtrate. The filtrate was then concentrated under reduced pressure to a paste/liquid at RT and triturated with 25.0 mL of anhydrous ether (for every gram of the resin used). The suspension was then centrifuged and the ether layer was decanted. This procedure was repeated two more times and the colorless precipitate after ether wash was purified by preparative HPLC.

**Example XIV - Figure 21**

**Synthesis of L201**

0.5 g of the Fmoc-Q(Trt)-W(Boc)-A-V-G-H(Trt)-M-Resin (0.4 mmol/g, 0.5g, 0.2 mmol) (Resin A) was used. The rest of the amino acid units were added as described in the general procedure to prepare (1R)-1-(Bis{2-[bis(carboxymethyl)amino]ethyl}amino)propane-3-carboxylic acid-1-carboxyl-glycyl-4-aminobenzoyl-L-glutaminyl-L-tryptophyl-L-alanyl-L-valyl-glycyl-L-histidyl-L-leucyl-L-methioninamide (L201), Yield: 17.0 mg (5.4%)

**Example XV - Figures 22A and 22B**

**Synthesis of L202**

4-Fmoc-hydrazinobenzoic acid (Fig 22A):

A suspension of 4-hydrazinobenzoic acid (5.0 g, 32.9 mmol) in water (100 ml) was treated with cesium carbonate (21.5 g, 66.0 mmol). Fmoc-Cl (9.1 g, 35.0 mmol) in THF (25 mL) was added dropwise to the above solution with stirring over a period of 1h. The solution was stirred for 4h more after the addition and the reaction mixture was concentrated to about 75 mL and extracted with ether (2 x 100 mL). The ether layer was discarded and the aqueous layer was acidified with 2N HCl. The separated solid was filtered, washed with water (5 x 100 mL) and then recrystallized from acetonitrile to yield the product (compound B) as a colorless solid. Yield: 11.0 g (89%). $^1$H NMR (DMSO-$d_6$) : δ 4.5 (m, 1H, Ar-C<u>H</u>$_2$-CH), 4.45 (m, 2H, Ar-C<u>H</u>$_2$), 6.6 (bs, 1H, Ar-<u>H</u>), 7.4 - 7.9 (m, 9, Ar-H and Ar-C<u>H</u>$_2$), 8.3 (s, 2H, Ar-<u>H</u>), 9.6 (s, 2H, Ar-<u>H</u>). M. S. - m/z 373.2 [M-H] 0.5 g of the Fmoc-Q(Trt)-W(Boc)-A-V-G-H(Trt)-M-Resin (0.4 mmol/g, 0.5g, 0.2 mmol) (Resin A) was used. The amino acid units were added as described in the general procedure, including Compound B to prepare *N*-[(3β, 5β, 12α)-3-[[[[4,7,10-Tris(carboxymethyl)-1,4,7,10-tetraazacyclododec-1-yl]acetyl]amino]acetyl]amino]-4-hydrazinobenzoyl-L-glutaminyl-L-tryptophyl-L-alanyl-L-valyl-glycyl-L-histidyl-L-leucyl-L-methioninamide (L202) (Fig. 22B), Yield: 25.0 mg (8.3%)

**Example XVI - Figures 23A and Fig. 23B**

**Synthesis of L203**

Preparation of 4-Boc-aminobenzyl benzoate Compound B Fig 23A:

A suspension of 4-boc-aminobenzoic acid (0.95 g, 4.0 mmol) in dry acetonitrile (10.0 mL) was treated with powdered cesium carbonate (1.3 g, 4.0 mmol) and stirred vigorously under nitrogen. Benzyl bromide (0.75 g, 4.4 mmol) was added and the reaction mixture was refluxed for 20h under nitrogen. The reaction mixture was then poured into ice cold water (200 mL) and the solid separated was filtered and washed with water (5 x 50 mL). The crude material was then recrystallized from aqueous methanol to yield the product as a colorless solid (Compound B). Yield: 0.8 g (61%). $^1$H NMR ($CDCl_3$): δ 1.5 (s, 9H, Tertiary methyls), 5.4 (s, 2H, Ar-C$\underline{H}_2$), 7.4 (m, 7H, Ar-$\underline{H}$) and 8.0 (m, 2H, Ar-$\underline{H}$). M. S. - m/z 326.1 [M+H]. 4-Aminobenzyl benzoate Compound C Fig 23A:

4-Boc-aminobenzyl benzoate (0.8 g, 2.5 mmol) was dissolved in DCM (20 mL) containing TFA (25% by volume) and stirred for 2h at RT. The reaction mixture was poured into 100.0 g of crushed ice and neutralized with saturated sodium bicarbonate solution until the pH reached about 8.5. The organic layer was separated and the aqueous layer was extracted with DCM (3 x 20 mL) and all the organic layers were combined. The DCM layer was then washed with 1 x 50 mL of saturated sodium bicarbonate, water (2 x 50 mL) and dried (sodium sulfate). Removal of the solvent yielded a colorless solid (compound C) that was taken to the next step without further purification. Yield: 0.51 g (91%). $^1$H NMR ($CDCl_3$): δ 5.3 (s, 2H, Ar-C$\underline{H}_2$), 6.6 (d, 2H, Ar-$\underline{H}$, *j* =1.0 Hz), 7.4 (m, 5H, Ar-$\underline{H}$, *J* = 1.0 Hz) and 7.9 (d, 2H, Ar-$\underline{H}$, *J* = 1.0 Hz).

4-(2-Chloroacetyl)aminobenzyl benzoate compound D Fig. 23A:

The amine (0.51 g, 2.2 mmol) was dissolved in dry dimethylacetamide (5.0 mL) and cooled in ice. Chloroacetyl chloride (0.28 g, 2.5 mmol) was added dropwise via a syringe and the solution was allowed to come to RT and stirred for 2h. An additional, 2.5 mmol of chloroacetyl chloride was added and stirring was continued for 2h more. The reaction mixture was then poured into ice cold water (100 mL). The precipitated solid was filtered and washed with water and then recrystallized from hexane/ether to yield a colorless solid (compound D). Yield: 0.38 g (56%). $^1$H NMR ($CDCl_3$): δ 4.25 (s, 2H, C$\underline{H}_2$-Cl), 5.4 (s, 2H, Ar-$\underline{H}$), 7.4 (m, 5H, Ar-$\underline{H}$), 7.6 (d, 2H, Ar-$\underline{H}$), 8.2 (d, 2H, Ar-$\underline{H}$) and 8.4 (s, 1H, -CON$\underline{H}$). *ter*-Butyl 2-{1,4,7,10-tetraaza-7,10-bis{[(tert-butyl)oxycarbonyl]methyl}-4-[(N-{4-[benzyloxycarbonyl]phenyl}carbamoyl] cyclododecyl}acetate, compound E Fig. 23A: DO3A-tri-*t*-butyl ester.HCl (5.24 g, 9.5 mmol) was suspended in 30.0 mL of dry acetonitrile and anhydrous potassium carbonate (2.76 g, 20 mmol) was added and stirred for 30 min. The chloroacetamide D (2.8 g, 9.2 mmol) in dry acetonitrile (20.0 mL) was then added dropwise to the above mixture for 10 min. The reaction mixture was then stirred overnight. The solution was filtered and then concentrated under reduced pressure to a paste. The paste was dissolved in about 200.0 mL of water and extracted with 5 x 50 mL of ethyl acetate. The combined organic layer was washed with water (2 x 100 mL) and dried (sodium sulfate). The solution was filtered and evaporated under reduced pressure to a paste and the paste was chromatographed over flash silica gel (600.0 g). Elution with 5% methanol in DCM eluted the product. All the fractions that were homogeneous on TLC were pooled and evaporated to yield a colorless gum. The gum was recrystallized from isopropylether and DCM to prepare compound E. Yield: 4.1 g (55%). $^1$H NMR ($CDCl_3$): δ 1.5 (s, 27H, methyls), 2.0 - 3.75 (m, 24H, NC$\underline{H}_2$s), 5.25 (d, 2H, Ar-C$\underline{H}_2$), 7.3 (m, 5H, Ar-$\underline{H}$), 7.8 (d, 2H, Ar-$\underline{H}$) and 7.95(d, 2H, Ar-$\underline{H}$). M. S. - m/z 804.3 [M+H].

Reduction of the above acid E to prepare compound F, Fig. 23A:

The benzyl ester E from above (1.0 g, 1.24 mmol) was dissolved in methanol-water mixture (10.0 mL, 95:5) and palladium on carbon was added (10%, 0.2 g). The solution was then hydrogenated using a Parr apparatus at 50.0 psi for 8h. The solution was filtered off the catalyst and then concentrated under reduced pressure to yield a colorless fluffy solid F. It was not purified further and was taken to the next step immediately. MS: m/z 714.3 [M+Na].

Preparation of L203 Fig. 23B

The above acid F was coupled to the amine on the resin [H-Q(Trt)-W(Boc)-A-V-G-H(Trt)-L-M-Resin] Resin A and F from above using standard coupling procedures described above. 0.5 g (0.2 mmol) of the resin yielded 31.5 mg of the final purified peptide (10.9%) *N*-[(3β,5β,12α)-3-[[[4,7,10-Tris(carboxymethyl)-1,4,7,10-tetraazacyclododec-1-yl] acetyl]amino]-4-aminobenzoyl-L-glutaminyl-L-tryptophyl-L-alanyl-L-valyl-glycyl-L-histidyl-L-leucyl-L-methioninamide (L203) (Fig. 23B).

**Example XVII - Figure 24**

**Synthesis of L204**

Fmoc-Q(Trt)-W(Boc)-A-V-G-H(Trt)-L-M-resin (0.5 g, 0.2 mmol) (resin A) was used. Fmoc-Gly-OH was loaded first followed by F from the above procedure (fig. 23A) employing standard coupling conditions. Yield: 24.5 mg (8.16%) of *N*-[(3β,5β,12α)-3-[[[4,7,10-Tris(carboxymethyl)-1,4,7,10-tetraazacyclododec-1-yl]acetyl]amino]-4-aminobenzoyl-glycyl-L-glutaminyl-L-tryptophyl-L-alanyl-L-valyl-glycyl-L-histidyl-L-leucyl-L-methioninamide (L204) (Fig. 24).

**Example XVIII - Figure 25**

**Synthesis of L205**

Fmoc-6-aminonicotinic acid[1] was prepared as described in the literature ("Synthesis of diacylhydrazine compiounds for therapeutic use". Hoelzemann, G.; Goodman, S. (Merck Patent G.m.b.H.., Germany). Ger.Offen. 2000, 16 pp. CODEN: GWXXBX DE 19831710 A1 20000120) and coupled with preloaded Fmoc-Q(Trt)-W(Boc)-A-V-G-H(Trt)-L-M-resin (0.5 g, 0.2 mmol) resin A, followed by the other amino groups as above to prepare *N*-[(3β,5β,12α)-3-[[[4,7,10-Tris(carboxymethyl)-1,4,7,10-tetraazacyclododec-1-yl]acetyl]amino]-4-aminobenzoyl-glycyl-L-glutaminyl-L-tryptophyl-L-alanyl-L-valyl-glycyl-L-histidyl-L-leucyl-L-methioninamide (L205) Yield: 1.28 mg (0.4%).

**Example XIX - Figures 26A and 26B**

**Synthesis of L206**

4'-Fmoc-amino-3'-methylbiphenyl-4-carboxylic acid B:

The amino acid (0.41 g, 1.8 mmol) was dissolved in a solution of cesium carbonate (0.98 g, 3.0 mmol) in 10.0 mL of water. See "Rational Design of Diflunisal Analogues with Reduced Affinity for Human Serum Albumin "Mao, H. et al J. Am. Chem. Soc., 2001, 123(43), 10429-10435. This solution was cooled in an ice bath and a solution of Fmoc-Cl (0.52 g, 2.0 mmol) in THF (10.0 mL) was added dropwise with vigorous stirring. After the addition, the reaction mixture was stirred at RT for 20h. The solution was then acidified with 2N HCl. The precipitated solid was filtered and washed with water (3 x 20 mL) and air dried. The crude solid was then recrystallized from acetonitrile to yield a colorless fluffy solid B Fig 26A. Yield: 0.66 g (75%). $^1$H NMR (DMSO-$d_6$): δ 2.2 (s, Ar-Me), 4.25 (t, 1H, Ar-C<u>H</u>, j = 5Hz), 4.5 (d, 2H, O-C<u>H</u>2, j = 5.0 Hz), 7.1 (bs, 1H, CON<u>H</u>), 7.4 - 8.0 (m, 8H, Ar-<u>H</u>) and 9.75 (bs, 1H, -COO<u>H</u>). M. S.: m/z 472.0 [M-H].

The acid B from above was coupled to Fmoc-Q(Trt)-W(Boc)-A-V-G-H(Trt)-L-M-resin (0.2g, 0.08 mmol) resin A with the standard coupling conditions. Additional groups were added as above to prepare *N*-[(3β,5β,12α)-3-[[[[[4,7,10-Tris(carboxymethyl)-1,4,7,10-tetraazacyclododec-1-yl]acetyl]amino]acetyl]amino]- [4'-Amino-2'-methyl biphenyl-4-carboxyl]-L-glutaminyl-L-tryptophyl-L-alanyl-L-valyl-glycyl-L-histidyl-L-leucyl-L-methioninamide (L206). yielded 30.5 mg (24%)

**Example XX - Figures 27A-B**

**Synthesis of L207**

3'-Fmoc-amino-biphenyl-3-carboxylic acid was prepared from the corresponding amine using the procedure described above. See "Synthesis of 3'-methyl-4-'-nitrobiphenylcarboxylic acids by the reaction of 3-methyl-4-nitrobenzenediazonium acetate with methyl benzoate", Boyland, E. and Gorrod, J., J. Chem. Soc., Abstracts (1962), 2209-11. 0.7G of the amine yielded 0.81 g of the Fmoc-derivative (58%) (Compound B, Fig. 27A). $^1$H NMR (DMSO-$d_6$): δ 4.3 (t, 1H, Ar-C<u>H</u>), 4.5 (d, 2H, O-C$\underline{H_2}$), 7.25-8.25 (m, 16H, Ar-<u>H</u>) and 9.9 (s, 1H, -COO<u>H</u>). M. S. - m/z 434 [M-H]

Fmoc-Q(Trt)-W(Boc)-A-V-G-H(Trt)-L-M-resin (0.2g, 0.08 mmol) resin A was coupled to the above acid B and additional groups as above (Fig. 27B). 29.0 mg of *N*-[(3β,5β,12α)-3-[[[[[4,7,10-Tris(carboxymethyl)-1,4,7,10-tetraazacyclododec-1-yl]acetyl]amino]acetyl]amino]- [3'-amino-biphenyl-3-carboxyl]-L-glutaminyl-L-tryptophyl-L-alanyl-L-valyl-glycyl-L-histidyl-L-leucyl-L-methioninamide (L207) was prepared (23%).

**Example XXI - Figure 28**

**Synthesis of L208**

Fmoc-Q(Trt)-W(Boc)-A-V-G-H(Trt)-L-M-resin (0.2g, 0.08 mmol) A was deblocked and coupled to terephthalic acid employing HATU as the coupling agent. The resulting acid on the resin was activated with DIC and NHS and then coupled to ethylenediamine. DOTA-mono acid was finally coupled to the amine on the resin. *N*-[(3β,5β,12α)-3-[[[[[4,7,10-Tris (carboxymethyl)- 1,4,7,10- tetraazacyclododec- 1- yl] acetyl] amino] acetyl] amino]-[1,2- diaminoethyl- terephthalyl]-L-glutaminyl-L-tryptophyl-L-alanyl-L-valyl-glycyl-L-histidyl-L-leucyl-L-methioninamide (L208) was prepared for a yield of 17.5 mg (14%)

**Example XXII - Figures 29A-B**

**Synthesis of L209**

Boc-Glu(G-OBn)-G-OBn:

Boc-Glutamic acid (5.0 g, 20.2 mol) was dissolved in THF (50.0 mL) and cooled to 0° C in an ice bath. HATU (15.61 g, 41.0 mmol) was added followed by DIEA (6.5 g, 50.0 mmol). The reaction mixture was stirred at 0° C for 30 min. Benzyl ester of glycine [8.45 g, 50 mmol, generated from neutralizing benzyl glycine hydrochloride with sodium carbonate and by extraction with DCM and solvent removal] was added in THF (25.0 mL). The reaction mixture was allowed to come to RT and stirred for 20h at RT. All the volatiles were removed under reduced pressure. The residue was treated with saturated sodium carbonate solution (100 mL) and extracted with ethyl acetate (3 x 100 mL). The organic layers were combined and washed with 1N HCl (2x 100 mL) and water (2 x 100 mL) and dried (sodium sulfate).

The solution was filtered and solvent was removed under reduced pressure to yield a paste that was chromatographed over flash silica gel (500.0 g). Elution with 2% methanol in DCM yielded the product as a colorless paste (compound B, Figure 29A). Yield: 8.5 g (74.5%). $^1$H NMR ($CDCl_3$): δ 1.4 (s, 9H, -C$\underline{H}_3$s), 2.0 - 2.5 (m, 4H,-CH-C$\underline{H}_2$ and CO-CH), 4.2 (m, 5H, N-C$\underline{H}_2$ -CO), 5.15 (s, 4H, Ar-C$\underline{H}_2$), 5.45 (bs, 1H, Boc-N$\underline{H}$), 7.3 (m, 10H, Ar-$\underline{H}$) and 7.6 (2bs, 2H, CON$\underline{H}$). M. S. - m/z 564.1 [M+H]. Analytical HPLC retention time - 8.29 min (>97% pure, 20-65%B over 15 min). H-Glu (G-OBn)-G-OBn:

The fully protected glutamic acid derivative (1.7 g, 3.2 mmol) B from above was dissolved in DCM/TFA (4:1, 20 mL) and stirred until the starting material disappeared on TLC (2h). The reaction mixture was poured into ice cold saturated sodium bicarbonate solution (200 mL) and the organic layer was separated and the aqueous layer was extracted with 2 x 50 mL of DCM and combined with the organic layer. The DCM layer was washed with saturated sodium bicarbonate (2 x 100 mL), water (2 x 100 mL) and dried (sodium sulfate). The solution was filtered and evaporated under reduced pressure and the residue was dried under vacuum to yield a glass (compound C, Figure 29A) that was taken to the next step without further purification. Yield: 0.72 g (95%). M. S. - m/z 442.2 [M+H]. (DOTA-tri-t-butyl)-Glu-(G-OBn)-G-OBn:

The amine C from above (1.33 g, 3 mmol) in anhydrous DCM (10.0 mL) was added to an activated solution of DOTA-tri-t-butyl ester [2.27 g, 3.6 mmol was treated with HBTU, 1.36 g, 3.6 mmol and DIEA 1.04 g, 8 mmol and stirred for 30 min at RT in 25 mL of dry DCM] and stirred at RT for 20h]. The reaction mixture was diluted with 200 mL of DCM and washed with saturated sodium carbonate (2 x 150 mL) and dried (sodium sulfate). The solution was filtered and solvent was removed under reduced pressure to yield a brown paste. The crude product was chromatographed over flash silica gel (500.0 g). Elution with 2% methanol in DCM furnished the product as a colorless gum (compound D, Figure 29A). Yield: 1.7 g (56.8%). $^1$H NMR ($CDCl_3$): δ 1.3 and 1.4 (2s, 9H, three methyls each from the free base and the sodium adduct of DOTA), 2.0 - 3.5 (m, 20H, N-CH and -CH-$CH_2$ and CO-C$\underline{H}_2$), 3.75 - 4.5 (m, 13H, N-C$\underline{H}_2$-CO), 5.2 (m, 4H, Ar-C$\underline{H}_2$) and 7.25 (m, 10H, Ar-$\underline{H}$). M. S. m/z - 1018.3 [M+Na] and 996.5 [M+H] and 546.3 [M+Na+H]/2. HPLC - Retention Time: 11.24 min (>90%, 20-80% B over 30 min). (DOTA-tri-*t*-butyl)-Glu-(G-OH)-G-OH:

The bis benzyl ester (0.2 g, 0.2 mmol) D from above was dissolved in methanol-water (20 mL, 9:1) and hydrogenated at 50 psi in the presence of 10% Pd/C catalyst (0.4 g, 50% by wt. water). After the starting material disappeared on HPLC and TLC (4h), the solution was filtered off the catalyst and the solvent was removed under reduced pressure and the residue was dried under high vacuum for about 20h (<0.1 mm) to yield the product as a colorless foam (Compound E, Figure 29A). Yield: 0.12 g (73.5%). $^1$H NMR (DMSO-$d_6$): δ 1.3 and 1.4 (2s, 9H corresponding to methyls of free base and the sodium adduct of DOTA), 1.8 - 4.7 (m, 33H, NC$\underline{H}_2$s, COC$\underline{H}_2$ and CH-$CH_2$ and NH-C$\underline{H}$-CO), 8.1, 8.2 and 8.4 (3bs, N$\underline{H}$CO). M. S.: m/z - 816.3 [M+H] and 838.3 [M+Na]. HPLC Retention Time: 3.52 min (20-80% B over 30 min, > 95% pure).

H-8-amino-3,6-dioxaoctanoyl-8-amino-3,6-dioxaoctanoyl-Gln-Trp-Ala-Val-Gly-His-Leu-Met-$NH_2$:

Fmoc-Q(Trt)-W(Boc)-A-V-G-H(Trt)-L-M-resin (0.5 g, 0.2 mmol) A was deblocked and coupled twice sequentially to 8-amino-3,6-dioxaoctanoic acid to yield the above deprotected peptide (compound F, Figure 29B) after preparative HPLC purification. Yield: 91.0 mg (37%).

HPLC Retention Time: 8.98 min (>95% purity, 10-40% B in over 10 min). M. S.: m/z - 1230.6 [M+H], 615.9 [M+2H]/2.

Solution phase coupling of the bis-acid E and the amine F from above (Figure 29B) The bis-acid (13.5 mg, 0.0166 mmol) E was dissolved in 100μL of dry acetonitrile and treated with NHS (4.0 mg, 0.035 mmol) and DIC (5.05 mg, 0.04 mmol) and stirred for 24h at RT. To the above activated acid, the free amine F (51.0 mg, 0.41 mmol)[generated from the TFA salt by treatment with saturated sodium bicarbonate and freeze drying the solution to yield the amine as a fluffy solid] was added followed by 100μL of NMP and the stirring was continued for 40h more at RT. The solution was diluted with anhydrous ether (10 mL) and the precipitate was collected by centrifugation and washed with 2 x 10 mL of anhydrous ether again. The crude solid was then purified by preparative HPLC to yield the product as a colorless fluffy solid L209 as in Figure 29B with a yield of 7.5 mg (14.7%).

## Example XXIII - Figures 30A-B

### Synthesis of L210

H-8-aminooctanoyl-8-aminooctanoyl-Gln-Trp-Ala-Val-Gly-His-Leu-Met-$NH_2$: This was also prepared exactly the same way as in the case of compound F Fig. 29B, but using 1-aminooctanoic acid and the amine (compound B, Fig. 30A) was purified by preparative HPLC. Yield: 95.0 mg (38.9%). HPLC Retention Time: 7.49 min (>95% purity; 10-40%B over 10.0 min). M. S.: m/z - 1222.7 [M+H], 611.8 [M+2H]/2. (DOTA-tri-t-butyl)-Glu-(G-OH)-G-OH (0.0163 g, 0.02 mmol) was converted to its bis-NHS ester as in the case of L209 in 100μL of acetonitrile and treated with the free base, Compound B (60.0 mg, 0.05 mmol) in 100μL of NMP and the reaction was continued for 40h and then worked up and purified as above to prepare L210 Fig. 30B for a yield of 11.0 mg (18%).

## Example XXIV - Figure 31

### Synthesis of L211

Prepared from 0.2 g 0f the Fmoc-Q(Trt)-W(Boc)-A-V-G-H(Trt)-L-M-resin (0.08 mmol) using standard protocols. *N*-[(3β, 5β, 12)- 3-[[[4,7,10- Tris (carboxymethyl)- 1,4,7,10- tetraazacyclododec- 1- yl] acetyl] amino]-glycyl- glycyl- 4- aminobenzoyl-L-glutaminyl-L-tryptophyl-L-alanyl-L-valyl-glycyl-L-histidyl-L-leucyl-L-m ethioninamide L211 was prepared in a yield of 4.7 mg (3.7%) (Fig. 31).

## Example XXV - Figure 32

### Synthesis of L212

Prepared from Rink Amide Novagel resin (0.47 mmol/g, 0.2 g, 0.094 mmol) by building the sequence on the resin by standard protocols. *N*-[(3β,5β,12α)-3-[[[4,7,10-Tris(carboxymethyl)-1,4,7,10-tetraazacyclododec-1-yl]acetyl] amino]-glycyl- 4- aminobenzoyl- L- glutamyl- L- tryptophyl- L- alanyl- L- valyl- glycyl- L- histidyl- L- leucyl- L- methioninamide L212 was prepared for a yield of 25.0 mg (17.7%) (Fig. 32).

## Example XXVI - Figure 33

### Synthesis of L213

Prepared from Fmoc-Met-2-chlorotrityl chloride resin (NovaBioChem, 0.78 mmol/g, 0.26 g, 0.2 mmol) and the rest of the sequence were built using standard methodology. N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-tryptophyl-L-alanyl-L-valyl-glycyl-L-histidyl-L-leucyl-L-methionine **L213** was prepared for a yield of 49.05 mg (16.4%) (Fig. 33).

**Example XXVII - Figure 34**

**Synthesis of L214**

Fmoc-Q(Trt)-W(Boc)-A-V-G-H(Trt)-L-M-resin (0.2 g, 0.08 mmol) A was used to prepare N-[(3β,5β,12α)-3-[[[4,7,10-Tris(carboxymethyl)-1,4,7,10-tetraazacyclododec-1-yl]acetyl]amino]-glycyl-4-aminobenzoyl-D-phenylalanyl-L-glutaminyl-L-tryptophyl-L-alanyl-L-valyl-glycyl-L-histidyl-L-leucyl-L-methioninamide L214 using standard conditions. 8.5 mg of the product (6.4%) was obtained (Fig. 34).

**Example XXVIII - Figure 35**

**Synthesis of L215**

Fmoc-Q(Trt)-W(Boc)-A-V-G-H(Trt)-L-M-resin (0.2 g, 0.08 mmol) A was used to prepare N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-arginyl-L-leucyl-glycyl-L-asparginyl-L-glutaminyl-L-tryptophyl-L-alanyl-L-valyl-glycyl-L-histidyl-L-leucyl-L-methioninamide L215. 9.2 mg (5.5%) was obtained (Figure 35).

**Example XXIX - Figure 36**

**Synthesis of L216**

Fmoc-Q(Trt)-W(Boc)-A-V-G-H(Trt)-L-M-resin (0.2 g, 0.08 mmol) A was used to prepare N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-arginyl-L-tyrosinyl-glycyl-L-asparginyl-L-glutaminyl-L-tryptophyl-L-alanyl-L-valyl-glycyl-L-histidyl-L-leucyl-L-methioninamide L216. 25.0 mg (14.7%) was obtained (Figure 36).

**Example XXX - Figure 37**

**Synthesis of L217**

Fmoc-Q(Trt)-W(Boc)-A-V-G-H(Trt)-L-M-resin A (0.2g, 0.08 mmol) was used to prepare N-[(3β,5β,12α)-3-[[[4,7,10-Tris(carboxymethyl)-1,4,7,10-tetraazacyclododec-1-yl]acetyl]amino]-glycyl-4-aminobenzoyl-L-glutaminyl-L-lysyl-L-tyrosinyl-glycyl-L-glutaminyl-L-tryptophyl-L-alanyl-L-valyl-glycyl-L-histidyl-L-leucyl-L-methioninamide L217. 58.0 mg (34.7%) was obtained (Figure 37).

**Example XXXI - Figure 38**

**Synthesis of L218**

Fmoc-Q(Trt)-W(Boc)-A-V-G-H(Trt)-L-M-resin A (0.2g, 0.08 mmol) was used. Fmoc-Lys(ivDde) was employed for the introduction of lysine. After the linear sequence was completed, the protecting group of the lysine was removed using 10% hydrazine in DMF (2 x 10 mL; 10 min each and then washed). The rest of the amino acids were then introduced using procedures described in the "general" section to complete the required peptide sequence. L218 in Figure 38 as obtained in a yield of 40.0 mg (23.2%).

**Example XXXII- Figure 39**

**Synthesis of L219**

4-Sulfamylbutyryl AM Novagel resin was used (1.1 mmol/g; 0.5 g; 0.55 mmol). The first amino acid was loaded on to this resin at -20° C for 20h. The rest of the sequence was completed utilizing normal coupling procedures. After washing, the resin was alkylated with 20.0 eq. of iodoacetonitrile and 10.0 equivalents of DIEA for 20h. The resin was then drained of the liquids and washed and then cleaved with 2.0 eq. of pentylamine in 5.0 mL of THF for 20h. The resin was then washed with 2x5.0 mL of THF and all the filtrates were combined. THF was then evaporated under reduced pressure and the residue was then deblocked with 10.0 mL of Reagent B and the peptide *N*-[(3β,5β,12α)-3-[[[4,7,10-Tris(carboxymethyl)-1,4,7,10-tetraazacyclododec-1-yl]acetyl] amino]-glycyl-4-aminobenzoyl-D-phenylalanyl-L-glutaminyl-L-tryptophyl-L-alanyl-L-valyl-glycyl-L-histidyl-L-leucyl-aminopentyl, L219 was purified as previously described. 28.0

mg (2.8%) was obtained (Figure 39)

**Example XXXIII- Figure 40**

**Synthesis of L220**

NovaSyn TGR (0.25 mmol/g; 0.15 g, 0.05 mmol) resin A was used to prepare N-[(3β,5β,12α)-3-[[[4,7,10-Tris (carboxymethyl)-1,4,7,10-tetraazacyclododec-1-yl]acetyl]amino]-glycyl-4-aminobenzoyl-L-glutaminyl-L-tryptophyl-L-alanyl-L-valyl-D-alanyl-L-histidyl-L-leucyl-L-methioninamide, L220. 31.5 mg (41.4%) was obtained (Figure 40).

**Example XXXIV - Figure 41**

**Synthesis of L221**

NovaSyn TGR (0.25 mmol/g; 0.15 g, 0.05 mmol) resin A was used to prepare N-[(3β,5β,12α)-3-[[[4,7,10-Tris (carboxymethyl)-1,4,7,10-tetraazacyclododec-1-yl]acetyl]amino]-glycyl-4-aminobenzoyl-D-phenylalanyl-L-glutaminyl-L-tryptophyl-L-alanyl-L-valyl-glycyl-L-histidyl-L-leucyl-L-leucinamide, L221. 28.0 mg (34.3%) was obtained (Figure 41).

**Example XXXV - Figure 42**

**Synthesis of L222**

NovaSyn TGR (0.25 mmol/g; 0.15 g, 0.05 mmol) resin A was used to prepare N-[(3β,5β,12α)-3-[[[4,7,10-Tris (carboxymethyl)-1,4,7,10-tetraazacyclododec-1-yl]acetyl]amino]-glycyl-4-aminobenzoyl-D-tyrosinyl-L-glutaminyl-L-tryptophyl-L-alanyl-L-valyl-betaalanyl-L-histidyl-L-phenylalanyl-L-norleucinamide, L222. 34.0 mg (40.0%) was obtained (Figure 42).

**Example XXXVI - Figure 43**

**Synthesis of L223**

NovaSyn TGR (0.25 mmol/g; 0.15 g, 0.05 mmol) resin A was used to prepare N-[(3β,5β,12α)-3-[[[4,7,10-Tris (carboxymethyl)-1,4,7,10-tetraazacyclododec-1-yl]acetyl]amino]-glycyl-4-aminobenzoyl-L-phenylalanyl-L-glutaminyl-L-tryptophyl-L-alanyl-L-valyl-betaalanyl-L-histidyl-L-phenylalanyl-L-norleucinamide, L223. 31.2 mg (37.1%) was obtained (Figure 43).

**Example XXXVII - Figure 44**

**Synthesis of L224**

NovaSyn TGR (0.25 mmol/g; 0.15 g, 0.05 mmol) resin A was used to prepare N-[(3β,5β,12a)-3-[[[4,7,10-Tris (carboxymethyl)-1,4,7,10-tetraazacyclododec-1-yl]acetyl]amino]-glycyl-4-aminobenzoyl-L-glutaminyl-L-tryptophyl-L-alanyl-glycyl-L-histidyl-L-phenylalanyl-L-leucinamide, L224. 30.0 mg (42.2%) was obtained (Figure 44).

**Example XXXVIII - Figure 45**

**Synthesis of L225**

NovaSyn TGR (0.25 mmol/g; 0.15 g, 0.05 mmol) resin A was used to prepare N-[(3β,5β,12α)-3-[[[4,7,10-Tris (carboxymethyl)-1,4,7,10-tetraazacyclododec-1-yl]acetyl]amino]-glycyl-4-aminobenzoyl-L-leucyl-L-tryptophyl-L-alanyl-L-valinyl-glycyl-L-serinyl-L-phenylalanyl-L-methioninamide, L225. 15.0 mg (20.4%) was obtained (Figure 45).

**Example XXXIX - Figure 46**

**Synthesis of L226**

NovaSyn TGR (0.25 mmol/g; 0.15 g, 0.05 mmol) resin A was used to prepare N-[(3β,5β,12α)-3-[[[4,7,10-Tris (carboxymethyl)-1,4,7,10-tetraazacyclododec-1-yl]acetyl]amino]-glycyl-4-aminobenzoyl-L-histidyl-L-tryptophyl-L-alanyl-L-valyl-glycyl-L-histidyl-L-leucyl-L-methioninamide, L226. 40.0 mg (52.9%) was obtained (Figure 46).

**Example XL - Figure 47**

**Synthesis of L227**

NovaSyn TGR (0.25 mmol/g; 0.15 g, 0.05 mmol) resin A was used to prepare *N*-[(3β,5β,12α)-3-[[[4,7,10-Tris (carboxymethyl)-1,4,7,10-tetraazacyclododec-1-yl]acetyl]amino]-glycyl-4-aminobenzoyl-L-leucyl-L-tryptophyl-L-alanyl-L-threonyll-glycyl-L-histidyl-L-phenylalanyl-L-methioninamide L227. 28.0 mg (36.7%) was obtained (Figure 47).

**Example XLI- Figure 48**

**Synthesis of L228**

NovaSyn TGR (0.25 mmol/g; 0.15 g, 0.05 mmol) resin A was used to prepare N-[(3β,5β,12α)-3-[[[4,7,10-Tris (carboxymethyl)-1,4,7,10-tetraazacyclododec-1-yl]acetyl]amino]-glycyl-4-aminobenzoyl-L-glutaminyl-L-tryptophyl-L-alanyl-L-valyl-glycyl-L-histidyl-L-phenylalanyl-L-methioninamide, L228. 26.0 mg (33.8%) was obtained (Figure 48).

**EXAMPLE XLII - Synthesis of Additional GRP Compounds**

A. General procedure for the preparation of 4,4'-Aminomethylbiphenylcarboxylic acid (**B2**) and 3,3'-aminomethylbiphenylcarboxylic acid (**B3**):

1. Methyl-hydroxymethylbiphenylcarboxylates:

Commercially available (Aldrich Chemical Co.) 4-hydroxymethylphenylboric acid or 3-hydroxymethylphenylboric acid (1.0 g, 6.58 mmol) was stirred with isopropanol (10 mL) and 2M sodium carbonate (16 mL) until the solution became homogeneous. The solution was degassed by passing nitrogen through the solution and then treated with solid methyl-3-bromobenzoate, or methyl-4-bromobenzoate (1.35 g, 6.3 mmol) followed by the Pd (0) catalyst {$[(C_6H_5)_3P]_4Pd$; 0.023g, 0.003 mmol}. The reaction mixture was kept at reflux under nitrogen until the starting bromobenzoate was consumed as determined by TLC analysis (2-3 h). The reaction mixture was then diluted with 250 mL of water and extracted with ethyl acetate (3 x 50 mL). The organic layers were combined and washed with saturated sodium bicarbonate solution (2 x 50 mL) and dried ($Na_2SO_4$). The solvent was removed under reduced pressure and the residue was chromatographed over flash silica gel (100 g). Elution with 40% ethyl acetate in hexanes yielded the product either as a solid or oil.

Yield:

B2 -0.45g (31%); m. p. -170-171° C.

B3 - 0.69 g (62%); oil.

$^1H$ NMR ($CDCl_3$) δ B2- 3.94 (s,3H, $-COOCH_3$), 4.73 (s, 2H, $-CH_2$-Ph), 7.475 (d, 2H, J= 5Hz), 7.6 (d, 2H, J = 10 Hz), 7.65 (d, 2H, J = 5Hz) and 8.09 (d, 2H, J = 10 Hz). M. S. - m/e - 243.0 [M+H]

B3 - 3.94 (s, 3H, $-COOCH_3$), 4.76 (s, 2H, $-CH_2$-Ph), 7.50 (m, 4H), 7.62 (s, 1H), 7.77 (s, 1H), 8.00 (s, 1H) and 8.27 (s, 1H).

M. S. - m/e - 243.2 [M+H]

2. Azidomethylbiphenyl carboxylates:

The above biphenyl alcohols (2.0 mmol) in dry dichloromethane (10 mL) were cooled in ice and treated with diphenylphosphoryl azide (2.2 mol) and DBU (2.0 mmol) and stirred under nitrogen for 24h. The reaction mixture was diluted with water and extracted with ethyl acetate (2 x 25 mL).The organic layers were combined and washed successfully with 0.5 M citric acid solution (2 x 25 mL), water (2 x 25 mL) and dried ($Na_2SO_4$). The solution was filtered and evaporated under reduced pressure to yield the crude product. The 4,4'-isomer was crystallized from hexane/ether and the 3,3'-isomer was triturated with isopropyl ether to remove all the impurities; the product was homogeneous as determined on TLC analysis and further purification was not required.

Yield:

Methyl-4-azidomethyl-4-biphenylcaroxylate - 0.245 g (46%); m. p. - 106-108° C. Methyl-4-azidomethyl-4-biphenylcaroxylate - 0.36 g (59%, oil)

$^1$H NMR ($CDCl_3$) δ - 4,4'-isomer - 3.95 (s, 3H, $-COOCH_3$), 4.41 (s, 2H, $-CH_2N_3$), 7.42 (d, 2H, J = 5 Hz), 7.66 (m, 4H) and 8.11 (d, 2H, J = 5 Hz) 3,3'-Isomer - 3.94 (s, 3H, $-COOCH_3$), 4.41 (s, 2H, $-CH_2N_3$), 7.26-7.6 (m, 5H), 7.76 (d, 1H, J = 10 Hz), 8.02 (d, 1H, J = 5 Hz) and 8.27 (s, 1H).

3. Hydrolysis of the methyl esters of biphenylcarboxylates:

About 4 mmol of the methyl esters were treated with 20 mL of 2M lithium hydroxide solution and stirred until the solution was homogeneous (20-24 h). The aqueous layer was extracted with 2 x 50 mL of ether and the organic layer was discarded. The aqueous layer was then acidified with 0.5 M citric acid and the precipitated solid was filtered and dried. No other purification was necessary and the acids were taken to the next step.

Yield:

4,4'-isomer - 0.87 g of methyl ester yielded 0.754 g of the acid (86.6%); m. p. - 205-210° C 3,3'-isomer - 0.48 g of the methyl ester furnished 0.34 g of the acid (63.6%); m. p. - 102-105° C.

$^1$H NMR (DMSO-$d_6$) δ : 4,4'-isomer - 4.52 (s, 2H, $-CH_2N_3$), 7.50 (d, 2H, J = 5 Hz), 7.9 (m, 4H), and 8.03 (d, 2H, J = 10 Hz)

3,3'-isomer - 4.54 (s, 2H, $-CH_2N_3$), 7.4 (d, 1H, J = 10 Hz), 7.5-7.7 (m, 4H), 7.92 (ABq, 2H) and 8.19 (s, 1H).

4. Reduction of the azides to the amine:

This was carried out on the solid phase and the amine was never isolated. The azidocarboxylic acid was loaded on the resin using the standard peptide coupling protocols. After washing, the resin containing the azide was shaken with 20 equivalents of triphenylphosphine in THF/water (95:5) for 24 h. The solution was drained under a positive pressure of nitrogen and then washed with the standard washing procedure. The resulting amine was employed in the next coupling.

5. (3β, 5β, 7α, 12α)-3-[{(9H-Flouren-9ylmethoxy)amino]acetyl}amino-7,12-dihydroxycholan-24-oic acid:

Tributylamine (3.2 mL); 13.5 mmol) was added dropwise to a solution of Fmoc-glycine (4.0 g, 13.5 mmol) in THF (80 mL) stirred at 0° C. Isobutylchloroformate (1.7 mL; 13.5 mmol) was subsequently added and, after 10 min, a suspension of tributylamine (2.6 mL; 11.2 mmol) and (3β, 5β, 7α, 12α)-3-amino-7,12-dihydroxycholan-24-oic acid (4.5 g; 11.2 mmol) in DMF (80 mL) was added dropwise, over 1 h, into the cooled solution. The mixture was allowed to warm up to ambient temperature and after 6 h, the solution was concentrated to 120 mL, then water (180 mL) and 1N HCl (30 mL) were added (final pH 1.5). The precipitated solid was filtered, washed with water (2 x 100 mL), vacuum dried and purified by flash chromatography. Elution with chloroform/methanol (8:2) yielded the product as a colorless solid.

Yield: 1.9g (25%). TLC: $R_f$ 0.30 ($CHCl_3$/MeOH/$NH_4OH$ - 6:3:1).

**IN VITRO AND IN VIVO TESTING OF COMPOUNDS**

**Example XLIII: *In vitro* Binding Assay for GRP Receptors in PC3 Cell Lines-Figures 14 A-B**

To identify potential lead compounds, an in vitro assay that identifies compounds with high affinity for GRP-R was used. Since the PC3 cell line, derived from human prostate cancer, is known to exhibit high expression of GRP-R on the cell surface, a radio ligand binding assay in a 96-well plate format was developed and validated to measure the binding of $^{125}$I-BBN to GRP-R positive PC3 cells and the ability of the compounds of the invention to inhibit this binding. This assay was used to measure the $IC_{50}$ for RP527 ligand, L134 ligand (controls) and compounds of the invention which inhibit the binding of $^{125}$I-BBN to GRP-R. (RP527 =N,N-dimethylglycine-Ser-Cys(Acm)-Gly-5-aminopentanoic acid-BBN (7-14) [SEQ. ID.NO: 1], which has MS = 1442.6 and IC50 - 0.84). Van de Wiele C, Dumont F et al., Technetium-99m RP527, a GRP analogue for visualization of GRP receptor-expressing malignancies: a feasibility study. Eur.J.Nucl.Med., (2000) 27; 1694-1699.; L134= DO3A-monoamide-8-amino-octanoic acid-BBN (7-14) [SEQ. ID. NO: 1], which has MS=1467.0. L134 is DO3A monoamide-aminooctanyl-BBN[7-14].

The Radioligand Binding Plate Assay was validated for BBN and BBN analogues (including commercially available BBN and L1) and also using $^{99m}$Tc RP527 as the radioligand.

A. Materials and Method:

1. Cell culture:

PC3 (human prostate cancer cell line) were obtained from the American Type Culture Collection and cultured in RPMI 1640 in tissue culture flasks (Corning). This growth medium was supplemented with 10% heat inactivated FBS (Hyclone, SH30070.03), 10 mM HEPES (GibcoBRL, 15630-080), and antibiotic/antimycotic (GibcoBRL, 15240-062) for a final concentration of penicillin-streptomycin (100 units/mL), and fungizone (0.25 μg/mL). All cultures were maintained in a humidified atmosphere containing 5% $CO_2$/95% air at 37°C, and passaged routinely using 0.05% trypsin/EDTA (GibcoBRL 25300-054) where indicated. Cells for experiments were plated at a concentration of $2.0x10^4$ /well either in 96-well white /clear bottom microtiter plates (Falcon Optilux-I) or 96 well black/clear collagen I cellware plates (Beckton Dickinson Biocoat). Plates were used for binding studies on day 1 or 2 post-plating.

2. Binding buffer:

RPMI 1640 supplemented with 20mM HEPES, 0.1% BSA (w/v), 0.5 mM PMSF (AEBSF), bacitracin (50 mg/ 500 ml), pH 7.4.

$^{125}$I-BBN (carrier free, 2200 Ci/mmole) was obtained from Perkin-Elmer.

B. Competition assay with $^{125}$I-BBN for GRP-R in PC3 cells:

A 96-well plate assay was used to determine the $IC_{50}$ of various compounds of the invention to inhibit binding of $^{125}$I-BBN to human GRP-R. The following general procedure was followed:

All compounds tested were dissolved in binding buffer and appropriate dilutions were also done in binding buffer. PC3 cells (human prostate cancer cell line) for assay were plated at a concentration of $2.0x10^4$ /well either in 96-well white /clear bottomed microtiter plates (Falcon Optilux-I) or 96 well black/clear collagen I cellware plates (Beckton Dickinson Biocoat). Plates were used for binding studies on day 1 or 2 post-plating. The plates were checked for confluency (>90% confluent) prior to assay. For the assay, RP527 or L134 ligand, (controls), or compounds of the invention at concentrations ranging from $1.25x10^{-9}$ M to $5x10^{-9}$ M, was co-incubated with $^{125}$I-BBN (25,000 cpm/well). These studies were conducted with an assay volume of 75 μl per well. Triplicate wells were used for each data point. After the addition of the appropriate solutions, plates were incubated for 1 h at 4°C to prevent internalization of the ligand-receptor complex. Incubation was ended by the addition of 200 μl of ice-cold incubation buffer. Plates were washed 5 times and blotted dry. Radioactivity was detected using either the LKB CompuGamma counter or a microplate scintillation counter.

Competition binding curves for RP527 (control) and L70, a compound of the invention can be found in Figures 14A-B These data show that the IC50 of the RP527 control is 2.5nM and that of L70, a compound of this invention is 5nM. The IC50 of the L134 control was 5nM. IC50 values for those compounds of the invention tested can be found in Tables 1-3, supra, and show that they are comparable to that of the controls and thus would be expected to have sufficient affinity for the receptor to allow uptake by receptor bearing cells in vivo.

C. Internalization & Efflux assay:

These studies were conducted in a 96-well plate. After washing to remove serum proteins, PC3 cells were incubated with $^{125}$I-BBN, $^{177}$Lu-L134 or radiolabelled compounds of this invention for 40 min, at 37 °C. Incubations were stopped by the addition of 200 μl of ice-cold binding buffer. Plates were washed twice with binding buffer. To remove surface-bound radioligand, the cells were incubated with 0.2M acetic acid (in saline), pH 2.8 for 2 min. Plates were centrifuged and the acid wash media were collected to determine the amount of radioactivity which was not internalized. The cells were collected to determine the amount of internalized $^{125}$I-BBN, and all samples were analyzed in the gamma counter. Data for the internalization assay was normalized by comparing counts obtained at the various time points with the counts obtained at the final time point (T40 min).

For the efflux studies, after loading the PC3 cells with $^{125}$I-BBN or radiolabelled compounds of the invention for 40 min at 37°c, the unbound material was washed off, and the % of internalization was determined as above. The cells were then resuspended in binding buffer at 37°C for up to 3h At 0.5, 1, 2, or 3 h. the amount remaining internalized relative to the initial loading level was determined as above and used to calculate the percent efflux recorded in Table 5.

Table 5

| Internalisation and efflux ofI-BBN and the Lu-177 complexes of L134 (control) and compounds of this invention | | | | | |
|---|---|---|---|---|---|
| | **I-BBN** | **L134** | **L63** | **L64** | **L70** |
| **Internalisation (40 minutes)** | 59 | 89 | 64 | 69 | 70 |
| **Efflux (2h)** | 35 | 28 | 0 | 20 | 12 |

These data show that the compounds of this invention are internalized and retained by the PC3 cells to a similar extent to the controls.

**Example XLIV - Preparation of Tc-labeled GRP compounds.**

Peptide solutions of compounds of the invention identified in Table 6 were prepared at a concentration of 1 mg/mL in 0.1% aqueous TFA. A stannous chloride solution was prepared by dissolving $SnCl_2.2H_2O$ (20 mg/mL) in 1 N HCl. Stannous gluconate solutions containing 20 μg of $SnCl_2.2H_2O$/100 μL were prepared by adding an aliquot of the $SnCl_2$ solution (10 μL) to a sodium gluconate solution prepared by dissolving 13 mg of sodium gluconate in water. A hydroxypropyl gamma cyclodextrin [HP-γ-CD] solution was prepared by dissolving 50 mg ofHP-γ-CD in 1 mL of water.

The $^{99m}$Tc labeled compounds identified below were prepared by mixing 20 μL of solution of the unlabeled compounds (20 μg), 50 μL of HP-γ-CD solution, 100 μL of Sn-gluconate solution and 20 to 50 μL of $^{99m}$Tc pertechnetate (5 to 8 mCi, Syncor). The final volume was around 200 μL and final pH was 4.5 - 5. The reaction mixture was heated at 100°C for 15 to 20 min. and then analyzed by reversed phase HPLC to determine radiochemical purity (RCP). The desired product peaks were isolated by HPLC, collected into a stabilizing buffer containing 5 mg/mL ascorbic acid, 16 mg/mL HP-γ-CD and 50 mM phosphate buffer, pH 4.5, and concentrated using a speed vacuum to remove acetonitrile. The HPLC system used for analysis and purification was as follows: C18 Vydac column, 4.6 x 250 mm, aqueous phase: 0.1% TFA in water, organic phase: 0.085% TFA in acetonitrile. Flow rate: 1 mL/min. Isocratic elution at 20% - 25% acetonitrile/0.085% TFA was used, depending on the nature of individual peptide.

Labeling results are summarized in Table 6.

Table 6

| Compound[1] | Sequence[2] | HPLC retention time (min) | Initial RCP[3] (%) | RCP[4] (%) immediately following purification |
|---|---|---|---|---|
| **L2** | -RJQWAVGHLM | 5.47 | 89.9 | 95.6 |
| **L4** | -SJQWAVGHI,M | 5.92 | 65 | 97 |
| **L8** | -JKQWAVGHLM | 6.72 | 86 | 94 |
| **L1** | -KJQWAVGHLM | 5.43 | 88.2 | 92.6 |
| **L9** | -JRQWAVGHLM | 7.28 | 91.7 | 96.2 |
| **L7** | -aJQWAVGHLM | 8.47 | 88.6 | 95.9 |

n.d. = not detected
1: All compounds were conjugated with an N,N'-dimethylglycine-Ser-Cys-Gly metal chelator. The Acm protected form of the ligand was used. Hence, the ligand used to prepare the 99mTc complex of L2 was N,N'-dimethylglycine-Ser-Cys (Acm)-Gly-RJQWAVGHLM. The Acm group was removed during chelation to Tc.
2: In the Sequence, "J" refers to 8-amino-3,6-dioxaoctanoic acid and "a" refers to D-alanine.
3: Initial RCP measurement taken immediately after heating and prior to HPL purification.
4: RCP determined following HPLC isolation and acetonitrile removal via speed vacuum.

**Example XLV - Preparation of 177 Lu-L64 for cell binding and biodistribution studies:**

This compound was synthesized by incubating 10 μg L64 ligand (10 μL of a 1 mg/mL solution in water), 100 μL ammonium acetate buffer (0.2M, pH 5.2) and ~1-2 mCi of $^{177}LuCl_3$ in 0.05N HCl (MURR) at 90°C for 15 min. Free $^{177}$ Lu was scavenged by adding 20 μL of a 1% $Na_2EDTA.2H_2O$ (Aldrich) solution in water. The resulting radiochemical purity (RCP) was ~95%. The radiolabeled product was separated from unlabeled ligand and other impurities by HPLC,

using a YMC Basic C8 column [4.6 x 150 mm], a column temperature of 30°C and a flow rate of 1 mL/min, with a gradient of 68%A/32%B to 66%A/34%B over 30 min., where A is citrate buffer (0.02M, pH 3.0), and B is 80% $CH_3CN$/20% $CH_3OH$. The isolated compound had an RCP of ~100% and an HPLC retention time of 23.4 minutes.

Samples for biodistribution and cell binding studies were prepared by collecting the desired HPLC peak into 1000 μL of citrate buffer (0.05 M, pH 5.3, containing 1% ascorbic acid, and 0.1% HSA). The organic eluent in the collected eluate was removed by centrifugal concentration for 30 min. For cell binding studies, the purified sample was diluted with cell-binding media to a concentration of 1.5 μCi/mL within 30 minutes of the in vitro study. For biodistribution studies, the sample was diluted with citrate buffer (0.05 M, pH 5.3, containing 1% sodium ascorbic acid and 0.1% HSA) to a final concentration of 50 μCi/mL within 30 minutes of the in vivo study.

**Example XLVI - Preparation of 177 Lu-L64 for radiotherapy studies:**

This compound was synthesized by incubating 70 μg L64 ligand (70 μL of a 1 mg/mL solution in water), 200 μL ammonium acetate buffer (0.2M, pH 5.2) and ~30 - 40 mCi of $^{177}LuCl_3$ in 0.05N HCl (MLTRR) at 85°C for 10 min. After cooling to room temperature, free $^{177}Lu$ was scavenged by adding 20 μL of a 2% $Na_2EDTA.2H_2O$ (Aldrich) solution in water. The resulting radiochemical purity (RCP) was ~95%. The radiolabeled product was separated from unlabeled ligand and other impurities by HPLC, using a 300VHP Anion Exchange column (7.5 x 50 mm) (Vydac) that was sequentially eluted at a flow rate of 1 mL/min with water, 50% acetonitrile/water and then 1g/L aqueous ammonium acetate solution. The desired compound was eluted from the column with 50% $CH_3CN$ and mixed with ~1 mL of citrate buffer (0.05 M, pH 5.3) containing 5% ascorbic acid, 0.2% HSA, and 0.9% (v:v) benzyl alcohol. The organic part of the isolated fraction was removed by spin vacuum for 40 min, and the concentrated solution (~20-25 mCi) was adjusted within 30 minutes of the in vivo study to a concentration of 7.5 mCi/mL using citrate buffer (0.05 M, pH 5.3) containing 5% ascorbic acid, 0.2% HSA, and 0.9% (v:v) benzyl alcohol. The resulting compound had an RCP of>95%.

**Example XLVII - Preparation of $^{111}$In-L64:**

This compound was synthesized by incubating 10 μg L64 ligand (5 μL of a 2 mg/mL solution in 0.01 N HCl), 60 μL ethanol, 1.12 mCi of $^{111}InCl_3$ in 0.05N HCl (80 μL) and 155 μL sodium acetate buffer (0.5M, pH 4.5) at 85°C for 30 min. Free $^{111}In$ was scavenged by adding 20 μL of a 1% $Na_2EDTA.2H_2O$ (Aldrich) solution in water. The resulting radiochemical purity (RCP) was 87%. The radiolabeled product was separated from unlabeled ligand and other impurities by HPLC, using a Vydac C18 column, [4.6x250 mm], a column temperature of 50°C and a flow rate of 1.5 mL/min. with a gradient of 75%A/25%B to 65%A/35%B over 20 min where A is 0.1% TFA in water, B is 0.085% TFA in acetonitrile. With this system, the retention time for $^{111}$In-L64 is 15.7 min. The isolated compound had an RCP of 96.7%.

**Example XLVIII - Preparation of $^{177}$Lu-L134 (Control)**

A stock solution of peptide was prepared by dissolving L134 ligand (prepared as described in US Application Publication No. 2002/0054855 and WO 02/87637, both incorporated by reference) in 0.01 N HCl to a concentration of 1 mg/mL. $^{177}$ Lu-L134 was prepared by mixing the following reagents in the order shown.

| | |
|---|---|
| 0.2 M $NH_4OAc$, pH 6.8 | 100 μl |
| Peptide stock, 1 mg/mL, in 0.01 N HCl | 5 μl |
| $^{177}LuCl_3$ (MLTRR) in 0.05M HCl | 1.2 μl (1.4 mCi) |

The reaction mixture was incubated at 85 °C for 10 min. After cooling down to room temperature in a water bath, 20 μl of a 1% EDTA solution and 20 μl of EtOH were added. The compound was analyzed by HPLC using a C18 column (VYDAC Cat # 218TP54) that was eluted at flow rate of 1 mL/min with a gradient of 21 to 25% B over 20 min, where A is 0.1%TFA/$H_2O$ and B is 0.1%TFA/$CH_3CN$). $^{177}$Lu-L134 was formed in 97.1% yield (RCP) and had a retention time of ~16.1 min on this system.

**Example XLIX - Preparation of $^{177}$ Lu-L63**

This compound was prepared as described for $^{177}$ Lu-L134. The compound was analyzed by HPLC using a C18 column (VYDAC Cat # 218TP54) that was eluted at flow rate of 1 mL/min with a gradient of 30-34% B over 20 min (where solvent is A. 0.1%TFA/$H_2O$ and B is 0.1%TFA/$CH_3CN$). The $^{177}$Lu-L63 that formed had an RCP of 97.8% and a retention time of ~14.2 min on this system.

**Example L - Preparation of $^{177}$ Lu-L70 for cell binding and biodistribution studies:**

This compound was prepared following the procedures described above, but substituting L70 (the ligand of Example II). Purification was performed using a YMC Basic C8 column (4.6 x 150 mm), a column temperature of 30˚C and a flow rate of 1 mL/min. with a gradient of 80%A/20%B to 75%A/25%B over 40 min., where A is citrate buffer (0.02M, pH 4.5), and B is 80% $CH_3CN$/20% $CH_30H$. The isolated compound had an RCP of ~100% and an HPLC retention time of 25.4 min.

**Example LI - Preparation of $^{177}$ Lu- L70 for radiotherapy studies:**

This compound was prepared as described above for L64.

**Example LII - Preparation of $^{111}$In-L70 for cell binding and biodistribution studies:**

This compound was synthesized by incubating 10 μg L70 ligand (10 μL of a 1 mg/mL solution in 0.01 N HCl), 180 μL ammonium acetate buffer (0.2M, pH 5.3), 1.1 mCi of $^{111}InCl_3$ in 0.05N HCl (61 μL, Mallinckrodt) and 50 μL of saline at 85˚C for 30 min. Free $^{111}$In was scavenged by adding 20 μL of a 1% $Na_2EDTA.2H_2O$ (Aldrich) solution in water. The resulting radiochemical purity (RCP) was 86%. The radiolabeled product was separated from unlabeled ligand and other impurities by HPLC, using a Waters XTerra C18 cartridge linked to a Vydac strong anion exchange column [7.5 x 50 mm], a column temperature of 30˚C and a flow rate of 1 mL/min. with the gradient listed in the Table below, where A is 0.1 mM NaOH in water, pH 10.0, B is 1 g/L ammonium acetate in water, pH 6.7 and C is acetonitrile. With this system, the retention time for $^{111}$In-L70 is 15 min while the retention time for L70 ligand is 27 to 28 min. The isolated compound had an RCP of 96%.

Samples for biodistribution and cell binding studies were prepared by collecting the desired HPLC peak into 500 μL of citrate buffer (0.05 M, pH 5.3, containing 5% ascorbic acid, 1 mg/mL L-methionine and 0.2% HSA). The organic part of the collection was removed by spin vacuum for 30 min. For cell binding studies, the purified, concentrated sample was used within 30 minutes of the in vitro study. For biodistribution studies, the sample was diluted with citrate buffer (0.05 M, pH 5.3, containing 5% sodium ascorbic acid and 0.2% HSA) to a final concentration of 10 μCi/mL within 30 minutes of the in vivo study.

| Time, min | A | B | C |
|---|---|---|---|
| 0-10 | 100% | | |
| 10-11 | 100-50% | | 0-50% |
| 11-21 | 50% | | 50% |
| 21-22 | 50-0% | 0-50% | 50% |
| 22-32 | | 50% | 50% |

**Example LIII - *In vivo* Pharmacokinetic Studies**

A. Tracer dose biodistribution:

Low dose pharmacokinetic studies (*e.g.*, biodistribution studies) were performed using the below-identified compounds of the invention in xenografted, PC3 tumor-bearing nude mice ([Ncr]-Foxnl<nu>). In all studies, mice were administered 100 μL of $^{177}$ Lu-labelled test compound at 200 μCi/kg, i.v., with a residence time of 1 and 24 h per group (n=3-4). Tissues were analyzed in an LKB 1282 CompuGamma counter with appropriate standards.

**Table 7**

Pharmacokinetic comparison at 1 and 24 h in PC3 tumor-bearing nude mice (200 μCi/kg; values as % ID/g) of Lu-177 labelled compounds of this invention compared to control

| Tissue | L134 control | | L63 | | L64 | | L70 | |
|---|---|---|---|---|---|---|---|---|
| | 1 hr | 24 hr | 1 hr | 24 hr | 1 hr | 24 hr | 1 hr | 24 hr |
| Blood | 0.44 | 0.03 | 7.54 | 0.05 | 1.87 | 0.02 | 0.33 | 0.03 |
| Liver | 0.38 | 0.04 | 12.15 | 0.20 | 2.89 | 0.21 | 0.77 | 0.10 |
| Kidneys | 7.65 | 1.03 | 7.22 | 0.84 | 10.95 | 1.45 | 6.01 | 2.31 |
| Tumor | 3.66 | 1.52 | 9.49 | 2.27 | 9.83 | 3.60 | 6.42 | 3.50 |
| Pancreas | 28.60 | 1.01 | 54.04 | 1.62 | 77.78 | 6.56 | 42.34 | 40.24 |

Whereas the distribution of radioactivity in the blood, liver and kidneys after injection of L64 and L70 is similar to that of the control compound, L134, the uptake in the tumor is much higher at 1 and 24 h for both L64 and L70. L63 also shows high tumour uptake although with increased blood and liver values at early times. Uptake in the mouse pancreas, a normal organ known to have GRP receptors is much higher for L64, L70 and L63 than for L134.

**Example LIV-Receptor Subtype Specificity**

Currently, four mammalian members of the GRP receptor family are known: the GRP-preferring receptor (GRP-R), neuromedin-B preferring receptor (NMB-R), the bombesin receptor subtype 3 (BB3-R) and the BB4 receptor subtype. The receptor subtype specificity of $^{177}$Lu-L245 was investigated. The results indicate $^{177}$Lu-L245 binds specifically to GRP-R and NMB-R, and has little affinity for BB3-R.

The subtype specificity of the Lutetium complex of L245 [$^{177}$Lu-L24] (prepared as described supra) was determined by in vitro receptor autoradiography using the procedure described in Reubi et al., "Bombesin Receptor Subtypes in Human Cancers: Detection with the Universal Radioligand (125)I-[D-TYR(6), beta-ALA, PHE(13), NLE(14)] Bombesin (6-14)", Clin.Cancer Res. 8:1139-1146 (2002) and tissue samples that had been previously found to express only one subtype of GRP receptor. Human ileal carcinoid tissue was used as a source for NMB-R, human prostate carcinoma for GRP-R and human bronchial carcinoid for BB3-R subtype receptors. A compound known as the Universal ligand, $^{125}$I-[DTyr$^6$, ßAl$_a$$^{11}$, Phe$^{13}$, Nle$^{14}$]-BBN(6-14), which binds to all three subsets of GRP-R, was used as a positive control. The following results were seen.

Table 8

| Receptor Subtype Specificity of $^{177}$Lu-L245 as Measured by Competition Assay with Universal Ligand on Human Tissue Samples | | | |
|---|---|---|---|
| | IC$_{50}$ (nM) | | |
| Compound | NMB-R | GRP-R | BB3-R |
| **L245-19** | **0.9 ± 0.1 (4)** | **0.8 ± 0.1 (4)** | >**1,000 (3)** |
| **Universal ligand** | **0.8 ± 0.1 (3)** | **0.7 ± 0.1 (3)** | **1.1 ± 0.1 (3)** |

These results indicate that L245 derivatives are expected to bind well to human prostate carcinoma, which primarily expresses GRP-R. They also indicate that L245 derivatives are not expected to bind well to normal human pancreas (which primarily expresses the BB3-R receptor), or to cancers which primarily express the BB3-R receptor subtype.

**Example LV - Radiotherapy Studies**

A. Short Term Efficacy Studies:

Radiotherapy studies were performed using the PC3 tumor-bearing nude mouse model. Lu-177 labelled compounds of the invention **L64, L70, L63** and the treatment control compound **L134** were compared to an untreated control group. (n=12 for each treatment group and n=36 for the untreated control group). For the first study, mice were administered 100 μL of $^{177}$Lu-labelled compound of the invention at 30 mCi/kg, i.v, or s.c. under sterile conditions. The subjects were housed in a barrier environment for up to 30 days. Body weight and tumor size (by caliper measurement) were collected on each subject 3 times per week for the duration of the study. Criteria for early termination included: death;

loss of total body weight (TBW) equal to or greater than 20%; tumor size equal to or greater than 2 $cm^3$. Results are displayed in Figure 15A. These results show that animals treated with L70, L64 or L63 have increased survival over the control animals given no treatment and over those animals given the same dose of L134.

A repeat study was performed with L64 and L70 using the same dose as before but using more animals per compound (n=46) and following them for longer. The results of the repeat study are displayed in Figure 15B. Relative to the same controls as before (n=36), both L64 and L70 treatment give significantly increased survival ($p<0.0001$) with L70 being better than L64 (p0.079).

**Example LVI**

**Alternative Preparation of L64 and L70 Using Segment Coupling**

Compounds **L64** and **L70** can be prepared employing the collection of intermediates generally represented by **A-D (Figure 19)**, which themselves are prepared by standard methods known in the art of solid and solution phase peptide synthesis (Synthetic Peptides - A User's Guide 1992, Grant, G., Ed. WH. Freeman Co., NY, Chap 3 and Chap 4 pp 77 - 258; Chan, W.C. and White, P.D. Basic Procedures in Fmoc Solid Phase Peptide Synthesis - A Practical Approach 2002, Chan, W.C. and White, P.D. Eds Oxford University Press, New York, Chap. 3 pp 41 - 76; Barlos, K and Gatos, G. Convergent Peptide Synthesis in Fmoc Solid Phase Peptide Synthesis - A Practical Approach 2002, Chan, W.C. and White, P.D. Eds Oxford University Press, New York, Chap. 9 pp 216 - 228.) which are incorporated herein by reference.

These methods include Aloc, Boc, Fmoc or benzyloxycarbonyl-based peptide synthesis strategies or judiciously chosen combinations of those methods on solid phase or in solution. The intermediates to be employed for a given step are chosen based on the selection of appropriate protecting groups for each position in the molecule, which may be selected from the list of groups shown in Figure 1. Those of ordinary skill in the art will also understand that intermediates, compatible with peptide synthesis methodology, comprised of alternative protecting groups can also be employed and that the listed options for protecting groups shown above serves as illustrative and not inclusive, and that such alternatives are well known in the art.

This is amply illustrated in **Figure 20** which outlines the approach. Substitution of the intermediate **C2** in place of **C1** shown in the synthesis of **L64,** provides **L70** when the same synthetic strategies are applied.

**EXAMPLE LVII - Figures 49 and 50**

**Synthesis of L69**

Summary: Reaction of (3β,5β,7α,12α)-3-amino-7,12-dihydroxycholan-24-oic acid A with Fmoc-Cl gave intermediate B. Rink amide resin functionalised with the octapeptide GlnTrpAlaValGlyHisLeuMet$NH_2$ (BBN[7-14]) (**A**), was sequentially reacted with B, Fmoc-8-amino-3,6-dioxaoctanoic acid and DOTA tri-t-butyl ester. After cleavage and deprotection with reagent B the crude was purified by preparative HPLC to give L230 B 24280. Overall yield: 4.2%.

A. <u>(3β,5β,7α,12α)-3-(9H-Fluoren-9-ylmethoxy)amino-7,12-dihydroxycholan-24-oic acid, **B** (FIG 49)</u>

A solution of 9-fluorenylmethoxycarbonyl chloride (1.4 g; 5.4 mmol) in 1,4-dioxane (18 mL) was added dropwise to a suspension of (3β,5β,7α,12α)-3-amino-7,12-dihydroxycholan-24-oic acid A (2.0 g; 4.9 mmol) (3) in 10% aq. $Na_2CO_3$ (30 mL) and 1,4-dioxane (18 mL) stirred at 0 °C. After 6 h stirring at room temperature H2O (100 mL) was added, the aqueous phase washed with $Et_2O$ (2 x 90 mL) and then 2 M HCl (15 mL) was added (final pH: 1.5). The precipitated solid was filtered, washed with $H_2O$ (3 x 100 mL), vacuum dried and then purified by flash chromatography to give B as a white solid (2.2 g; 3.5 mmol). Yield 71%.

B. <u>N-[3β,5β,7α,12α)-3-[[[2-[2-[[[4,7,10-Tris(carboxymethyl)-1,4,7,10-tetraazacyclododec-1-yl]acetyl]amino]ethoxy]ethoxy]acetyl]amino]-7,12-dihydroxy-24-oxocholan-24-yl]-L-glutaminyl-L-tryptophyl-L-alanyl-L-valyl-glycyl-L-histidyl-L-leucyl-L-methioninamide, L**69** (FIG 50)</u>

Resin A (0.5 g; 0.3 mmol) was shaken in a solid phase peptide synthesis vessel with 50% morpholine in DMA (7 mL) for 10 min, the solution flushed off and fresh 50% morpholine in DMA (7 mL) was added. The suspension was stirred for another 20 min then the solution was flushed off and the resin washed with DMA (5 x 7 mL). (3β,5β,7α, 12α)-3-(9H-Fluoren-9-ylmethoxy)amino-7,12-dihydroxycholan-24-oic acid B (0.75 g; 1.2 m mol), N-hydroxybenzotriazole (HOBt) (0.18 g; 1.2 mmol), N,N'-diisopropylcarbodiimide (DIC) (0.19 mL; 1.2 mmol) and DMA (7 mL) were added to the resin, the mixture shaken for 24 h at room temperature, emptied and the resin washed with DMA (5 x 7 mL). The resin was then shaken with 50% morpholine in DMA (7 mL) for 10 min, the solution emptied, fresh 50% morpholine in DMA (7 mL) was added and the mixture shaken for another 20 min. The solution was emptied and the resin washed with DMA (5 x 7 mL). Fmoc-8-amino-3,6-dioxaoctanoic acid (0.79 g; 1.2 mmol), HOBt (0.18 g; 1.2

mmol), DIC (0.19 mL; 1.2 mmol) and DMA (7 mL) were added to the resin. The mixture was shaken for 3 h at room temperature, emptied and the resin washed with DMA (5 x 7 mL). The resin was then shaken with 50% morpholine in DMA (7 mL) for 10 min, the solution flushed off, fresh 50% morpholine in DMA (7 mL) was added and the mixture shaken for another 20 min. The solution was flushed off and the resin washed with DMA (5 x 7 mL) 1,4,7,10-Tetraazacyclododecane-1,4,7,10-tetraacetic acid tris(1,1-dimethylethyl) ester adduct with NaCl (0.79 g; 1.2 mmol), HOBt (0.18 g; 1.2 mmol), DIC (0.19 mL: 1.2 mmol), N-ethyldiisopropylamine (0.40 mL; 2.4 mmol) and DMA (7 mL) were added to the resin. The mixture was shaken for 24 h at room temperature, flushed off and the resin washed with DMA (5 x 7 mL), CH2C12 (5 x 7 mL) and vacuum dried. The resin was shaken in a flask with Reagent B (25 mL) (2) for 4.5 h. The resin was filtered and the solution was evaporated under reduced pressure to afford an oily crude that after treatment with $Et_2O$ (20 mL) gave a precipitate. The precipitate was collected by centrifugation and washed with $Et_2O$ (3 x 20 mL) to give a solid (248 mg) which was analysed by HPLC . An amount of crude (50 mg) was purified by preparative HPLC. The fractions containing the product were lyophilised to give L69 (6.5 mg; 3.5 x $10^3$ mmol) (FIG 50) as a white solid. Yield 5.8%.

**EXAMPLE LVIII - Figure 51**

**Synthesis of L144**

Summary: Rink amide resin functionalised with the octapeptide GlnTrpAlaValGlyHisLeuMetNH2 (BBN[7-14]) (A) was reacted with 4-[2-hydroxy-3-[4,7,10-tris[2-(1,1-dimethylethoxy)-2-oxoethyl]-1,4,7,10-tetrazacyclododec-1-yl] propoxy] benzoic acid. After cleavage and deprotection with reagent B (2) the crude was purified by preparative HPLC to give **L144.** Overall yield: 12%.

A. *N*-[4-[2-Hydroxy-3-[4,7,10-tris(carboxymethyl)-1,4,7,10-tetraazacyclododec-1-yl]propoxy]benzoyl]-L-glutaminyl-L-tryptophyl-L-alanyl-L-valyl-glycyl-L-histidyl-L-leucyl-L-methioninamide, **L144 (FIG 51)**

Resin **A** (0.4 g; 0.24 mmol) was shaken in a solid phase peptide synthesis vessel with 50% morpholine in DMA (7 mL) for 10 min, the solution flushed off and fresh 50% morpholine in DMA (7 mL) was added. The suspension was stirred for another 20 min then the solution was flushed off and the resin washed with DMA (5 x 7 mL). 4-[2-Hydroxy-3-[4,7,10-tris[2-(1,1-dimethylethoxy)-2-oxoethyl]-1,4,7,10-tetrazacyclododec-1-yl]propoxy] benzoic acid **B** (0.5 g; 0.7 mmol), HOBt (0.11 g; 0.7 mmol), DIC (0.11 mL; 0.7 mmol)), *N*-ethyldiisopropylamine (0.24 mL; 1.4 mmol) and DMA (7 mL) were added to the resin. The mixture was shaken for 24 h at room temperature, emptied and the resin washed with DMA (5 x 7 mL), $CH_2Cl_2$ (5 x 7 mL) and vacuum dried. The resin was shaken in a flask with Reagent B (25 mL) (2) for 4.5 h. The resin was filtered and the solution was evaporated under reduced pressure to afford an oily crude that after treatment with $Et_2O$ (20 mL) gave a precipitate. The precipitate was collected by centrifugation and washed with $Et_2O$ (3 x 20 mL) to give a solid (240 mg) which was analysed by HPLC . An amount of crude (60 mg) was purified by preparative HPLC. The fractions containing the product were lyophilised to give **L144** (10.5 mg; 7.2 x $10^{-3}$ mmol) as a white solid. Yield 12%.

## Claims

**1.** A compound of the general formula:

M-N-O-P-G

wherein :

M is an optical label or a metal chelator, optionally complexed with a radionuclide;
N is 0, an alpha amino acid, a substituted bile acid or other linking group;
O is an alpha amino acid or a substituted bile acid; and
P is 0, an alpha amino acid, a substituted bile acid or other linking group; and
G is a GRP receptor targeting peptide, and

wherein at least one of N, O or P is a substituted bile acid.

**2.** The compound of claim 1, wherein G is an agonist or a peptide which confers GRP-R agonist activity.

**3.** The compound of claim 1-2, wherein the substituted bile acid is selected from the group consisting of:

3β-amino-3-deoxycholic acid;
(3β,5β)-3-aminocholan-24-oic acid;
(3β,5β,12α)-3-amino-12-hydroxycholan-24-oic acid;
(3β,5β,7β,12α)-3-amino-7,12-dihydroxycholan-24-oic acid;
Lys-(3,6,9)-trioxaundecane-1,11 -dicarbonyl-3,7-dideoxy- 3-aminocholic acid);
(3β,5β,7α,12α)-3-amino-7-hydroxy-12-oxocholan-24-oic acid; and
(3β,5β,7α)-3-amino-7-hydroxycholan-24-oic acid.

**4.** The compound of claim 3 wherein at least one of N, O or P is (3β,5β,12α)-3-amino-12-hydroxycholan-24-oic acid.

**5.** The compound of claim 3 wherein at least one of N, O or P is 3β-amino-3-deoxycholic acid.

**6.** The compound of any one of claims 1-6, wherein the GRP receptor targeting peptide (G) is selected from the group consisting of: SEQ ID NO 1, QWAVGHLM-OH, QRLGNQWAVGHLM-$NH_2$, QRYGNQWAVGHLM-$NH_2$, QKYGN-QWAVGHLM-NH2, QWAVGHL-NH-Pentyl, QWSVaHLM-$NH_2$, QWAVGHLL-$NH_2$, QWAV-Bala-HF-Nle-$NH_2$ QWAV-Bala-HF-Nle-$NH_2$, QWAGHFL-$NH_2$, LWAVGSFM-$NH_2$, HWAVGHLM-$NH_2$, LWAVGSFM-$NH_2$, and QWAVGHFM-$NH_2$.

**7.** The compound of any one of claims 1-6, wherein M is selected from the group consisting of: DTPA, DOTA, DO3A, HPDO3A, EDTA, TETA, CMDOTA, EHPG, HBED and derivatives thereof.

**8.** The compound of claim 7, wherein said EPGH derivatives is selected from the group consisting of :5-Cl-EHPG, 5-Br-EHPG, 5-Me-EHPG, 5-t-Bu-EHPG, and 5-sec-Bu-EHPG.

**9.** The compound of claim 7, wherein M is selected from the group consisting of benzodiethylenetriamine pentaacetic acid (benzo-DTPA) and derivatives thereof.

**10.** The compound of claim 9, wherein said benzo-DTPA derivative is selected from the group consisting of: dibenzo-DTPA, phenyl-DTPA, diphenyl-DTPA, benzyl-DTPA, and dibenzyl DTPA.

**11.** The compound of claim 1, wherein M is a macrocyclic compound which contains at least 3 carbon atoms and at least two heteroatoms (O and/or N), which macrocyclic compounds consist of one ring, or two or three rings joined together at the hetero-ring elements.

**12.** The compound of claim 11, wherein M is selected from the group consisting of benzo-DOTA, dibenzo-DOTA, and benzo-NOTA, benzo-TETA, benzo-DOTMA, and benzo-TETMA, derivatives of 1,3-propylenediaminetetraacetic acid (PDTA) and triethylenetetraaminehexaacetic acid (TTHA); derivatives of 1,5,10-N,N',N"-tris(2,3-dihydroxybenzoyl)-tricatecholate (LICAM) and 1,3,5-N,N',N"-tris(2,3-dihydroxybenzoyl) aminomethylbenzene (MECAM).

**13.** The compound according to any one of claims 7-12 selected from the group consisting of:

D03A-monoamide-Gly-(3β,5β)-3-aminocholan-24-oic acid-BBN(7-14) wherein the BBN(7-14) sequence is SEQ. ID NO: 1;
DO3A-monoamide-Gly-(3β,5β,12α)-3-amino-12-hydroxycholan-24-oic acid-BBN(7-14) wherein the BBN(7-14) sequence is SEQ. ID NO: 1;
DO3A-monoamide-Gly-(3β,5β,7α,12α)-3-amino-7,12-dihydroxycholan-24-oic acid-BBN(7-14) wherein the

BBN(7-14) sequence is SEQ. ID NO: 1;
D03A-monoamide-Gly-Lys-(3,6,9)-trioxaundecane-1,11-dicarbonyl-3,7-dideoxy-3-aminocholic acid)-Arg-BBN(7-14) wherein the BBN(7-14) sequence is SEQ. ID NO: 1;
(3β,5β,7α,12α)-3-amino-7,12-dihydroxycholan-24-oic acid-3,6,9-trioxaundecane-1,11-dicarbonyl Lys(DO3A-monoamide-Gly)-Arg-BBN(7-14) wherein the BBN(7-14) sequence is SEQ. ID NO: 1;
DO3A-monoamide-Gly-(3β,5β,7α,12α)-3-amino-12-oxocholan-24-oic acid-BBN(7-14) wherein the BBN(7-14) sequence is SEQ. ID NO: 1; and
DO3A-monoamide-1-amino-3,6-dioxactanoic acid-(3β,5β,7α,12α)-3-amino-7,12-dihydroxycholan-24-oic acid-BBN(7-14) wherein the BBN(7-14) sequence is SEQ. ID NO: 1.

**14.** The compound according to claim 13 which is DO3A-monoamide-Gly-(3p,5p,7a,12a)-3-amino-7,12-dihydroxycholan-24-oic acid-BBN(7-14) wherein the BBN(7-14) sequence is SEQ. ID NO: 1.

**15.** The compound according to any one of claims 7-12 selected from the group consisting of:

DOTA-Gly-3-amino-3-deoxycholic acid-Q-W-A-V-a-H-L-M-NH2
DOTA-Gly-3-amino-3-deoxycholic acid-f Q-W-A-V-Gly-H-L-M-NH2
DOTA-Gly-3-amino-3-deoxycholic acid-f Q-W-A-V-Gly-H-L-L-NH2
DOTA-Gly-3-amino-3-deoxycholic acid-f-Q-W-A-V-Gly-H-L-NH-pentyl
DOTA-Gly-3-amino-3-deoxycholic acid-y-Q-W-A-V-Bala-H-F-Nle-NH2
DOTA-Gly-3-amino-3-deoxycholic acid-f-Q-W-A-V-Bala-H-F-Nle-NH2
DOTA-Gly-3-amino-3-deoxycholic acid-Q-W-A-V-Gly-H-F-L-NH2
DOTA-Gly-3-amino-3-deoxycholic acid-Q-W-A-V-Gly-NMeHis-L-M-NH2
DOTA-Gly-3-amino-3-deoxycholic acid-L-W-A-V-Gly-S-F-M-NH2
DOTA-Gly-3-amino-3-deoxycholic acid-H-W-A-V-Gly-H-L-M-NH2
DOTA-Gly-3-amino-3-deoxycholic acid-L-W-A-T-Gly-H-F-M-NH2
DOTA-Gly-3-amino-3-deoxycholic acid-Q-W-A-V-Gly-H-F-M-NH2
DOTA-Gly-3-amino-3-deoxycholic acid-Q-R-L-Gly-N-Q-W-A-V-Gly-H-L-M-NH2
DOTA-Gly-3-amino-3-deoxycholic acid-Q-R-Y-Gly-N-Q-W-A-V-Gly-H-L-M-NH2
DOTA-Gly-3-amino-3-deoxycholic acid-Q-K-Y-Gly-N-Q-W-A-V-Gly-H-L-M-NH2
Pglu-Q-Lys(DOTA-Gly-3-amino-3-deoxycholic acid)-L-Gly-N-Q-W-A-V-Gly-H-L-M-NH2

**16.** The compound according to any one of claims 7-15 chelated with a diagnostic or therapeutic radionuclide.

**17.** The compound of any one of claims 1-6, wherein M is an optical label selected from the group consisting of organic chromophores, organic fluorophores, light-absorbing compounds, light-reflecting compounds, light-scattering compounds, and bioluminescent molecules.

**18.** Compounds according to claim 16 for use in radiotherapy wherein the metal is selected in the groups consisting of : $^{64}Cu$, $^{90}Y$, $^{105}Rh$, $^{111}In$, $^{117m}Sn$, $^{149}Pm$, $^{153}SM$, $^{161}Tb$, $^{166}Dy$, $^{166}Ho$, $^{175}Yb$, $^{177}Lu$, $^{186/188}Re$, and $^{199}Au$, with $^{177}Lu$ and $^{90}Y$.

**19.** Compounds according to claim 16 for use in radioimaging wherein the metal is selected in the groups consisting of: $^{64}Cu$, $^{67}Ga$, $^{68}Ga$, $^{99m}Tc$, and $^{111}In$, with $^{99m}Tc$, and $^{111}In$.

**20.** Compounds according to any one of claims 16- 19 for use in radiotherapy, radioimaging or optical imaging of cancers wherein GRP-R is upregulated.

**21.** Compounds according to claim 20 wherein said cancers are selected in the group consisting of: prostate cancer (primary and metastatic), breast cancer (primary and metastatic), colon cancer, gastric cancer, pancreatic cancer, non small cell lung cancer, small cell lung cancer, gastrinomas, melanomas, glioblastomas, neuroblastomas, uterus leiomyosarcoma tumors, prostatic intraepithelial neoplasias [PIN], and ovarian cancer.

**22.** Pharmaceutical composition comprising the compound according to any one of claims 1-19.

**23.** The pharmaceutical composition according to claim 22 further comprising other chemotherapic or therapeutic agents.

**24.** A method of imaging GRP-R expressing tissue in a patient comprising imaging a patient pre-administered with the

compounds of any one of claims 16 - 17 or the composition according to any one of claims 22-23.

**25.** The compound according to claim 17 for use in phototherapy.

**26.** A method of preparing a radiotherapeutic agent comprising the step of adding to an injectable medium a substance comprising the compound of claim 18 wherein M is a metal chelator complexed with a radionuclide.

**27.** A method of synthesizing the compound of claim 14 (D03A-monoamide-Gly-(3β,5β,7α,12α)-3-amino-7,12-dihydroxycholan-24-oic acid-BBN(7-14) wherein the BBN(7-14) sequence is SEQ. ID NO: 1) comprising the steps of:

(a) shaking a solution in a solid phase peptide synthesis vessel, said solution comprising a resin and at least one peptide building ingredient,
(b) flushing said solution, and
(c) washing said resin with DMA,

wherein said at least one peptide building ingredient includes DMA morpholine, (3β,5β,7α,12α)-3-[[(9H-fluoren-9-ylmethoxy)amino]acetyl]amino-7,12-dihydroxycholan-24-oic acid, HOBT, DIC, HATU or mixtures thereof, and wherein each of steps (a), (b) and (c) are repeated until the compound DO3A-monoamide-Gly-(3β,5β,7α,12α)-3-amino-7,12-dihydroxycholan-24-oic acid-BBN(7-14) wherein the BBN(7-14) sequence is SEQ. ID NO: 1 is obtained.

**28.** A method for labeling the compound of claim 14 (DO3A-monoamide-Gly-(3β,5β,7α,12α)-3-amino-7,12-dihydroxycholan-24-oic acid-BBN(7-14) wherein the BBN(7-14) sequence is SEQ. ID NO: 1) comprising the steps of incubating a first solution comprising D03A-monoamide-Gly-(3β,5β,7α,12α)-3-amino-7,12-dihydroxycholan-24-oic acid-BBN (7-14) wherein the BBN(7-14) sequence is SEQ. ID NO: 1, ammonium acetate, a radioactive metal precursor selected from the group consisting of $^{177}LuCb$ or $^{111}InCl_3$, HCl, and adding to said first solution a second solution comprising $Na_2EDTA\text{-}2H_2O$ and water to obtain a radiochemical purity greater than 95%.

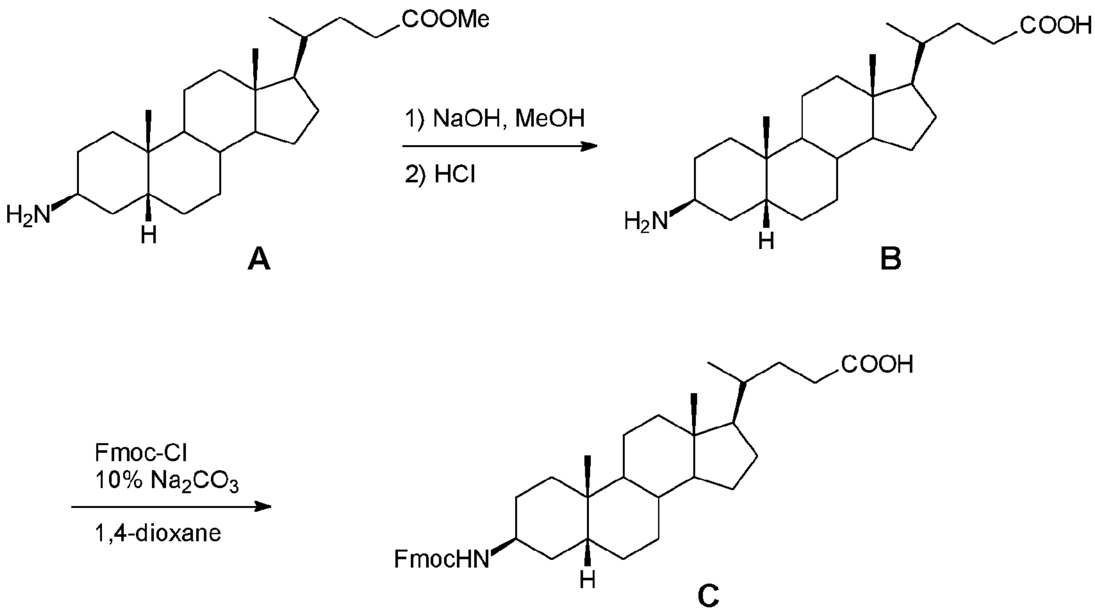


A

B

C

FIG. 1A

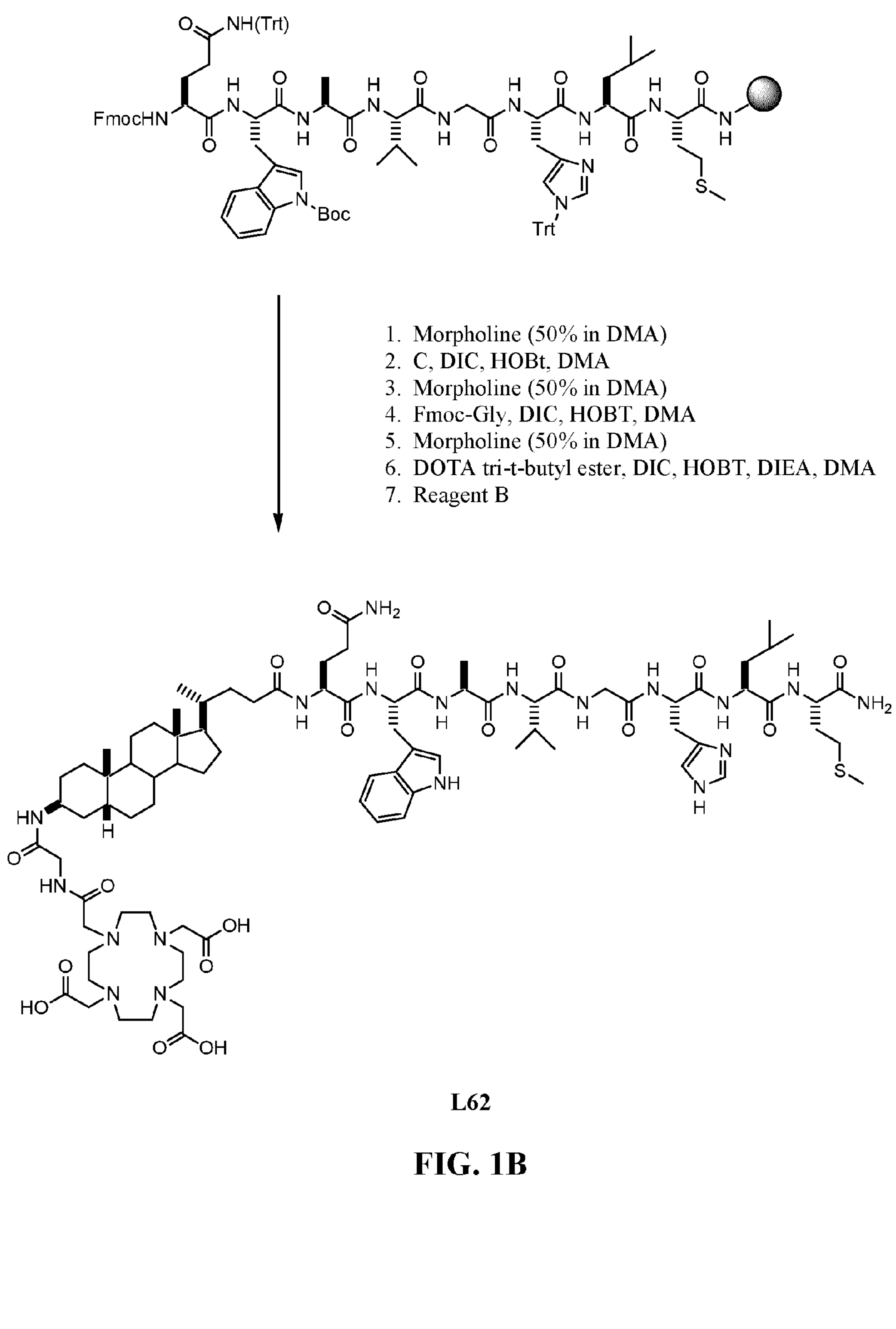
1. Morpholine (50% in DMA)
2. C, DIC, HOBt, DMA
3. Morpholine (50% in DMA)
4. Fmoc-Gly, DIC, HOBT, DMA
5. Morpholine (50% in DMA)
6. DOTA tri-t-butyl ester, DIC, HOBT, DIEA, DMA
7. Reagent B

**L62**

**FIG. 1B**

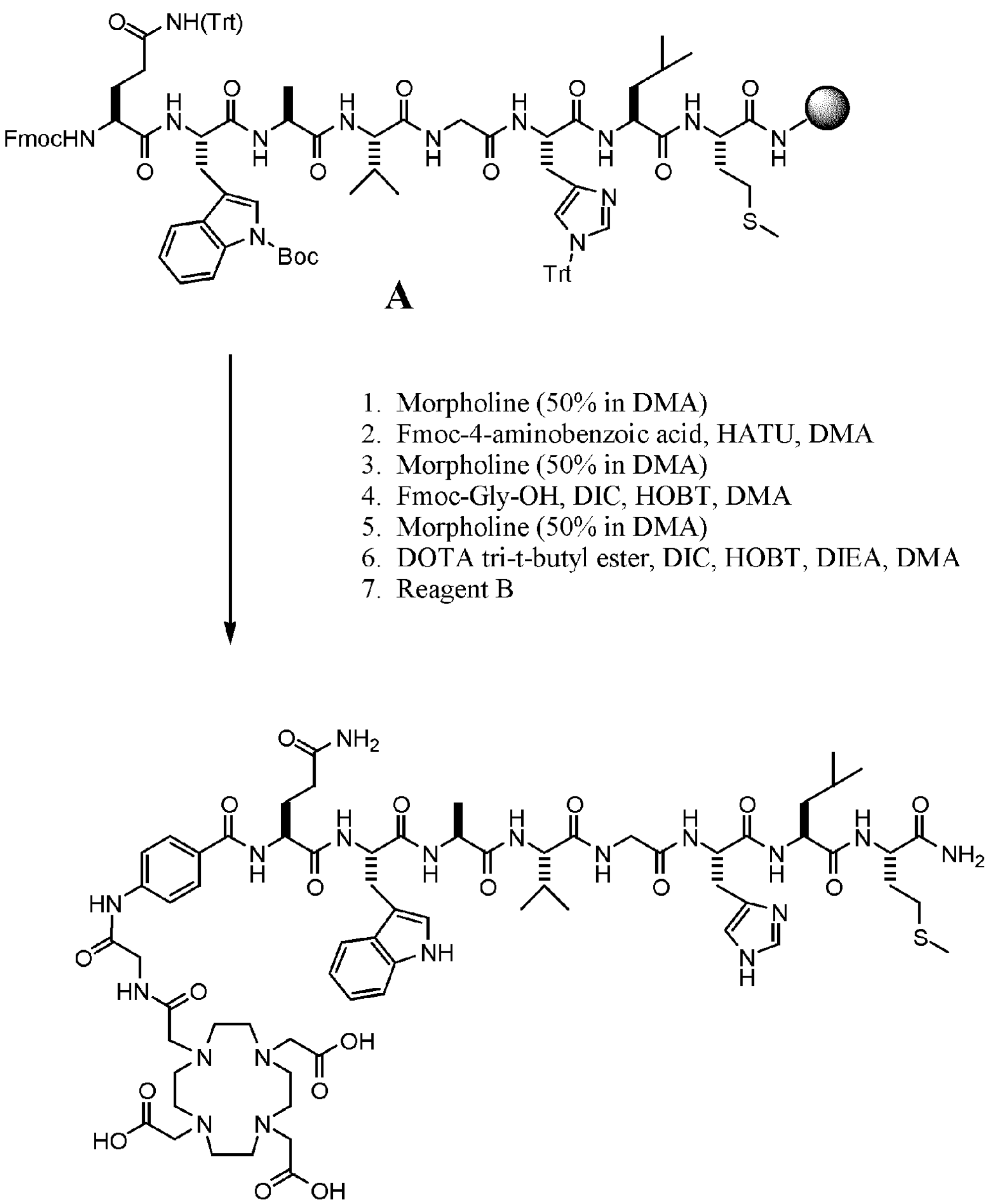
A
1. Morpholine (50% in DMA)
2. Fmoc-4-aminobenzoic acid, HATU, DMA
3. Morpholine (50% in DMA)
4. Fmoc-Gly-OH, DIC, HOBT, DMA
5. Morpholine (50% in DMA)
6. DOTA tri-t-butyl ester, DIC, HOBT, DIEA, DMA
7. Reagent B

L70

**FIG. 2A**

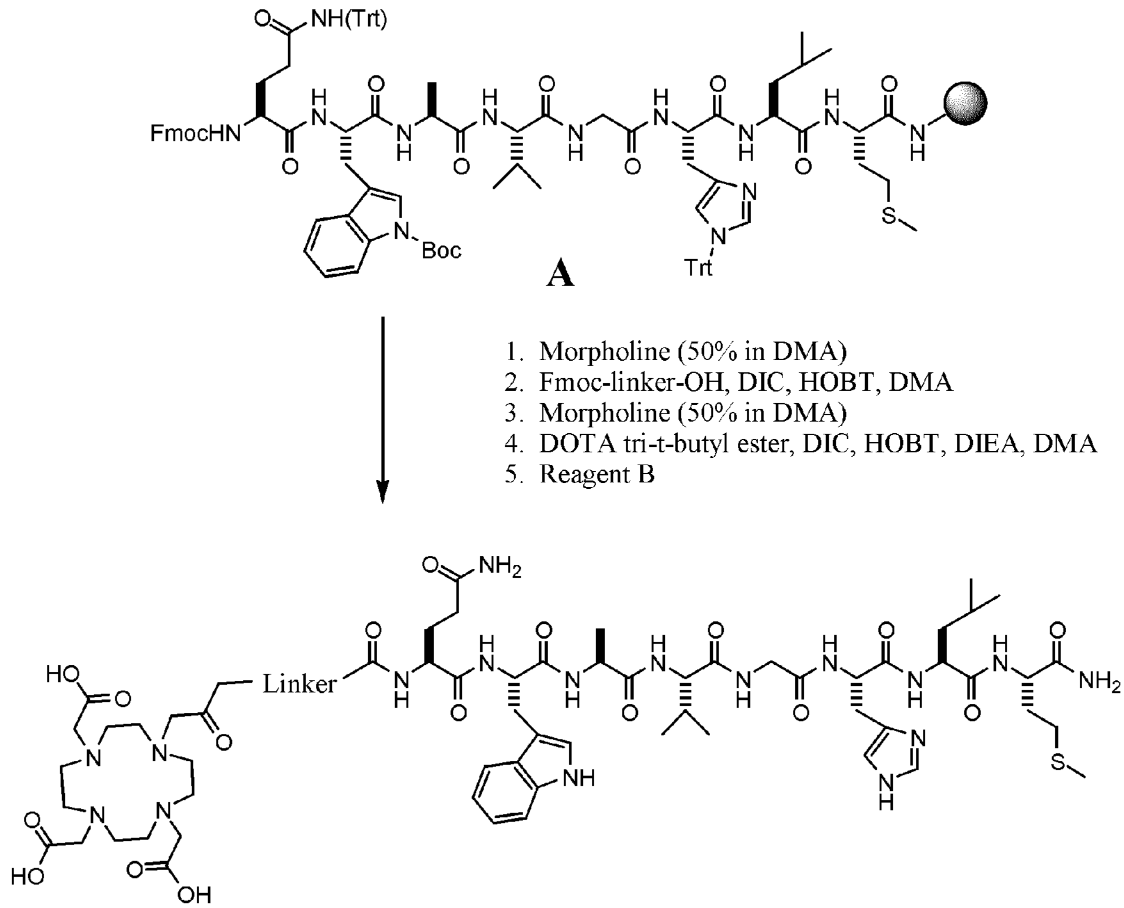


**FIG. 2B**

LINKERS:

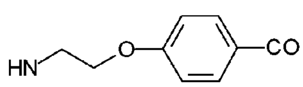

**L73**

**FIG. 2C**

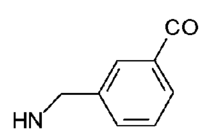

**L115**

**FIG. 2D**

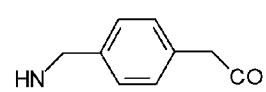

**L116**

**FIG. 2E**

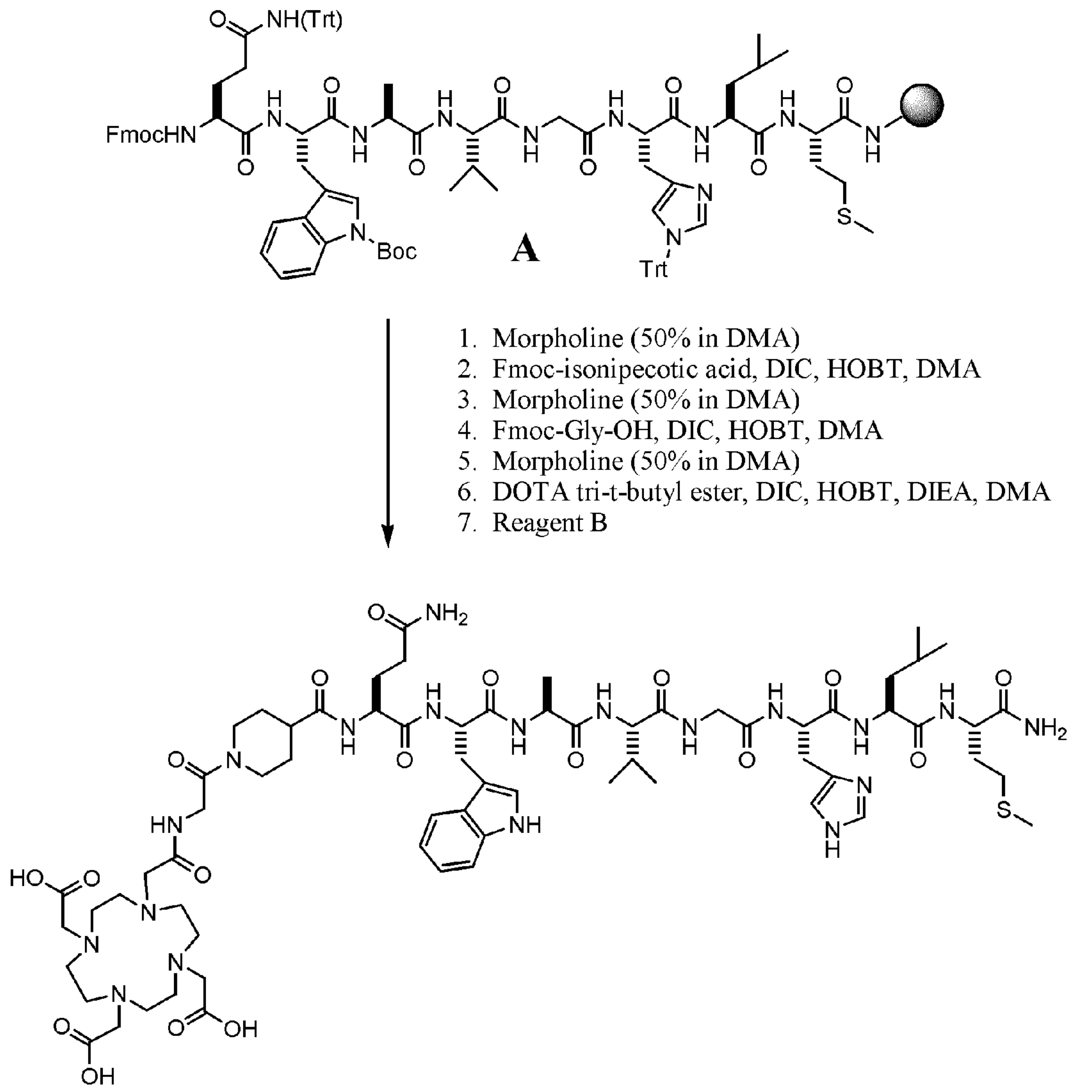
1. Morpholine (50% in DMA)
2. Fmoc-isonipecotic acid, DIC, HOBT, DMA
3. Morpholine (50% in DMA)
4. Fmoc-Gly-OH, DIC, HOBT, DMA
5. Morpholine (50% in DMA)
6. DOTA tri-t-butyl ester, DIC, HOBT, DIEA, DMA
7. Reagent B

A

L74

**FIG. 2F**

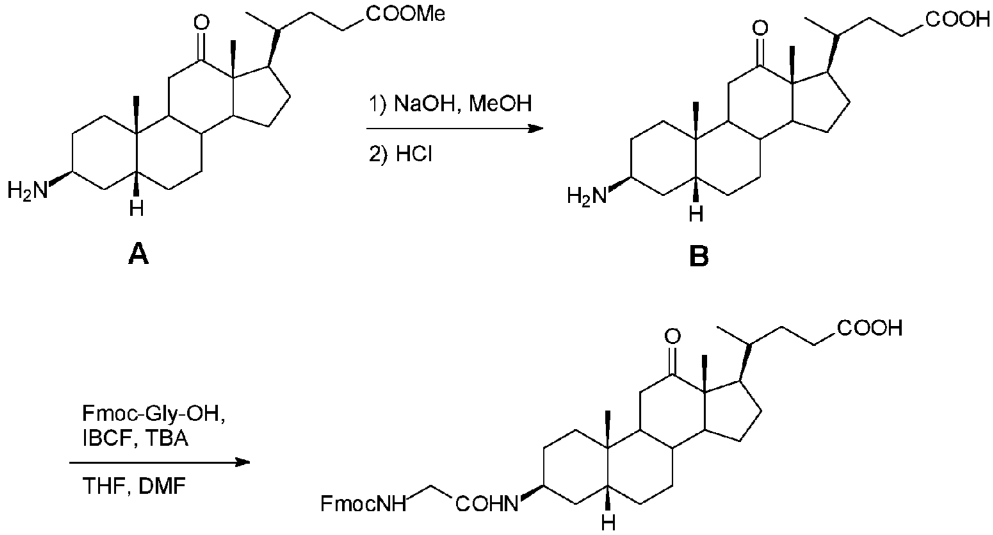


A

B

C

FIG. 3A

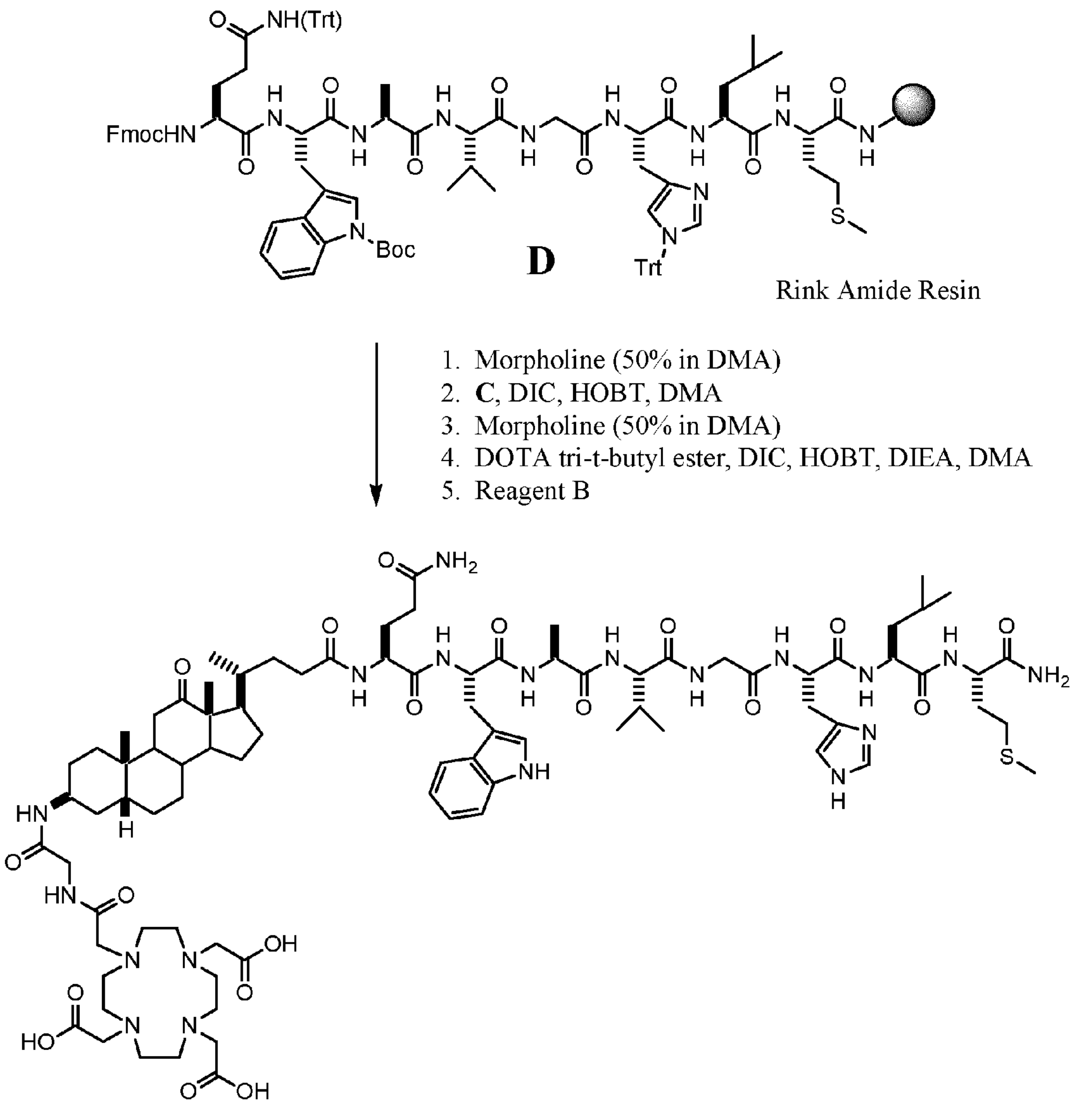
D
Rink Amide Resin
1. Morpholine (50% in DMA)
2. C, DIC, HOBT, DMA
3. Morpholine (50% in DMA)
4. DOTA tri-t-butyl ester, DIC, HOBT, DIEA, DMA
5. Reagent B

L67

FIG. 3B

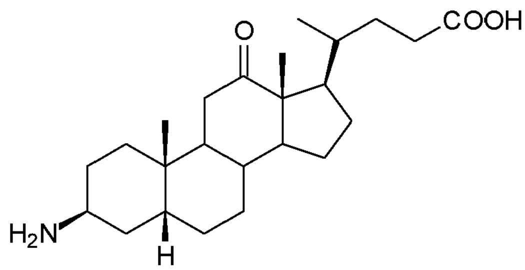

**B**

# FIG. 3C

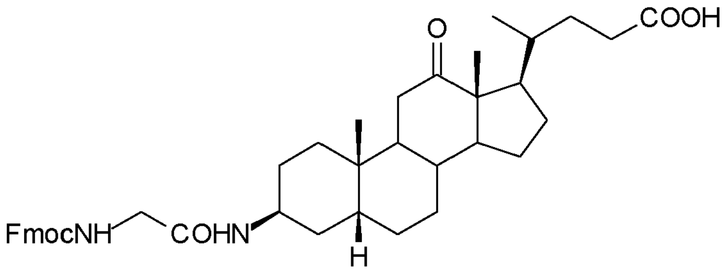

**C**

# FIG. 3D

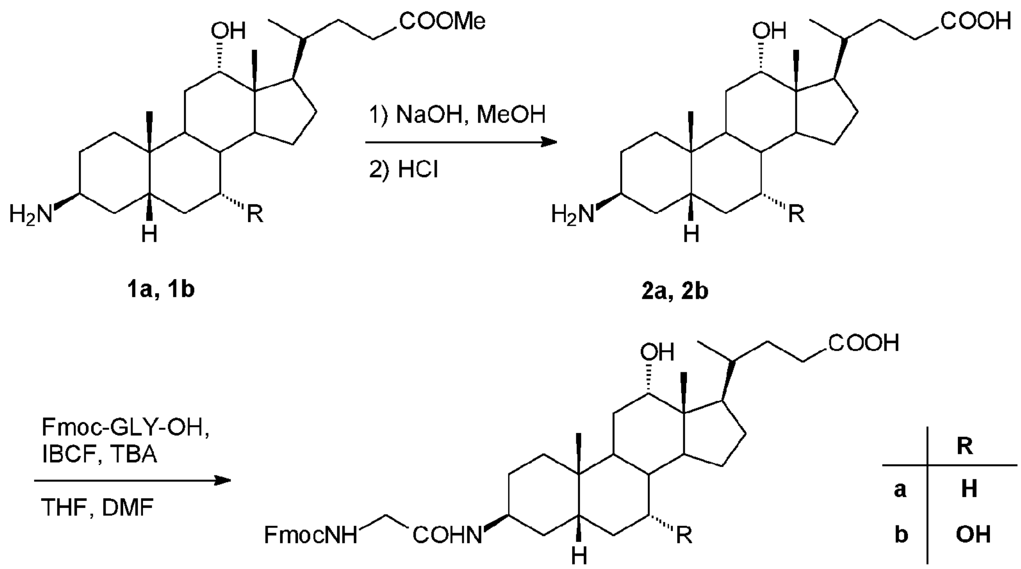


1a, 1b

2a, 2b

3a, 3b

| | R |
|---|---|
| a | H |
| b | OH |

FIG. 4A

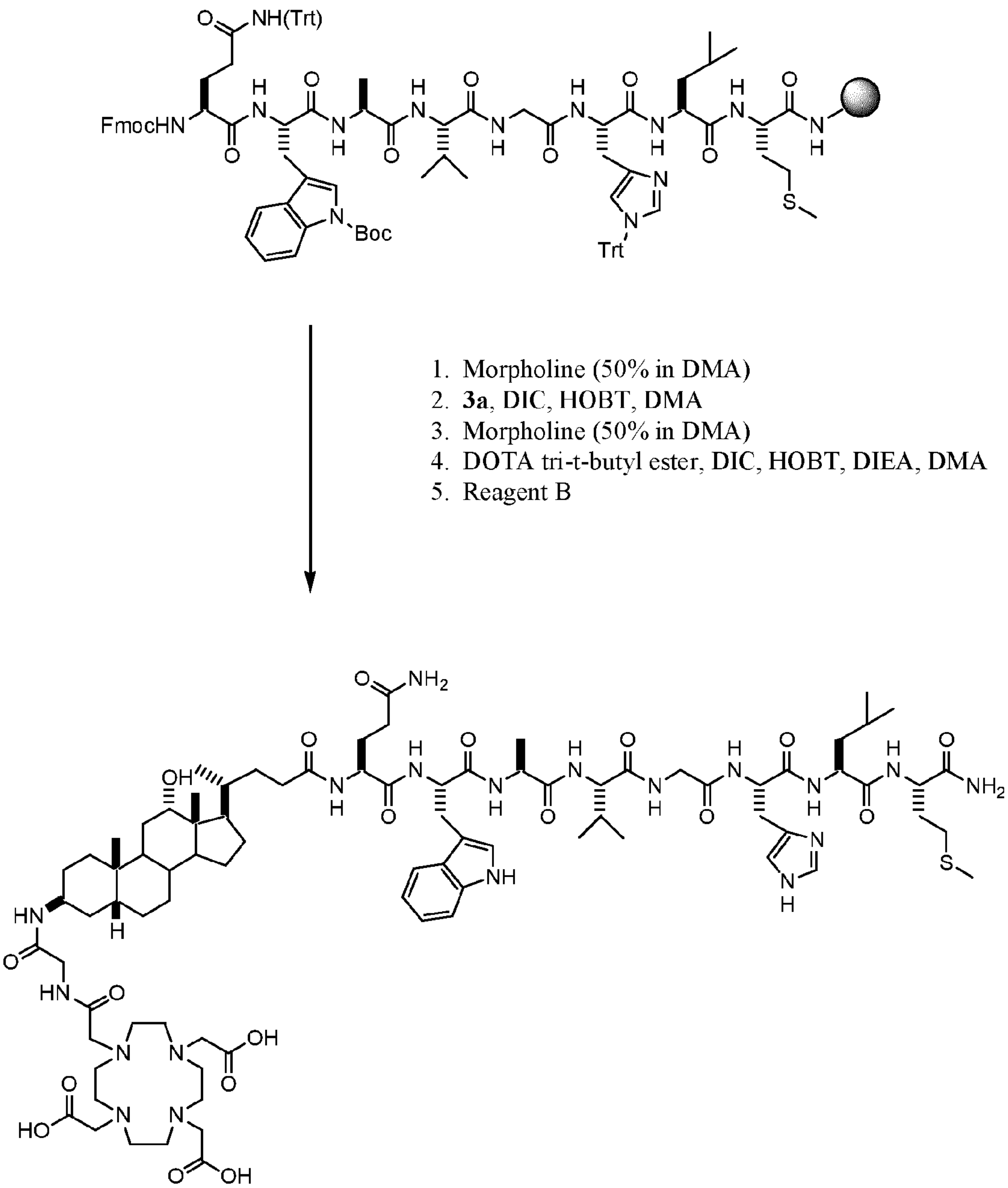
1. Morpholine (50% in DMA)
2. **3a**, DIC, HOBT, DMA
3. Morpholine (50% in DMA)
4. DOTA tri-t-butyl ester, DIC, HOBT, DIEA, DMA
5. Reagent B

**L63**

**FIG. 4B**

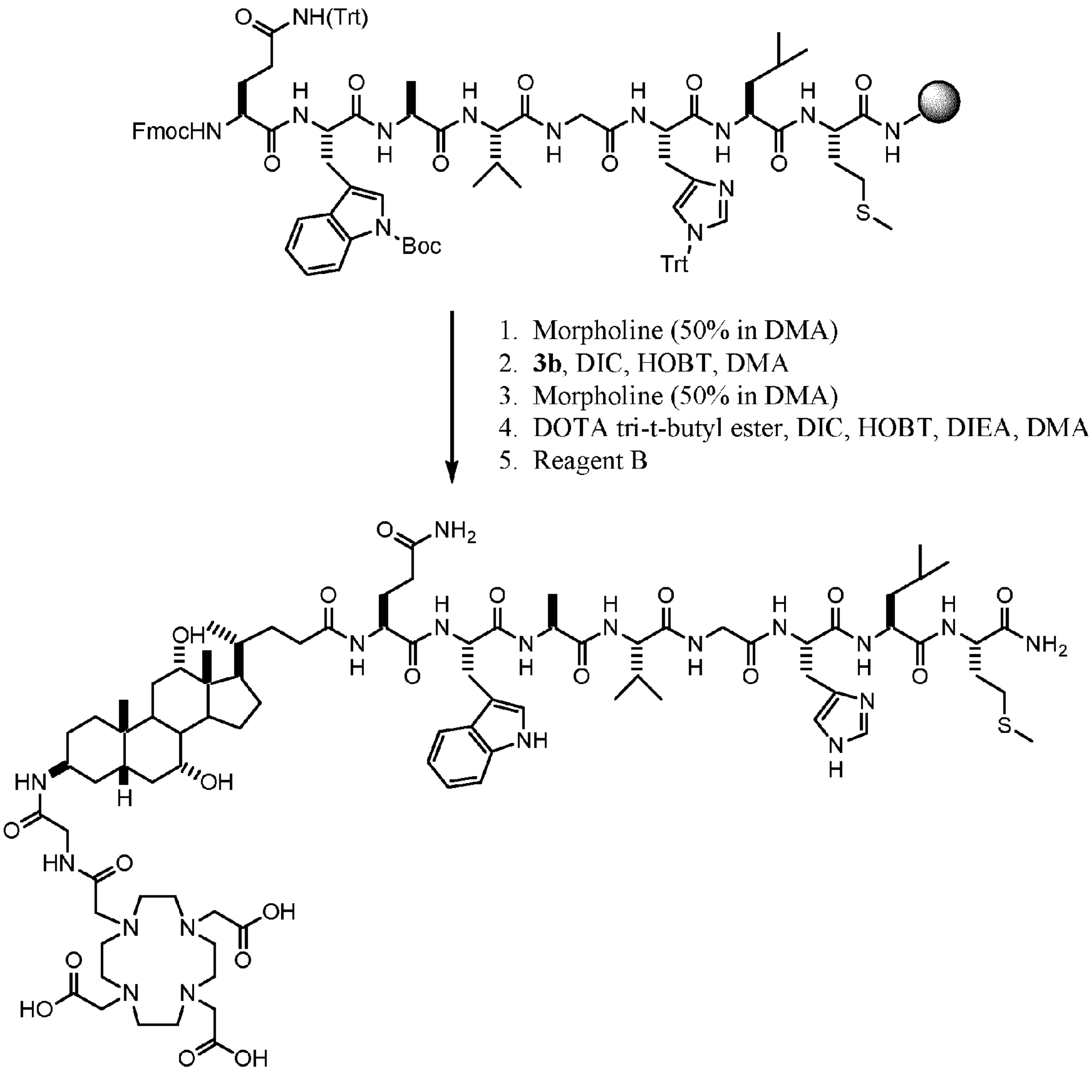
1. Morpholine (50% in DMA)
2. **3b**, DIC, HOBT, DMA
3. Morpholine (50% in DMA)
4. DOTA tri-t-butyl ester, DIC, HOBT, DIEA, DMA
5. Reagent B

**L64**

**FIG. 4C**

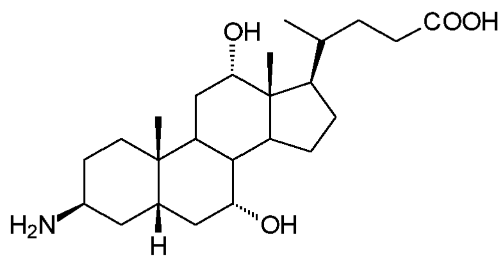

2b

FIG. 4D

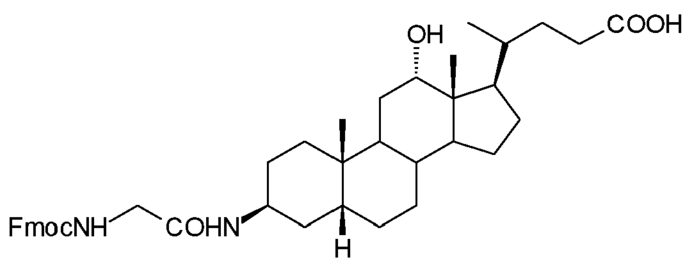

3a

FIG. 4E

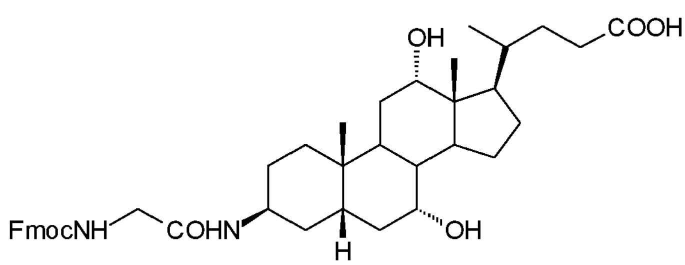

3b

FIG. 4F

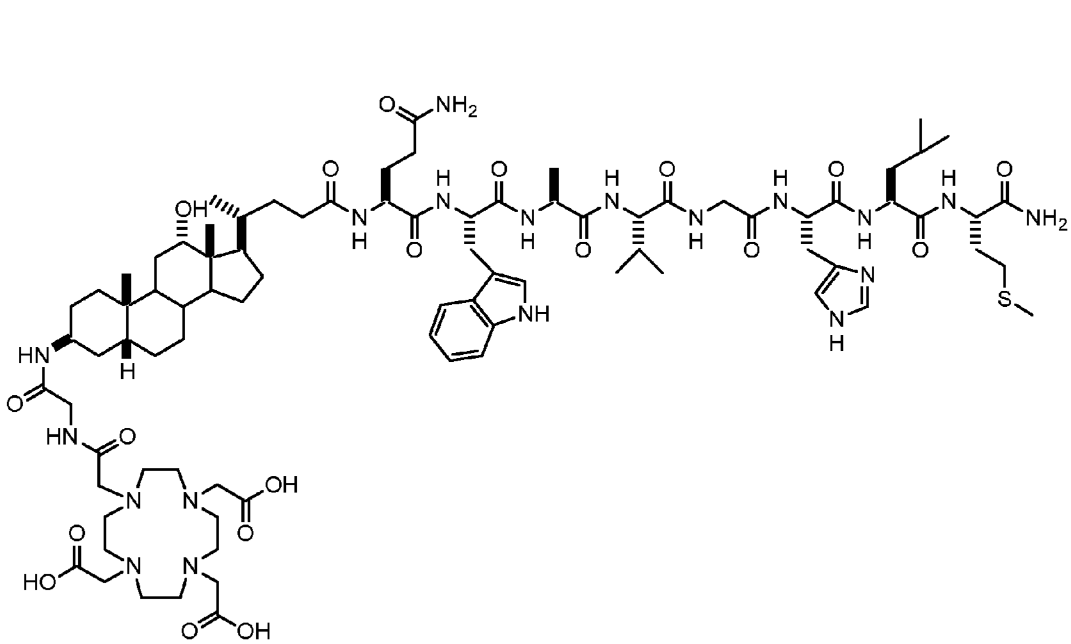

L63

FIG. 4G

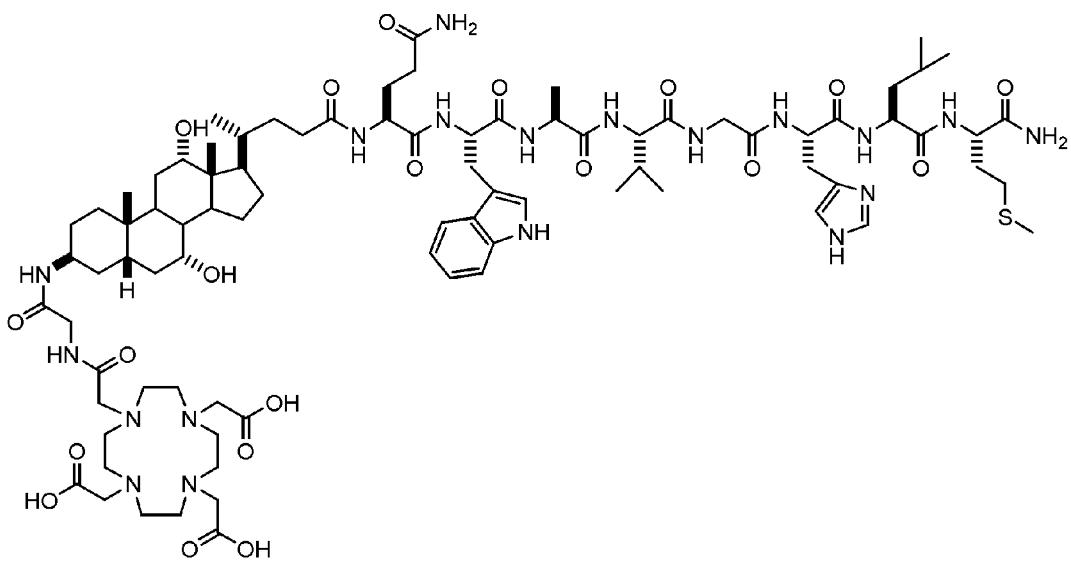

L64

FIG. 4H

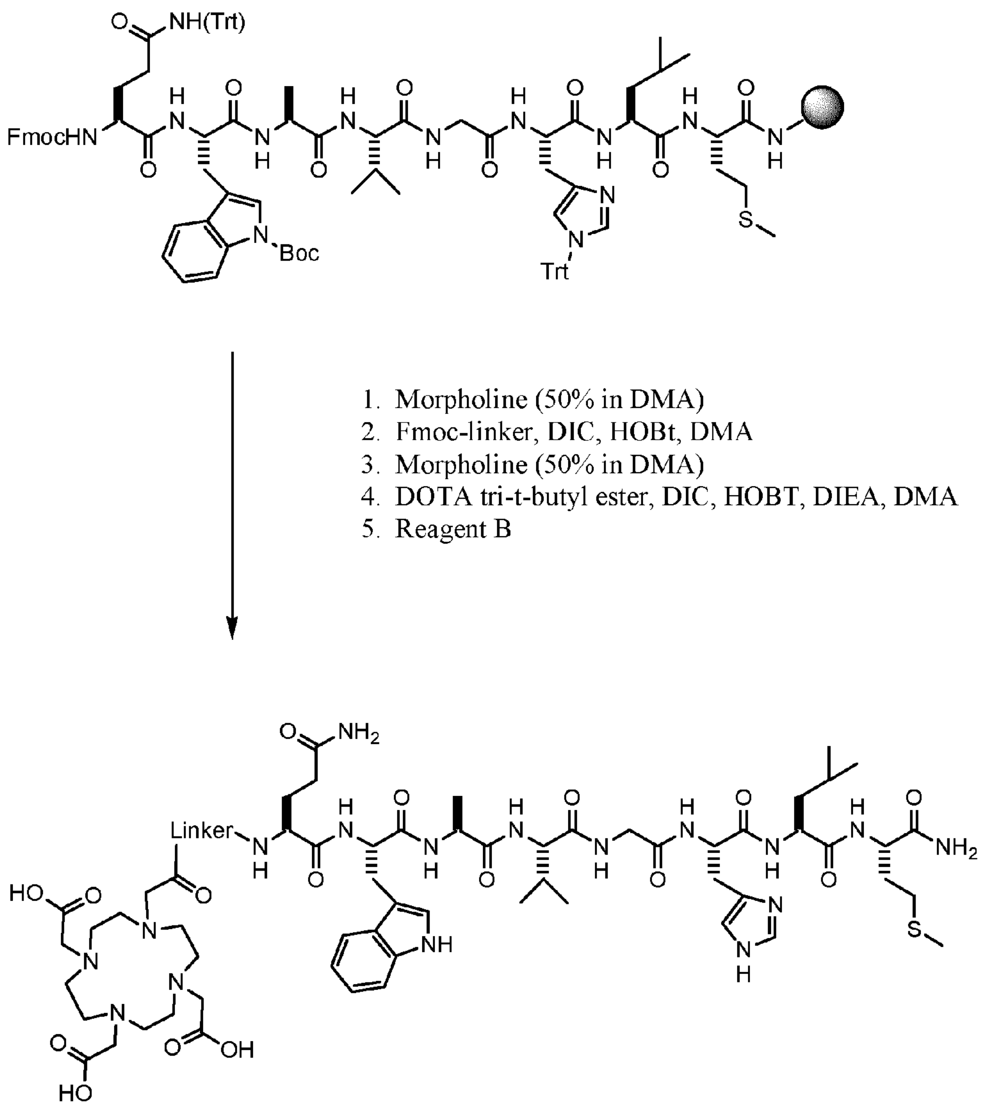
1. Morpholine (50% in DMA)
2. Fmoc-linker, DIC, HOBt, DMA
3. Morpholine (50% in DMA)
4. DOTA tri-t-butyl ester, DIC, HOBT, DIEA, DMA
5. Reagent B

**FIG. 5A**

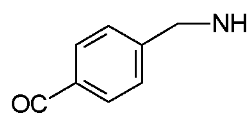

L71 linker

FIG. 5B

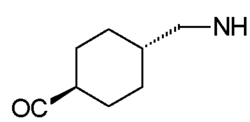

L72 linker

FIG. 5C

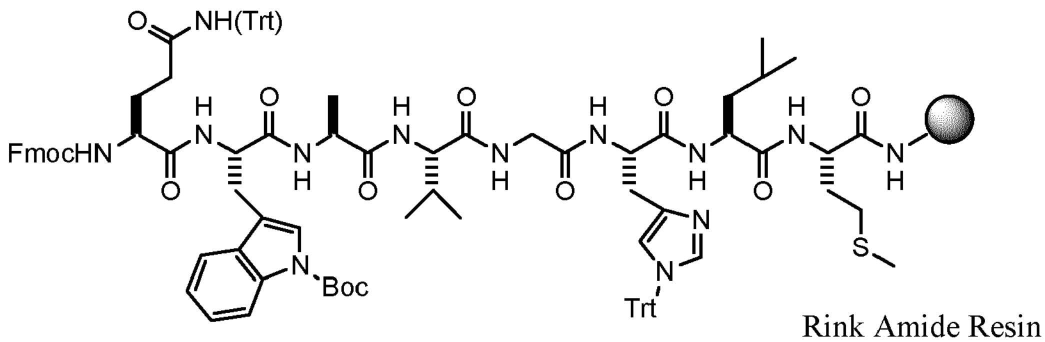

Rink Amide Resin

B

FIG. 5D

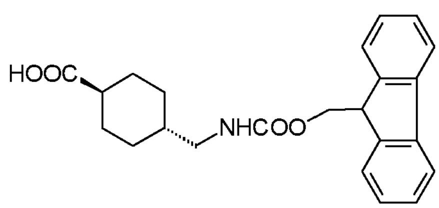

D

FIG. 5E

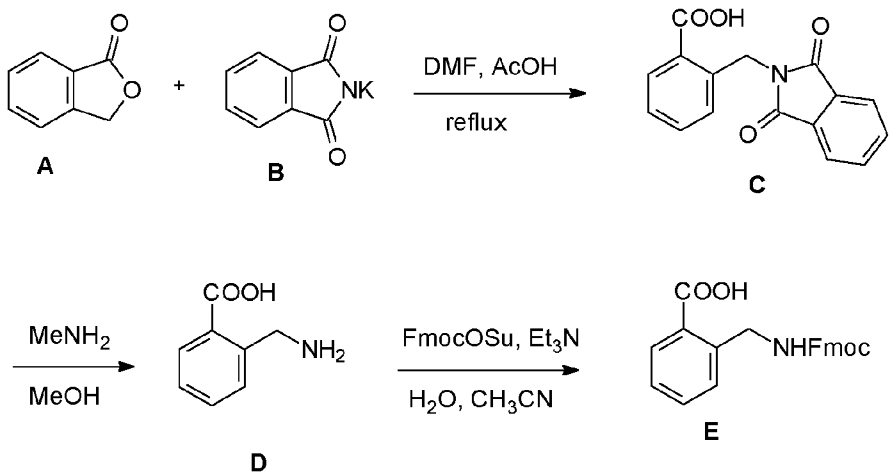


A B C D E

FIG. 6A

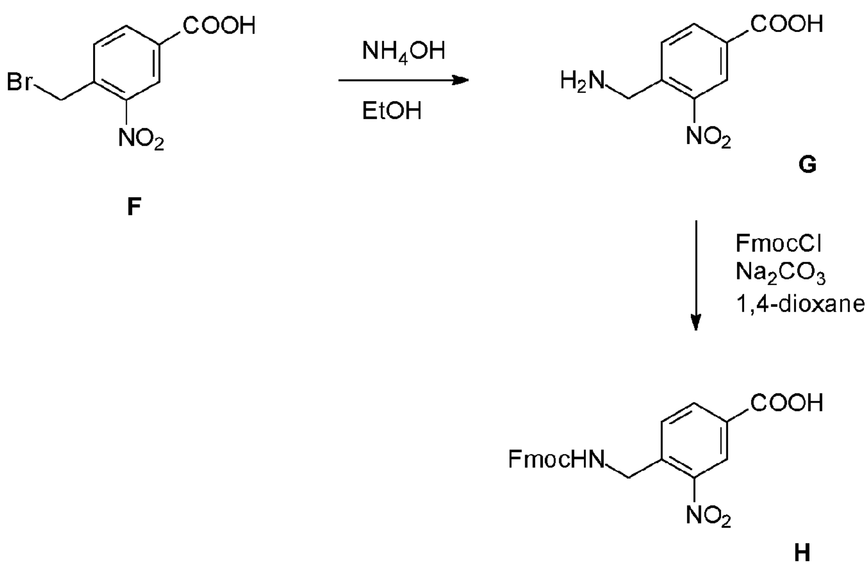


F G H

FIG. 6B

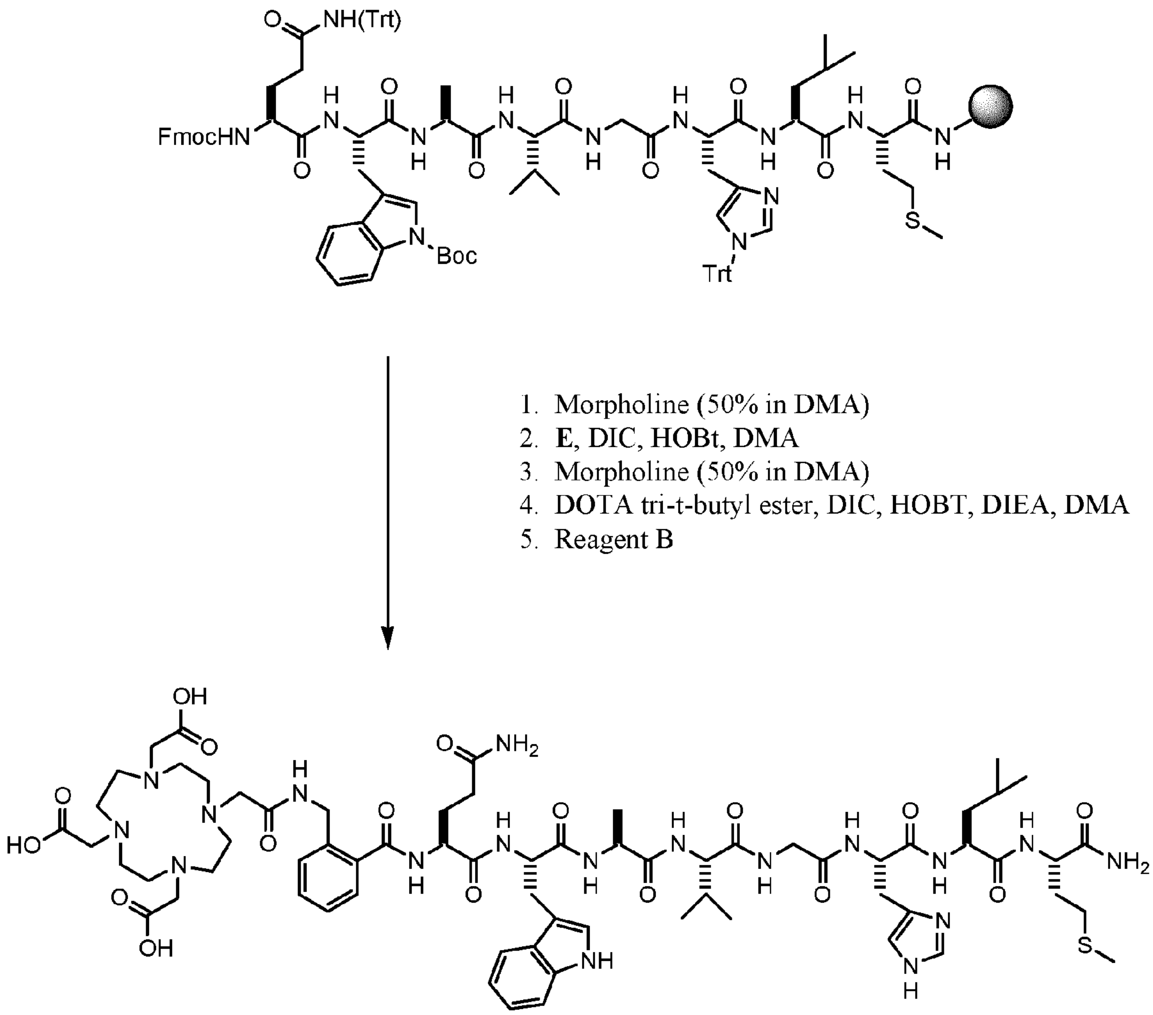
1. Morpholine (50% in DMA)
2. **E**, DIC, HOBt, DMA
3. Morpholine (50% in DMA)
4. DOTA tri-t-butyl ester, DIC, HOBT, DIEA, DMA
5. Reagent B

**L75**

# FIG. 6C

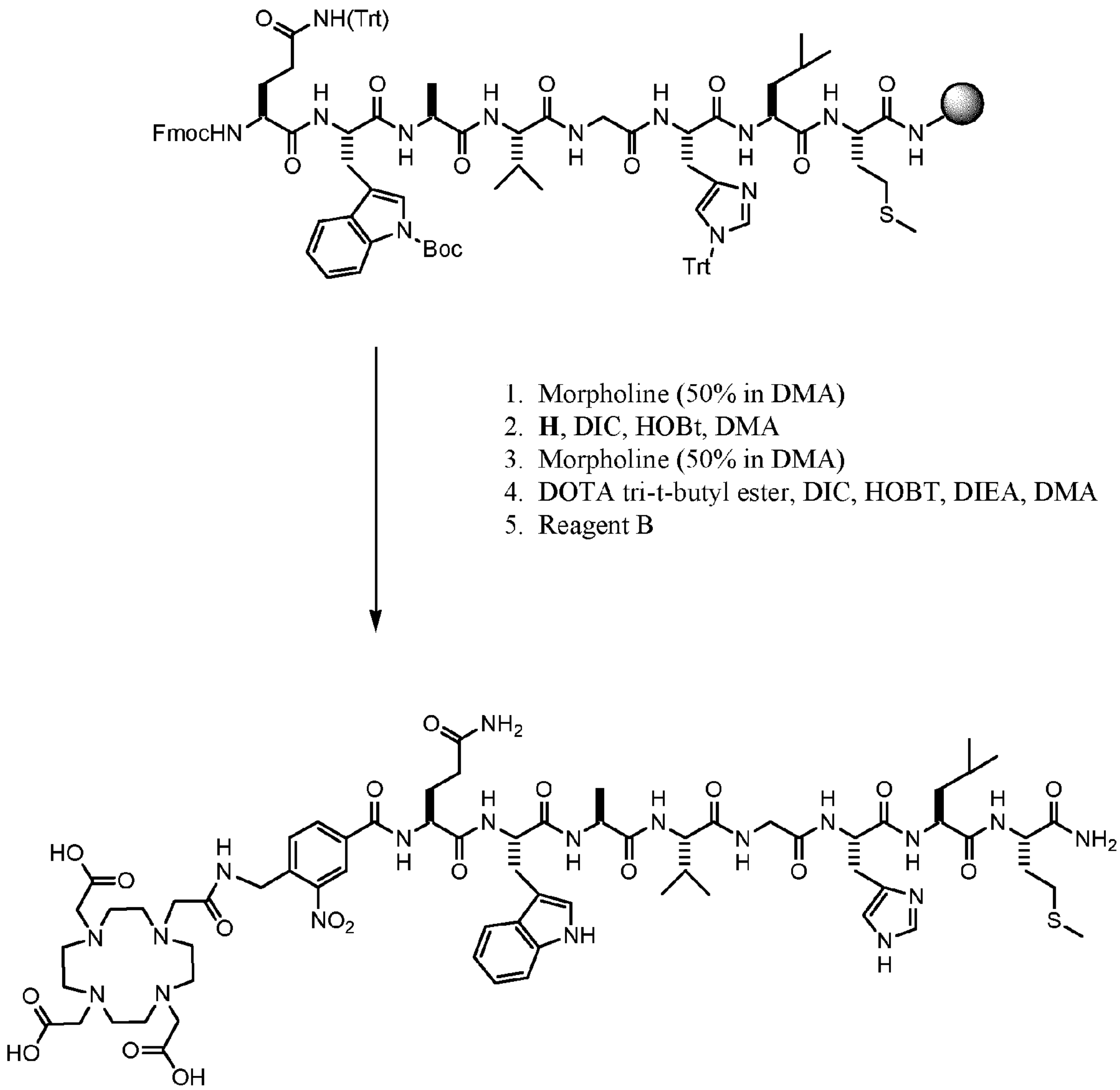
1. Morpholine (50% in DMA)
2. **H**, DIC, HOBt, DMA
3. Morpholine (50% in DMA)
4. DOTA tri-t-butyl ester, DIC, HOBT, DIEA, DMA
5. Reagent B

**L76**

# FIG. 6D

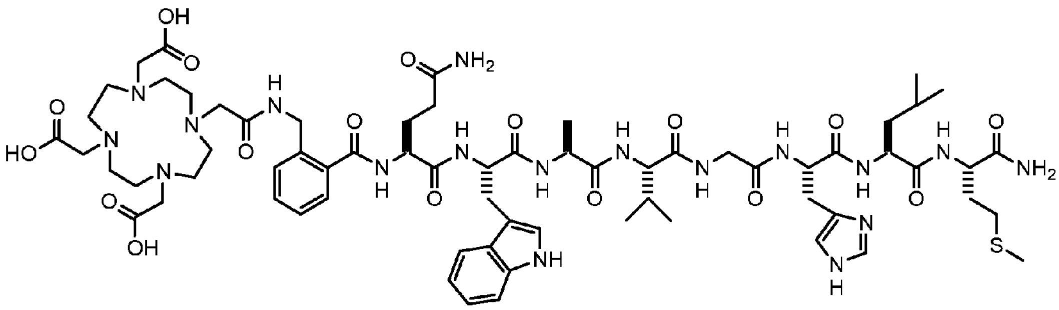

L75

**FIG. 6E**

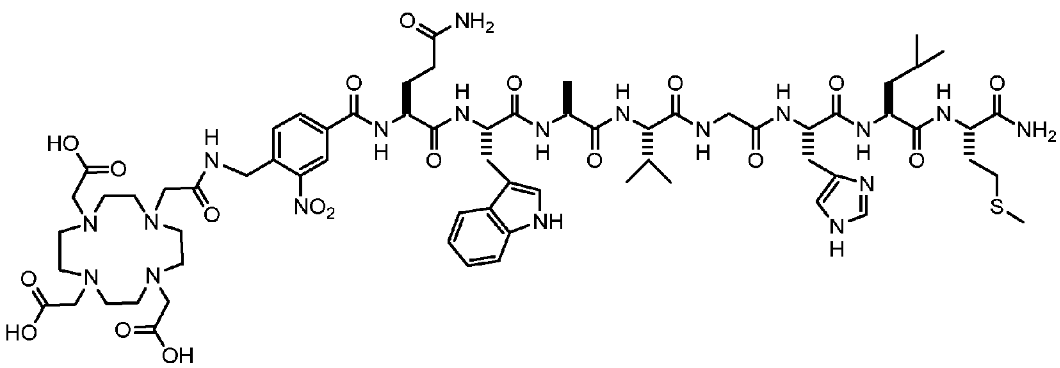

L76

**FIG. 6F**

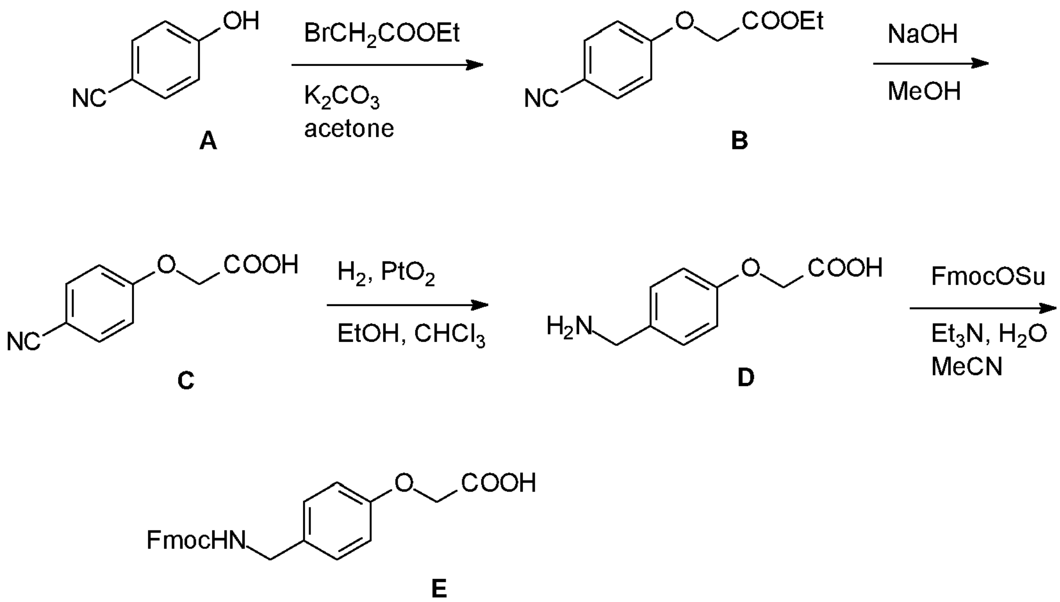
BrCH2COOEt
K2CO3
acetone
NaOH
MeOH
H2, PtO2
EtOH, CHCl3
FmocOSu
Et3N, H2O
MeCN

A B C D E

**FIG. 7A**

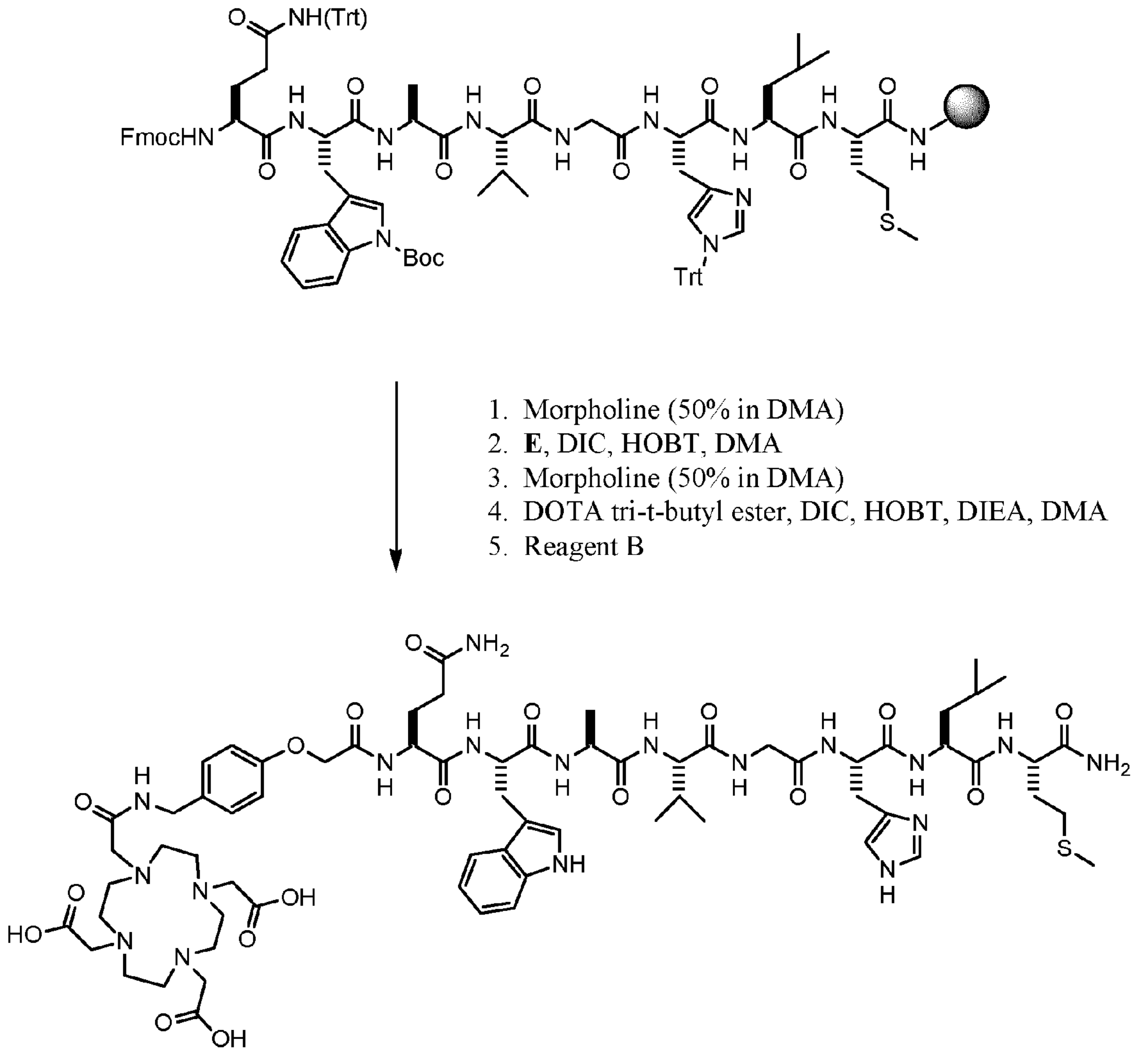
1. Morpholine (50% in DMA)
2. **E**, DIC, HOBT, DMA
3. Morpholine (50% in DMA)
4. DOTA tri-t-butyl ester, DIC, HOBT, DIEA, DMA
5. Reagent B

**L124**

**FIG. 7B**

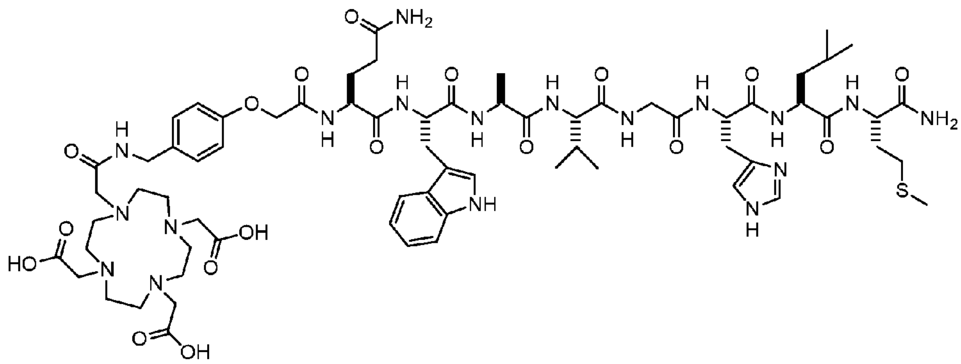

L124

FIG. 7C

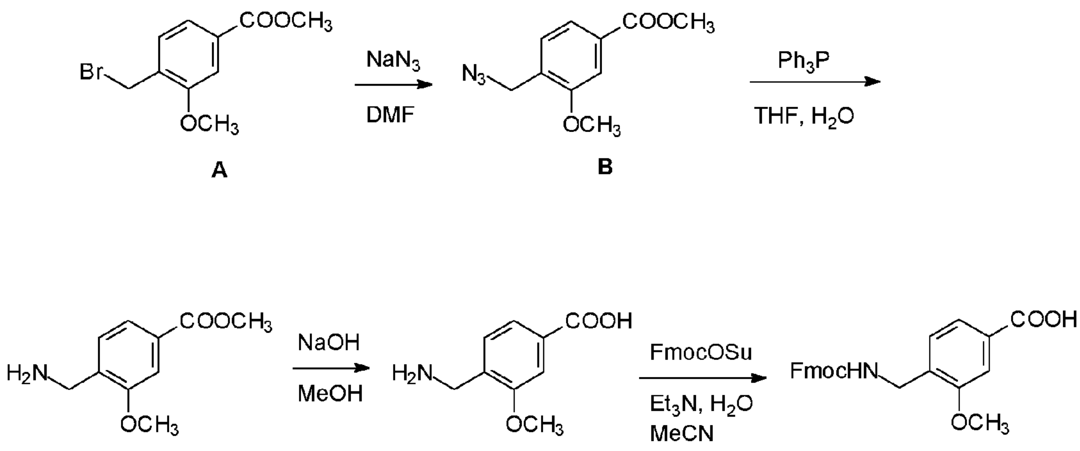

A B C D E

FIG. 8A

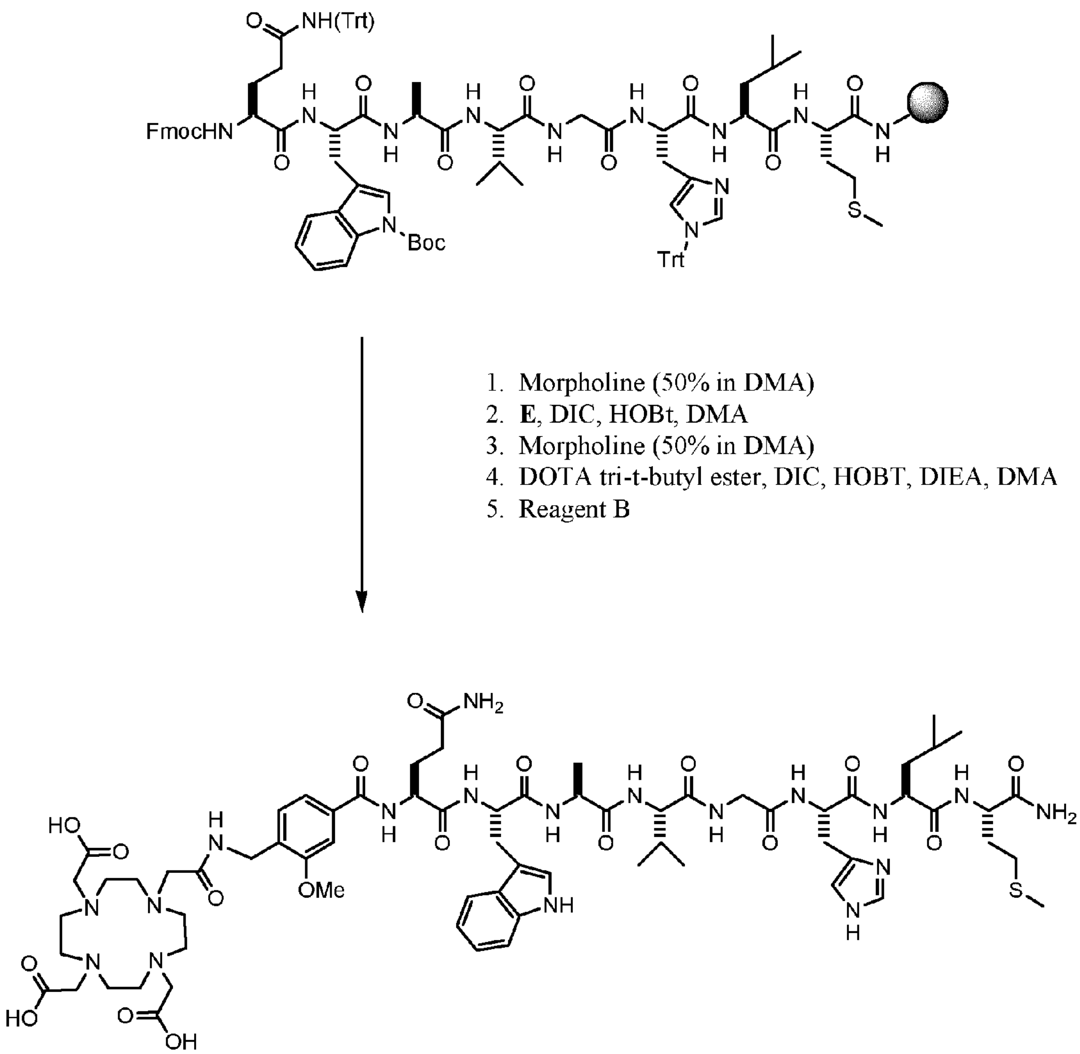
1. Morpholine (50% in DMA)
2. E, DIC, HOBt, DMA
3. Morpholine (50% in DMA)
4. DOTA tri-t-butyl ester, DIC, HOBT, DIEA, DMA
5. Reagent B

**L125**

# FIG. 8B

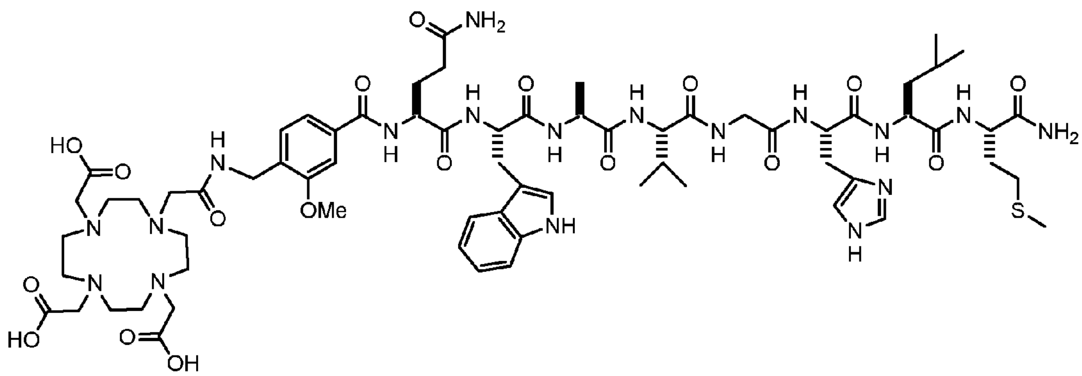

L125

FIG. 8C

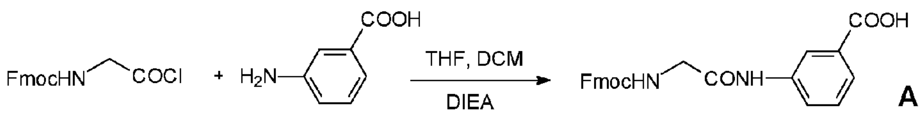


A

**FIG. 9A**

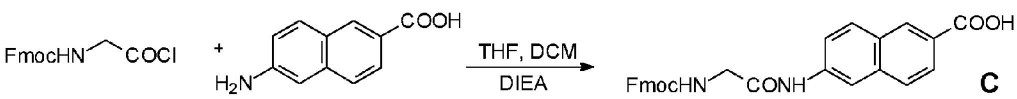


C

**FIG. 9B**

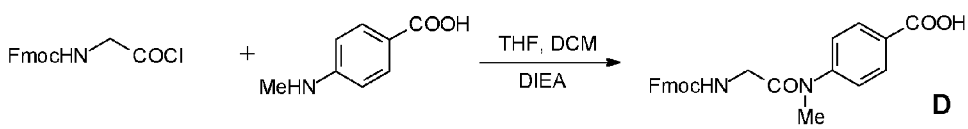


D

**FIG. 9C**

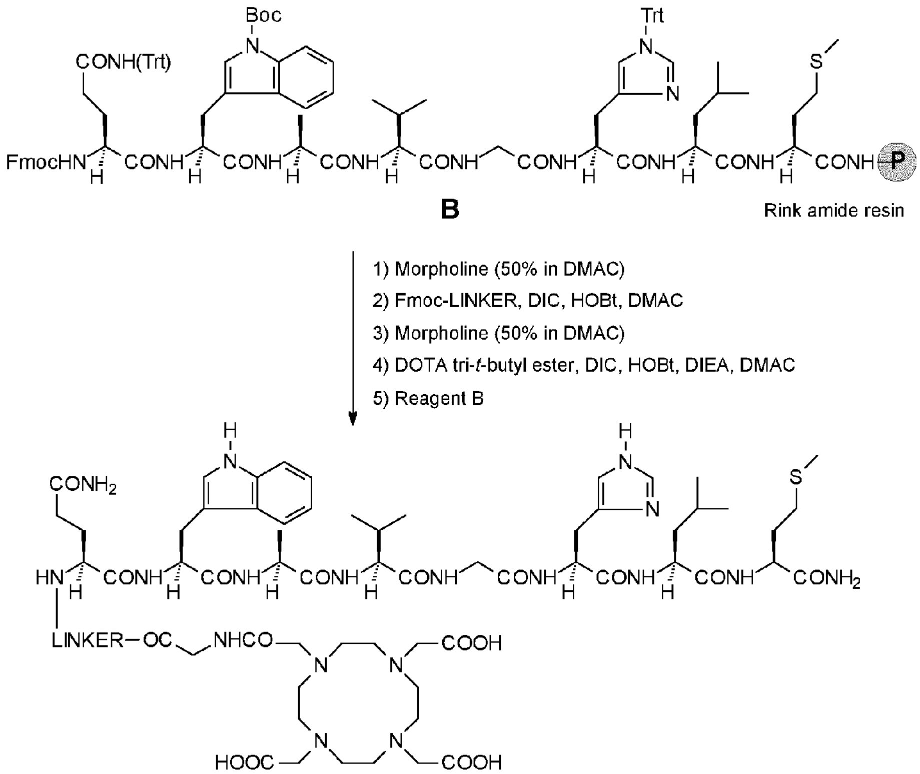


| LINKER | PRODUCT |
|---|---|
| 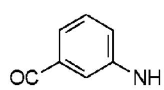 | **L233** |
| 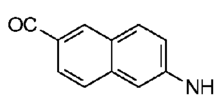 | **L234** |
| 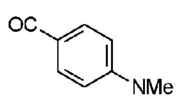 | **L235** |

**FIG. 9D**

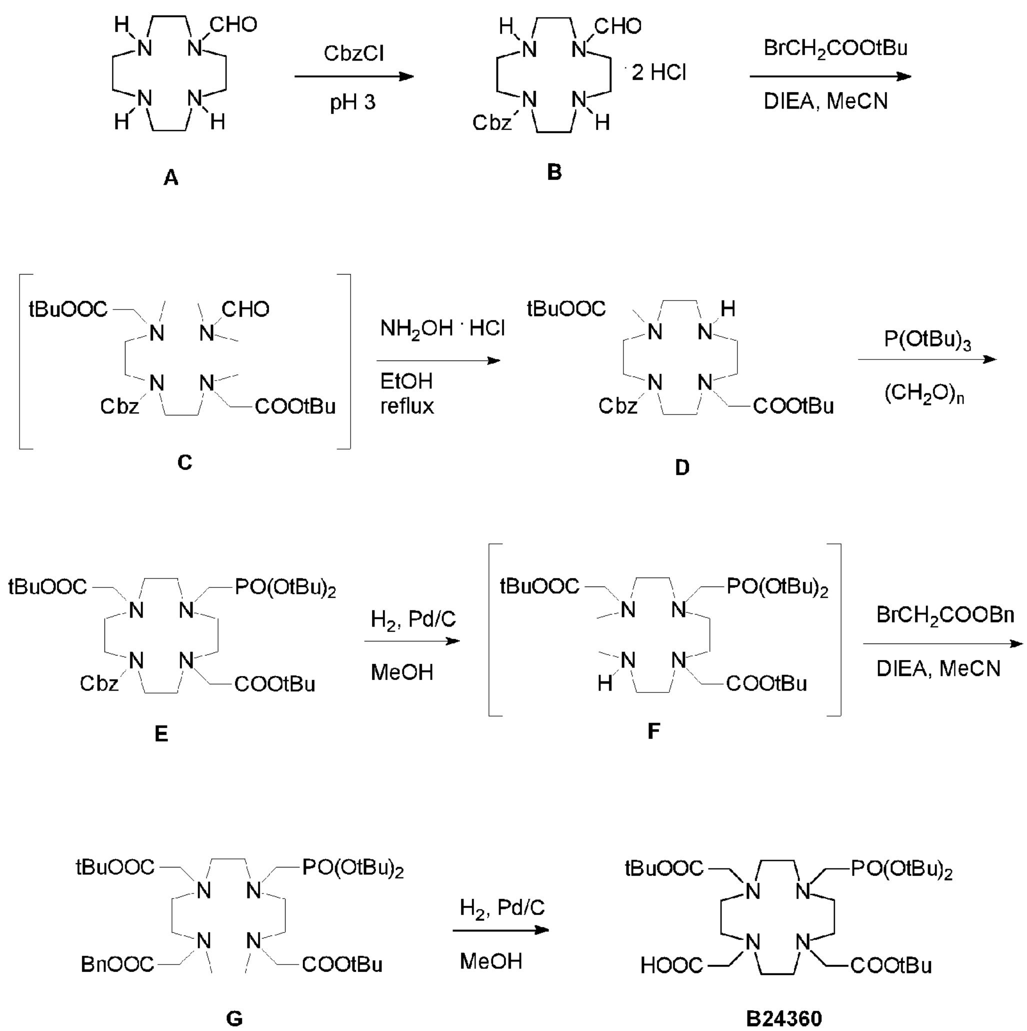
CbzCl
pH 3
· 2 HCl
BrCH$_2$COOtBu
DIEA, MeCN
A
B
NH$_2$OH · HCl
EtOH
reflux
P(OtBu)$_3$
(CH$_2$O)$_n$
C
D
H$_2$, Pd/C
MeOH
BrCH$_2$COOBn
DIEA, MeCN
E
F
H$_2$, Pd/C
MeOH
G
B24360

**FIG. 10A**

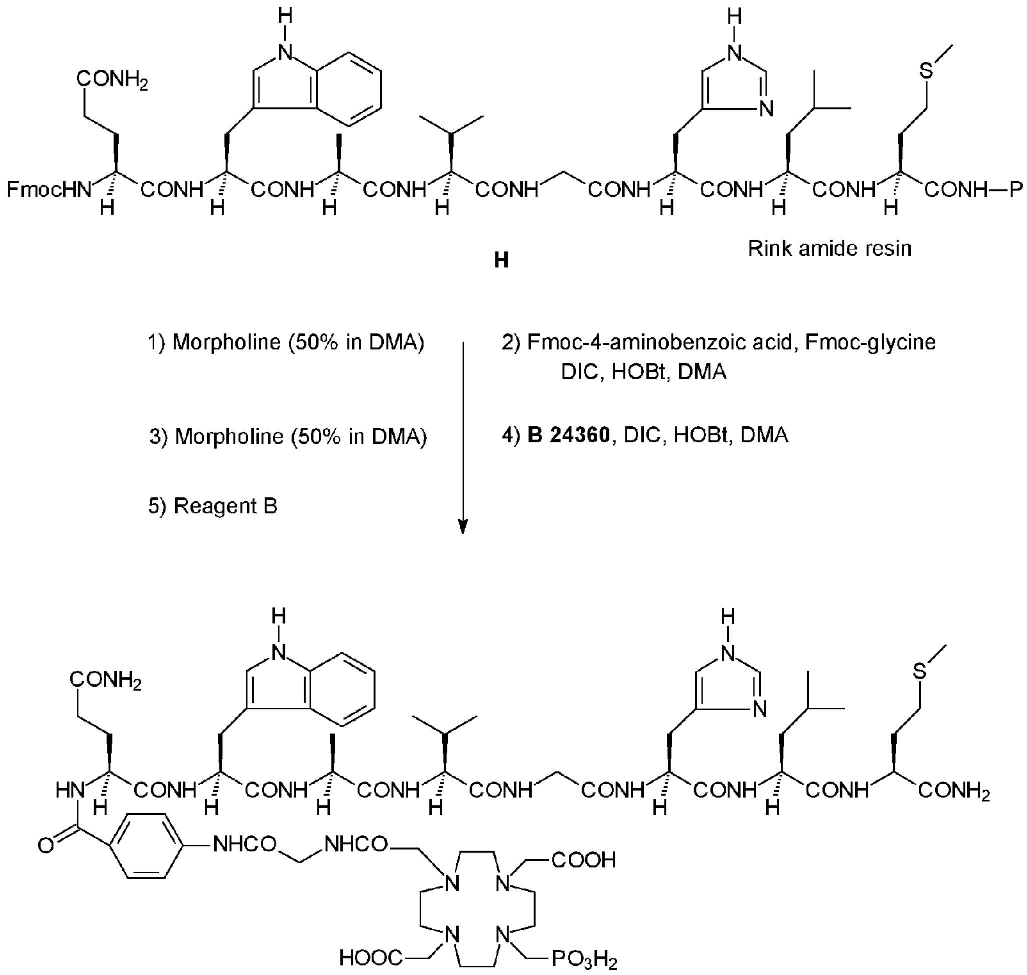


**H**

**B24370**

# FIG. 10B

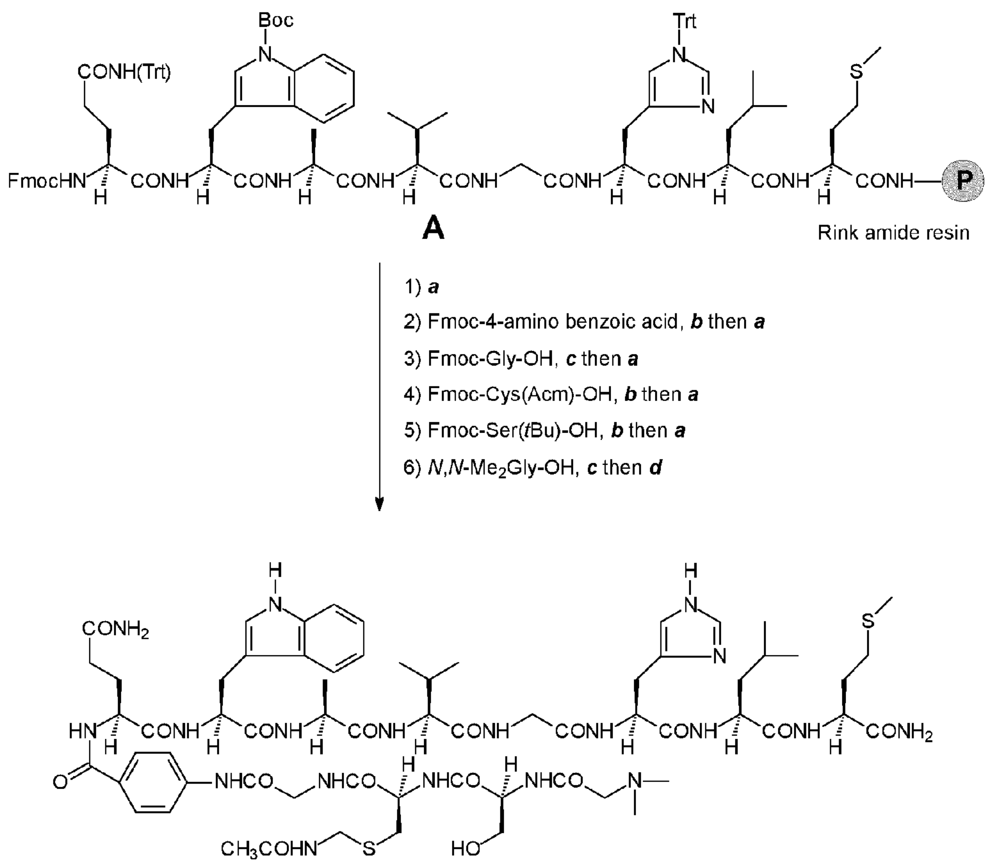


**B 24390**

Conditions: *a* = 50% morpholine in DMAC; *b* = DIC, HOBt, DMAC; *c* = HATU, DIEA, DMAC;
*d* = Reagent B

# FIG. 11A

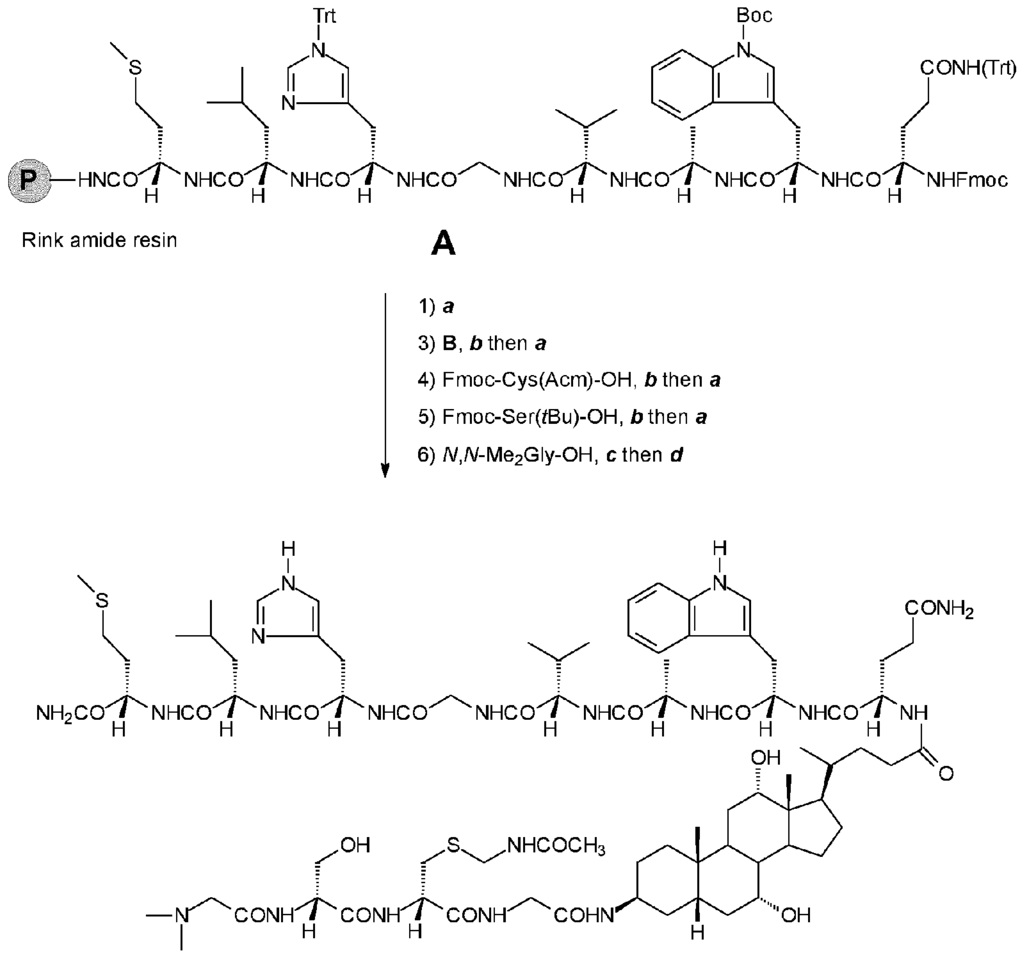


**B 24400**

Conditions: ***a*** = 50% morpholine in DMAC; ***b*** = DIC, HOBt, DMAC; ***c*** = HATU, DIEA, DMAC;
***d*** = Reagent B

# FIG. 11B

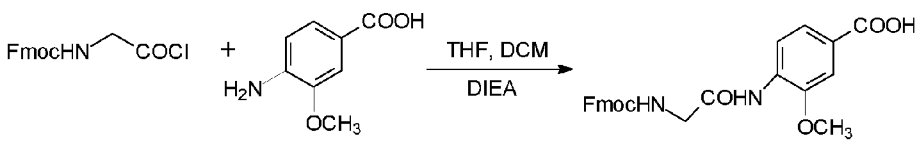


A

FIG. 12A

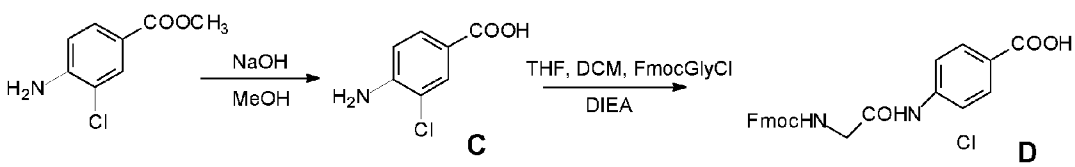


C

D

FIG. 12B

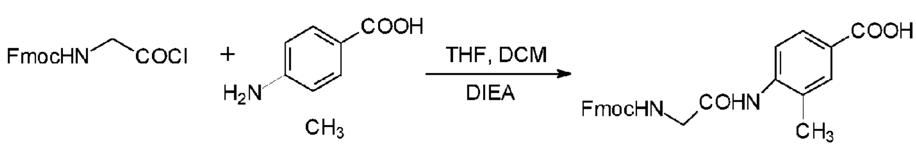


E

FIG. 12C

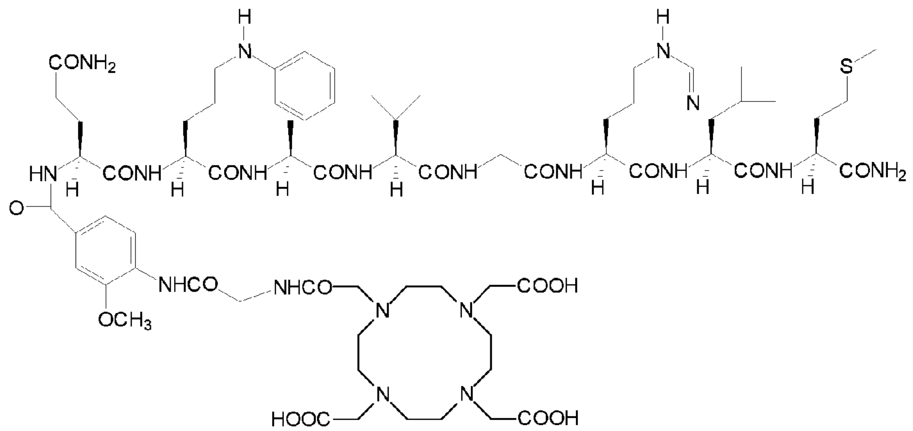

FIG. 12D

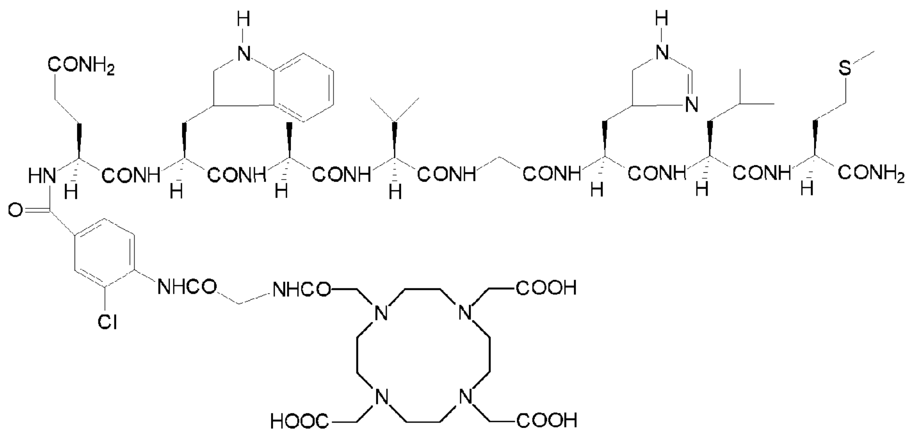

FIG. 12E

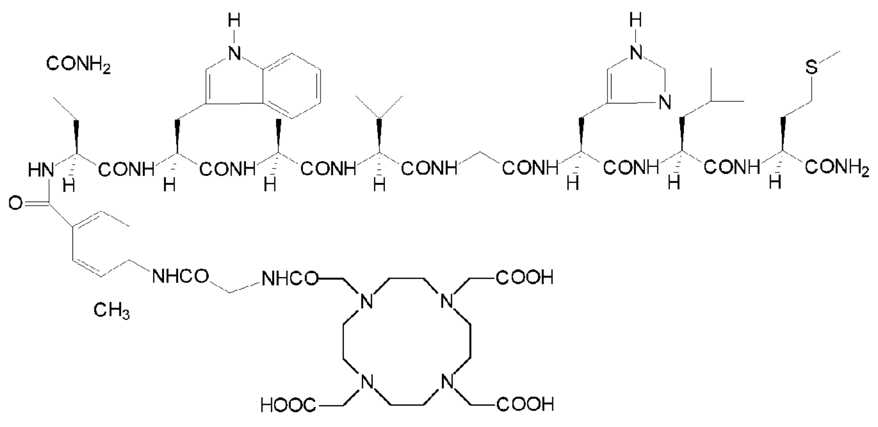

FIG. 12F

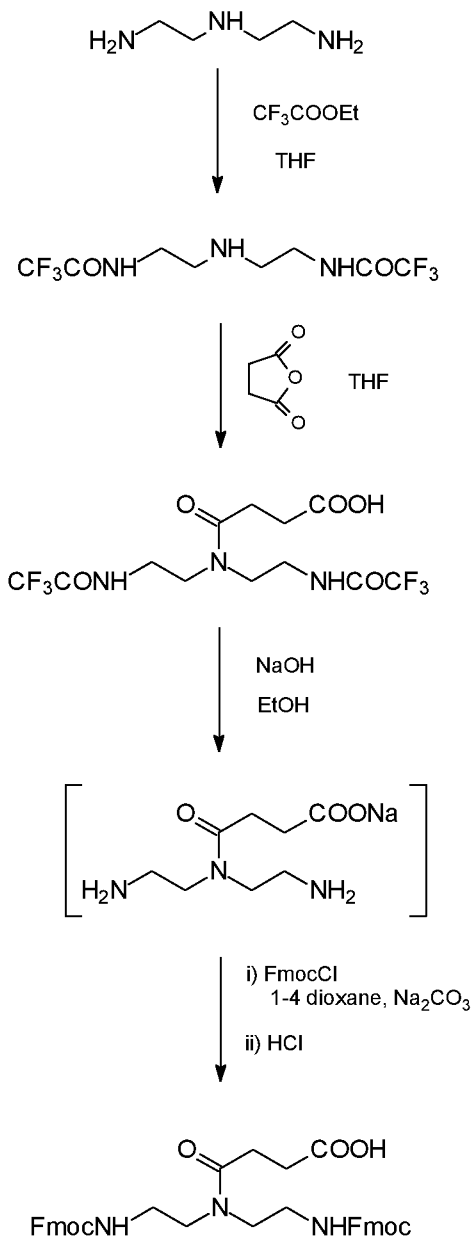


A

B

C

D

FIG. 13A

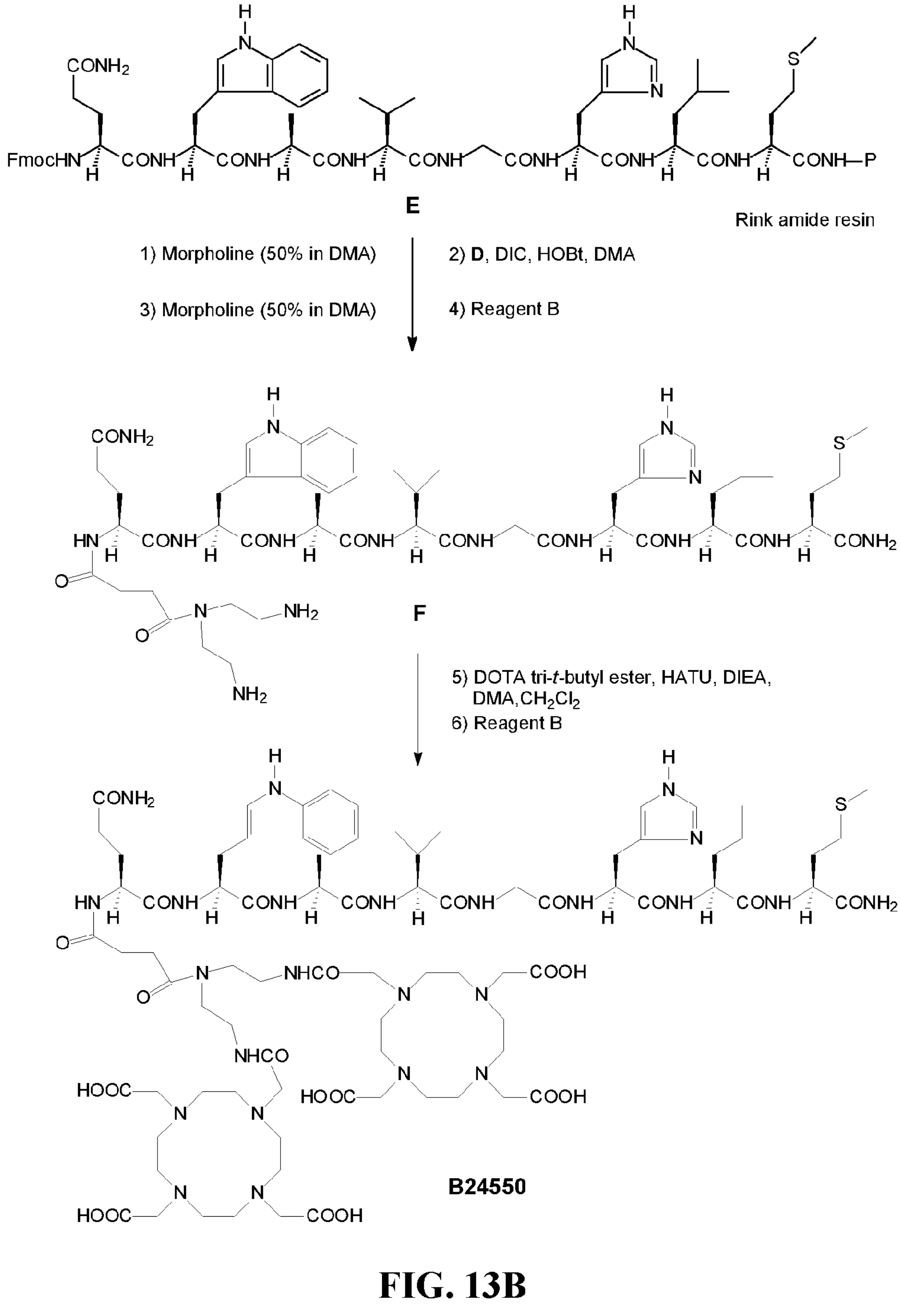
E
Rink amide resin
1) Morpholine (50% in DMA)
2) D, DIC, HOBt, DMA
3) Morpholine (50% in DMA)
4) Reagent B
F
5) DOTA tri-t-butyl ester, HATU, DIEA, DMA,$CH_2Cl_2$
6) Reagent B
B24550

# FIG. 13B

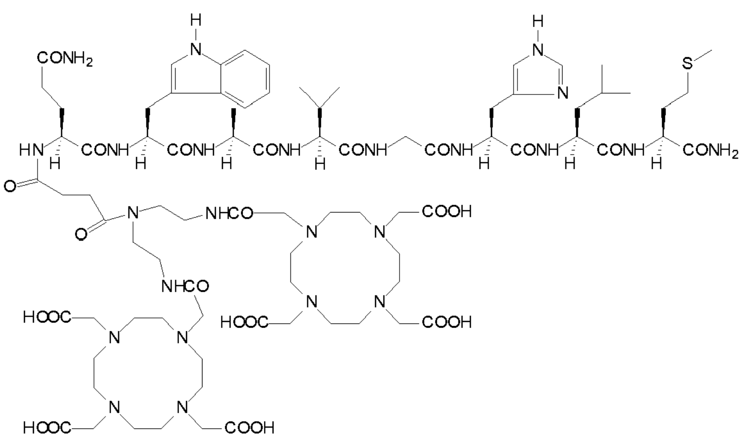

FIG. 13C

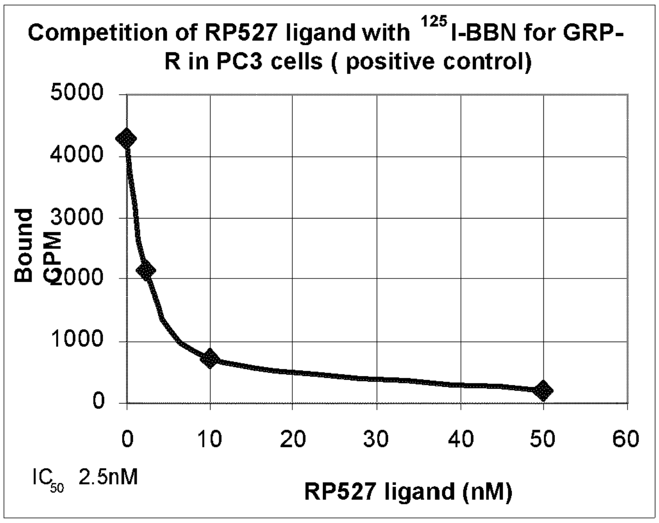


FIG. 14A

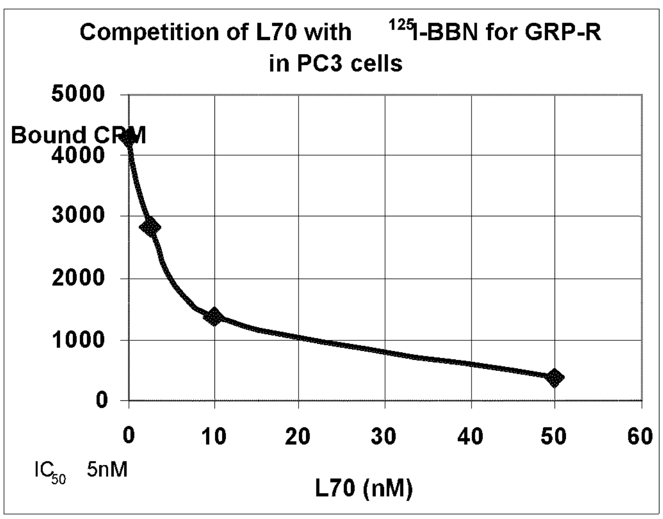


FIG. 14B

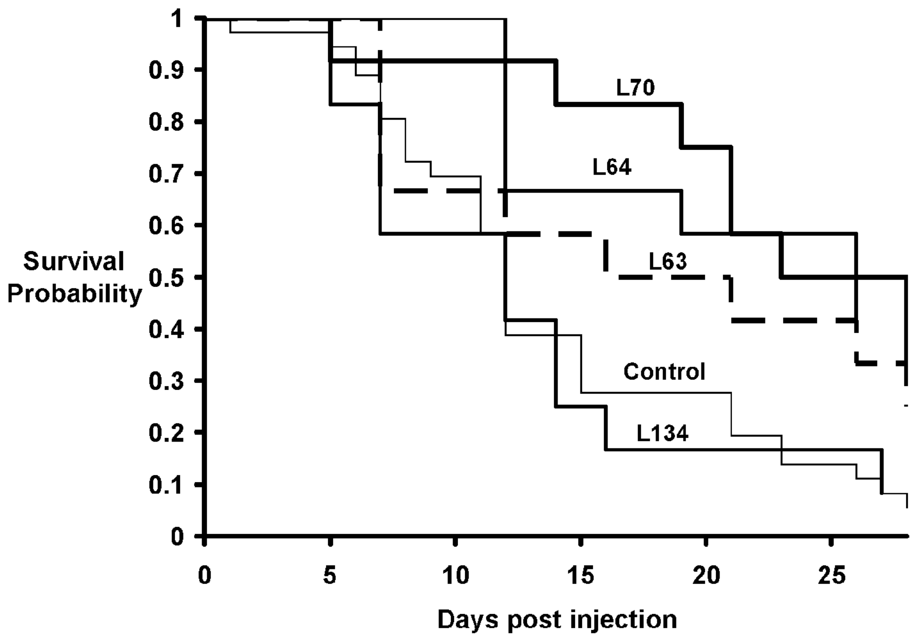


FIG. 15A

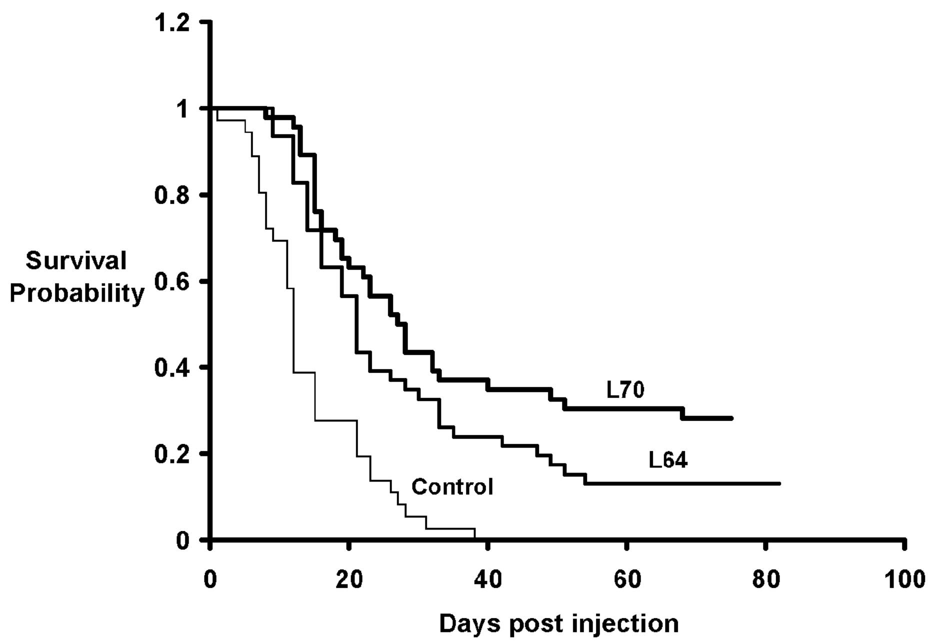


FIG. 15B

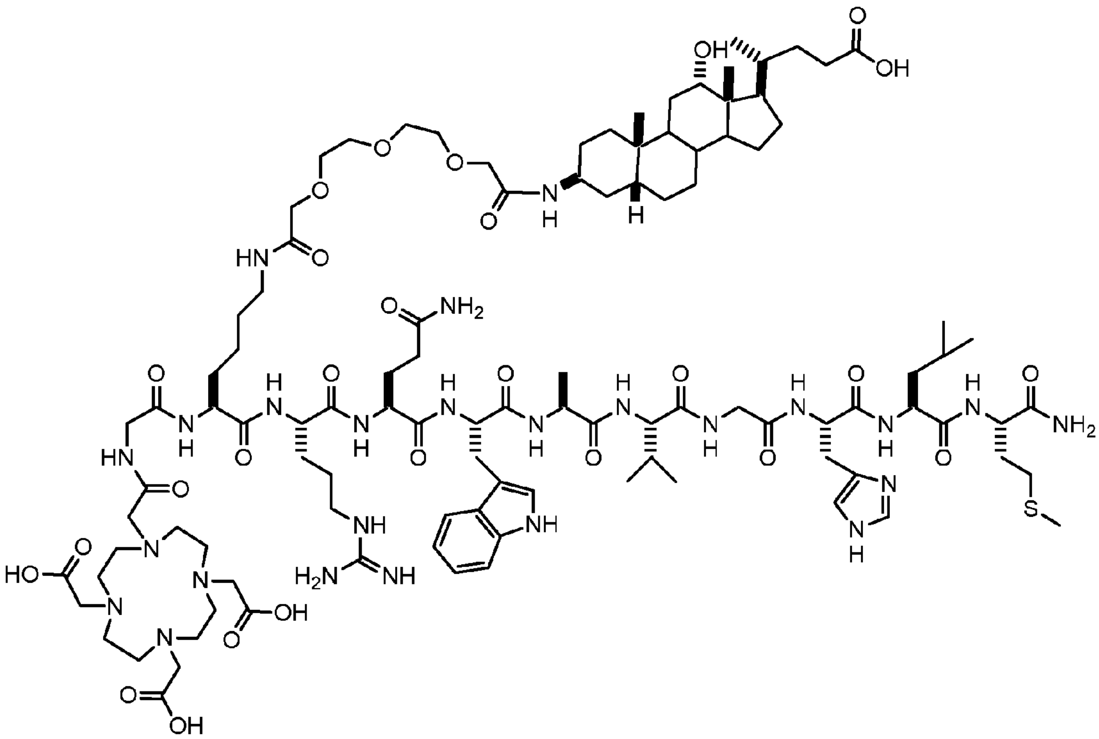

L65

FIG. 16

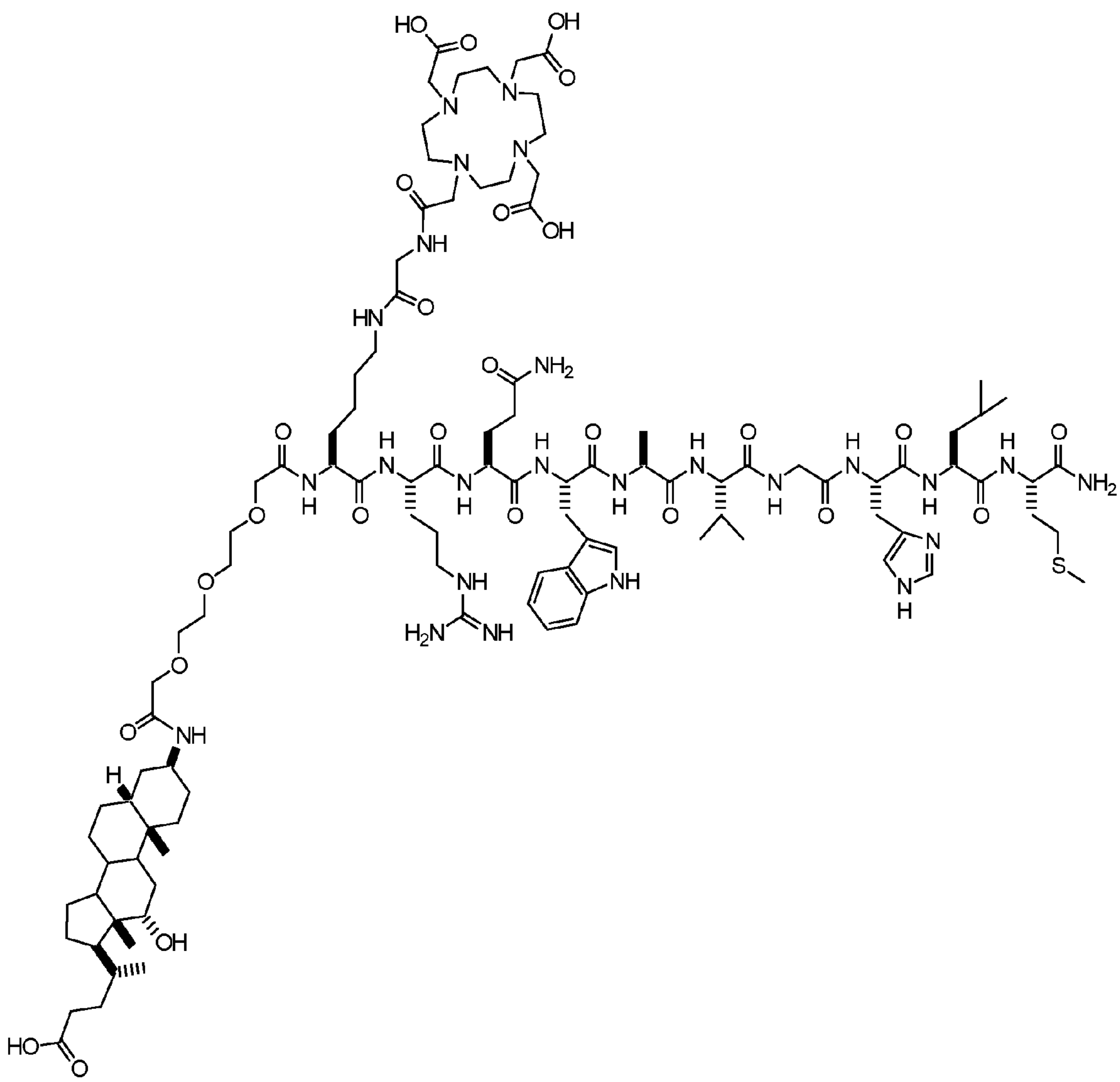

L66

FIG. 17

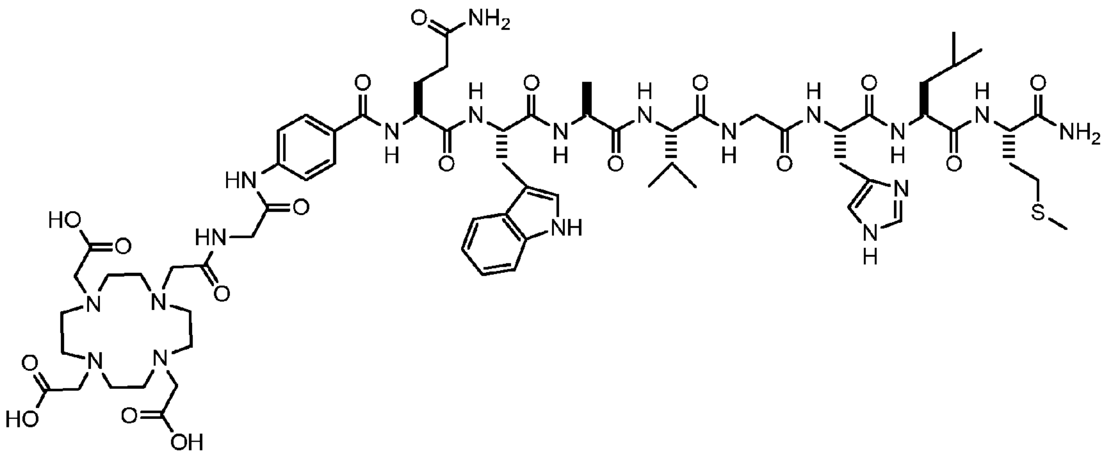

L70

FIG. 18A

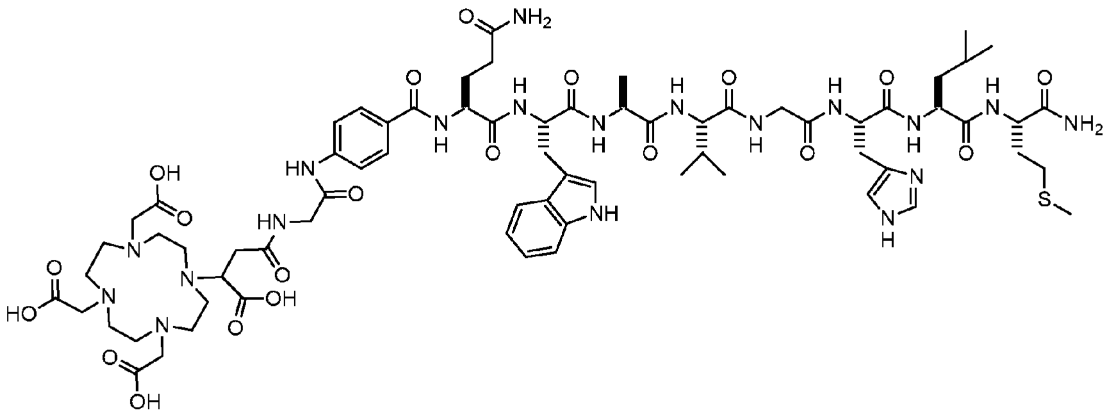

L114

FIG. 18B

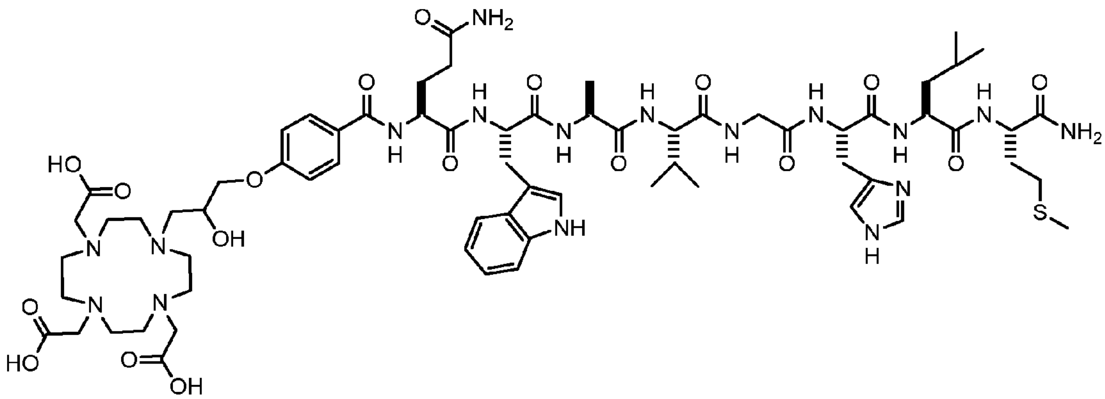

L144

FIG. 18C

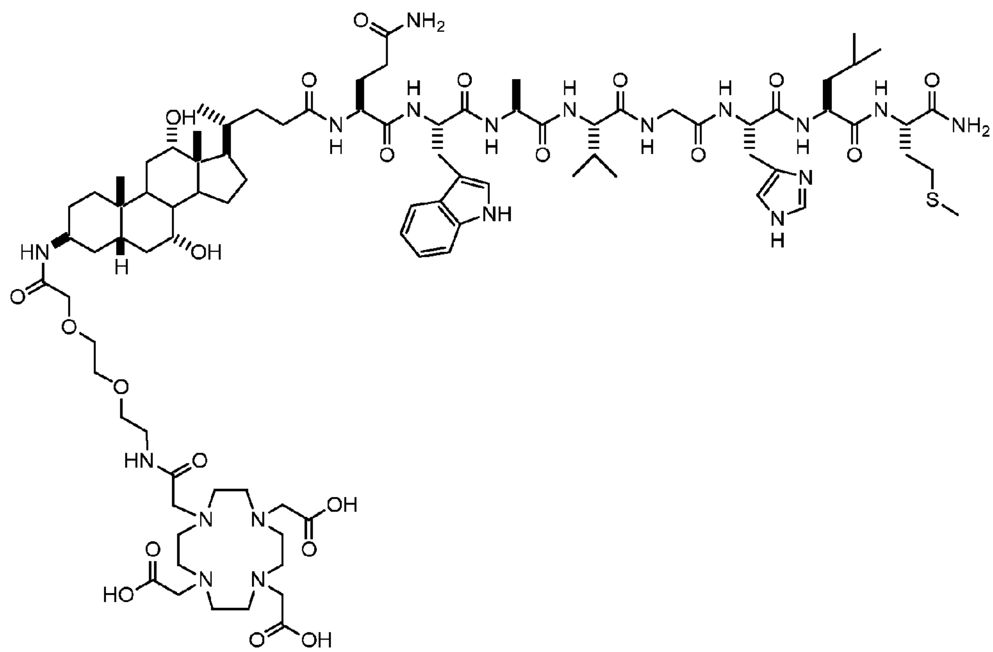

L69

FIG. 18D

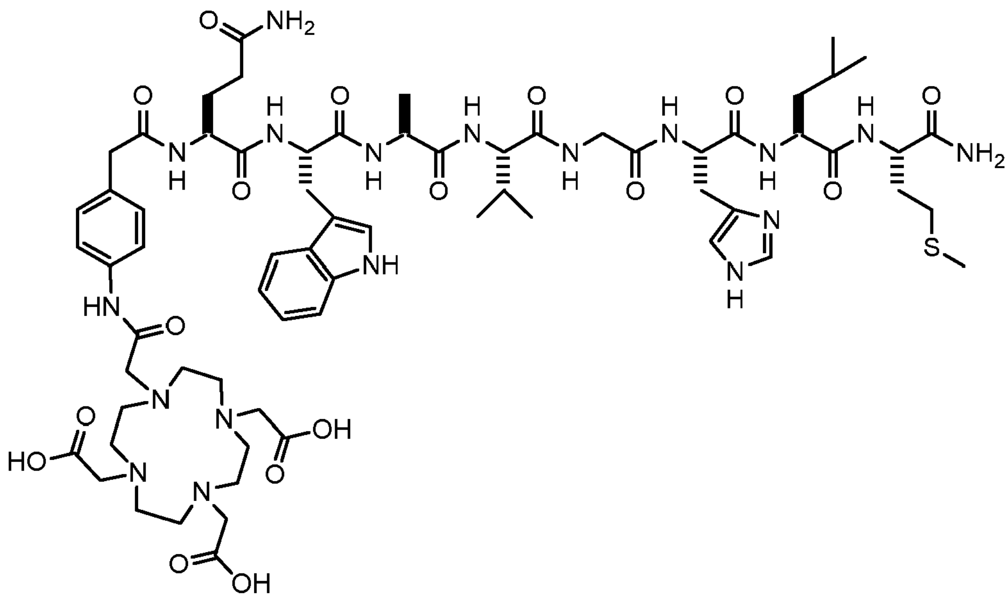

**L146**

# FIG. 18E

**Chart 1**

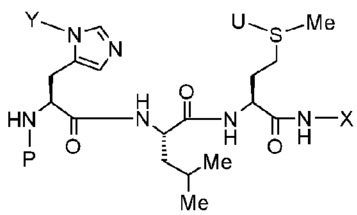

**A**

X = H, Tmob, Xan, Trt
U = →O or null
Y= Trt, Bum, Boc, Cbz
P = Fmoc, Boc, Aloc, H, Cbz

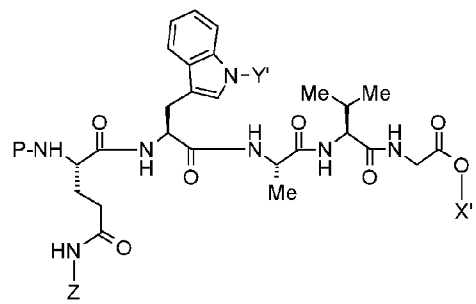

**B**

X' = H, t-Bu, Bz, 2-Cl-Trt, Me, Et
Y' = CHO, Boc, H, 9-PhF, CBz
Z = H, Xan, Tmob, Trt
P' = Fmoc, Boc, Aloc, H, Cbz

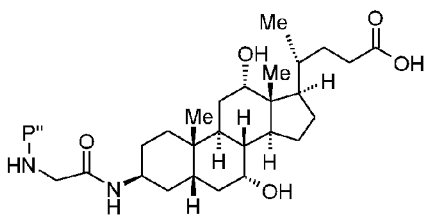

**C1**

P" = Fmoc, Boc, Aloc, H, Cbz

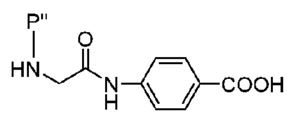

**C2**

P" = Fmoc, Boc, Aloc, H, Cbz

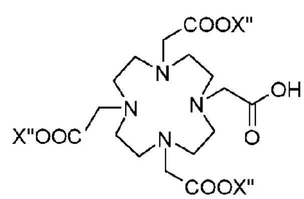

**D**

X" = t-Bu, Me, Bz, H

**FIG. 19**

**Scheme 1**

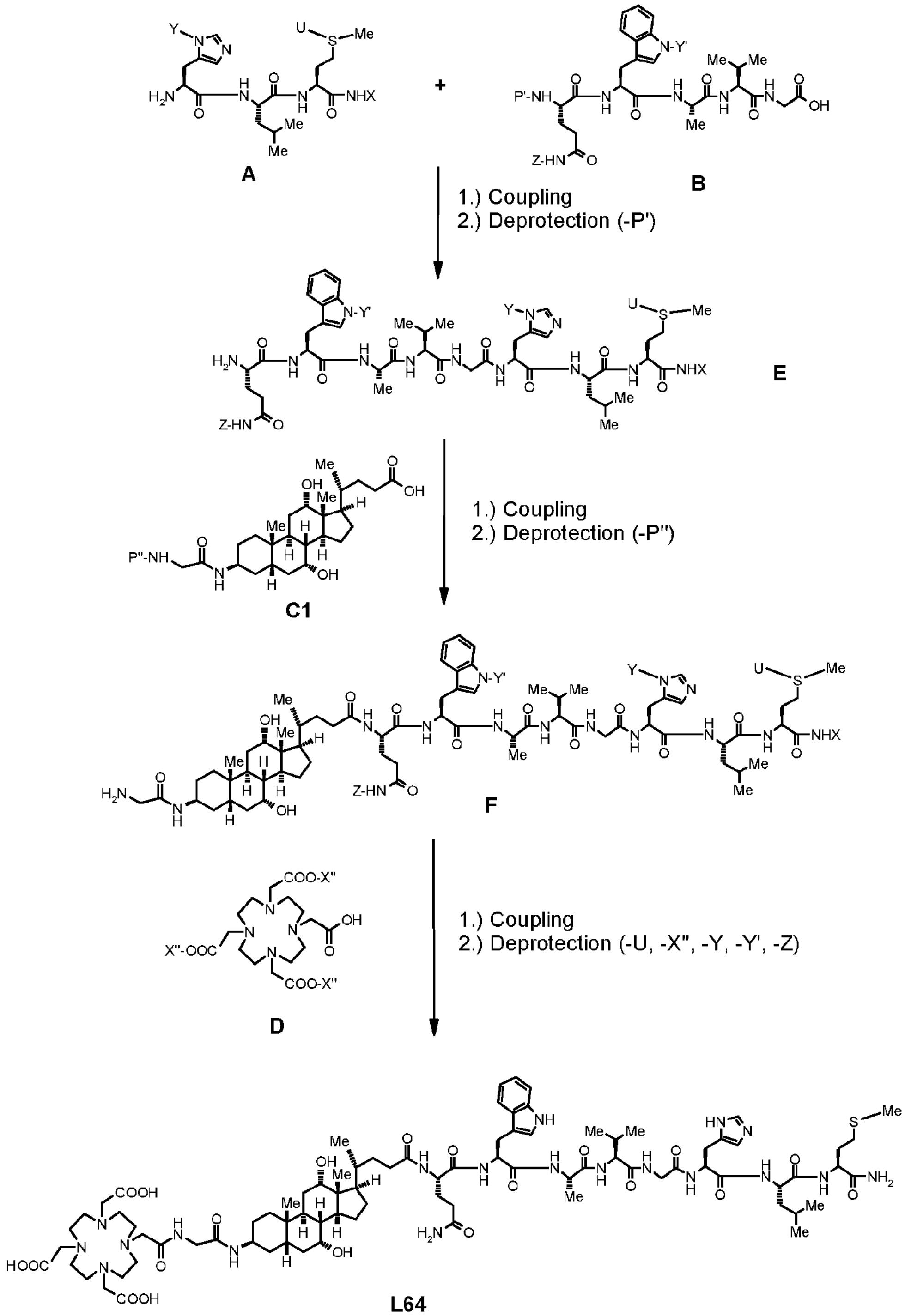


A B E C1 F D L64

FIG. 20

Europäisches Patentamt
European Patent Office
Office européen des brevets

# EUROPEAN SEARCH REPORT

Application Number
EP 12 15 9175

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2002/176819 A1 (HOFFMAN TIMOTHY J [US] ET AL) 28 November 2002 (2002-11-28) | 1-3, 6-13, 16-26 | INV. A61K49/00 C07K7/08 |
| A | * figures 1-7 * | 4,5,14, 15,27,28 | |
| | ----- | | |
| Y | WO 01/09163 A2 (UNIV GLASGOW [GB]; MORRISON JAMES DUNCAN [GB]; LUCAS MICHAEL LESLIE [G) 8 February 2001 (2001-02-08) | 1-3, 6-13, 16-26 | |
| A | * page 3, line 15 - line 23; claim 12 * | 4,5,14, 15,27,28 | |
| | ----- | | |

TECHNICAL FIELDS SEARCHED (IPC)
C07K

The present search report has been drawn up for all claims

2

| Place of search | Date of completion of the search | Examiner |
|---|---|---|
| The Hague | 10 August 2012 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 9175

This annex lists the patent family members relating to the patent documents cited in the above-mentioned European search report.
The members are as contained in the European Patent Office EDP file on
The European Patent Office is in no way liable for these particulars which are merely given for the purpose of information.

10-08-2012

| Patent document cited in search report | Publication date | Patent family member(s) | Publication date |
|---|---|---|---|
| US 2002176819 A1 | 28-11-2002 | CA 2283854 A1 | 29-10-1998 |
| | | EP 0977579 A1 | 09-02-2000 |
| | | JP 2002506424 A | 26-02-2002 |
| | | US 6200546 B1 | 13-03-2001 |
| | | US 2002176819 A1 | 28-11-2002 |
| | | US 2005163710 A1 | 28-07-2005 |
| | | US 2007065362 A1 | 22-03-2007 |
| | | WO 9847524 A1 | 29-10-1998 |
| WO 0109163 A2 | 08-02-2001 | AU 6173900 A | 19-02-2001 |
| | | DE 60032926 T2 | 25-10-2007 |
| | | DK 1228093 T3 | 14-05-2007 |
| | | EP 1228093 A2 | 07-08-2002 |
| | | ES 2280232 T3 | 16-09-2007 |
| | | GB 2355009 A | 11-04-2001 |
| | | US 7153930 B1 | 26-12-2006 |
| | | WO 0109163 A2 | 08-02-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5662885 A, Pollak
- US 5976495 A, Pollak
- US 6200546 B
- US 20020054855 A, Hoffman
- US 5367080 A
- US 5364613 A
- US 5021556 A
- US 5075099 A
- US 5886142 A
- US 5720934 A
- US 6143274 A
- US 6093382 A
- US 5608110 A
- US 5665329 A
- US 5656254 A
- US 5688487 A
- CA 9400395 W
- CA 9400479 W
- CA 9500249 W
- US 5780006 A
- US 5183653 A
- US 5387409 A
- US 5118797 A
- WO 9818496 A
- WO 8606605 A
- WO 9103200 A
- WO 9528179 A
- WO 9623526 A
- WO 9736619 A
- US 9801473 W
- US 9820182 W
- US 4899755 A
- US 5474756 A
- US 5846519 A
- US 5627286 A
- US 6334996 B
- WO 9818497 A
- WO 9818495 A
- WO 9818498 A
- WO 9853857 A
- WO 9617628 A
- WO 9718841 A
- WO 9623524 A
- WO 9847538 A
- US 5453505 A
- WO 9848846 A
- WO 9822146 A
- WO 9848838 A
- US 6663847 B
- US 6656451 B
- US 6641798 B
- US 6485704 B
- US 6423547 B
- US 6395257 B
- US 6280703 B
- US 6277841 B
- US 6264920 B
- US 6264919 B
- US 6228344 B
- US 6217848 B
- US 6190641 B
- US 6183726 B
- US 6180087 B
- US 6180086 B
- US 6180085 B
- US 6013243 A
- US 2003185756 A
- US 20031656432 A
- US 2003158127 A
- US 2003152577 A
- US 2003143159 A
- US 2003105300 A
- US 2003105299 A
- US 2003072763 A
- US 2003036538 A
- US 2003031627 A
- US 2003017164 A
- US 2002169107 A
- US 2002164287 A
- US 2002156117 A
- US 20030224998 A
- WO 9962563 A
- US 5171298 A
- WO 9857666 A
- US 4885363 A
- WO 980005 A, Archimbault, P.; LeClerc, G.; Strosberg, A. D.; Pietri-Rouxel, F.
- DE 19831710 A1
- WO 0287637 A

**Non-patent literature cited in the description**


- **DONALD J. BUCHSBAUM.** Cancer Therapy with Radiolabeled Antibodies; Pharmacokinetics of Antibodies and Their Radiolabels; Experimental Radioimmunotherapy and Methods to Increase Therapeutic Efficacy. CRC Press, 1995, 115-140
- **FISCHMAN et al.** A Ticket to Ride: Peptide Radiopharmaceuticals. *The Journal of Nuclear Medicine,* December 1993, vol. 34 (12
- **HOFFKEN, K.** *Peptides in Oncology II, Somatostatin Analogues and Bombesin Antagonists,* 1993, 87-112
- **DAVIS et al.** Metabolic Stability and Tumor Inhibition of Bombesin/GRP Receptor Antagonists. *Peptides,* 1992, vol. 13, 401-407
- **DUNCAN et al.** *Indium-111-Diethylenetriaminepentaacetic Acid-Octreotide Is Delivered in Vivo to Pancreatic, Tumor Cell, Renal, and Hepatocyte Lysosomes, Cancer Research,* 15 February 1997, vol. 57, 659-671
- **LIU ; EDWARDS.** *Chem Rev.,* 1999, vol. 99, 2235-2268
- **R. B. MUJUMDAR ; L. A. ERNST ; S. R. MUJUMDAR et al.** Cyanine dye labeling reagents: Sulfoindocyanine succinimidyl esters. *Bioconjugate Chemistry,* 1993, vol. 4 (2), 105-111
- **D. A. BELLNIER et al.** Murine pharmacokinetics and antitumor efficacy of the photodynamic sensitizer 2-[1-hexyloxyethyl]-2-devinyl pyropheophorbide-a. *J. Photochem. Photobiol.,* 1993, vol. 20, 55-61
- **G. A. WAGNIERES et al.** In vivo fluorescence spectroscopy and imaging for oncological applications. *Photochem. Photobiol.,* 1998, vol. 68, 603-632
- **J. S. REYNOLDS et al.** Imaging of spontaneous canine mammary tumors using fluorescent contrast agents. *Photochem. Photobiol.,* 1999, vol. 70, 87-94
- Optical properties of tissues. **B. C. WILSON.** Encyclopedia of Human Biology. 1991, vol. 5, 587-597
- **Y-L. HE ; H. TANIGAMI ; H. UEYAMA ; T. MASHIMO ; I. YOSHIYA.** Measurement of blood volume using indocyanine green measured with pulse-spectrometry: Its reproducibility and reliability. *Critical Care Medicine,* 1998, vol. 26 (8), 1446-1451
- **J. CAESAR ; S. SHALDON ; L. CHIANDUSSI et al.** The use of Indocyanine green in the measurement of hepatic blood flow and as a test of hepatic function. *Clin. Sci.,* 1961, vol. 21, 43-57
- **R. B. MUJUMDAR ; L. A. ERNST ; R. MUJUMDAR et al.** Cyanine dye labeling reagents: Sulfoindocyanine succinimidyl esters. *Bioconjugate Chemistry,* 1993, vol. 4 (2), 105-111
- **BRECHBIEL MW ; GANSOW OA.** Backbone-substituted DTPA ligands for Y radioimmunotherapy. *Bioconj. Chem.,* 1991, vol. 2, 187-194
- **LI, WP ; MA DS ; HIGGINBOTHAM C ; HOFFMAN T ; KETRING AR ; CUTLER CS ; JURISSON, SS.** Development of an in vitro model for assessing the in vivo stability of lanthanide chelates. *Nucl. Med. Biol.,* 2001, vol. 28 (2), 145-154
- **KASOKAT T ; URICH K. ; ARZNEIM.-FORSCH.** *Quantification of dechelation of gadopentetate dimeglumine in rats,* 1992, vol. 42 (6), 869-76
- **CUTLER C S ; SMITH CJ ; EHRHARDT GJ. ; TYLER TT ; JURISSON SS ; DEUTSCH E.** Current and potential therapeutic uses of lanthanide radioisotopes. *Cancer Biother. Radiopharm.,* 2000, vol. 15 (6), 531-545
- Convergent Peptide Synthesis. **BARLOS, K. ; GATOS, D.** Fmoc Solid Phase Synthesis - A Practical Approach. Oxford University Press, 2002, 215-228
- **SUN, J-H. ; DENEKER, W. F.** *Synth. Commun.,* 1998, vol. 28, 4525-4530
- **HOELZEMANN, G. ; GOODMAN, S.** *Synthesis of diacylhydrazine compiounds for therapeutic use,* 2000, 16
- **MAO, H. et al.** Rational Design of Diflunisal Analogues with Reduced Affinity for Human Serum Albumin. *J. Am. Chem. Soc.,* 2001, vol. 123 (43), 10429-10435
- **BOYLAND, E. ; GORROD, J.** Synthesis of 3'-methyl-4-'-nitrobiphenylcarboxylic acids by the reaction of 3-methyl-4-nitrobenzenenediazonium acetate with methyl benzoate. *J. Chem. Soc., Abstracts,* 1962, 2209-11
- **VAN DE WIELE C ; DUMONT F et al.** Technetium-99m RP527, a GRP analogue for visualization of GRP receptor-expressing malignancies: a feasibility study. *Eur.J.Nucl.Med.,* 2000, vol. 27, 1694-1699
- **REUBI et al.** Bombesin Receptor Subtypes in Human Cancers: Detection with the Universal Radioligand (125)I-[D-TYR(6), beta-ALA, PHE(13), NLE(14)] Bombesin (6-14. *Clin.Cancer Res.,* 2002, vol. 8, 1139-1146
- Synthetic Peptides - A User's Guide. WH. Freeman Co, 1992, 77-258
- **CHAN, W.C. ; WHITE, P.D.** Basic Procedures in Fmoc Solid Phase Peptide Synthesis - A Practical Approach. Oxford University Press, 2002, 41-76
- **BARLOS, K ; GATOS, G.** Convergent Peptide Synthesis in Fmoc Solid Phase Peptide Synthesis - A Practical Approach. Oxford University Press, 2002, 216-228